(19) Europäisches Patentamt
European Patent Office
Office européen des brevets

(11) EP 3 805 405 A1

(12) # EUROPÄISCHE PATENTANMELDUNG

(43) Veröffentlichungstag:
**14.04.2021 Patentblatt 2021/15**

(51) Int Cl.:
***C12Q 1/682** (2018.01)* ***B01L 3/00** (2006.01)*
***G01N 33/58** (2006.01)*

(21) Anmeldenummer: **20200076.6**

(22) Anmeldetag: **14.12.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(62) Dokumentnummer(n) der früheren Anmeldung(en) nach Art. 76 EPÜ:
**17207476.7 / 3 498 865**

(71) Anmelder: **Ludwig-Maximilians-Universität München**
**80539 München (DE)**

(72) Erfinder:
- **GRABMAYR, Heinrich Ernst Paul**
  **80337 München (DE)**
- **WÖHRSTEIN, Johannes Benedikt**
  **80538 München (DE)**

(74) Vertreter: **Vossius & Partner**
**Patentanwälte Rechtsanwälte mbB**
**Siebertstrasse 3**
**81675 München (DE)**

Bemerkungen:
Diese Anmeldung ist am 05-10-2020 als Teilanmeldung zu der unter INID-Code 62 erwähnten Anmeldung eingereicht worden.

(54) **EINZELMOLEKÜLNACHWEIS BZW. -QUANTIFIZIERUNG DURCH DNA-NANOTECHNOLOGIE IN MIKRO-WELLS**

(57) Die vorliegende Erfindung betrifft ein Verfahren sowie eine DNA-Nanostruktur für den Nachweis einer Zielstruktur. Insbesondere betrifft die vorliegende Erfindung eine DNA-Nanostruktur, die durch geschickte Wahl ihrer Form und der Platzierung von auf ihr befestigten Markermolekülen eine, bevorzugt lineare, Abhängigkeit von Anzahl der Markermoleküle und Messsignal unabhängig von der örtlichen Anordnung mehrerer solcher DNA-Nanostrukturen gewährleistet. Des Weiteren betrifft die Erfindung die Nutzung dieser DNA-Nanostrukturen und anderer Nanoreporter, bevorzugt in Kombination mit Adaptern, die spezifisch an Zielmoleküle binden, in einem Verfahren zur, bevorzugt simultanen, Quantifizierung einer Vielzahl von Zielmolekülen durch ein Muliplexverfahren zu quantifizieren. Insbesondere betrifft das Verfahren eine Einzelzellanalyse.

Fig. 1

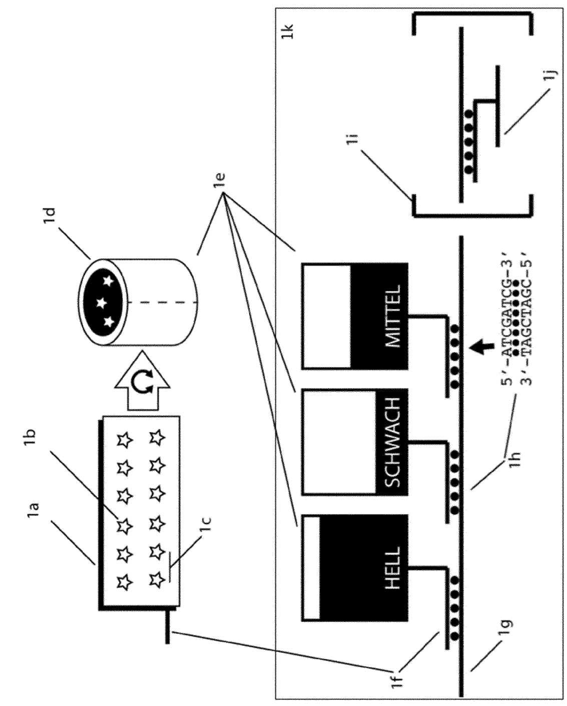


EP 3 805 405 A1

## Beschreibung

Die vorliegende Erfindung betrifft ein Verfahren sowie eine DNA-Nanostruktur für den Nachweis einer Zielstruktur. Insbesondere betrifft die vorliegende Erfindung eine DNA-Nanostruktur, die durch geschickte Wahl ihrer Form und der Platzierung von auf ihr befestigten Markermolekülen eine, bevorzugt lineare, Abhängigkeit von Anzahl der Markermoleküle und Messsignal unabhängig von der örtlichen Anordnung mehrerer solcher DNA-Nanostrukturen gewährleistet. Des Weiteren betrifft die Erfindung die Nutzung dieser DNA-Nanostrukturen und anderer Nanoreporter, bevorzugt in Kombination mit Adaptern, die spezifisch an Zielmoleküle bindenden, in einem Verfahren zur, bevorzugt simultanen, Quantifizierung einer Vielzahl von Zielmolekülen durch ein Muliplexverfahren zu quantifizieren.

Eine häufig auftretende Fragestellung in der Forschung wie auch in der angewandten Medizin und Biotechnologie ist die Analyse, also der Nachweis, bestimmter Zielstrukturen. Beispielsweise kann im Falle einer Sepsis eines Patienten der Nachweis der relevanten Toxine notwendig sein, im Falle einer Erkrankung mit verändertem Zellbild, z.B. bei einer Krebserkrankung, kann eine quantitative Analyse der Umsetzung genetischer Information erforderlich sein.

Die quantitative Analyse der Umsetzung genetischer Information, die sogenannte Genexpressionsanalyse ("Gene Expression Profiling", GEP), erfordert die Detektion und Identifikation einer Anzahl unterschiedlicher Ziel-mRNA-Moleküle (Stadler, Z. K. et al. Critical Reviews in Oncology Hematology 69, 1-11 (2009)). Hierbei ist mRNA die allgemein gebräuchliche Abkürzung für den auch im Deutschen gebräuchlichen englischen Begriff *messenger RNA* (zu Deutsch Boten-RNA). Da die mRNA-Sequenzen im Expressionsprozess der entsprechenden Gene spezifisch produziert werden, kann die Genexpression des untersuchten Materials so quantifiziert werden.

Man kann durch GEP Aufschluss über den Zustand oder Typ einzelner Zellen oder auch einer Gewebeprobe (Vielzahl von Zellen) bekommen und dadurch z.B. die molekulare Diagnostik von Tumorgewebe ermöglichen. Dabei umfasst die GEP die Analyse der Expression mindestens eines Gens, bevorzugt aber die Analyse der Expression einer Vielzahl von Genen. Um dieses ultimative Ziel zu erreichen, wird eine Technologie benötigt, die gleichzeitig eine quantitative Analyse mit höchstmöglicher Spezifität und Sensitivität, bevorzugt für eine möglichst hohe Anzahl an Genen ermöglicht. Für die flächendeckende Einsetzbarkeit im klinischen Alltag muss sich diese Technologie zusätzlich durch schnelle Analysezeiten, geringe Kosten und einfache Benutzung auszeichnen.

Es existiert eine Vielzahl GEP-Techniken, die je nach Fragestellung verschiedene Vor- und Nachteile besitzen.

*Microarrays.* GEP-Analyse mittels Microarrays basiert auf der Oberflächenhybridisierung von fluoreszenten DNA-Molekülen zu spezifischen, örtlich separierten Zielsequenzen auf einem Chip (Trevino, V. et al. Mol Med 13, 527-541 (2007)). Diese Moleküle werden in einem vorgelagerten Schritt durch reverse Transkription und "Polymerase-Chain-Reaction" (PCR)-Amplifikation von RNA-Molekülen erzeugt. Microarrays erlauben dadurch die parallele Detektion von bis zu mehreren tausend Sequenzen. Allerdings ist eine exakte Quantifizierung unmöglich, da enzymatische Reaktionen wie reverse Transkription und PCR-Amplifikation zu systematischen Fehlern führen. Des Weiteren benötigen Microarray-basierte Methoden eine recht lange Prozesszeit von etwa einem Tag und sind daher für eine Großzahl klinischer Anwendungen ungeeignet.

*qPCR/dPCR.* Mittels PCR lassen sich spezifische DNA-Moleküle in einem enzymatischen Prozess exponentiell vervielfältigen (VanGuilder, H. D. et al. Biotechniques 44, 619-626 (2008)). mRNA-Ziele müssen für PCR-Reaktionen generell enzymatisch mittels reverser Transkription von RNA nach DNA umgewandelt werden. Durch Zugabe fluoreszierender, DNA-bindender Moleküle lässt sich die Amplifikation messen und die ursprüngliche Konzentration grob bestimmen (qPCR). Da die Amplifikationsrate empfindlich von vielen Parametern (u.a. Ziel-Sequenz, Ziel-Länge, Primer, Instrumentation, Reagenzien, Reaktionsvolumen) abhängt, muss diese Quantifizierung genau kalibriert werden. Um diese Nachteile zu kompensieren wurde die digitale PCR (dPCR) entwickelt. Diese erlaubt präzisere Quantifizierung durch Kompartmentalisierung einzelner Zielmoleküle (Baker, M. Nature Methods 9, 541-544 (2012)). Allerdings wird diese hohe dPCR-Genauigkeit durch Limitation auf typischerweise zwei Ziele erkauft. dPCR ist dadurch für GEP-Analysen ungeeignet.

*RNA-Seq/NGS.* Beim sogenannten Next-Generation-Sequencing (NGS), auf dem auch die RNA-Sequenzierung (RNA-Seq) basiert, werden Sequenzen in mehreren Schritten pro Base sequenziell ausgelesen und identifiziert (Wang, Z. et al. Nat Rev Genet 10, 57-63 (2009)). Auch bei RNA-seq muss RNA zunächst enzymatisch in DNA umgewandelt werden. Der Vorteil und das Alleinstellungsmerkmal von NGS in der Transkriptions-Analyse ist die Möglichkeit neuartige oder veränderte Sequenzen zu erkennen. Aufgrund der vielen komplexen Prozessschritte sind die Analysegeräte komplex und teuer und die Prozessdauer lang. Das verhindert einen Einsatz im Point-of-Care-Bereich. Wie in den zuvor beschriebenen Methoden verhindern enzymatische Prozesse die exakte Quantifizierbarkeit.

*nCounter.* Das nCounter-System von NanoString Technologies basiert auf der Detektion von RNA-Molekülen durch Hybridisierung mit einem fluoreszenten Reporter (Barcode) einerseits, sowie einem zweiten DNA-Molekül (Capture-Strang) zum Anbinden an einen Mikroskopiechip andererseits (Geiss, G. K. et al. Nat Biotechnol 26, 317-325 (2008)). Die fluoreszenten Reporter sind mikrometerlange, lineare geometrische Barcodes, die aus der Aneinanderreihung verschiedener mit Fluoreszenzfarbstoffen markierten Bereichen bestehen. Einer der Vorteile des nCounter-Systems ist die enzymfreie und quantitative Detektion von bis zu 800 verschiedenen RNA-Zielmolekülen. Ein Nachteil der Methode ist die Notwendigkeit, nach Target-Hybridisierung und Oberflächenimmobilisierung das geometrische Barcodemolekül zu strecken, um den entsprechenden Barcode auslesen zu können. Dies hat zwei wichtige Implikationen: (1) Das elektrophoretische Strecken der Reporter stellt einen komplexen Prozessschritt dar, (2) Bedingt durch die niedrige Effizienz dieses Schrittes kommt es zum Ausschluss von ca. 80 % der Zielmoleküle durch nicht identifizierbare Barcodes. Des Weiteren sind komplexe und zeitintensive Aufreinigungsschritte notwendig, z.B. mit magnetischen Partikeln. Zusammen genommen führen diese Prozessschritte zu teuren Analysegeräten (>200 000 Euro) und langen Messzeiten (24-48 Stunden).

Diese traditionellen Methoden werden somit nicht all den oben genannten bevorzugten Anforderungen an eine GEP gerecht und opfern z.B. teilweise Quantifizierbarkeit für notwendige Sensitivität.

Ein weiterer vielversprechender Analyseansatz für GEPs basiert auf dem direkten Markieren einzelner Ziel-mRNA-Sequenzen durch sogenannte Hybridisierung mit fluoreszenten Reportern. Fluoreszenzmethoden erlauben die sensitive Detektion einzelner Moleküle (Joo, C. et al. Annu Rev Biochem 77, 51-76 (2008)). Klassische Methoden sind jedoch nicht in der Lage eine große Anzahl unterschiedlicher Reporter zu bestimmen.

Eine quantitative GEP auf Einzelzellbasis ist mit den verfügbaren Verfahren nur limitiert möglich, wäre aber sowohl für wissenschaftliche Forschung wie auch für industrielle Forschung und Entwicklung, und die klinische Anwendung von großem Vorteil.

Wichtige wünschenswerte Merkmale einer GEP wären die exakte Quantifizierung und die effiziente Durchführung der Analyse. Damit ergeben sich folgende bevorzugte Vorgaben für eine Verbesserung einer GEP:

- Direkte Detektion der mRNA, insbesondere keine Umwandlung in DNA, z.B. durch reverse Transkription
- Keine Vervielfältigung der Zielemoleküle, da eine nicht exakte Amplifikation die Quantifizierung beeinträchtigt
- Damit: Detektion einzelner Ziel-Moleküle
- möglichst schnelle Analyse (Größenordnung von Minuten)
- möglichst einfache Analyse, insbesondere: wenige Arbeitsschritte
- Möglichkeit der Korrelation der Analysenergebnisse mit den ursprünglichen Probenbestandteilen

Neben Nukleinsäuren und Proteinen gibt es noch eine Fülle von weiteren Problemstellungen, bei denen Einheiten in der Größenordnung von einigen Nanometern bis mehreren zehn Nanometern nachgewiesen und bevorzugt gezählt werden müssen, bzw. ihre Konzentration bestimmt werden soll; wie beispielsweise anorganische Komplexe und Nanopartikel.

All diese Problemstellungen haben gemein, dass Moleküle oder andere Strukturen schnell, einfach und genau nachgewiesen und bevorzugt gezählt werden sollen. Ein genaues Zählen beinhaltet insbesondere, dass die Wahrscheinlichkeit der falsch positiven und/oder falsch negativen Zählereignisse möglichst gering gehalten werden muss. Auf eine Verbesserung einer oder mehrerer dieser Eigenschaften, insbesondere auf eine Reduzierung der Wahrscheinlichkeit der falsch positiven und/oder falsch negativen Zählereignisse, zielt die vorliegende Erfindung ab.

Diese Aufgabe wird durch die vorliegende Erfindung gemäß den unabhängigen Ansprüchen gelöst.

Die vorliegende Erfindung ermöglicht nun die Lösung der vorgenannten Aufgaben aufgrund einer Vielzahl von gegenüber dem Stand der Technik vorteilhaften Aspekten.

Die vorliegende Erfindung betrifft ein Verfahren, das die Analyse einzelner Zellen oder anderer zu untersuchenden Strukturen dieser oder einer ähnlichen Größenordnung ermöglicht. Ein Aspekt des Verfahrens, der sich hier besonders vorteilhaft auswirkt, ist die Vereinzelung der zu untersuchenden Strukturen in Mikro-Wells. Hierdurch kann eine Verfälschung des Ergebnisses durch Diffusionsverluste, Einträge von außen und/oder Mischung der eigentlich einzeln zu untersuchenden Strukturen reduziert werden. Die Verwendung der Mikro-Wells bietet weiterhin den Vorteil, dass falsch bestückte Mikro-Wells schnell und einfach verworfen werden können, was zu einer Verbesserung der Genauigkeit des Analyseergebnisses führt.

Gemäß einem weiteren Aspekt der Erfindung kann trotz der einzelnen Analyse der zu untersuchenden Strukturen eine Vielzahl von zu untersuchenden Strukturen parallel analysiert werden.

Das Verfahren beinhaltet, verglichen mit den eingangs erwähnten bereits bekannten Methoden, eine geringe Zahl von Schritten, wodurch die Gefahr der Artefaktbildung durch den Analyseprozess selbst reduziert wird.

Die Verfahrensschritte zur Vereinzelung der zu untersuchenden Strukturen sind in vorteilhafter Weise einfach gehalten, was die Handhabung des Verfahrens im Vergleich zu anderen Vereinzelungstechniken, wie beispielsweise die Separation in einzelnen Droplets, wesentlich vereinfacht.

Ein besonders vorteilhafter Aspekt der vorliegenden Erfindung ist darin zu sehen, dass die einzelnen zu untersuchenden Strukturen nicht nur molekular analysiert werden können, sondern auch ihr Phänotyp hinsichtlich beispielsweise ihrer Morphologie analysiert werden kann. Diese beiden Analyseergebnisse können einander zugeordnet werden, so dass eine Zuordnung zwischen den molekularen Eigenschaften und anderen Phänotyp-Merkmalen der zu untersuchenden Strukturen erfolgen kann, was weitere Auswertungen, beispielsweise Korrelationsanalysen, ermöglicht.

Demnach richtet sich die vorliegende Erfindung auf ein Verfahren zum Nachweis (bevorzugt Quantifizierung) einer Zielstruktur, umfassend:

p1) Einbringen einer Gruppe von Wirtskörpern inklusive eines Wirtskörpers mit der Zielstruktur in ein Mikro-Well Array derart, dass in mindestens einem Mikro-Well genau ein Wirtskörper vorhanden ist;
p2) Einbringen von mindestens zwei 3D-DNA-Nanostrukturen in das mindestens eine Mikro-Well, wobei jede der 3D-DNA-Nanostrukturen ein oder mehrere innenliegende Fluoreszenz-Farbstoffmoleküle aufweist;

a) Ausbilden einer Identifizierungsstruktur in dem mindestens einen Mikro-Well, die aufweist:

(i) die Zielstruktur, und
(ii) die mindestens zwei 3D-DNA-Nanostrukturen, wobei jede der 3D-DNA-Nanostrukturen spezifisch an die Zielstruktur gebunden ist, und wobei die 3D-DNA-Nanostrukturen an paarweise unterschiedliche Regionen der Zielstruktur gebunden sind;

b) Nachweis der Zielstruktur durch Messen mindestens eines Fluoreszenzsignals,

wobei die 3D-DNA-Nanostrukturen und die Parameter der Fluoreszenzmessung derart ausgewählt sind, dass das mindestens eine gemessene Fluoreszenzsignal der in a) ausgebildeten Identifizierungsstruktur von dem Fluoreszenzsignal jeder der mindestens zwei isolierten 3D-DNA-Nanostrukturen unterscheidbar ist, wenn diese nicht in der Identifizierungsstruktur gebunden sind, wobei die Identifizierungsstruktur an eine erste Oberfläche, bevorzugt den Boden des mindestens einen Mikro-Wells gebunden ist.

Bevorzugt haben die 3D-DNA-Nanostrukturen jeweils für sich betrachtet dasselbe Fluoreszenzsignal unabhängig davon, ob sie isoliert (d.h. nicht in einer Identifizierungsstruktur gebunden) oder in einer Identifizierungsstruktur vorliegen. Das mindestens eine in Schritt b) gemessene Fluoreszenzsignal unterscheidet sich also kraft der Kombination der mindestens zwei 3D-DNA-Nanostrukturen gegenüber den individuellen Fluoreszenzsignalen der ungebundenen (d.h. isolierten) 3D-DNA-Nanostrukturen. Es ist also nicht notwendig, obwohl dies auch möglich ist, dass eine 3D-DNA-Nanostruktur ihr Fluoreszenzsignal durch Binden in der Identifizierungsstruktur verändert, beispielsweise durch eine Verschiebung des Spektrums und/oder eine Veränderung der Intensität.
Als Wirtskörper wird hier die zu untersuchende Struktur bezeichnet, die die zu untersuchende Zielstruktur aufweisen kann. Beispielsweise kann der Wirtskörper eine Zelle, etwa eine prokariotische, eukariotische, bakterielle, pflanzliche und/oder tierische Zelle, ein Virus, ein Exosom, ein Vesikel und/oder ein Droplet sein. Die zu untersuchende Zielstruktur kann sich auf der Oberfläche des Wirtskörpers und/oder in dessen Inneren befinden.
Demnach richtet sich die Erfindung auch auf eine Verfahrensvariante, bei dem die Zielstruktur in einem Wirtskörper vorliegt, wobei das Verfahren ferner umfasst:
e) Aufschließen der Wirtskörper, vorzugsweise durch Flüssigkeitsaustausch mit einem Aufschlusspuffer, um die Zielstruktur aus dem Wirtskörper zu befreien.
Für den Fall, dass die Gruppe von Wirtskörpern Zellen, Vesikel und/oder Exosome enthält, umfasst der Aufschluss-Schritt e) bevorzugt eine Lyse, besonders bevorzugt eine hypotone oder eine Detergensbasierte Lyse (siehe bspw. "Thermo Scientific Pierce Cell Lysis Technical Handbook" (2 ed.), Thermo Scientific; D. Liu und L. Huang, "Trypsin-induced lysis of lipid vesicles: effect of surface charge and lipid composition", Anal Biochem 1992, 202(1), 1-5; L Turnbull et al, "Explosive cell lysis as a mechanism for the biogenesis of bacterial membrane vesicles and biofilms", Nat Commun 2016 Apr 14; 7:11220; C. Lässer et al, "Isolation and Characterization of RNA-Containing Exosomes", J Vis Exp 2012, 59 3037 und/oder Pin Li et al, "Progress in Exosome Isolation Techniques", Theranostics 2017, 7(3): 789-804), vorzugsweise unter Verwendung eines Lyse-Puffers, oder eine physikalische Lyse, beispielsweise eine laserinduzierte (siehe bspw. MD. Dhawan et al, "Development of a laser-induced cell lysis system", Anal Bioanal Chem 2002, 373(3): 421-6; K. Rau et al., "Pulsed Laser Microbeam-induced Cell Lysis: Time-Resolved Imaging and Analysis of Hydrodynamic Effects", Biophys J. 2006, 91(1): 317-329) oder akustische (siehe bspw. "Thermo Scientific Pierce Cell Lysis Technical Handbook" (2 ed.), Thermo Scientific) Lyse. Für den Fall, dass die Gruppe von Wirtskörpern Droplets einer Wasser-in-Öl-in-Wasser-Emulsion aufweist, kann ein der Lyse von Zellen ähnliches und/oder gleichartiges Verfahren verwendet werden, das dem Fachmann bekannt ist (siehe bspw. M. Chavez-Paez et al., "Coalescence in double emulsions", Langmuir 2012, 28, 5934-5939). Für Wirtskörper in Form von Droplets einer Wasser-in-Öl-Emulsion geschieht deren Aufschluss automatisch, da ein solches Droplet direkt an die hydrophile Mikro-Well-Begrenzung, also Wand und/oder Boden, fusioniert, so dass dieser Verfahrensschritt lediglich das Abwarten einer Inkubationszeit, bevorzugt von 5-120 Minuten, besonders bevorzugt von 15 Minuten, beinhaltet. Für den Fall, dass die Gruppe von Wirtskörpern Viren aufweist, kann ein Verfahren zur Virus-Lyse verwendet werden, das beispielsweise einen Lyse-Puffer umfasst, das beispielsweise Natriumdodecylsulfat (SDS) und/oder Formamid enthält (siehe bspw. G. F. Steward und A. I. Culley, "Extraction and purification of nucleic acids from viruses", Manual of aquatic viral ecology Kapitel 16, American Society of Limnology and Oceanography, 2010, pp. 154-165).

Die Lyse kann z.B. mechanisch, durch Lyse-Puffer, enzymatisch und/oder chemisch oder durch Licht erfolgen. Eine auf dem Gebiet der Technik bekannte Methode zur Lyse von Zellen wird beispielsweise in "Thermo Scientific Pierce Cell Lysis Technical Handbook" (2 ed.), Thermo Scientific genannt und beinhaltet die Verwendung des Tensids Triton X als Lyse-Puffer. Die Einzelheiten des Verfahrens werden daher in der vorliegenden Schrift nicht mehr näher erläutert. Ähnliche Verfahren sind auch für den Aufschluss von Droplets bekannt. Beispielsweise beschreibt M. Chavez-Paez et al., "Coalescence in double emulsions", Langmuir 2012, 28, 5934-5939 den Aufschluss von Droplets mithilfe von Tensiden, beispielsweise SDS oder Span 80.

Ein weiterer Aspekt der vorliegenden Erfindung betrifft ein Mikro-Well Array. Das Mikro-Well Array isoliert die Wirtskörper voneinander und lokalisiert die Zielstrukturen der einzelnen Wirtskörper großteils (je nach Geometrie bspw. zu 70%, bevorzugt 85%, besonders bevorzugt 99%) auf das zugehörige Mikro-Well. Das Mikro-Well Array kann eine bevorzugte Ausführungsform eines Trägers sein. Insbesondere kann die 1. Schicht des Mikro-Well Arrays als Träger fungieren. Träger werden im Verlauf dieser Beschreibung noch genauer beschrieben und alle Merkmale, die im Zusammenhang mit einem Träger genannt werden, gelten insbesondere auch für die Träger-Ausführungsform Mikro-Well Array.

Das Mikro-Well Array ist bevorzugt für die Fluoreszenzmikroskopie geeignet. Das Mikro-Well Array ist ein Array von Wells, also Vertiefungen, mit bevorzugten Dimensionen von 10 bis 500 Mikrometer, wobei jedes Mikro-Well einen Boden und eine Umfangswand aufweist. Die Böden der Mikro-Wells sind bevorzugt für Fluoreszenzmikroskopie geeignet. Jedes der Mikro-Wells weist bevorzugt eine offene Oberseite auf.

Bevorzugt weist das Mikro-Well Array eine 1. Schicht, die bevorzugt die Böden der Mikro-Wells bildet, auf, wobei die 1. Schicht bevorzugt für Fluoreszenzmikroskopie geeignet ist und vorzugsweise ein Deckgläschen ist. Als Materialien für die 1. Schicht kommen sämtliche in dem Gebiet der Technik bekannten Materialien in Betracht, beispielsweise Glas oder Spezialkunststoffe. Das Mikro-Well Array weist ferner bevorzugt eine 2. Schicht, die an der 1. Schicht angebracht ist, auf, wobei die 2. Schicht die Wände der Mikro-Wells bildet. In einer bevorzugten Ausführungsform werden die Böden der Mikro-Wells durch die 1. Schicht gebildet und sind frei von dem Material der 2. Schicht. In einer anderen Ausführungsform können hingegen die Böden der Mikro-Wells wenigstens abschnittsweise mit Material der 2. Schicht, bevorzugt mit einer dünnen Schicht von 0 - 5 Mikrometer, stärker bevorzugt 0-1 Mikrometer, noch stärker bevorzugt 0-100 Nanometer, bedeckt sein. In einer bevorzugten Ausführungsform weist die 1. Schicht ein anderes Material auf als die 2. Schicht.

Das Material der 2. Schicht wird bevorzugt so ausgewählt, dass es eine möglichst geringe Autofluoreszenz in den für die Analyse relevanten Wellenlängen aufweist, so dass die Analyse nicht durch die Autofluoreszenz des Mikro-Well Arrays gestört wird. Bevorzugt ist das Fluoreszenzsignal durch die Autofluoreszenz der 2. Schicht in einer Fluoreszenzmessung gemäß dem Analyseverfahren nicht größer als das Fluoreszenzsignal durch eine 3D-DNA-Nanostruktur mit 5 Fluoreszenzmolekülen, bevorzugt 4 Fluoreszenzmolekülen, stärker bevorzugt 3 oder weniger Fluoreszenzmolekülen.

Um eine phänomenologische Autofluoreszenz $\delta$ der zweiten Schicht eines Mikro-Well Arrays zu messen, werden die experimentellen Verfahrensschritte einer Einzelzell-Genexpressionsanalyse nach dem weiter unten beschriebenen Beispiel 6 (also exklusive der in Beispiel 6 beschriebenen Auswertungsschritte) durchgeführt mit dem Unterschied, dass die 2. Schicht des Mikro-Well Arrays aus dem zu untersuchenden Material besteht. Die Aufnahme geschieht mithilfe eines Nikon Eclipse Ti Epifluoreszenzmikroskops. Als Lichtquelle dient eine Sola SE von Lumencor, Inc., Beaverton, OR, USA. Als Kamera dient eine pco.edge 4.2 von PCO GmbH, Kehlheim, Deutschland. Als Strahlenteiler wird Artikel F73-832 von AHF Analysentechnik AG, Tübingen, Deutschland genutzt. Für den blauen Kanal sind Anregungs- und Emissionsfilter F39-480 und F37-527; für den grünen Kanal sind es F39-563 sowie F39-637, und für den roten Kanal werden F39-640 und F37-698 genutzt. Als Objektiv kommt der FI Apochromat TIRF 60x Öl 1.49 von Nikon GmbH, Düsseldorf, Deutschland zum Einsatz. Zur Messung wird auf die obere Oberfläche der ersten Schicht fokussiert, sodass die 3D-DNA Nanostrukturen im Fokus sind. Für jeden Kanal werden zwei Aufnahmen mit Belichtungsdauer von 1 Sekunde bei maximaler Intensität der Lichtquelle gemacht: Eine von einem Bereich eines Mikro-Well-Bodens mit 3D-DNA Nanostrukturen, und eines von einem Bereich, der von der 2. Schicht ausgefüllt ist, gemacht. Die phänomenologische Autofluoreszenz $\delta$ der 2. Schicht wird für jeden Kanal separat berechnet und errechnet sich aus dem Quotienten der mittleren Pixelintensität des Bilds der 2. Schicht und dem Mittelwert der Maximalpixelwerte aller im Bild im entsprechenden Kanal zu sehenden 3D-DNA Nanostrukturen.

Die phänomenologische Autofluoreszenz $\delta$ beträgt bevorzugt 0 - 0,1, stärker bevorzugt 0 - $10^{-2}$, noch stärker bevorzugt 0 - $10^{-4}$, und ganz besonders bevorzugt 0 - $10^{-6}$.

Um ein Material, das für die 2. Schicht des Mikro-Well Arrays infrage kommt auf für das erfindungsgemäße Verfahren relevante Autofluoreszenz zu untersuchen sind folgende Überlegungen zu Absorption und Fluoreszenz hilfreich:

Im Allgemeinen lässt sich die Absorption in homogenen Materialien, die durch absorbierende Teilchen hervorgerufen wird, wie bspw in Demtröder, "Experimentalphysik 2 - Elektrizität und Optik", Springer 1995, ISBN 3-540-57095-0, pp. 212ff und P. Atkins: Physikalische Chemie, 5. Auflage, 2013, Wiley-VCH, Weinheim zu lesen, beschreiben durch die Reduktion der Strahlungsintensität I

$$I = I_0 * \exp(-A)$$

vom Anfangswert $I_0$, wobei sich die Absorption A darstellen lässt als

$$A = b * d = \varepsilon_{abs} * c * d = N * \sigma_{abs}$$

wobei d die Dicke des bestrahlten Materials ist, b der Absorptionskoeffizient, $\varepsilon_{abs}$ der konzentrationsbezogene Absorptionskoeffizient (meist Absorptionskoeffizient genannt, wobei dem Fachmann aus dem Kontext die Unterscheidung zwischen b und $\varepsilon_{abs}$ klar ist) und c die Konzentration der absorbierenden Teilchensorte.

In Anlehnung an den Streuquerschnitt (siehe bspw. Demtröder, "Experimentalphysik 2 - Elektrizität und Optik", Springer 1995, ISBN 3-540-57095-0, pp. 218ff) kann man für einzelne absorbierende Teilchen einen Absorptionsquerschnitt $\sigma_{abs}$ definieren, also eine gedachte Fläche um das Teilchen innerhalb derer jedes einfallende Photon absorbiert wird. Damit ist die Absorption

$$A = N * \sigma_{abs} / F = N * \sigma_{abs} * d / V$$

wobei N die bestrahlte Teilchenzahl, F die bestrahlte Probenfläche, d die Dicke des bestrahlten Materials und V das bestrahlte Probenvolumen bezeichnet.

Mit diesen Beziehungen lassen sich konzentrationsbezogener Absorptionskoeffizient und Absorptionsquerschnitt verbinden:

$$\sigma_{abs} = \varepsilon_{abs} / N_A$$

wobei $N_A$ die Avogadrokonstante von 6,022 * $10^{23}$ $mol^{-1}$ ist.

Wird ein einzelnes fluoreszierendes Teilchen mit einer Anregungsintensität $I_{ex}$ bestrahlt, wobei

$$I_{ex} = (h * \nu_{ex}) * N_{exphot} / (t * F),$$

also ein Fluss von $N_{exphot}$ Photonen, deren Energie h * $\nu_{ex}$ groß ist, in der Zeit t durch eine Fläche F, so gilt nach obiger Definition des Absorptionsquerschnitts $\sigma_{abs,}$ hier bezogen auf das Fluoreszierende Teilchen mit $\sigma_{abs,fluo}$ benannt, dass

$$N_{absphot} = N_{exphot} * \sigma_{abs,fluo} / F$$

Photonen in dieser Zeit absorbiert werden. Die Anzahl emittierter Photonen $N_{emphot}$ ist das Produkt aus Anzahl absorbierter Photonen und der Fluoreszenz-Quanteneffizienz $\eta_{fluo}$ des fluoreszierenden Teilchens (siehe bspw. P. Atkins: Physikalische Chemie, 5. Auflage, 2013, Wiley-VCH, Weinheim):

$$N_{emphot} = \eta_{fluo} * N_{absphot}$$

Dementsprechend gilt für den emittierten Photonenfluss $N_{emphot}$ / t eines fluoreszierenden Teilchens, der zum mit dem Mikroskop aufgenommenen und durch das Mikroskop-Bild repräsentierten Fluoreszenzsignal proportional ist:

$$N_{emphot} / t = \eta_{fluo} * \sigma_{abs,fluo} * I_{ex} / (h * \nu_{ex}) = \eta_{fluo} * (\varepsilon_{abs,\,fluo} / N_A) * I_{ex} / (h * \nu_{ex})$$

$\varepsilon_{abs,fluo}$ ($\varepsilon_{abs}$ für das fluoreszierende Teilchen) und $\eta_{fluo}$ sind Kenngrößen für Fluoreszenzmarker und beide werden vom Hersteller angegeben. Sie können auch durch Fluoreszenz- und Absorptionsspektroskopie selbstständig in einem Verfahren, das dem Fachmann geläufig ist, ermittelt werden. Hierfür können handelsübliche und dem Fachmann geläufige Geräte, bspw. die folgenden Geräte, verwendet werden: FluoroMax-4 und Quanta-Phi von Horiba Scientific (Vertrieb über Horiba Jobin Yvon GmbH, Bensheim, Deutschland). Dabei ist das FluorMax-4 für die Messung von $\varepsilon_{abs,fluo}$ und beide Geräte gemeinsam für die Messung von $\eta_{fluo}$ nötig.

Für ein Materialstück mit dem Absorptionskoeffizienten $b_{mat}$ (unten auch b_mat geschrieben) gilt wie oben beschrieben, dass die eingestrahlte Intensität I mit der Materialdicke d wie folgt vom Wert der eingestrahlten Intensität $I_{ex}$ abnimmt:

$$I = I_{ex} * e^{-b_mat*d}$$

Aufgrund der Energieerhaltung wird also folgende Intensität absorbiert:

$$I_{abs}{}^{mat} = I_{ex} * (1- e^{-b_mat*d})$$

Die Anzahl der auf einer Fläche $F_b$ in der Zeit t absorbierten Photonen $N_{absphot}{}^{mat}$ ist dann

$$N_{absphot}{}^{mat} / t = I_{abs} / (h * \nu_{ex}) * F_b = I_{ex} * (1- e^{-b_mat*d}) / (h * \nu_{ex}) * F_b$$

Die Anzahl emittierter Photonen $N_{emphot}{}^{mat}$ errechnet sich aus der Anzahl absorbierter Photonen durch Reduktion mit der Fluoreszenzquantenausbeute des Materials $\eta_{mat}$

$$N_{emphot}{}^{mat} / t = \eta_{mat} * N_{absphot} / t = \eta_{mat} * I_{ex} * (1- e^{-b_mat*d}) / (h * \nu_{ex}) * F_b$$

Der emittierte Photonenfluss $N_{emphot}{}^{mat}$ / t eines Materialstücks ist proportional zum im Mikroskop aufgenommenen und durch das Mikroskop-Bild repräsentierte Fluoreszenzsignal.

Um ein Material auf der Messung entgegenstehende Autofluoreszenz zu testen, müssen sein Absorptionskoeffizient $b_{mat}$, sowie seine Fluoreszenzquantenausbeute $\eta_{mat}$ bestimmt werden. Dazu wird, wie dem Fachmann geläufig ist, ein Probenstück davon in einer für Fluoreszenzspektroskopie günstigen Form hergestellt, in vielen Fällen beispielsweise in einem 1cm x 1cm x 3 cm großen Quader. Dieses wird mit handelsüblichen und dem Fachmann geläufigen Geräten, bspw. im FluoroMax-4 und dem Quanta-Phi von Horiba Scientific (Vertrieb über Horiba Jobin Yvon GmbH, Bensheim, Deutschland), vermessen und $b_{mat}$, sowie $\eta_{mat}$, für verschiedene Wellenlängenbereiche bestimmt. Die für das erfindungsgemäße Verfahren relevanten Wellenlängenbereiche sind die mit den in den 3D-DNA Nanostrukturen verbauten Fluoreszenz-Farbstoffmolekülen verwendeten Anregungs- und Emissionswellenlängenbereiche. Anregungswellenlängen können beispielsweise in den Bereichen 510-550 Nanometer, 570-630 Nanometer, und/oder 660-720 Nanometer liegen.

Die Messung von b und $\eta$ für ein Material kann mit dem Quanteneffizienz-Messsystem von Horiba, bestehend aus FluoroMax-4 mit CCD und Quanta-Phi erfolgen. Dazu wird der Material-Quader in die Küvettenhalterung des Flüssig-Probenhalters für den Quanta-Phi eingelegt. Der Probenhalter wird anschließend in die Photometerkugel des Quanta-Phi gesetzt und dieser verschlossen. Anschließend wird die optische Faser zwischen Quanta-Phi und FluoroMax gemäß Bedienungsanleitung verlegt. Die Messung des Materials geschieht mithilfe der Software FluorEssence™ von Horiba Scientific entsprechend der Quanta-Phi Bedienungsanleitung ("Quanta-phi F-3029 Integrating Sphere Operation Manual - Part number J81089 rev. C", Horiba Scientific 2010). Als Hintergrundsprobe wird eine leere Probenkammer vermessen. Für diese wird in der Software der Rayleigh excitation peak auf $10^6$ Counts/s eingestellt. Anregungswellenlängen werden für Messungen in den Kanälen Blau, Grün und Rot auf 488 Nanometer, 561 Nanometer, und 647 Nanometer eingestellt. Für die Hintergrundmessung wird der Emissionsbereich von 290 Nanometer bis 1100 Nanometer eingestellt. Integrationszeit und Anzahl von Akkumulationen wird wie im Handbuch beschrieben so eingestellt dass der Messwert bei der Anregungswellenlänge bei $10^6$ Counts/s liegt. Anschließend wird die Messung mit den selben Messparametern am Materialprobenquader durchgeführt. Die Auswertung wird mit der Software wie im Benutzerhandbuch angegeben gemacht und die Anregungswellenlängen 480-495, 555-570 und 640-655 Nanometer für Blau, Grün und Rot sowie die Emissionswellenlängen 500-540, 575-620, und 660-700 Nanometer eingestellt. Die Software errechnet dann einen Wert für die Quantenteffizienz $\eta$.

Um die Absorptionskonstante b zu messen wird statt der optischen Faser wieder die normale Küvettenprobenhalterung in das FluoroMax-4 eingebaut und eine Absorptionsmessung nach Benutzerhandbuch ("FluoroMax-4 & FluoroMax-4P with USB Operation Manual - Part number J810005 rev. D", Horiba Scientific 2012) mit oben angegebenen Anregungswellenlängen für die Verschiedenen Kanäle durchgeführt. Der Messwert für die Hintergrundmessung, die wieder ohne Probe statt findet, wird wieder auf $10^6$ Counts/s eingestellt.

Wie störend sich die Autofluoreszenz des Materials der 2. Schicht auf die Messung auswirkt, kann durch das Verhältnis $\gamma$ von Fluoreszenzsignal einer beugungsbegrenzten Fläche $F_b$ von Material der 2. Schicht der Dicke d, die der Höhe der Miko-Wells entspricht, zum Fluoreszenzsignal eines Fluoreszenzfarbstoffes ermittelt werden:

$$\gamma = \eta_{mat} * N_{Avogadro} * F_b * (1 - e^{-b_mat*d}) / (\varepsilon_{abs,fluo} * \eta_{fluo})$$

Für die beugungsbegrenzte Fläche wird eine Airy-Scheibe (Y. Soskind, "Field Guide to Diffractive Optics", SPIE Press 2011, Bellingham, Washington USA, ISBN 978-0-8194-8690-5, p. 14) mit Radius $r_{airy}$ verwendet:

$$F_b = \pi\, r_{airy}^2 = \pi * (0{,}61 * \lambda / NA)^2$$

wobei $\pi$ die Kreiszahl (3,1415...), $\lambda$ die Emissionswellenlänge, bzw. die Zentralwellenlänge des Emissions-Wellenlängenbereichs, und NA die numerische Apertur des verwendeten Mikroskops ist.

$\gamma$ wird im Folgenden als fluorophorbezogene Autofluoreszenz bezeichnet und ist bevorzugt kleiner als 5, stärker bevorzugt kleiner als 2, besonders bevorzugt kleiner als 0,5, ganz besonders bevorzugt kleiner als 0,01 für mindestens einen der Fluoreszenzkanäle.

Bevorzugt weist die 2. Schicht SU-8, bevorzugt PEG-DA, besonders bevorzugt PDMS, stärker bevorzugt Cytop, und besonders bevorzugt Liquid Glass auf. Besonders bevorzugt besteht die 2. Schicht aus einem dieser Materialien. Liquid Glass bezeichnet ein Nanokomposit aus Lösungsmittel, Kunststoff und Silica Nanopulver, das bei Raumtemperatur flüssig ist und durch Sintern im Ofen zu fast reinem, bevorzugt reinem Siliziumdioxid/Glas gebacken wird (siehe bspw. F. Kotz et al, "Three-dimensional printing of transparent fused silica glass", Nature 2017, 544 (7650), 337-339 sowie F.Kotz et al., "Liquid Glass: A Facile Soft Replication Method for Structuring Glass", Advanced Materials 2016, 28, 4646-4650).

In einer bevorzugten Ausführungsform besteht die 2. Schicht aus einem elastischen Material, bevorzugt PDMS, und schließt das Mikro-Well nach oben hin ab, während die 1. und 2. Schicht reversibel, bevorzugt durch Druck, miteinander verbunden sind.

Alternativ oder zusätzlich kann ein Mikro-Well auch in eine Schicht, insbesondere wenn es sich um Glas handelt, geätzt werden. Im Resultat liegt wieder eine erste, untere Schicht vor, die durchgängig ausgebildet ist, und eine zweite, obere Schicht mit einer geätzten Ausnehmung als Mikro-Well, wobei die zweite Schicht stoffschlüssig mit der 1. Schicht verbunden ist und die Umfangswand des Mikro-Wells bildet.

Die Formen der einzelnen Wells sind bevorzugt einheitlich, können sich aber auch unterscheiden. Die Form eines Wells in Draufsicht kann beispielsweise quadratisch, kreisförmig, elliptisch, hexagonal, rund, dreieckig, polygonal und/oder von unregelmäßiger Form sein. Der Boden eines Mikro-Wells hat bevorzugt einen Durchmesser eines einbeschriebenen Kreises von 10 $\mu$m -1000 $\mu$m, bevorzugt 20 $\mu$m - 500 $\mu$m, stärker bevorzugt 50 $\mu$m -200 $\mu$m, und/oder eine Fläche von 1.000.000 $\mu m^2$ oder kleiner, vorzugsweise 250.000 $\mu m^2$ oder kleiner, besonders bevorzugt 40.000 $\mu m^2$ oder kleiner. Die Tiefe eines Mikro-Wells ist vorzugsweise gleich oder größer als der Durchmesser des einbeschriebenen Kreises des jeweiligen Mikro-Wells.

In einem Aspekt richtet sich die vorliegende Erfindung auch auf ein Mikro-Well Array, wobei ein oder mehrere Träger-Adapter am Boden mindestens eines der Mikro-Wells gebunden ist. Bevorzugt ist bzw. wird die Zielstruktur vermittelt durch einen Träger-Adapter, der an die Zielstruktur spezifisch bindet, an den Träger bzw. die erste Oberfläche des Trägers gebunden. Bevorzugt ist bzw. wird also die Zielstruktur an den Boden des mindestens einen Mikro-Wells gebunden, wobei diese Bindung bevorzugt durch einen spezifisch an die Zielstruktur bindenden Träger-Adapter vermittelt wird.

In einer besonders bevorzugten Ausführungsform kann das Mikro-Well Array als Teil einer Mikro-Well Kammer ausgebildet sein. In der Mikro-Well Kammer weist das Mikro-Well Array eine 3. Schicht, die an der 2. Schicht angebracht ist, auf. Die Anordnung der 1., 2. und 3. Schicht ist dabei bevorzugt derart, dass sich die 2. Schicht zwischen der 1. und der 3. Schicht befindet. Die 3. Schicht weist einen zusätzlichen Hohlraum auf, der sich direkt an die Mikro-Wells des Mikro-Well Arrays anschließt, d.h., der zusätzliche Hohlraum der 3. Schicht und die durch die Mikro-Wells gebildeten Hohlräume bilden einen zusammenhängenden Arbeitshohlraum, der durch die 1. Schicht, die 2. Schicht und die 3. Schicht gegen die Umgebung abgeschlossen ist.

Der Hohlraum der 3. Schicht ist ferner bevorzugt über einen Einlass und einen Auslass, die sich jeweils durch die 3. Schicht hindurch erstrecken, mit der Umgebung verbunden. Ein- und Auslass sind bevorzugt dafür vorgesehen, den Arbeitshohlraum effizient mit Flüssigkeit zu befüllen bzw. überschüssige Flüssigkeit abzuführen, insbesondere im Rahmen der Verfahrensschritte der vorliegenden Erfindung. Hierzu kann der Einlass und/oder der Auslass mit einem Standard-Verbinder, beispielsweise einem Luer-Verbinder, versehen sein.

Diese Ausführungsform ist insbesondere deshalb vorteilhaft, weil dadurch einerseits die Einfachheit der bereits beschriebenen Arbeitsschritte durch die nach oben offenen Mikro-Wells beibehalten wird, andererseits aber das Fließverhalten und das Volumen der verwendeten Flüssigkeiten besser kontrolliert werden kann, so dass letztendlich der Verbrauch an Reagenzien und Probenmaterialien minimiert werden kann.

Der Verfahrensschritt des Einbringens einer Gruppe von Wirtskörpern inklusive eines Wirtskörpers mit der Zielstruktur in ein Mikro-Well Array derart, dass in mindestens einem Mikro-Well genau ein Wirtskörper vorhanden ist, stellt einen Vereinzelungsschritt der Wirtskörper dar. Bevorzugt wird hierzu ein Volumen einer geeigneten Flüssigkeit mit der Gruppe von Wirtskörpern in geeigneter Konzentration verwendet. Bevorzugt wird die Wirtskörper- Flüssigkeit in die Mikro-Wells eingebracht und anschließend den Wirtskörpern Zeit gegeben, durch Wirkung der Schwerkraft auf den Boden der Mikro-Wells zu sinken. Für HeLa-Zellen in PBS (Rezeptur siehe bspw. Beispiel 6) kann die Zeit für das Absinken bspw. 15-30 min betragen. Bevorzugt erfolgt das Einbringen der Wirtskörper-Flüssigkeit in die Mikro-Wells, indem die Wirtskörper-Flüssigkeit auf die offene Seite des Mikro-Well Arrays aufgebracht wird. Für den Fall dass die Wirtskörper Bakterien, Viren oder andere Wirtskörper sind, die nicht durch Schwerkraft absinken, kann die Oberfläche der 1. und/oder 2. Schicht des Mikro-Well Arrays mit einem kationischen Polymer wie beispielsweise Poly-L-Lysin (PLL) versehen sein, sodass basierend auf statistischer Bewegung und/oder Diffusion solcher Wirtskörper ein hinreichender Anteil nahe genug an das kationische Polymer gelangt, sodass die Wirtskörper daran haften (siehe bspw. B. Fang et al., "Surfaces for Competitive Selective Bacterial Capture from Protein Solutions", ACS Appl. Mater. Interfaces, 2015, 7 (19), pp. 10275-10282). Das Versehen des Mikro-Well Arrays mit einem kationischen Polymer erfolgt bevorzugt gemeinsam mit, vor und/oder nach einem gegebenenfalls vorhandenen Schritt des Versehens des Mikro-Well Arrays mit Trägeradapter, bevorzugt vor dem Verfahrensschritt p1). Beispielsweise kann in einem zusätzlichen Verfahrensschritt cc) vor Verfahrensschritt p1) eine Lösung mit 0,01-5 mg/ml, bevorzugt 0,1-1 mg/ml Poly-L-Lysin mit einer molekularen Masse von bevorzugt 20000 g/mol (wie beispielsweise von Sigma-Aldrich kommerziell erhältlich) in einem Puffer, bevorzugt RX07, eingespült und für 5-120 Minuten, bevorzugt 15-45 Minuten inkubiert werden.

Um eine etwaige ungewollte Bindung der 3D-DNA Nanostrukturen an das kationische Polymer zu verhindern kann optional ein weiterer Verfahrensschritt dd) nach Verfahrensschritt p1) und vor Verfahrensschritt p2) eingeführt werden, in dem die postitiven Ladungen des auf dem Mikro-Well Array befindlichen kationischen Polymers durch ein anionisches Polymer abgesättigt wird. Beispielsweise kann hierin eine Lösung von 0,01-5 mg/ml, bevorzugt 0,1-1 mg/ml PSS (Polystyrolsulfonat) mit einer Molekularen Masse von bevorzugt 10 000-200 000 g/mol, stärker bevorzugt 20 000 g/mol in einem Puffer, bevorzugt RX07, eingespült und für 5-120 Minuten, bevorzugt 15-45 Minuten inkubiert werden.

Das Einbringen der Wirtskörper-Flüssigkeit in die Mikro-Wells kann, insbesondere bei ausreichender Größe des Mikro-Well Arrays von bevorzugt mindestens 1 mm x 1 mm, stärker bevorzugt mindestens 2 mm x 2 mm, stärker bevorzugt mindestens 5 mm x 5 mm, stärker bevorzugt 1 cm x 1 cm, besonders bevorzugt 1,5 cm x 1,5 cm, durch Pipettieren mit gängigen Pipetten und Pipettenspitzen erfolgen.

Alternativ oder zusätzlich kann das Einbringen der Wirtskörper-Flüssigkeit in die Mikro-Wells mittels eines Schlauchs und/oder eines anderen Flüssigkeitskanals erfolgen.

Die Eignung einer Flüssigkeit für die Wirtskörper-Flüssigkeit hängt von der Art des Wirtskörpers sowie von den übrigen am Verfahren beteiligten Substanzen ab und wird vom Fachmann passend gewählt. Bevorzugt handelt es sich um eine wässrige und/oder hydrophile Flüssigkeit. Beispielsweise kann es sich um eine Zellsuspension handeln, bei der Zellen (z.B. HeLa-Zellen) in PBS vorliegen. Geeignete Wirtskörper-Konzentrationen richten sich nach der Geometrie des Mikro-Well Arrays, also nach Form, Größe und Anordnung (insbesondere Abstände) der Mikro-Wells, und nach der Form und Größe der Wirtskörper. Die Konzentration sollte groß genug sein, um in einer ausreichenden Zahl von Mikro-Wells einen einzelnen Wirtskörper vorliegen zu haben. Die Konzentration sollte aber soweit reduziert sein, dass die Zahl jener Mikro-Wells, die mehr als einen Wirtskörper aufweisen, möglichst gering ist. Ein guter Ansatzpunkt für die zu wählende Konzentration ist eine Zelle pro Mikro-Well in der Befüllungslösung zu haben. Beispielsweise kann ein Mikro-Well Array mit einer Fläche von 1 $cm^2$ und einer Million Mikro-Wells mit einer Zellsuspension befüllt werden, die 1 Millimeter hoch auf dem Mikro-Well Array steht und 10 Millionen Zellen pro Milliliter enthält. Eine weitere Befüllungsmöglichkeit ist einen Mikro-Well Array mit zehntausend Mikro-Wells pro Quadratzentimeter mit einer Wirtskörpersuspension zu befüllen, die 100 Mikrometer hoch steht und 1 Million Wirtskörper pro Milliliter enthält.

In einer bevorzugten Ausführungsform kann die Befüllung der Mikro-Wells mithilfe einer Schablone durchgeführt werden. Dazu kann eine vorgeformte Schicht mit Vertiefungen, bevorzugt aus PDMS, hergestellt werden, wobei die Vertiefungen das 0,2-5-fache, bevorzugt das 0,5-2-fache, stärker bevorzugt das 0,8-1,2-fache des mittleren Volumens der Wirtskörper besitzen (siehe bspw. Y. Wang et al., "Trapping cells on a stretchable microwell array for single-cell analysis", Anal Bioanal Chem 2012, 402(3): 1065-72; C.Probst et al., "Polydimethylsiloxane (PDMS) Sub-Micron Traps for Single-Cell Analysis of Bacteria). Die Abstände zwischen den Vertiefungen können gleich groß sein wie die zwischen den Mikro-Wells auf dem Mikro-Well Array. Diese Schablone kann durch Überschütten mit einer dichten Wirtskörper-Suspension (beispielsweise 1 Million - 100 Milliarden Wirtskörper pro Milliliter, bevorzugt 10 Millionen - 1 Milliarde Wirtskörper pro Milliliter) befüllt werden, wobei die meisten Vertiefungen (80%, bevorzugt 90%, stärker bevorzugt 95%, noch stärker bevorzugt 99%) von genau einem Wirtskörper besetzt sind, da die Vertiefungen für mehr als einen Wirtskörper keinen Platz bieten. Daraufhin kann die Schablone mit der offenen Seite nach unten auf den Mikro-Well Array gegeben werden und die Mikro-Wells optional durch Druck mit der Schablone verschlossen werden. Aufgrund der übereinstimmenden Abstände von Vertiefungen und Mikro-Wells befindet sich nun genau eine Vertiefung über einem Mikro-Well, und die Wirtskörper sinken durch Schwerkraft von der Schablone in die Mikro-Wells. Diese Ausführungsform hat den Vorteil einer effizienteren Einzelbelegung der Mikro-Wells durch Wirtskörper.

In einer bevorzugten Ausführungsform des erfindungsgemäßen Verfahrens kann nach Schritt e) ein Verfahrensschritt h) durchgeführt werden, bei dem die Umfangswände der Mikro-Wells, vorzugsweise die 2. Schicht entfernt wird.

Beispielsweise kann hierzu eine Ausführungsform des erfindungsgemäßen Mikro-Well Arrays genutzt werden, bei der die 2. Schicht aus einem elastischen Material, bevorzugt PDMS besteht und die Mikro-Wells nach oben hin verschließt. Die Mikro-Wells dieser 2. Schicht kann umgedreht wie oben beschrieben in Schritt p1) mit Wirtskörpern befüllt werden und anschließend mit einer mit Trägeradaptern versehenen 1. Schicht reversibel, beispielsweise durch Druck, verschlossen werden. Daraufhin können die Wirtskörper in Schritt e) bevorzugt physikalisch, besonders bevorzugt laserinduziert oder akustisch, lysiert werden. Insbesondere kann das Mikro-Well Array beispielsweisse mit einem 400 Nanometer - 2000 Nanometer, bevorzugt 1064 Nanometer-Laser mit 50-500 Milliwatt Leistung für 1 Minute - 120 Minuten, bevorzugt 15 Minuten beleuchtet und dadurch die Zellen lysiert werden. Die nun freien Zielstrukturen können nun innerhalb einer Inkubationszeit von 5 Minuten bis 24 Stunden, bevorzugt 10 -120 Minuten, stärker bevorzugt 15-45 Minuten, an die Trägeradapter der ersten Schicht binden. Nun kann in Verfahrensschritt h) die zweite Schicht von der 1. Schicht entfernt werden. Sie kann in einer bevorzugten Variante komplett entfernt werden, sodass die weiteren Verfahrensschritte ohne sie statt finden. Alternativ kann sie in einer stark bevorzugten Variante um 10 Mikrometer - 5 Millimeter, bevorzugt 100-200 Mikrometer weit entfernt werden, sodass sich eine Flüssigkeitskammer bildet, durch die hindurch die anschließenden mit Flüssigkeitsaustausch einhergehenden Verfahrensschritte realisiert werden können.

Vorteil dieser Ausführungsform ist, dass der Flüssigkeits- und Komponentenaustausch in den folgenden Verfahrensschritten freier und weniger durch die Barrieren der 2. Schicht behindert geschehen kann. Dadurch können die jeweiligen Verfahrensschritte schneller und damit ökonomischer vonstatten gehen. Der Schritt des Einbringens von mindestens zwei 3D-DNA-Nanostrukturen in das mindestens eine Mikro-Well kann auf die gleiche Art und Weise erfolgen wie das Einbringen der Wirtskörper-Flüssigkeit, also beispielsweise durch Pipettieren und/oder Applizieren mittels eines Schlauchs oder Ähnlichem, wie oben und später beschrieben. Das Einbringen der 3D-DNA-Nanostrukturen kann vor, gleichzeitig mit und/oder nach dem Einbringen der Wirtskörper erfolgen. Die Reihenfolge hängt u.a. davon ab, ob und ggf. welche der am Verfahren beteiligten Strukturen an den Träger gebunden werden sollen.

In einer bevorzugten Ausführungsform der Erfindung umfasst das Verfahren ferner:

f) Schließen der Oberseite der Mikro-Wells durch Aufbringen einer bevorzugt fluorinierten Ölschicht auf die 2. Schicht.

Der Schritt des Aufbringens einer bevorzugt fluorinierten Ölschicht auf die 2. Schicht erfolgt nach Schritt p1) und bevorzugt durch Überschütten des mit bevorzugt wässriger Wirtskörper-Flüssigkeit befüllten Mikro-Well Arrays mit bevorzugt fluoriniertem Öl. Dadurch wird überschüssige wässrige Flüssigkeit abgespült und nur die wässrige Flüssigkeit, die sich bereits zuvor in den Mikro-Wells befunden hat, verbleibt dort. Die bereits zuvor in den Mikro-Wells befindliche wässrige Flüssigkeit verbleibt dort, weil das bevorzugt fluorinierte Öl aufgrund seiner Hydrophobizität durch die wässrige Flüssigkeit innerhalb der Mikro-Wells von einem Eindringen in die Mikro-Wells abgehalten wird. Somit hat das bevorzugt fluorinierte Öl keinen Angriffspunkt, um die wässrige Flüssigkeit innerhalb der Mikro-Wells aus den Mikro-Wells herauszuspülen. Zusätzlich ist die Dichte des bevorzugt fluorinierten Öls geringer als die der wässrigen Lösung und wirkt somit einer Befüllung der Mikro-Wells durch bevorzugt fluoriniertes Öl entgegen. Nach Beendigung des Überschüttens mit bevorzugt fluoriniertem Öl verbleibt eine bevorzugt fluorinierte Ölschicht, die die Mikro-Wells gegen die Umgebung abschließt. Eine Vermischung mit der wässrigen Flüssigkeit innerhalb der Mikro-Wells findet aufgrund der bereits erwähnten Hydrophobizität des bevorzugt fluorinierem Öls nicht statt.

In einer bevorzugten Ausführungsform ist das Öl mit Lipiden versetzt. Lipide haben, wie allgemein bekannt ist, einen hydrophoben und einen hydrophilen Abschnitt. Dadurch bildet sich an der Grenzfläche zwischen dem Öl und den Mikro-Wells eine Lipidschicht aus. Bei Ersetzen der Ölschicht durch wässrige Lösung legt sich über die Einzel-Lipidschicht eine zweite Lipidschicht, sodass eine Lipid-Doppelschicht entsteht.

Eine bevorzugte Ausführungsform des Verfahrens umfasst daher ferner:

g) Abziehen des Öls durch Überschütten des Mikro-Well Arrays mit wässriger Lösung.

Dadurch wird das nicht weiter benötigte Öl von dem Mikro-Well Array entfernt, wobei eine Lipid-Doppelschicht zurückbleibt. Die Mikro-Wells bleiben somit mit einer Lipid-Doppelschicht verschlossen.

In einer bevorzugten Ausführungsform enthält die wässrige Lösung in Verfahrensschrit g) Membrankanäle, beispielsweise Ionenkanäle. Diese bauen sich selbstständig in die Lipid-Doppelschicht, die das Mikro-Well abschließt, ein (siehe bspw. R Watanabe et al., "Arrayed lipid bilayer chambers allow single-molecule analysis of membrane transporter activity", Nat. Commun. 5, ncomms5519(2014)). Dadurch kann die Lipid-Doppelschicht für Ionen durchlässig, für größere Moleküle aber undurchlässig gemacht werden. So kann im Anschluss die Salzkonzentration in den Mikro-Wells durch Austausch der wässrigen Lösung außerhalb der Mikro-Wells durch eine hypotonische Lösung (mit geringerer Salzkonzentration) gesenkt werden und damit die Wirtskörper hypotonisch lysiert werden (also durch Osmose zum Platzen gebracht werden, siehe bspw. C. J. Schröteret al., "A rapid method to separate endosomes from lysosomal contents using differential centrifugation and hypotonic lysis of lysosomes", Journal of immunological methods 1999, 227 (1-2), pp. 161-168) unter Sicherstellung, dass die Zielstrukturen in dem Mikro-Well bleiben und nicht hinausdiffundieren.

In einer bevorzugten Ausführungsform des Verfahrens umfasst daher der Schritt g):
g) Abziehen des Öls durch Überschütten des Mikro-Well Arrays mit wässriger Lösung, die mit Membrankanälen versetzt ist.

Um die Lipid-Doppelschicht mit Membrankanälen zu versehen kann beispielsweise 0,01-5 mg/ml $\alpha$-Haemolysin ($\alpha$HL, beispielsweise von Sigma-Aldrich kommerziell erhältlich), bevorzugt 0,5-1 mg/ml aHL in einem Puffer, vorzugsweise PBS oder RX07, in Verfahrensschritt g) verwendet werden und für 5-120 Minuten, bevorzugt 15-45 Minuten inkubiert werden (siehe bspw. X. Zhang et al., "Natural channel protein inserts and functions in a completely artificial, solid-supported bilayer membrane", Scientific Reports 2013, 3: 2196, DOI: 10.1038/srep02196).
In einer alternativen erfindungsgemäßen Ausführungsform kann in Schritt g) der Membrankanal TRPM8 in die Lipid-Doppelschicht inkorporiert werden. Dazu wird mizellare RGPM8-Lösung (beschrieben in E. Zakharian, "Recording of Ion Channel Activity in Planar Lipid Bilayer Experiments", Methods Mol Biol. 2013, 998, pp. 109-118) in Verfahrensschritt g) als membrankanalhaltige wässrige Lösung verwendet. Wenigstens ein Teil der membrankanalhaltigen wässrigen Lösung verbleibt auf dem Mikro-Well Array und wird für 5 Minuten - 24 Stunden, bevorzugt 2 Stunden, inkubiert, sodass sich Membrankanäle aus der membrankanalhaltigen wässrigen Lösung in die Lipid-Doppelschicht einbauen.

Das Verschließen der Mikro-Wells mittels einer Ölschicht bzw. einer Lipid-Doppelschicht (Verfahrensschritte f) und g)) geschieht bevorzugt vor dem Einbringen der DNA-Nanostrukturen und/oder nach dem Aufschluss der Wirtskörper, um ein Entweichen der noch ungebundenen Zielstrukturen zu verhindern und die Effizienz zu erhöhen, mit der sie an die Träger-Adapter binden. Nach einer ausreichenden Inkubationszeit von 15 Minuten, bevorzugt 30 Minuten, besonders bevorzugt 1 Stunde, ganz besonders bevorzugt 24 Stunden zwischen den Verfahrensschritten f) und g) sind fast alle, bevorzugt alle Zielstrukturen an den Träger-Adapter gebunden und laufen nicht mehr Gefahr, aus dem Mikro-Well heraus zu diffundieren. Somit können die 3D-DNA Nanostrukturen nach Verfahrensschritt g) hinzugefügt werden. Hierbei ist zu beachten, dass dies im Falle eines Öls ohne Lipide nach Schritt g) geschehen kann, weil dann die verschließende Ölschicht bereits in Schritt g) abgespült wird. Für den Fall, dass das Verfahren die Ausbildung einer Lipidschicht (also die Verwendung lipidhaltigen Öls) aufweist, muss zunächst die Lipid-(Doppel)schicht entfernt werden, bevor die 3D-DNA Nanostrukturen hinzugefügt werden. Das Entfernen der Lipid-Doppelschicht kann erfolgen, indem mit einem Lysepuffer für ein Detergens-basiertes Lyseverfahren (siehe bspw. "Thermo Scientific Pierce Cell Lysis Technical Handbook" (2 ed.), Thermo Scientific) gespült und für 5 - 120 Minuten, bevorzugt 15-45 Minuten inkubiert wird. Anschließend können die 3D-DNA Nanostrukturen in die nun wieder von der Öl- bzw. Lipid-Doppelschicht befreiten Mikro-Wells eingebracht werden.

Die oben erwähnten Ausführungsformen des Verschließens der Mikro-Wells mittels einer Ölschicht bzw. einer Lipid-Doppelschicht (Verfahrensschritte f) und g)) erfolgen bevorzugt unter Verwendung einer Mikro-Well Kammer. Hierbei werden alle Schritte wie bereits beschrieben durchgeführt, wobei das Einbringen sämtlicher Flüssigkeiten in die Mikro-Wells über den Einlass der Mikro-Well Kammer erfolgt und überschüssige bzw. abgespülte Flüssigkeit durch den Auslass der Mikro-Well Kammer entweicht.

Wie bereits beschrieben, bietet die Mikro-Well Kammer den Vorteil, dass die Fließrichtung der eingespülten und ausgespülten Flüssigkeit(en) besonders kontrolliert und besonders vorteilhaft ist. Dies gilt insbesondere für das wechselseitige Spülen mit wässrigen Lösungen und Öl. Das Abspülen der überschüssigen wässrigen Lösung durch Öl von der Oberfläche des Mikro-Well Arrays wird durch die abscherende Wirkung eines parallel zur Oberfläche der 2. Schicht erfolgenden Flusses, wie er durch die Anordnung in der Mikro-Well Kammer begünstigt wird, besonders gefördert. Das gleiche gilt für das Abspülen des Öls mittels einer wässrigen Lösung unter Zurückbleiben der Lipid-Doppelschicht.

Ferner richtet sich die Erfindung u.a. auf eine 3D-DNA-Nanostruktur, auf der mindestens ein Fluoreszenz-Farbstoffmolekül angebracht ist, wobei die Form der 3D-DNA-Nanostruktur verhindert, dass bei Annäherung an eine zweite 3D-DNA-Nanostruktur, auf der mindestens ein Fluoreszenz-Farbstoffmolekül angebracht ist, die Fluoreszenz-Farbstoffmoleküle der beiden 3D-DNA-Nanostrukturen signifikant wechselwirken.

Bevorzugt ist die erfindungsgemäße 3D-DNA-Nanostruktur eine 3D-DNA-Nanostruktur mit einem Hohlraum und mindestens einem innenliegenden Fluoreszenz-Farbstoffmolekül, wobei der Abstand des mindestens einen innenliegenden Fluoreszenz-Farbstoffmoleküls zum Rand der 3D-DNA-Nanostruktur mindestens 2 nm, bevorzugt mindestens 3 nm und besonders bevorzugt mindestens 5 nm beträgt.

Durch die geschickte Wahl der Form der 3D-Nanostrukturen und der Anordnung der Fluoreszenz-Farbstoffmoleküle im Inneren/im Hohlraum der 3D-Nanostruktur wird sichergestellt, dass die Farbstoffmoleküle nicht mit der Umgebung sterisch wechselwirken. Wie in den angefügten experimentellen Beispielen gezeigt, wechselwirken die erfindungsgemäßen 3D-Nanostrukturen dadurch z.B. deutlich geringer mit Oberflächen, wie einer Glasoberfläche, als 2D-Nanostrukturen mit den gleichen Farbstoffen. Dies ist von großem Vorteil für die Verwendung einer solchen 3D-DNA Nanostruktur im erfindungsgemäßen Verfahren. Beispielsweise kann so die Rate von falsch-positiven Nachweisen einer Zielstruktur verringert werden, insbesondere wenn eine Trägerstruktur, bspw. das Mikro-Well Array Anwendung findet. Ferner ist ein Vorteil der Abschirmung der Fluoreszenz-Farbstoffmoleküle, dass die Fluoreszenz-Farbstoffmoleküle der beiden 3D-DNA-Nanostrukturen nicht signifikant wechselwirken (optisch und/oder sterisch). Dies ermöglicht, dass das Fluoreszenzsignal einer erfindungsgemäßen 3D-DNA-Nanostruktur nicht signifikant durch eine sehr naheliegende benachbarte 3D-DNA-Nanostruktur beeinflusst wird. Ferner wird die über Fluoreszenz-Farbstoffmoleküle vermittelte unspezifische Interaktion von 3D-DNA-Nanostrukturen verhindert. All dies zusammen ist von großem Vorteil bei der Verwendung dieser 3D-Nanostrukturen zum Nachweis von Zielstrukturen. Insbesondere erlaubt dies, wie im erfindungsgemäßen Verfahren, mindestens zwei 3D-DNA Nanostrukturen an eine Zielstruktur zu binden, ohne dass das Fluoreszenzsignal der einzelnen 3D-DNA-Nanostrukturen durch die Nähe zueinander beeinflusst wird (z.B. durch Quenching/Fluoreszenzauslöschung oder FRET).

Die Verwendung von mindestens zwei DNA-Nanostrukturen kann je nach Messverfahren sogar notwendig sein, um die Identifizierungsstruktur aus Zielstruktur und gebundenen DNA-Nanostrukturen von den freien DNA-Nanostrukturen zu unterscheiden. Dies ist vor allem bei Messverfahren, die in Lösung ablaufen, der Fall. Wird im Verfahren die Identifizierungsstruktur zum Nachweis an einen Träger bzw. eine Trägeroberfläche gebunden (wie in einer bevorzugten Ausführungsform des erfindungsgemäßen Verfahrens, insbesondere unter Verwendung des Mikro-Well Arrays als Träger), ermöglicht dies freie DNA-Nanostrukturen mit einem oder mehreren Waschschritten zu entfernen. Die Verwendung von mindestens zwei DNA-Nanostrukturen, die an eine Zielstruktur binden, hat in einem Verfahren, das einen Träger benutzt, den Vorteil, dass die Rate an falsch positiven Nachweisen verringert werden kann. Zum Einen kann eine Identifizierungsstruktur mit mindestens zwei DNA-Nanostrukturen eindeutig von unspezifisch mit der Oberfläche interagierenden DNA-Nanostrukturen unterschieden werden. Dies ist in einem analogen Verfahren, in dem nur eine DNA-Nanostruktur die Zielstruktur erkennt, nicht möglich. Zum zweiten, kann durch die Verwendung von mindestens zwei DNA-Nanostrukturen, die beide unterschiedliche Regionen der Zielstruktur erkennen, die Wahrscheinlichkeit, dass fälschlicherweise andere ähnliche Zielstrukturen vermeintlich als Zielstruktur nachgewiesen werden, massiv verringert und in vielen Fällen ausgeschlossen werden.

Zudem kann im Fall der unabhängigen Kopplung mindestens zweier 3D-DNA Nanostrukturen (z.B. verschiedener orthogonal messbarer Nanoreporter) an Zielmoleküle die Sensitivität der Messung drastisch erhöht werden. Sie erhöht sich mit der Potenz der Anzahl unabhängiger Kopplungsreaktionen. Das ist insbesondere für eine hohe Dynamik, also beispielsweise das simultane Messen sehr niedrig exprimierter und sehr hoch exprimierter Gene von großem Vorteil.

In einer bevorzugten Ausführungsform, richtet sich die vorliegende Erfindung auch auf ein Verfahren für den Nachweis bzw. die Quantifizierung von mindestens zwei verschiedenen Zielstrukturen (z.B. zwei m-RNAs, die von unterschiedlichen Genen abstammen, d.h. eine unterschiedliche Nukleinsäuresequenz aufweisen), bevorzugt einer Vielzahl von verschiedenen Zielstrukturen. Die verschiedenen Zielstrukturen sind dabei paarweise unterscheidbar.

Dieses Verfahren umfasst bevorzugt folgende Schritte:

a) Ausbilden jeweils einer Identifizierungsstruktur für jede der mindestens zwei verschiedenen Zielstrukturen, die aufweist:

(i) die jeweilige Zielstruktur, und
(ii) mindestens zwei 3D-DNA-Nanostrukturen, wobei jede der mindestens zwei 3D-DNA-Nanostrukturen ein oder mehrere innenliegende Fluoreszenz-Farbstoffmoleküle aufweist und wobei jede der mindestens zwei 3D-DNA-Nanostrukturen spezifisch an die jeweilige Zielstruktur gebunden ist, und wobei die mindestens zwei 3D-DNA-Nanostrukturen an paarweise unterschiedliche Regionen der jeweiligen Zielstruktur gebunden sind;

b) Nachweis der mindestens zwei Zielstrukturen durch Messen mindestens eines Fluoreszenzsignals, wobei alle 3D-DNA-Nanostrukturen und die Parameter der Fluoreszenzmessung derart ausgewählt sind, dass das mindestens eine gemessene Fluoreszenzsignal der in a) ausgebildeten Identifizierungsstrukturen von dem Fluoreszenzsignal aller isolierter 3D-DNA-Nanostrukturen unterscheidbar ist, wenn diese nicht in einer der jeweiligen Identifizierungsstrukturen gebunden sind, und dass die gemessenen Fluoreszenzsignale der für die einzelnen verschiedenen Zielstrukturen ausgebildeten Identifizierungsstrukturen paarweise voneinander unterscheidbar sind.

Bevorzugt wird dabei jede der verschiedenen Zielstrukturen mehrmals nachgewiesen, d.h. es wird jede der einer bestimmten Zielstruktur zugewiesenen Identifizierungsstrukturen mehrfach ausgebildet.

Insbesondere ist eine Kombination mit den Verfahrensschritten p1) und p2) bevorzugt.

Alle oben genannten Vorteile des erfindungsgemäßen Verfahrens gelten hier *mutatis mutandis.* DNA-Nanostrukturen bieten bei entsprechender Form die Möglichkeit, eine Vielzahl von gleichen und/oder verschiedenartigen Fluoreszenz-Farbstoffmolekülen anzubringen. Wie aus WO2016/140727 A2 und WO2016/140726 A2 bekannt, kann so eine Vielzahl an DNA-Nanostrukturen mit unterscheidbarem Fluoreszenzsignal erzeugt werden. Jedoch hängt die Menge der unterscheidbaren Kombinationen entscheidend von der Größe der DNA-Nanostruktur ab, die die Zahl der Fluoreszenz-Farbstoffmoleküle, die daran angebracht werden können, ohne miteinander wechselzuwirken, begrenzt. Große DNA-Nanostrukturen sind aber aufwendiger und teurer in der Herstellung und zudem in einem Verfahren zum Nachweis einer Zielstruktur kinetisch schlechter als kleinere Strukturen. Das erfindungsgemäße Verfahren bietet auf Grund der Verwendung von mindestens zwei DNA-Nanostrukturen, die Teil der Identifizierungsstruktur werden, den Vorteil, dass auch mit wesentlich kleineren DNA-Nanostrukturen eine ähnliche Vielzahl an Kombinationen von Fluoreszenzmolekülen, die an einer Zielstruktur gebunden sind, erzeugt werden kann. Ferner ermöglicht die Verwendung von mindestens zwei DNA-Nanostrukturen in einer Identifizierungsstruktur, die Anzahl der verschiedenen unterscheidbaren Kombinationen an Fluoreszenz-Farbstoffmolekülen an einer Zielstruktur bzw. in einer Identifizierungsstruktur bei gleicher Maximalgröße wie in WO2016/140727 A2 und WO2016/140726 A2 beschrieben weiter zu vergrößern. Dadurch können im Prinzip noch deutlich mehr verschiedene Zielstrukturen in einem Verfahren nachgewiesen werden. Entscheidend hierfür sind neben der Verwendung von mindestens zwei DNA-Nanostrukturen insbesondere die Form der 3D-Nanostruktur und die Ausrichtung der Fluoreszenz-Farbstoffmoleküle ins Innere der Struktur. Erst dadurch wird, wie oben erwähnt, eine Wechselwirkung der Farbstoffe aus zwei DNA-Nanostrukturen verhindert und das Signal einer Kombination aus zwei oder mehr 3D-DNA-Nanostrukturen verlässlich vorhersehbar und messbar.

Erfindungsgemäß werden Nukleinsäure-Nanostrukturen, auch DNA-Nanostrukturen oder DNA-Origami genannt, verwendet. Nukleinsäure-Nanostrukturen, auch DNA-Nanostrukturen oder DNA-Origami genannt, sind zwei- oder dreidimensionale Strukturen, die unter anderem aus Nukleinsäuren bestehen. Der Ausdruck "Origami" beschreibt dabei, dass ein oder mehrere Stränge oder Bauteile aus Nukleinsäuren, vorzugsweise DNA in eine nahezu beliebige, vordefinierte Struktur oder Form gefaltet werden kann/können. Ein solcher DNA-Strang wird auch als Gerüst-Strang (*scaffold strand*) bezeichnet. Der eine oder die mehreren Gerüst-Stränge werden durch (relativ zu dem mindestens einen Gerüst-Strang) kürzere Nukleinsäure-Stränge, die auch Klammer-Stränge (*staple strand*) genannt werden, in Form gehalten. Von großer Bedeutung ist, dass die kürzeren Stränge (Klammer-Stränge) hierbei sehr präzise an wohldefinierte Postionen auf dem DNA-Origami platziert werden. DNA-Origami werden beispielsweise in Rothemund, "Folding DNA to create nano-scale shapes and patterns", Nature, March 2006, pp. 297-302, vol. 440; Douglas et al., Nature, 459, pp. 414-418 (2009); und Seeman, "Nanomaterials based on DNA", An. Rev. Biochem. 79, pp.65-87 (2010) genauer beschrieben. Auf all diese Schriften wird hier in ihrer Ganzheit als Teil der Anmeldung verwiesen.

Diese DNA-Origami Nanostrukturen können mit photoaktiven Molekülen, im speziellen mit einem oder mehreren fluoreszenten Farbstoff-Molekülen, funktionalisiert werden. Dadurch wird das DNA-Origami in seiner Gesamtheit ein fluoreszierender Partikel. Derartige DNA-Nanostrukturen, die auf DNA Origami basieren, sind in WO 2016/140727 A2 und WO2016/1407726 A2 beschrieben. Auf all diese Schriften wird hier in ihrer Ganzheit als Teil der Anmeldung verwiesen.

Bevorzugt ist eine erfindungsgemäße DNA-Nanostruktur eine 3D-DNA-Nanostruktur, auf der mindestens ein Fluoreszenz-Farbstoffmolekül angebracht ist, wobei die Form der 3D-DNA-Nanostruktur verhindert, dass bei Annäherung an eine zweite gleichartige oder identische 3D-DNA-Nanostruktur, auf der mindestens ein Fluoreszenz-Farbstoffmolekül angebracht ist, die Fluoreszenz-Farbstoffmoleküle der beiden 3D-DNA-Nanostrukturen signifikant wechselwirken. Die Geometrie der DNA-Nanostrukturen verhindert damit durch sterische Hinderung, dass sich Fluoreszenz-Farbstoffmoleküle unterschiedlicher DNA-Nanostrukturen zu nahe kommen.

Eine DNA-Nanostruktur besteht, wie schon am Namen ersichtlich, vorzugsweise aus DNA. Im Prinzip kann eine erfindungsgemäße DNA-Nanostruktur aber auch andere Nukleinsäuren wie z.B RNA, RNA-Analoga wie LNA, oder DNA-Analoga enthalten oder aus diesen bestehen. Folglich umfasst die vorliegende Erfindung auch sämtliche hier beschriebenen Ausführungsformen, in denen die DNA-Nanostruktur(en) bzw. die 3D-DNA-Nanostruktur(en), Nukleinsäure-Nanostrukturen bzw. 3D-Nukleinsäure-Nanostrukturen sind. Eine aus DNA geformte Nanostruktur ist bevorzugt. z.B. auf Grund der deutlich geringeren Herstellungskosten für solche Nanostrukturen.

Ein Fluoreszenz-Farbstoffmolekül kann beispielsweise RFP, GFP, YFP oder deren Derivate (siehe beispielsweise PJ Cranfill et al., "Quantitative assessment of fluorescent proteins", Nature Methods, 13, 557-562 (2016)), ein Atto-Farbstoff (z.B. Atto647N, Atto565 oder Atto488), ein Alexa-Farbstoff, DAPI, Rhodamin, Rhodamin-Derivat, Cyanin, Cyanin-Derivat, Coumarin, Coumarin-Derivat (für verschiedenste organische Fluoreszenz-Farbstoffe siehe beispielsweise Q. Zheng and L Lavis, "Development of photostable fluorophores for molecular imaging", Curr. Op. Chem. Biol. 39 p32-38 (2017)), oder Quantenpunkt (quantum dot, siehe E. Petryayeva et al, "Quantum dots in Bioanalysis: a review of applications across various platforms for fluorescence spectroscopy and imaging", Appl. spectroscopy 67 (2013)) sein.

Unter einer 3D-DNA-Nanostruktur wird bevorzugt eine Struktur verstanden, die sich substantiell in drei Dimensionen erstreckt und sich dadurch von einer im Wesentlichen zweidimensionalen Struktur wie z.B. einer leicht gekrümmten, rauen oder welligen Fläche unterscheidet. Mit anderen Worten betragen die Mindestausmaße in drei zueinander senkrechte Raumrichtungen mindestens 2 nm, bevorzugt mindestens 5 nm, stärker bevorzugt mindestens 10 nm und besonders bevorzugt mindestens 20 nm.

Das Wechselwirken zweier Fluoreszenz-Farbstoffmoleküle kann insbesondere Fluoreszenzauslöschung und/oder FRET umfassen.

Insbesondere können auf der 3D-DNA-Nanostruktur mindestens zwei Fluoreszenz-Farbstoffmoleküle angebracht sein, wobei der Abstand zwischen den mindestens zwei Fluoreszenz-Farbstoffmolekülen paarweise größer als derjenige ist, bei dem die Fluoreszenz-Farbstoffmoleküle paarweise signifikant wechselwirken. Für den Beispielfall einer 3D-DNA-Nanostruktur mit zwei Fluoreszenz-Farbstoffmolekülen heißt das, dass die beiden Fluoreszenz-Farbstoffmoleküle einen Abstand haben, der größer als der Wechselwirkungsabstand ist. Für den Beispielfall einer 3D-DNA-Nanostruktur mit drei Fluoreszenz-Farbstoffmolekülen heißt das, dass jedes Fluoreszenz-Farbstoffmolekül zu den beiden anderen Fluoreszenz-Farbstoffmolekülen einen Abstand hat, der größer als der jeweilige Wechselwirkungsabstand ist. Für mehr Fluoreszenz-Farbstoffmoleküle gilt das gesagte entsprechend. Das heißt, egal welches Fluoreszenz-Farbstoffmolekül-Paar der 3D-DNA-Nanostruktur betrachtet wird, der Abstand der Fluoreszenz-Farbstoffmoleküle des betrachteten Paares ist immer größer als der Wechselwirkungsradius des betrachteten Paares. "Wechselwirkungsradius" bezeichnet den Abstand, den Fluoreszenz-Farbstoffmoleküle mindestens haben müssen, um vernachlässigbare Wechselwirkungen (wie z.B. Fluoreszensauslöschung oder FRET) zu zeigen. Welches Farbstoffpaar welchen Wechselwirkungsradius aufweist, ist dem Fachmann bekannt. Beispielsweise werden entsprechende Wechselwirkungsradien in Novotny, Lukas: Principles of nano-optics 2.ed, Cambridge Univ. Press, 2013, Kapitel "Optical Interactions", pp 224ff; Peter Atkins: Physikalische Chemie, 5. Auflage, 2013, Wiley-VCH, Weinheim, Kapitel 17 "Wechselwirkungen zwischen Molekülen", pp 657ff; Förster T: Zwischenmolekulare Energiewanderung und Fluoreszenz. In: Ann. Physik. 437, 1948, S. 55. doi: 10.1002/andp. 19484370105 beschrieben. Bezogen auf FRET kann ein Wechselwirkungsradius z.B. der Abstand, bei dem eine FRET-Rate von 50% vorliegt, sein.

Die DNA-Nanostrukturen dienen bevorzugt dem Zweck der präzisen Anordnung einer wohldefinierten Anzahl an Markermolekülen (bevorzugt Fluoreszenz-Farbstoffmolekülen) einer oder mehrerer Arten. Die geometrische Anordnung ist von zentraler Wichtigkeit, um Wechselwirkungen zwischen Markermolekülen (bevorzugt Fluoreszenz-Farbstoffmolekülen) zu verhindern. Die definierte Anzahl an Markermolekülen (bevorzugt Fluoreszenz-Farbstoffmolekülen) ermöglicht die Programmierung der Intensitätswerte und damit die spätere eindeutige Identifikation. Die Anzahl verschiedener Kombinationen ist N = k^m, wobei k die Anzahl an Intensitätslevels, und m die Anzahl verschiedenartiger (hier orthogonal messbarer) Markermoleküle (bevorzugt Fluoreszenz-Farbstoffmoleküle) sei. Durch dieses Multiplexing kann also eine große Anzahl unterschiedlicher DNA-Nanostrukturen unterschieden werden, ohne Multiplexing wäre die Anzahl nur m. In einem Anwendungsbeispiel haben wir 5 Intensitätsstufen und drei Markermolekültypen, und somit 124 statt 3 simultan unterscheidbare Spezies. Die DNA-Nanostrukturen sind daher Nanoreporter.

Die Bezeichnung "Nanoreporter" verwenden wir hier somit um mit Markermolekülen besetzte Strukturen, die messbare und identifizierende Signaturen haben, mit Abmessungen im Nanometerbereich zu bezeichnen. Insbesondere können die erfindungsgemäßen DNA-Nanostrukturen Nanoreporter sein. Auch andere DNA-Origami, z.B. nach Rothemund können Nanoreporter sein, ebenso wie Kaskaden an fluoreszierenden Proteinen.

Mit dem Ausdruck "orthogonal messbar" beschreiben wir die Eigenschaft, dass von einer Linearkombination an Messwerten von Markermolekülen (z.B. Fluoreszenz-Farbstoffmoleküle) eindeutig auf eine Linearkombination der zugrundeliegenden Markermoleküle, bzw. auf eine Kombination von Nanostrukturen geschlossen werden kann. Im Beispiel von Fluoreszenzdetektion kann das durch spektral weit auseinanderliegende Farbstoffe und somit weit separierter Anregungs- und Detektionswellenlänge (z.B. blau und rot) realisiert sein, oder aber auch durch näher aneinanderliegende Farbstoffe in Kombination mit Multispektraler Detektion, sodass durch "Linear Unmixing" die orthogonalen Komponenten errechnet werden können.

Eine erfindungsgemäße DNA-Nanostruktur ist bevorzugt dadurch gekennzeichnet, dass auf ihr eines oder mehrere Markermoleküle (z.B. Fluoreszenz-Farbstoffmoleküle) von einer oder mehreren Arten von Markermolekülen so angebracht sind, dass der Abstand zwischen zwei Markermolekülen größer als derjenige ist, bei dem sie signifikant wechselwirken, und deren Form verhindert, dass bei Annäherung an eine gleichartige DNA-Nanostruktur ihre Markermoleküle mit jenen der anderen DNA-Nanostruktur signifikant wechselwirken. Bevorzugt ist eine erfindungsgemäße DNA-Nanostruktur ist eine DNA-Nanostruktur, auf der eines oder mehrere Markermoleküle von einer oder mehreren Arten von Markermolekülen so angebracht ist/sind, dass der Abstand zwischen identischen Markermolekülen größer als derjenige ist, bei dem Fluoreszenzauslöschung geschieht, und der Abstand zwischen unterschiedlichen Markermolekülen größer als der Förster-Resonante-Energietransfer (FRET)-Radius ist, und deren Form verhindert, dass bei Annäherung an eine gleichartige DNA-Nanostruktur ihr/e Markermolekül/e mit einem oder mehreren Markermolekülen der anderen DNA-Nanostruktur mittels Fluoreszenzauslöschung oder FRET wechselwirken.

"Markermoleküle" bezeichnen Teilchen, die messbare Signale aussenden. Insbesondere sind Fluoreszenz-Farbstoffmoleküle Markermoleküle. Das Signal von Fluoreszenz-Farbstoffmolekülen kann die Intensität sein, aber auch Lebenszeit, Polarisation, Blink-Kinetik oder ähnliche Größen. Auch Goldpartikel sind in diesem Sinne Markermoleküle.

Zwei Markermoleküle, insbesondere zwei Fluoreszenz-Farbstoffmoleküle, wechselwirken signifikant, wenn das von mindestens einem von ihnen ausgesandte Signal deutlich von der An- oder Abwesenheit des anderen abhängt. Für unsere Zwecke kann man beispielsweise einen Intensitätsabfall des Signals um 1% durch die Anwesenheit eines anderen Moleküls vernachlässigen, während ein Abfall um 20% nicht vernachlässigbar ist. Im ersten Fall wechselwirken die Markermoleküle (insbesondere Fluoreszenz-Farbstoffmoleküle) also nicht signifikant, im zweiten sehr wohl. Im Falle von gleichartigen Fluoreszenz-Farbstoffmolekülen ist der für uns dominante Wechselwirkungsmechanismus die Fluoreszenzauslöschung, die je nach Fluoreszenz-Farbstoffmolekül unterschiedlich, aber ab einem Abstand von etwa 2 - 3 Nanometer zwischen den Fluoreszenz-Farbstoffmolekülen vernachlässigbar wird. Für verschiedenartige Fluoreszenz-Farbstoffmoleküle ist der dominante Wechselwirkungsmechanismus Förster Resonanter Energietransfer (FRET), und der Abstand, ab dem die Fluoreszenzfarbstoffmolekülpaare nicht mehr signifikant wechselwirken (auch als FRET-Abstand bezeichnet), liegt je nach Paar unterschiedlich im Bereich von 2 - 20 Nanometer. FRET-Abstände sind für eine Vielzahl von Fluoreszenz-Farbstoff-Paaren bekannt, beispielsweise veröffentlichen viele Farbstoff-Hersteller Listen für FRET-Abstände ihrer Farbstoffe (z.B. http://www.attotec.com/fileadmin/user_upload/Katalog_Flyer_Support/R_0_-Tabelle_2016_web.pdf). Überdies können FRET-Abstände über die spektralen Eigenschaften der Farbstoffmoleküle berechnet werden (s. Atkins, Physikalische Chemie).

Bevorzugt wird unter einer signifikanten Wechselwirkung zwischen zwei Flureszenz-Farbstoffmolekülen eine Wechselwirkung verstanden, bei der das gemessene Fluoreszenzsignal (unter üblicher Anregung) des einen Moleküls durch die Anwesenheit des jeweils anderen Moleküls gegenüber einem gemessenen Fluoreszenzsignals des einen Moleküls in Abwesenheit des anderen Moleküle auf maximal 80 %, bevorzugt maximal 90 %, besonders bevorzugt maximal 95 %, ganz besonders bevorzugt maximal 99 % reduziert wird.

Eine erfindungsgemäße 3D-DNA-Nanostruktur weist bevorzugt einen Hohlraum auf, wobei der Hohlraum ein Volumen von mindestens 0,1 Zeptoliter (1e-22 Liter), bevorzugt mindestens 10 Zeptoliter und besonders bevorzugt mindestens 100 Zeptoliter hat.

Eine erfindungsgemäße 3D-DNA-Nanostruktur kann im Wesentlichen als hohle zylinderförmige DNA-Nanostruktur, d.h. als Hohlzylinder, ausgebildet sein, wobei mindestens ein Fluoreszenz-Farbstoffmolekül im Inneren der hohlzylinderförmigen DNA-Nanostruktur angebracht ist. Der Hohlraum des Hohlzylinders hat vorzugsweise ein oben angegebenes Volumen.

Der Teil der 3D-DNA-Nanostruktur, der dem Mantel des Hohlzylinders entspricht, kann hierbei Lücken aufweisen. Die Teile der 3D-DNA-Nanostruktur, die den Deckflächen des Hohlzylinders entsprechen, sind bevorzugt offen, sie können jedoch auch jeweils mindestens teilweise geschlossen und/oder vollständig geschlossen sein. Der Mantel und ggf. die Deckflächen des Hohlzylinders sind durch die 3D-DNA-Nanostruktur gebildet, das heißt, es handelt sich nicht um einen mathematisch präzisen Zylinder, da z.B. die einzelnen Helices der Nanostruktur unebene Oberflächen mit Ausbuchtungen und Vertiefungen bilden. Die Einhüllende der 3D-Nanostruktur hat jedoch im Wesentlichen die Form eines Zylinders.

Bevorzugt sind in einer erfindungsgemäßen 3D-DNA-Nanostruktur mindestens 2, stärker bevorzugt mindestens 3, stärker bevorzugt mindestens 4, stärker bevorzugt mindestens 5, stärker bevorzugt mindestens 6, stärker bevorzugt mindestens 15, besonders bevorzugt mindestens 30, besonders bevorzugt mindestens 60 Fluoreszenz-Farbstoffmoleküle angebracht. Bevorzugt sind die Fluoreszenz-Farbstoffmoleküle auf einer 3D-DNA-Nanostruktur gleichartig, das heißt vom gleichen Typ.

Bevorzugt weist eine erfindungsgemäße 3D-DNA-Nanostruktur einen Hohlraum und mindestens ein innenliegendes Fluoreszenz-Farbstoffmolekül auf, wobei der Abstand des mindestens einen innenliegenden Fluoreszenz-Farbstoffmoleküls zum Rand bzw. Außenoberfläche der Einhüllenden der 3D-DNA-Nanostruktur mindestens 2 nm, bevorzugt mindestens 3 nm und besonders bevorzugt mindestens 5 nm beträgt. Dies bietet den Vorteil, dass die Farbstoffe der 3D-DNA-Nanostruktur aufgrund der Anordnung im Inneren der 3D-DNA-Nanostruktur und des Mindestabstands zum Rand der 3D-DNA-Nanostruktur auch diesen Mindestabstand zu anderen Strukturen, mit denen die 3D-DNA-Nanostruktur während eines Verfahrens in Wechselwirkung treten können, aufweisen. Beispielsweise haben die Farbstoffe auch zu einer Oberfläche eines Trägers, beispielsweise eines Substrats, einen Abstand größer oder gleich dem Mindestabstand, wenn die 3D-DNA-Nanostruktur in Kontakt mit dieser Oberfläche kommt.

Bevorzugt weist eine erfindungsgemäße 3D-DNA-Nanostruktur mindestens zwei innenliegende Fluoreszenz-Farbstoffmoleküle auf, wobei der paarweise Abstand der mindestens zwei innenliegenden Fluoreszenz-Farbstoffmoleküle mindestens 2 nm, bevorzugt mindestens 5 nm und besonders bevorzugt mindestens 9 nm beträgt. Für den Beispielfall einer 3D-DNA-Nanostruktur mit zwei Farbstoff-Molekülen heißt das, dass die beiden Farbstoffmoleküle einen Abstand von mindestens 2 nm, bevorzugt mindestens 5 nm und besonders bevorzugt mindestens 9 nm aufweisen. Für den Beispielfall einer 3D-DNA-Nanostruktur mit drei Farbstoffmolekülen heißt das, dass jeder Farbstoff zu den beiden anderen Farbstoffen jeweils einen Abstand von mindestens 2 nm, bevorzugt mindestens 5 nm und besonders bevorzugt mindestens 9 nm aufweist. Für mehr Farbstoffe gilt das gesagte entsprechend. Das heißt, egal welches Farbstoff-Paar der 3D-DNA-Nanostruktur betrachtet wird, der Abstand der Farbstoffe des betrachteten Paares beträgt immer mindestens 2 nm, bevorzugt mindestens 5 nm und besonders bevorzugt mindestens 9 nm.

Der Abstand der Farbstoffmoleküle kann beispielsweise dadurch bestimmt werden, dass die Struktur der 3D-DNA-Nanostruktur identifiziert wird, was Rückschlüsse darauf zulässt, an welchen Positionen die Farbstoffmoleküle sitzen. Hierfür kann beispielsweise eine Sequenzanalyse erforderlich sein. Dazu wird eine Lösung mit DNA-Nanostrukturen beispielsweise durch Reduktion der Salzkonzentration in eine Lösung mit einzelnen Nukleinsäuresträngen überführt. Bei Reduktion der Salzkonzentration werden die negativen Ladungen des DNA-Rückgrats aufgrund geringerer Abschirmung dominanter im Vergleich zu den konstant bleibenden Bindeenergien der Basenpaar-bildenden Wasserstoffbrückenbindungen. So werden mit Reduktion der Salzkonzentration zunächst die DNA-Nanostrukuren destabilisiert und bei weiterer Reduktion brechen die einzelnen DNA-Doppelhelices auf. und beispielsweise mit MySeq oder einer anderen von Illumina, Inc. zur Verfügung gestellten Methode und dem vom Hersteller angegebenen Protokoll sequenziert. Damit werden die Sequenzen aller in der Probe vorhandenen Nukleinsäurestränge bekannt. Mit dieser Sequenzinformation kann die Form der DNA-Nanostruktur rekonstruiert werden. Hierzu kann jeder Sequenzeditor (z.B. ApE 2.0 (http://biologylabs.utah.edu/jorgensen/wayned/ape/), zur Hilfe genommen werden. Der längste DNA-Strang der Analyse ist der Gerüst-Strang. Er wird in den Editor geladen und für jede einzelne Klammersequenz werden die Regionen mit dem Gerüststrang komplementärer Sequenz berechnet und notiert. Dies sind je Klammerstrang zwei unterschiedliche Regionen am Gerüststrang. Mit dieser Information kann wiederum wie an anderer Stelle in dieser Schrift beschrieben die Topologie in CaDNAno definiert und somit das DNA-Nanostrukturdesign rekonstruiert werden. Im nächsten Schritt muss eruiert werden welche der Klammerstränge farbstoffbesetzt sind. Im Fall der Nutzung von Farbstoff-Adaptern ist dies einfach, da dann eine bestimmte Anzahl von Klammersträngen weitere identische Sequenzen haben, die nach der obigen Rekonstruktion der DNA-Nanostruktur einzelsträngig bleiben. Im Fall von direkter Modifikation der Klammerstränge müssen die einzelnen Klammerstränge isoliert werden und auf ihre Fluoreszenzeigenschaften untersucht werden. Jeder einzelne Klammerstrang kann dann durch Hybridisierung an einen Komplementärstrang mit zusätzlichem Molekulargewicht beladen werden in einer Agarose-Gelelektrophorese aus obiger Lösung mit einzelnen Nukleinsäursträngen isoliert werden. Eine Fluoreszenzmessung zeigt schließlich jeweils welcher Klammerstrang mit Fluorophor besetzt ist. Diese können in caDNAno identifiziert und die Abstände zwischen Fluoreszenzmolekülen berechnet werden. Alternativ zu dieser Methode kann mittels Massenspektrometrie (Sauer S, Lechner D, Berlin K et al. (2000) Full flexibility genotyping of single nucleotide polymorphisms by the GOOD assay. Nucleic Acids Res 28:E100; Haff LA, Smirnov IP (1997) Single-nucleotide polymor- phism identification assays using a thermostable DNA poly- merase and delayed extraction MALDI-TOF mass spectrome- try. Genome Res 7:378-388; Wenzel T, Elssner T, Fahr K et al. (2003) Genosnip: SNP genotyping by MALDI-TOF MS using photocleavable oligonucleotides. Nucleosides Nucleotides Nucleic Acids 22:1579- 1581; Braun A, Little DP, Koster H (1997) Detecting CFTR gene mutations by using primer oligo base extension and mass spectrometry. Clin Chem 43:1151-1158; Sun X, Ding H, Hung K et al. (2000) A new MALDI-TOF based minisequencing assay for genotyping of SNPS. Nucleic Acids Res 28:E68) die Lösung mit DNA-Nanostrukturen direkt sequenziert werden und simultan erkannt werden, welche Klammerstränge mit Fluoreszenzmolekülen besetzt sind. Daraufhin kann wieder analog zu oben mittels Komplementärsequenzanalyse von Klammersträngen und Gerüststrang (der wieder der längste Strang ist) die Topologie der DNA-Struktur in CaDNAno rekonstruiert werden. Mit der mitgemessenen Information, an welchen Strängen die Fluoreszenzmoleküle angebracht sind, kann direkt dazu verwendet werden mithilfe von caDNAno die Abstände der Fluoreszenzmoleküle zu berechnen.

In einer bevorzugten Ausführungsform ist die 3D-DNA-Nanostruktur im Wesentlichen als elliptischer Zylinder, bevorzugt im Wesentlichen als Kreiszylinder ausgebildet.

Wie bereits oben ausgeführt, handelt es sich bei den erfindungsgemäßen Strukturen nicht um einen mathematisch präzisen Zylinder, da die Oberfläche der Struktur auf dem Level der Einzelatome extrem uneben ist und z.B. auf dem Level der einzelnen Helices der Nanostruktur Unebenheiten in der Größenordnung eines Helixradius oder -durchmesser aufweisen kann. Für den Fachmann sind diese Strukturen dennoch zylindrisch, da z.B. die Einhüllende der 3D-Nanostruktur im Wesentlichen die Form eines Zylinders haben kann.

In einer bevorzugten Ausführungsform ist der Hohlzylinder der 3D-DNA-Nanostruktur ein Kreiszylinder und weist einen Außenradius von mindestens 5 nm, mindestens 10 nm, bevorzugt von mindestens 20 nm, bevorzugt von mindestens 20 nm, bevorzugt einen Außenradius von 30 nm bis 80 nm" bevorzugt von 50 nm bis 70 nm und besonders bevorzugt einen Außenradius von 60 nm auf. Dabei wird unter dem Außenradius der Struktur der Außenradius der Einhüllenden verstanden.

In einer bevorzugten Ausführungsform weist der Hohlzylinder der 3D-DNA-Nanostruktur, der bevorzugt ein Kreiszylinder ist, eine Höhe von maximal 200 nm, bevorzugt eine Höhe von 60 nm - 30 nm, besonders bevorzugt eine Höhe von 30 nm auf. Dabei wird unter der Höhe der Struktur die Höhe der Einhüllenden verstanden.

Die bevorzugten Ausführungsformen gewährleisten eine hinreichende Größe der 3D-DNA-Nanostruktur für das Anbringen einer ausreichenden Zahl von Farbstoff-Molekülen bei ausreichendem Abstand der Farbstoff-Moleküle untereinander und bevorzugt im Inneren der 3D-DNA-Nanostruktur.

In einer bevorzugten Ausführungsform weist der Hohlzylinder der 3D-DNA-Nanostruktur eine Wandstärke von mindestens 2 nm, bevorzugt 2 nm bis 7 nm, besonders bevorzugt 5 nm auf. Dabei wird unter der Wandstärke der Struktur die Differenz zwischen Außenradius und Innenradius verstanden. Mit anderen Worten entspricht die Wandstärke dem Abstand zwischen der äußeren Einhüllenden des Zylinders und der inneren Einhüllenden des Zylinderhohlraums.

Bevorzugt weist eine erfindungsgemäße 3D-DNA-Nanostruktur eine Wandstärke auf, die mindestens dem Durchmesser einer DNA-Doppelhelix entspricht. Bevorzugt bietet die Wandstärke 2-4, besonders bevorzugt 3 DNA-Doppelsträngen Platz, wobei die DNA-Stränge bevorzugt mit ihrer Länge senkrecht zur Wandstärke angeordnet sind und zusätzlich bevorzugt auf benachbarten Gitterplätzen eines Honigwabengitters angeordnet sind.

Der Innendurchmesser des Hohlzylinders ist als die Differenz aus Außendurchmesser des Hohlzylinders und der Wandstärke definiert. Bevorzugte Innendurchmesser ergeben sich daher aus den hierin gemachten Angaben zum Außendurchmesser und zur Wandstärke.

Die beschriebenen Mindestwandstärken in Kombination mit der Form der 3D-DNA-Nanostruktur, die die Farbstoffe gegen die Umgebung abschirmt, trägt ebenfalls dazu bei, dass eine Wechselwirkung der Farbstoffe der 3D-DNA-Nanostruktur mit einer Oberfläche eines Trägers und/oder einem anderen Farbstoff, der in einer anderen 3D-DNA-Nanostruktur angebracht sein kann, durch sterische Hinderung verhindert wird. Insbesondere wird so Quenchen und/oder FRET mit einem anderen Farbstoff und/oder unspezifisches Binden an einen Träger, insbesondere eine Substratoberfläche verhindert.

Die erfindungsgemäßen 3D-Nanostrukturen weisen, wie für DNA-Origami üblich, bevorzugt einen DNA-Einzelstrang als Gerüst-Strang (englisch "scaffold-strand") auf. Der Gerüst-Strang weist bevorzugt mindestens 3000 Basen, bevorzugt 5000 - 50000 Basen, besonders bevorzugt 10000 - 11000 Basen auf. Eine andere Anzahl von Basen ist jedoch möglich. Der Gerüst-Strang ist bevorzugt zirkulär. Zirkulär bedeutet hierbei, dass der DNA-Strang kein offenes 5'-Ende und kein offenes 3'-Ende aufweist. Ein nicht-limitierendes Beispiel für einen Gerüststrang wird in den angehängten Beispielen genannt und ist in SEQ ID NO 1258 gezeigt.

Ferner weisen die 3D-Nanostrukturen, wie für DNA-Origami üblich, bevorzugt eine Vielzahl von weiteren kürzeren einzelsträngigen DNA-Molekülen auf. Diese werden bevorzugt als Klammerstränge (englisch "staple strands") bezeichnet. Die Anzahl der Klammerstränge der 3D-DNA-Nanostruktur wird erfindungsgemäß bevorzugt so ausgewählt, dass sie an die Länge des mindestens einen Gerüst-Strangs der 3D-DNA-Nanostruktur angepasst ist. Bevorzugt weist die 3D-DNA-Nanostruktur 100 - 500 Klammerstränge auf. Ein Klammerstrang weist bevorzugt 30 - 100 Basen auf. Nicht limitierende Beispiele für Klammerstränge sind in den angehängten Beispielen genannt und/oder in SEQ ID NOs. 1 bis 504 gezeigt

Die 3D-DNA-Nanostrukturen können eine Zylinderform haben, bei der die Markermoleküle innenliegend mit einem Abstand zum Rand von mindestens dem halben Wechselwirkungsradius angebracht sind. Dies garantiert dass die Markermoleküle unterschiedlicher DNA-Strukturen nicht näher als der Wechselwirkungsradius aneinander liegen, unabhängig von der Lage der DNA-Strukturen relativ zu einander. Hierbei bietet ein Zylinder mit etwa quadratischer Seitenansicht ein gutes Verhältnis aus sterischer Hinderung (und damit Abschirmung), kleinem hydrodynamischem Radius (und damit Diffusionsgeschwindigkeit) und innenliegender Markermolekül-Positionen mit ausreichendem Abstand zwischen Markermolekülen. Insbesondere kommt hier eine DNA-Nanostruktur-Hohlzylinder mit einem Durchmesser von etwa 30-90 Nanometern und einer Höhe von etwa 30-90 Nanometern in Betracht.

Alternativ können die DNA-Strukturen eine Körbchenform haben, mit Farbstoffen auf dem "Boden" des Körbchens und einem Körbchenrand, der mindestens so groß wie der größte Wechselwirkungsradius aller Kombinationen von Markermolekültypen ist. Der Körbchenrand kann in einen beliebigen Winkel zum Körbchenboden aufweisen, bevorzugt liegt der Winkel jedoch in einem Bereich von -45° bis +45°. Bevorzugt hat der Körbchenrand eine Höhe von maximal 200 nm, bevorzugt eine Höhe von 60 nm bis 30 nm, besonders bevorzugt eine Höhe von 30 nm auf. Dabei wird wieder unter der Höhe der Struktur die Höhe der Einhüllenden verstanden.

Die offene Seite des Körbchens weist bevorzugt einen Außenradius von mindestens 5 nm, bevorzugt einen Innenradius von 30 nm bis 60 nm, besonders bevorzugt einen Innenradius von 60 nm auf. Ein Hohlzylinder mit einer offenen und einer geschlossenen Deckfläche ist ein Spezialfall eines Körbchens.

Eine erfindungsgemäße 3D-DNA-Nanostruktur kann eine im Wesentlichen quadratische Projektion haben, d.h. ein Verhältnis aus Höhe zu Breite bzw. Durchmesser ist im Bereich von 0,8 bis 1,2. Insbesondere kann eine als Hohlzylinder und/oder Körbchen gestaltete 3D-DNA-Nanostruktur eine im Wesentlichen quadratische Projektion haben.

Eine erfindungsgemäßen 3D-DNA-Nanostruktur kann die Form einer Hohlkugel aufweisen und bevorzugt mit innenliegenden Markermolekülen versehen sein.

Erfindungsgemäße 3D-DNA-Nanostruktur sind auch Wireframe-Geometrien, wie Tetraeder, Oktaeder, Würfel, Hexaeder, Pyramiden, usw.

Das Grundprinzip der Herstellung von DNA-Origami und damit DNA-Nanostrukturen in verschiedenen Formen ist wohlbekannt, bspw. aus Rothemund, "Folding DNA to create nano-scale shapes and patterns", Nature, March 2006, pp. 297-302, vol. 440; Douglas et al., Nature, 459, pp. 414-418 (2009); und Seeman, "Nanomaterials based on DNA", An. Rev. Biochem. 79, pp.65-87 (2010). Wie bereits erwähnt, beruht das Herstellungsprinzip für DNA-Origami darauf, mindestens einen Gerüst-Strang, der vorzugsweise ein Einzelstrang ist, und eine Vielzahl von Klammer-Strängen gemeinsam zu inkubieren. Die Klammer-Stränge weisen wenigstens zwei Bindungs-Abschnitte auf, die dazu dienen, jeweils an komplementäre Abschnitte des Gerüst-Strangs zu binden. Während der Inkubation, die vorzugsweise bei einer Temperatur von 50-70°C beginnt und daraufhin reduziert wird, binden die Klammer-Stränge und der Gerüst-Strang mittels ihrer jeweiligen komplementären Bindungs-Abschnitte. Dies führt dazu, dass sich die so erzeugte DNA-Nanostruktur in eine Konformation faltet. Durch gezieltes Design des Gerüst-Strangs und der Klammer-Stränge sowie deren komplementäre Bindungs-Abschnitte kann ein DNA-Origami nach dem Bedarf des Anwenders designed und hergestellt werden. Zum Designen der DNA-Origami steht frei verfügbare Software zur Verfügung. Beispielsweise kann das Programm CaDNAno 2.5 (Source-Code verfügbar unter: https://github.com/cadnano; Nutzeranleitungen verfügbar: http://cadnano.org/docs.html bzw. http://cando-dna-origami.org/tutorial/ (siehe auch S.M. Douglas et al, "Rapid prototyping of 3D DNA-origami shapes with caDNAno, Nucleic Acids Res., 37(15), 2009) verwendet werden.

In diesem Programm gibt man eine beliebige Sequenz eines Gerüststranges vor, der vorzugsweise zur Herstellung der DNA-Nanostrukturen zur Verfügung steht; besonders bevorzugt die Gerüststränge p7308 (SEQ ID NO 1258) oder p7249 (SEQ ID NO 1257). Zusätzlich spezifiziert man die gewünschte Topologie der DNA-Nanostruktur. Im Speziellen berechnet CaDNAno die Anzahl und Sequenzen der benötigten Gerüststränge und Klammerstränge bei Vorgabe von (1) Länge und (2) Sequenz des Gerüststranges, (3) räumlichem Verlauf des Gerüststranges und (4) Startposition des Gerüststranges. Der räumliche Verlauf des Gerüststranges definiert die Form der geplanten Struktur und beinhaltet die Anzahl und Anordnung der Helices und den Verlauf des Gerüststranges durch diese Helices. Das Programm ist auf Knopfdruck in der Lage den vorgegebenen Gerüststrang selbstständig mit Klammersträngen zu verbinden. Anschließend lassen sich die Klammerstränge in Tabellenform ausgeben. Die Tabelle enthält vor allem Start- und Endposition des Klammerstranges (definiert durch Helix und Anzahl der Basen vom Rand der Helix, z.B. 7[128] bezeichnet eine Position auf der 7ten Helix, 128 Basen vom willkürlich aber einheitlich definierten linken Rand) zur eindeutigen Identifikation, die Anzahl der Basen (i.e. der Länge des Klammerstranges) und die Sequenz des Klammerstranges. Erwähnenswert ist, dass bei identischem Strukturdesign (Punkt (1)-(3)) aber unterschiedlicher Startposition des zirkulären Gerüststranges, andere Klammerstrang-Sequenzen generiert werden. Diese sind ohne Einschränkung genauso nutzbar wie die mit anderer Startpostition, aber nicht mit diesen kompatibel.

Für zuweilen in 3D-Strukturen nützliche Biegungen einer DNA-Helix können Positionen entlang des Gerüststranges definiert werden an denen der entsprechende Klammerstrang eine Base mehr oder weniger besitzt. Auch können zusätzliche Klammerstrang-Klammerstrang Paarungen definiert werden, die der Struktur über die vorgegebene Gerüststranglänge hinaus weitere DNA-Helix-Kontourlänge hinzufügen. Zu beachten ist, dass CaDNAno lediglich Repräsentationen in einer Ebene anzeigt, und 3D-Repräsentationen durch das räumliche Vorstellungsvermögen des Nutzers oder ein Plugin von CaDNAno für das Design-Programm Autodesk Maya (D Selnihin und ES Andersen, "Computer-aided design of DNA origami structures", Comp. Meth. Synth. Biol. 1244, pp 23-44 (2014)) gefertigt werden müssen. CaDNAno gibt schlussendlich eine Liste von Klammersträngen aus, mit denen die gewünschte Struktur hergestellt werden kann.

Jeder dieser Klammerstränge kann prinzipiell mit einem Fluorophor/Fluoreszenz-Farbstoffmolekül besetzt werden. Das Programm sieht aber keine Funktion zur Definition von Fluoreszenz-Farbstoffmolekül Positionen zur direkten Markierung in der exportierten Klammerstrang-Liste vor. Jedoch hilft das Programm bei der Visualisierung dieses Prozesses, da es die Start- und Endpositionen der Stränge, sowie den Verlauf des Gerüststranges und die Anordnung der Helices anzeigt. Damit kann der Nutzer relative Abstände von infrage kommenden Positionen an den Klammersträngen innerhalb der designten 3D-DNA-Nanostruktur einfach errechnen. Konkret und bevorzugt wählt der Nutzer Positionen an Klammersträngen aus, die modifiziert werden sollen. Dies seien beispielsweise die 5'-Enden oder die 3'-Enden. Dann sucht der Nutzer die Klammerstränge aus, die den Positionierungskriterien der Fluoreszenz-Farbstoffmolekül genügen, beispielsweise dass sie sich im inneren der Hohlstruktur befinden und den gewünschten, ausreichenden Abstand zum Rand der Struktur haben. Dies ist wie oben beschrieben in CaDNAno ersichtlich. Schließlich berechnet der Nutzer die Minimalabstände der zu modifizierenden Positionen an den noch zur Auswahl stehenden Klammersträngen, was sich aufgrund der Gitterstruktur des Designs und der Visualisierung in CaDNAno auf 2-3 Berechnungen beschränkt und einfach zu bewerkstelligen ist. Schließlich wählt der Nutzer je nach zu realisierender Anzahl von Fluoreszenz-Farbstoffmolekülen ein beliebiges Subset der zur Auswahl stehenden Klammerstränge aus, notiert die Klammerstrang-IDs und bestellt die entsprechenden Sequenzen in der exportierten Klammerstrang-Liste mit Fluoreszenz-Farbstoffmolekül-Modifikation an gewählter Position. Für eine auf Fluoreszenz-Farbstoffmolekül-Adapter basierende Besetzung der DNA-Nanostrukturen mit Fluoreszenz-Farbstoffmolekülen ist die Strategie analog, nur dass die exportierten Klammerstrangsequenzen nicht mit Fluoreszenz-Farbstoffmolekül-Modifikation sondern mit um die zu dem Fluoreszenz-Farbstoffmolekül-Adapter(n) komplementäre Adaptersequenz verlängerte Sequenz verwendet werden. Außerdem ist die gewählte Position entlang der Klammerstränge dann bevorzugt ein Ende der Klammerstränge, das besonders bevorzugt aus der Struktur ins Innere ragt (bzw. in den Hohlraum hineinragt). Bei dieser Adapter-basierten Fluoreszenz-Farbstoffmolekülbesetzung der DNA-Strukturen binden viele Fluoreszenz-Farbstoffmolekül-besetzte Adapterstränge gleicher Art (und SEQ ID NO, beispielsweise 1259-1261 für die verschiedenen Fluoreszenz-Farbstoffmolekülfarben) an viele verschiedene Klammerstränge, die mit jeweils der selben Komplementärsequenz für die Adapter verlängert sind, an der DNA-Nanostruktur. Bei der Verwendung von Fluoreszenz-Farbstoffmolekül -Adaptern ist das Fluoreszenz-Farbstoffmolekül bevorzugt an der zum Rand bzw. zur Wand der Struktur gerichteten Seite des Fluoreszenz-Farbstoffmolekül-Adapters gebunden, Dies dient dazu ihm eine geringere Bewegungsfreiheit zu gewähren. Zum Beispiel ist dies das 3'-Ende des Fluoreszenz-Farbstoffmolekül-Adapters, wenn das 5'-Ende der durch CaDNAno definierten Klammerstränge um die komplementäre Adaptersequenz verlängert wird. Der umgekehrte Fall ist aber auch denkbar.

Nach gleichem Muster wie für die Klammerstränge für Fluorophor-Adapter wird pro 3D-DNA-Nanostruktur ein Klammerstrang für Ziel-Adapter designt, wobei die Auswahl-Kriterien für die Klammerstrang-Position andere sein können, wie zum Beispiel Ausgesetztheit für gute Bindeeffizienz mit Zielstrukturen.

Die Herstellung von 3D-Nanostrukturen in verschiedenen 3D-Formen, z.B. in einer Hohlzylinderstruktur ist auch aus Knudsen J. et al. (Nature Nanotechnology 10, 892-898 (2015) doi:10.1038/nnano.2015.190) bekannt. Um auf dem dortigen Design basierend erfindungsgemäße 3D-DNA-Nanostrukturen herzustellen, werden bevorzugt alle oder ein Subset der Oligomere, die ausschließlich in die Innenseite des Hohlzylinders zeigenden Helices, die sich nicht am einer der Kanten (Rand) des Hohlzylinders befinden, binden, an einem Ende mit einem Farbstoff besetzt bzw. durch Adapter-Sequenzen (an die ein Fluoreszenz-Adapter, der ein Fluoreszenz-Farbstoffmolekül aufweist gebunden werden kann) verlängert. Für den Ziel- oder Träger-Adapter kann ein Oligomer gewählt werden, das in einer Helix integriert ist, die sich am Rand des Hohlzylinders befindet.

Ferner wird in den angehängten Beispielen (insbesondere in Beispiel 1) ein exemplarisches Beispiel für ein Herstellungsverfahren einer erfindungsgemäßen 3D-DNA-Nanostruktur erläutert. Basierend auf den dafür verwendeten Sequenzen, und dem bereitgestellten Verfahren zur Herstellung kann ein Fachmann auch ähnliche 3D-DNA-Nanostrukturen (z.B. mit völlig anderen Sequenzen (die aber z.B. an ähnlichen oder den gleichen Positionen komplementäre DNA Sequenzen aufweisen)) herstellen.

Die vorliegende Erfindung richtet sich in einem weiteren Aspekt auf ein Set aus mehreren 3D-DNA-Nanostrukturen (wobei es sich bevorzugt um 3D-DNA-Nanostrukturen wie in dieser Anmeldung beschrieben handelt), wobei das Set N voneinander verschiedene 3D-DNA-Nanostrukturen aufweist und wobei sich die N voneinander verschiedenen 3D-DNA-Nanostrukturen des Sets durch die Fluoreszenzfarbstoffmoleküle paarweise voneinander unterscheiden. Bevorzugt enthalten die N voneinander verschiedenen 3D-DNA-Nanostrukturen eine unterschiedliche Anzahl von Fluoreszenzfarbstoffmolekülen und/oder unterschiedliche Fluoreszenzfarbstoffmoleküle, so dass mit den N voneinander verschiedenen 3D-DNA-Nanostrukturen des Sets k voneinander unterscheidbarer Intensitätslevel und/oder m voneinander unterscheidbarer Farblevel erzeugt werden können. Bevorzugt ist mindestens ein Teil der N voneinander verschiedenen 3D-DNA-Nanostrukturen mehrmals in dem Set enthalten, so dass jedes der k Intensitätslevel durch eine Intensitätsverteilung gebildet wird und wobei die k Intensitätsverteilungen, bevorzugt statistisch, voneinander unterscheidbar sind. Bevorzugt ist mindestens ein Teil der N voneinander verschiedenen 3D-DNA-Nanostrukturen mehrmals in dem Set enthalten, so dass jedes der m Farblevel durch eine Farbverteilung gebildet wird und wobei die m Farbverteilungen, bevorzugt statistisch, voneinander unterscheidbar sind. Dabei ist der Überlapp benachbarter Verteilungen kleiner als 30 %, bevorzugt kleiner als 20%, bevorzugt kleiner als 10 %, stärker bevorzugt kleiner als 5 %, noch stärker bevorzugt kleiner als 2 % und besonders bevorzugt kleiner als 1 % ist.

Hier gilt bevorzugt: $k>2$, bevorzugt $k>3$, stärker bevorzugt $k>4$, noch stärker bevorzugt $k>5$, besonders bevorzugt $k>6$; und/oder $m>2$, bevorzugt $m>3$, stärker bevorzugt $m>4$, noch stärker bevorzugt $m>5$, besonders bevorzugt $m>6$.

In einem weiteren Aspekt richtet sich die vorliegende Erfindung auch auf ein Verfahren zum Nachweis einer Zielstruktur. Dieses Verfahren umfasst:

a) Ausbilden einer Identifizierungsstruktur, die aufweist:

(i) die Zielstruktur, und

(ii) mindestens zwei 3D-DNA-Nanostrukturen, wobei jede der 3D-DNA-Nanostrukturen ein oder mehrere innenliegende Fluoreszenz-Farbstoffmoleküle aufweist und wobei jede der 3D-DNA-Nanostrukturen (spezifisch) an die Zielstruktur gebunden ist,

b) Nachweis der Zielstruktur durch Messen mindestens eines Fluoreszenzsignals,

wobei die 3D-DNA-Nanostrukturen und die Parameter der Fluoreszenzmessung derart ausgewählt sind, dass das mindestens eine gemessene Fluoreszenzsignal der in a) ausgebildeten Identifizierungsstruktur von dem Fluoreszenzsignal jeder der mindestens zwei isolierten 3D-DNA-Nanostrukturen unterscheidbar ist, wenn diese nicht in der Identifizierungsstruktur gebunden sind.

Bevorzugt binden die mindestens zwei 3D-DNA-Nanostrukturen an paarweise unterschiedliche Regionen/Abschnitte des Zielmoleküls. So kann eine höhere Spezifität für den Nachweis einer Zielstruktur erreicht werden.

Insbesondere ist eine Kombination dieses Verfahrens mit den Verfahrensschritten p1) und p2) bevorzugt.

Eine Zielstruktur, die mit einem erfindungsgemäßen Verfahren nachgewiesen wird, kann ein Molekül, Komplex aus mehreren Molekülen oder ein Partikel sein. Eine Zielstruktur kann insbesondere eine DNA (vorzugsweise eine wenigstens teilweise einzelsträngige DNA), eine RNA (vorzugsweise eine wenigstens teilweise einzelsträngige RNA, z.B. m-RNA), eine LNA (vorzugsweise eine wenigstens teilweise einzelsträngige LNA) oder ein Protein sein. Denkbar ist aber auch eine Zielstruktur, die ein Komplex, der eine oder mehrere DNAs (vorzugsweise mindestens eine wenigstens teilweise einzelsträngige DNA), eine oder mehrere RNAs (vorzugsweise mindestens eine wenigstens teilweise einzelsträngige RNA), eine oder mehrere LNAs (vorzugsweise mindestens eine wenigstens teilweise einzelsträngige LNA) und/oder ein oder mehrere Proteine enthält bzw. daraus besteht. Grundsätzlich kann es sich bei der Zielstruktur aber auch um ein anorganisches Partikel handeln. Bevorzugt umfasst oder ist die Zielstruktur ein Polynukleotid (d.h. z.B.

eine DNA, eine RNA oder LNA), besonders bevorzugt ein mindestens teilweise einzelsträngiges Polynukleotid und besonders bevorzugt ein einzelsträngiges Polynukleotid. Besonders bevorzugt umfasst oder ist die Zielstruktur eine einzelsträngige DNA, eine einzelsträngige RNA oder eine einzelsträngige LNA. In einem bevorzugten Aspekt wird das erfindungsgemäße Verfahren zur Genexpressionsanalyse eingesetzt. In diesem Fall handelt es sich bei der Zielstruktur bevorzugt um eine m-RNA oder ein Protein, besonders bevorzugt um m-RNA. Folglich kann die Zielstruktur ein Protein oder eine m-RNA sein. Unter "teilweise" einzelsträngig ist zu verstehen, dass die PolyNukleinsäure (d.h. z.B. eine DNA, eine RNA oder LNA) einen einzelsträngigen Bereich von mindestens 10 Basen, bevorzugt mindestens 15 Basen und besonders bevorzugt von mindestens 21 Basen aufweist.

Folglich kann in einer bevorzugten Ausführungsform des Verfahrens die Zielstruktur ein Polynukleotid, bevorzugt ein teilweise einzelsträngiges Polynukleotid, bevorzugt ein einzelsträngiges Polynukleotid und besonders bevorzugt eine m-RNA aufweisen oder sein.

Folglich kann das erfindungsgemäße Verfahren in einer bevorzugten Ausführungsform ein Verfahren zur Genexpressionsanalyse sein, wobei eine m-RNA nachgewiesen wird (die m-RNA ist also Zielstruktur) und das Verfahren umfasst

a) Ausbilden einer Identifizierungsstruktur, die aufweist:

(i) eine m-RNA, und
(ii) mindestens zwei 3D-DNA-Nanostrukturen, wobei jede der 3D-DNA-Nanostrukturen ein oder mehrere innenliegende Fluoreszenz-Farbstoffmoleküle aufweist und wobei jede der 3D-DNA-Nanostrukturen (spezifisch) an paarweise unterschiedliche Sequenzabschnitte der m-RNA gebunden ist

b) Nachweis der Zielstruktur durch Messen mindestens eines Fluoreszenzsignals,

wobei die 3D-DNA-Nanostrukturen und die Parameter der Fluoreszenzmessung derart ausgewählt sind, dass das mindestens eine gemessene Fluoreszenzsignal der in a) ausgebildeten Identifizierungsstruktur von dem Fluoreszenzsignal jeder der mindestens zwei isolierten 3D-DNA-Nanostrukturen unterscheidbar ist, wenn diese nicht in der Identifizierungsstruktur gebunden sind.

Insbesondere ist eine Kombination mit den Verfahrensschritten p1) und p2) bevorzugt, sodass eine bevorzugte Ausführungsform ein Verfahren zur Genexpressionsanalyse ist, wobei eine m-RNA nachgewiesen wird (die m-RNA ist also Zielstruktur) und das Verfahren umfasst:

p1) Einbringen einer Gruppe von Wirtskörpern inklusive eines Wirtskörpers mit der m-RNA in ein Mikro-Well Array derart, dass in mindestens einem Mikro-Well genau ein Wirtskörper vorhanden ist;
p2) Einbringen von mindestens zwei 3D-DNA-Nanostrukturen in das mindestens eine Mikro-Well, wobei jede der 3D-DNA-Nanostrukturen ein oder mehrere innenliegende Fluoreszenz-Farbstoffmoleküle aufweist;

a) Ausbilden einer Identifizierungsstruktur, die aufweist:

(i) eine m-RNA, und
(ii) mindestens zwei 3D-DNA-Nanostrukturen, wobei jede der 3D-DNA-Nanostrukturen ein oder mehrere innenliegende Fluoreszenz-Farbstoffmoleküle aufweist und wobei jede der 3D-DNA-Nanostrukturen (spezifisch) an paarweise unterschiedliche Sequenzabschnitte der m-RNA gebunden ist

b) Nachweis der m-RNA durch Messen mindestens eines Fluoreszenzsignals,

wobei die 3D-DNA-Nanostrukturen und die Parameter der Fluoreszenzmessung derart ausgewählt sind, dass das mindestens eine gemessene Fluoreszenzsignal der in a) ausgebildeten Identifizierungsstruktur von dem Fluoreszenzsignal jeder der mindestens zwei isolierten 3D-DNA-Nanostrukturen unterscheidbar ist, wenn diese nicht in der Identifizierungsstruktur gebunden sind.

Eine "3D-DNA-Nanostruktur" meint im Zusammenhang mit dem erfindungsgemäßen Verfahren das Gleiche wie oben im Zusammenhang mit den erfindungsgemäßen 3D-DNA-Nanostrukturen definiert. In anderen Worten kann eine 3D-DNA-Nanostruktur als DNA-Origami bezeichnet werden.

Bevorzugt ist mindestens eine, bevorzugt sind alle, der mindestens zwei 3D-DNA-Strukturen, die die Identifizierungsstruktur aufweist, erfindungsgemäße 3-DNA-Strukturen, die hierin beschrieben sind.

Dem Fachmann ist bekannt, wie die 3D-DNA-Nanostrukturen und die Messparameter aufeinander abzustimmen sind und welche Fluoreszenzsignale sich mit welchen Messverfahren und welchen Messparametern unterscheiden lassen. So können sich die voneinander verschiedenen 3D-DNA-Nanostrukturen in ihrer Fluoreszenzantwort beispielsweise durch Farbe und/oder Intensität unterscheiden. Für den Fachmann ist klar, dass beim Einsatz unterschiedlicher Farben mit entsprechend geeigneten Anregungswellenlängen und auf die Farben abgestimmten Filtern gearbeitet werden muss. Die maximale Anzahl erreichbarer Farblevel m hängt dabei unter anderem davon ab, wie präzise zwei benachbarte Farbverteilungen messtechnisch aufgelöst werden können. Dasselbe gilt für die maximale Anzahl erreichbarer Intensitätslevel, da das Verfahren darauf beruht, dass sich zwei benachbarte Intensitätsverteilungen (der Intensitätslevel kl und kl+1) noch (zumindest statistisch) voneinander trennen lassen. Etwa können einzelne Intensitätsverteilungen modelliert werden und die Mischverteilung aus mehreren teilweise überlappenden solcher Intensitätsverteilungen durch Entfaltung oder statistischer, bevorzugt Bayes'scher Inferenz relative Populationsgrößen der einzelnen Verteilungen errechnet werden.

Anstelle von Farbe und/oder Intensität können jedoch auch andere Variablen zum Einsatz kommen, die dazu geeignet sind, die Fluoreszenzsignale unterschiedlicher Identifizierungsstrukturen zu unterscheiden wie z.B. die Bleichgeschwindigkeit des Farbmoleküls oder Fluoreszenzlebensdauer.

Der Nachweis einer Zielstruktur kann die Detektion, dass eine Zielstruktur vorhanden ist, umfassen. Im Prinzip kann der Nachweis allerdings auch den Ausschluss, das eine Zielstruktur nicht vorhanden ist, umfassen. Insbesondere ist das erfindungsgemäße Verfahren auch zur Quantifizierung der Zielstruktur geeignet. In anderen Worten umfasst der Nachweis der Zielstruktur bevorzugt das Quantifizieren einer Zielstruktur (z.B. in einer Probenlösung). Die Quantifizierung kann dabei relativ, d.h. im Verhältnis zu einer anderen Komponente (z.B. einer zweiten Struktur/Zielstruktur in einer Probenlösung), oder absolut (d.h. in Form einer Konzentration oder absoluten Zahl) sein. Beispielsweise kann absolut quantifiziert werden, wenn die Detektion der Identifizierungsstruktur(en) in Lösung geschieht, wie beispielsweise in Flow-Cytometry-, FCS-, oder Lichtblattmikroskopie-basierten Messgeometrien. Dann können alle Identifizierungsstrukturen in einem gegebenen Probenvolumen vermessen werden und eine absolute Anzahl bzw. Konzentration angegeben werden. Für exaktere Aussagen kann die Probe in verschiedenen Verdünnungsschritten mehrmals vermessen und/oder mithilfe statistischer Methoden, bevorzugt Bayes'scher Inferenz aussortierte Messereignisse (etwa aufgrund des Vorhandenseins von weniger als der erwarteten Anzahl von DNA-Nanostrukturen innerhalb eines Messereignisses) die Anzahl der übergangenen Zielstrukturen abgeschätzt werden. Bei Messung von an einen Träger bzw. eine Trägeroberfläche gebundene Identifizierungsstrukturen ist eine analoge Herangehensweise denkbar. Dazu müssen die Parameter derart gewählt werden, dass die Wahrscheinlichkeit gering ist, dass Identifizierungsstrukturen nicht an den Träger bzw. an die (erste) Trägeroberfläche binden. Dies kann zum Beispiel durch ein großes Verhältnis von Trägeroberfläche bzw. der ersten Trägeroberfläche zu Probenvolumen (beispielsweise 0.01/$\mu$m, bevorzugt 0.1/$\mu$m, stärker bevorzugt 0.5/$\mu$m, ganz stark bevorzugt 1/$\mu$m) und/oder durch lange Inkubationszeit (beispielsweise 30 min, bevorzugt 2h, stärker bevorzugt 10h, ganz stark bevorzugt 24h) erreicht werden. Dann können wieder alle Identifizierungsstrukturen vermessen und gegebenenfalls deren Anzahl durch das Probenvolumen geteilt werden. Wie im obigen Fall können weitere Analyseschritte die Abschätzung verfeinern. Bevorzugt kann die Messung in einer Probenkammer erfolgen, in der nur eine der Oberflächen Träger-Adapter aufweist (bevorzugt die am einfachsten zu messende Oberfläche) und alle anderen Oberflächen sind passiviert (z.B. wie anderswo hier beschrieben), sodass die Messung aller Identifizierungsstrukturen leichter von statten geht.

Die Quantifizierung kann auch basierend auf einem internen Standard oder auf empirische Daten erfolgen. Der interne Standard gibt bevorzugt einen Vergleichswert mit bekannter Konzentration an.

Das erfindungsgemäße Verfahren zum Nachweis einer Zielstruktur kann ferner umfassen, dass die in a) ausgebildete Identifizierungsstruktur an einen Träger gebunden ist oder wird. Folglich kann die ausgebildete Identifizierungsstruktur an einen Träger, bevorzugt an eine erste Oberfläche des Trägers, gebunden sein. Das Verfahren kann somit den Schritt des Bindens der ausgebildeten Identifizierungsstruktur an einen Träger, bevorzugt an eine erste Oberfläche des Trägers, umfassen.

Falls die Identifizierungsstruktur an einen Träger gebunden *ist,* so ist darunter bevorzugt zu verstehen, dass die Identifizierungsstruktur auf dem Träger ausgebildet wird. Die Bindung der Identifizierungsstruktur an den Träger kann dadurch vermittelt sein, dass die Zielstruktur und/oder eine der mindestens zwei 3D-DNA Nanostrukturen (bevorzugt die Zielstruktur) bereits vor Ausbildung der Identifizierungsstruktur an den Träger vorgebunden ist/sind oder gebunden wird/werden. In anderen Worten, kann die Zielstruktur und/oder eine der mindestens zwei 3D-DNA Nanostrukturen (bevorzugt die Zielstruktur) an den Träger (vor Ausbildung der Identifizierungsstruktur) gebunden sein. Das Verfahren kann somit weiterhin den Schritt des Bindens der Zielstruktur und/oder mindestens einer der mindestens zwei 3D-DNA Nanostrukturen (bevorzugt der Zielstruktur) an den Träger umfassen. Dieser Schritt kann beispielsweise die Inkubation des Trägers bzw. der ersten Trägeroberfläche mit einer Probenlösung, die die Zielstruktur (und ggf. auch weitere Komponenten wie z.B. weitere Polynukleotide, bevorzugt m-RNAs) enthält, umfassen. Ferner kann dieser Schritt einen oder mehrere Waschschritte (mit einer Pufferlösung) umfassen. Die Vorbindung der Zielstruktur an den Träger bzw. die erste Trägeroberfläche in Kombination mit dem mindestens einen Waschschritt hat den Vorteil, dass die Zielstruktur bereits vor dem Ausbilden der Identifizierungsstruktur aus dem Kontext der Probenlösung entfernt werden kann. Dies ist insbesondere bei komplexen Proben mit vielen und optional ähnlichen Komponenten von Vorteil.

Die Pufferlösung kann 1x bis 8x SSC, bevorzugt 3x bis 5x SSC, und besonders bevorzugt 4x SSC enthalten. Dabei bezeichnet SSC den sogenannten *saline sodium citrate*-Puffer, der aus einer wässrigen Lösung von 150 mM Natriumchlorid und 15 mM Trinatriumcitrat besteht, die mit HCl auf pH 7.0 gebracht wird. Als Pufferbasis sind alternativ zu einem Citratpuffer wie SSC auch Tris, oder PBS verwendbar. Der Puffer kann (bevorzugt alternativ zu SSC) auch NaCl und/oder $MgCl_2$ umfassen. Die Konzentration von NaCl ist bevorzugt 50 mM bis 1200 mM, besonders bevorzugt 200 mM bis 800 mM. Zum Beispiel kann die NaCl-Konzentration 600 mM, bevorzugt 500 mM und besonders bevorzugt 300 mM sein. Die Konzentration von $MgCl_2$ kann 2 mM bis 20 mM, bevorzugt 5 mM bis 15 mM, bevorzugt 8 mM bis 12 mM und besonders bevorzugt 10 mM betragen. Ferner kann die Pufferlösung 4% bis 6%, 2% bis 10%, 15%, oder 20% Dextran Sulfate umfassen. Bevorzugt umfasst der Puffer zum Beispiel 5% Dextran Sulfate. Die Pufferlösung kann auch Polyethylenglycol (PEG), z.B. PEG8000, PEG2000, PEG4000, PEG1000 umfassen. Auch kann der Puffer 0,01 bis 5% Tween-20 umfassen. Optional kann der Puffer auch EDTA, bevorzugt in einer Konzentration von 0,1 mM bis 5 mM, besonders bevorzugt 1 mM umfassen. Ein weiterer optionaler Bestandteil des Puffers ist "sheared salmon sperm" (kommerziell erhältlich), bevorzugt in einer Konzentration von 0,1 mg/ml. "Sheared salmon sperm" kann ggf. die Spezifität erhöhen. Ebenfalls kann der Puffer Denhardts Medium (bestehend aus einer wässrigen Lösung von 0,02% (w/v) BSA (Fraktion V), 0,02% Ficoll 400 (kommerziell erhältlich), sowie 0,02% Polyvinylpyrrolidon (PVP), siehe auch Cold Spring Harb Protoc 2008, doi:10.1101/pdb.rec11538) umfassen, bevorzugt in 1-facher, 2-facher, 3-facher, 4-facher oder 5-facher Konzentration. Ein besonders bevorzugter Puffer umfasst oder hat die Zusammensetzung 4x SSC, 5% Dextran Sulfate und 0.1% Tween 20. Dieser Puffer wird besonders bevorzugt verwendet, wenn es sich bei mindestens einer der, bevorzugt bei allen, nachzuweisenden Zielstrukturen um eine Polynukleinsäure, z.B. eine m-RNA handelt.

Falls die Identifizierungsstruktur an einen Träger gebunden *wird,* ist darunter bevorzugt zu verstehen, dass die Identifizierungsstruktur nicht nur ausgebildet sondern auch auf dem Träger, bevorzugt auf der ersten Oberfläche des Trägers, gebunden wird. In anderen Worten ist keine der Komponenten der Identifizierungsstruktur vorgebunden. Das Verfahren kann daher den Schritt des Bindens der Identifizierungsstruktur an den Träger, bevorzugt der ersten Oberfläche des Trägers, umfassen. Das Binden kann in einem Schritt wie die Ausbildung der Identifizierungsstruktur in a) erfolgen (d.h. Träger und alle zur Ausbildung der Identifizierungsstruktur notwendigen Komponenten (und optional Komponenten die zur Bindung an den Träger notwendig sind, z.B. der unten beschriebene Trägeradapter) werden in einem Schritt gemischt und inkubiert). Alternativ kann das Binden an den Träger anschließend (vorzugsweise vor dem Messen in b)) erfolgen. In beiden Fällen kann das Verfahren nach dem Binden der Identifizierungsstruktur an den Träger bzw. an die ersten Trägeroberfläche ferner mindestens einen Waschschritt (z.B. mit einer der oben genannten Pufferlösungen) aufweisen, um nicht gebundene Komponenten (z.B. 3D-DNA Nanostrukturen und/oder andere Komponenten, die in einer Probenlösung enthalten sind) vom Träger zu entfernen. Wird die Identifizierungsstruktur gebunden, kann die Bindung analog zum "Gebunden sein" durch die Zielstruktur und/oder eine der mindestens zwei 3D-DNA Nanostrukturen (bevorzugt die Zielstruktur) vermittelt sein.

Die Bindung (falls Identifizierungsstruktur an Träger gebunden *ist*) oder das Binden (falls Identifizierungsstruktur an Träger gebunden *wird*) der Identifizierungsstruktur kann durch die Zielstruktur und/oder eine der mindestens zwei 3D-DNA Nanostrukturen (bevorzugt die Zielstruktur) vermittelt sein. Besonders bevorzugt ist, dass die Bindung bzw. das Binden durch die Zielstruktur vermittelt ist.

Die Bindung bzw. das Binden der Identifizierungsstruktur an den Träger bzw. die erste Oberfläche des Trägers kann direkt oder durch einen Träger-Adapter vermittelt sein. Folglich kann die Bindung bzw. das Binden der Zielstruktur und/oder mindestens einer der in der Identifizierungsstruktur gebundenen 3D-DNA-Nanostrukturen (bevorzugt der Zielstruktur) an den Träger bzw. an die erste Trägeroberfläche direkt (z.B. durch eine kovalente oder nicht kovalente Bindung) sein, oder durch einen Trägeradapter vermittelt sein (d.h. indirekt sein). Dabei bindet der Trägeradapter die Zielstruktur (spezifisch) und ist bzw. wird an den Träger bzw. die erste Trägeroberfläche gebunden. Der Bindung des Träger-Adapters an den Träger bzw. die erste Trägeroberfläche kann wiederum selbst direkt (z.B. durch eine kovalente oder nicht kovalente Bindung) sein, oder durch einen Zwischen-Trägeradapter, der den Trägeradapter spezifisch bindet und selbst an den Träger bzw. an die erste Oberfläche des Trägers direkt oder indirekt bindet/gebunden ist, vermittelt sein. Prinzipiell sind auch noch weitere Zwischenträgeradapter nach dem gleichen Prinzip denkbar. Bevorzugt ist, dass der Trägeradapter selbst an den Träger oder die erste Trägeroberfläche direkt gebunden ist/wird. Der Träger bzw. die erste Trägeroberfläche kann beispielsweise mit dem Trägeradapter beschichtet sein.

"Träger-Adapter" bezeichnet bevorzugt ein Teilchen, das spezifisch an Teilregionen von Zielstrukturen (z.B. Zielmolekülen) oder einem Nanoreporter (d.h. einer der mindestens zwei 3D-DNA-Nanostrukturen) binden und an einen Träger gekoppelt werden kann (direkt oder indirekt). Die spezifische Bindung an die Zielstruktur kann beispielsweise durch Watson-Crick-Basenpaarung, durch Van-Der-Waals-Wechselwirkungen oder Wasserstoffbrücken geschehen und unter anderem mit Nukleinsäuren, Antikörpern, Aptameren, Adhirons oder Nanobodies realisiert werden (je nach Zielstruktur).

Ein Träger, an dem die Identifizierungsstruktur gebunden ist/wird, kann verschiedene Formen aufweisen und aus verschiedenen Materialien geformt sein. Bevorzugt weist der Träger eine erste Trägeroberfläche auf und die Identifizierungsstruktur ist an der ersten Trägeroberfläche gebunden. Folglich ist/wird im Verfahren zum Nachweis einer Zielstruktur die Identifizierungsstruktur bevorzugt an eine Trägeroberfläche gebunden. Die erste Trägeroberfläche eines Trägers kann eine komplette Seitenfläche einer Trägerstruktur umfassen. Ebenso kann nur ein Teil einer Seitenfläche einer Trägerstruktur die erste Oberfläche sein. Die erste Trägeroberfläche kann eine Fläche von mindestens 0,01 $mm^2$, bevorzugt mindestens 1 $mm^2$ oder besonders bevorzugt mindestens 10 $mm^2$ aufweisen. Die erste Trägeroberfläche kann eine Fläche von höchstens 1000 $mm^2$, bevorzugt höchstens 400 $mm^2$ oder besonders bevorzugt höchstens 100 $mm^2$ aufweisen. Eine Trägeroberfläche bzw. die erste Oberfläche des Trägers weist bevorzugt eine Glasoberfläche (z.B. eine Borosilikatglasoberfläche oder eine Quartzglasoberfläche) oder eine Polymeroberfläche (wie z.B. μ-Slide $VI^{0.1}$ von ibidi GmbH, oder auf andere für Fluoreszenzmikroskopie geeigneten Polymeren basierende Oberflächen) auf. Ebenso kann eine Trägeroberfläche bzw. die erste Oberfläche des Trägers eine Glasoberfläche (z.B. eine Borosilikatglasoberfläche oder eine Quartzglasoberfläche) oder eine Polymeroberfläche (wie z.B. μ-Slide $VI^{0.1}$ von ibidi GmbH, oder auf andere für Fluoreszenzmikroskopie geeigneten Polymeren basierende Oberflächen) sein. In anderen Worten handelt es sich bei der ersten Oberfläche des Trägers bevorzugt um eine Glasoberfläche (z.B. eine Borosilikatglasoberfläche oder eine Quartzglasoberfläche) oder eine Polymeroberfläche (wie z.B. μ-Slide $VI^{0.1}$ von ibidi GmbH, oder auf andere für Fluoreszenzmikroskopie geeigneten Polymeren basierende Oberflächen). Folglich kann ein Träger eine erste Oberfläche aufweisend oder bestehend aus einer Glasoberfläche (z.B. eine Borosilikatglasoberfläche oder eine Quartzglasoberfläche) oder einer Polymeroberfläche (wie z.B. μ-Slide $VI^{0.1}$ von ibidi GmbH, oder auf andere für Fluoreszenzmikroskopie geeigneten Polymeren basierende Oberflächen) aufweisen. Der Träger kann auch ein Polymernetzwerk aufweisen, welches bevorzugt eines oder eine Kombination der folgenden Materialien aufweist: Biopolymer, Agarose, Collagen. Ein bevorzugter Träger im Kontext der Erfindung ist ein Mikroskopiechip, ein Well einer Platte (bevorzugt für hochauflösende Flureszenzmikroskopie geeignet; z.B. Well einer μ-Plate von Ibidi) oder ein Deckgläschen, ganz bevorzugt ein Mikro-Well Array bzw. eine Mikro-Well Kammer.

Der Träger bzw. die erste Trägeroberfläche ist bevorzugt passiviert. "Passiviert" heißt, dass der Träger bzw. die erste Trägeroberfläche derart beschichtet oder behandelt ist, dass unspezifisches Binden einer Zielstruktur und/oder einer 3D-DNA-Nanostruktur und/oder optional weiterer Komponenten die mit dem Träger oder der ersten Trägeroberfläche in Verbindung kommen (z.B. weitere Bestandteile der Probe in der die Zielstruktur enthalten ist) minimiert wird. Bevorzugt kann passivieren heißen, dass die Oberfläche mit einer BSA-Lösung mit einer Konzentration von 0.1 mg/ml bis 10 mg/ml, bevorzugt 0,5 mg/ml bis 1 mg/ml in Kontakt gebracht bzw. gewaschen wird. Passivieren kann auch durch PEGylierung (beispielsweise beschrieben in folgenden Publikationen: S Chandradoss et al, Jove 2014, "Surface Passivation for Single-molecule Protein Studies", J Piehler et al, Biosensors & Bioelectronics 2000, "A high-density poly(ethylene glycol) polymer brush for immobilization on glass-type surfaces", R Schlapak et al, Langmuir 2006, "Glass Surfaces Grafted with High-Density Poly(ethylene glycol) as Substrates for DNA Oligonucleotide Microarrays") oder Silanisierung (beispielsweise beschrieben in folgenden Publikationen: B Hua et al. und Tj Ha, Nature Methods 2014, "An improved surface passivation method for single-molecule studies", A. Kumar et al, Nuc Ac Research 2000, "Silanized nucleic acids: a general platform for DNA immobilization", H Labit et al, BioTechniques 2008, "A simple and optimized method of producing silanized surfaces for FISH and replication mapping on combed DNA fibers") erfolgen. Ferner kann Passivieren auch Waschen des Trägers oder der Trägeroberfläche mit einer Lösung die Strukturen, die vom selben Typ wie die Zielstruktur sind (z.B. einzelsträngige Polynukleotide), aber nicht identisch mit der Zielstruktur sind, umfassen. Besonders bevorzugt wird der Träger bzw. die erste Oberfläche des Trägers passiviert, wenn die erste Trägeroberfläche eine Glasoberfläche ist oder aufweist. Bei Glasoberflächen wird bevorzugt mit einer BSA-Lösung, PEGylierung oder Silanisierung passiviert.

Ein Träger kann auch eine Matrix, wie z.B. eine Polymermatrix aufweisen. Dies ist besonders für Analysen auf Einzelzellebene von Vorteil. In diesem Fall ist es bevorzugt, dass die Identifizierungsstruktur in die Polymermatrix eingebettet ist/ oder wird. Alternativ oder zusätzlich kann die Identifizierungsstruktur an eine Oberfläche der Polymermatrix gebunden sein/werden. Als Ausgangsstoff für die Matrix bzw. Polymermatrix bieten sich insbesondere Agarose, Collagen, DNA bzw. eine Kombination der Selbigen an. Besonders bevorzugt ist Agarose. Verfahren zur Herstellung und Einbettung einer Identifizierungsstruktur bzw. einer Komponente einer Identifizierungsstruktur (im Falle der Vorbindung der Zielstruktur und/oder einer der mindestens zwei 3D-DNA-Nanostrukturen) sind dem Fachmann wohl bekannt. Hierzu kann die Agarose-Matrix aus einer Mischung von Agarose und biotinylierter Agarose (kommerziell erhältlich) oder Streptavidin-Agarose (kommerziell erhältlich) hergestellt werden, und Ziel-Adapter oder Träger-Adapter an diese Funktionalisierungen (im Falle von biotinylierter Agarose mit vorherigem Durchspülen von Streptavidin) gebunden werden. Alternativ und für die anderen optionalen Matrixmaterialien kann der Adapter an einen Antikörper gekoppelt (direkt oder via Biotin-Streptavidin) werden, der spezifisch an das Matrixmaterial bindet (also anti-Agarose, anti-Collagen, usw.). Eine weitere Möglichkeit ist, dass der Adapter mit Maleimid oder NHS-Ester modifiziert gekauft wird und direkt an die entsprechenden chemischen Gruppen des Matrixmaterials kovalent gebunden wird. Im Falle einer DNA-Polymermatrix als Träger kann diese an sich einzelsträngige Bereiche mit Trägersequenzen beinhalten.

Die Verwendung einer solchen Matrix kann in Kombination mit dem Mikro-Well Array erfolgen. Die oben genannte Polymermatrix kann die Diffusion von aus einer Zelle oder einem anderen Wirtskörper durch Lyse entlassenen Zielstrukturen einschränken. Die Verwendung einer solchen Matrix ist also insbesondere als Alternative, aber auch in Ergänzung, zu den Verfahrensschritten f) und g) vorteilhaft, um zu verhindern dass sich durch Eintrag von außen und/oder Abwanderung aus einen Mikro-Well hinaus, etwa durch Diffusion, der zu untersuchende Inhalt eines Mikro-Wells ändert und dadurch das Analyseergebnis verfälscht. Bei Verwendung einer solchen Träger-Matrix ist darauf zu achten, dass die Polymermatrix so schnell herzustellen ist, dass der Wirtskörper seine Zielstruktur-Komposition vor dem Wirtskörper-Aufschluss nicht verändern kann, oder dass die Polymermatrix derart herzustellen ist, dass die Zielstruktur-Komposition des Wirtskörpers dadurch nicht beeinflusst wird. Letzteres kann beispielsweise durch Verwendung von Niedrigtemperatur-Agarose (Low melting point agarose) gewährleistet werden.

In einer bevorzugten Ausführungsform wird in einem Verfahrensschritt aa), bevorzugt nach Verfahrensschrit p1), eine Träger-Matrix in dem Mikro-Well Array hergestellt und bevorzugt werden in einem Verfahrensschritt bb) Träger-Adapter an die Träger-Matrix befestigt.

Insbesondere ist eine Kombination der Verfahrensschritte aa) und bb) mit bereits beschriebenen Verfahrensschritten bevorzugt, sodass eine bevorzugte Ausführungsform ein Verfahren zur Genexpressionsanalyse ist, wobei eine m-RNA nachgewiesen wird (die m-RNA ist also Zielstruktur) und das Verfahren umfasst:

p1) Einbringen einer Gruppe von Wirtskörpern inklusive eines Wirtskörpers mit der m-RNA in ein Mikro-Well Array derart, dass in mindestens einem Mikro-Well genau ein Wirtskörper vorhanden ist;

aa) Herstellen einer Träger-Matrix in dem mindestens einen Mikro-Well.
bb) Befestigen von Träger-Adaptern an der Träger-Matrix.

p2) Einbringen von mindestens zwei 3D-DNA-Nanostrukturen in das mindestens eine Mikro-Well, wobei jede der 3D-DNA-Nanostrukturen ein oder mehrere innenliegende Fluoreszenz-Farbstoffmoleküle aufweist;

a) Ausbilden einer Identifizierungsstruktur, die aufweist:

(i) eine m-RNA, und
(ii) mindestens zwei 3D-DNA-Nanostrukturen, wobei jede der 3D-DNA-Nanostrukturen ein oder mehrere innenliegende Fluoreszenz-Farbstoffmoleküle aufweist und wobei jede der 3D-DNA-Nanostrukturen (spezifisch) an paarweise unterschiedliche Sequenzabschnitte der m-RNA gebunden ist

b) Nachweis der m-RNA durch Messen mindestens eines Fluoreszenzsignals,

wobei die 3D-DNA-Nanostrukturen und die Parameter der Fluoreszenzmessung derart ausgewählt sind, dass das mindestens eine gemessene Fluoreszenzsignal der in a) ausgebildeten Identifizierungsstruktur von dem Fluoreszenzsignal jeder der mindestens zwei isolierten 3D-DNA-Nanostrukturen unterscheidbar ist, wenn diese nicht in der Identifizierungsstruktur gebunden sind.

Es ist auch möglich, dieses Verfahren *mutatis mutandis* für andere Zielstrukturen als mRNA zu verwenden.

Das Polymer-Netzwerk weist bevorzugt eine mittlere Maschengröße von 1 $\mu$m bis 50 $\mu$m, bevorzugt 2 $\mu$m bis 10 $\mu$m auf. Dies dient zur Einschränkung der Diffusion, sowie zur Bereitstellung von ausreichend vielen Bindestellen für Träger-Adapter bzw. Träger-Adapter-Bindestellen. Der paarweise Mindestabstand der Träger-Adapter bzw. Träger-Adapter-Bindestellen kann im Wesentlichen 200 nm bis 10 $\mu$m, bevorzugt 500 nm bis 5 $\mu$m, besonders bevorzugt 2 $\mu$m bis 3 $\mu$m betragen. Im Wesentlichen heißt in diesem Zusammenhang, dass mindestens 80%, bevorzugt mindestens 90% und besonders bevorzugt mindestens 99% der Träger-Adapter bzw. Träger-Adapter-Bindestellen mindestens die oben angegebene Entfernung von benachbarten Träger-Adapter bzw. Träger-Adapter-Bindestellen haben.

Die Bindung bzw. das Binden der Identifizierungsstruktur an den Träger bzw. die Trägeroberfläche (z.B. mittels Zielstruktur, oder mindestens eine der mindestens zwei DNA Nanostrukturen einer Identifizierungsstruktur) kann unabhängig davon, ob sie direkt, mittels eines Trägeradapters oder eines an den Trägeradapter gebundenen Zwischenträgeradapter erfolgt, mittels verschiedener dem Fachmann bekannter kovalenter und nicht kovalenter Bindungen erfolgen. Zum Beispiel kann die Bindung mittels einer Biotin-Streptavidin-Kopplung erfolgen. Folglich kann die Komponente, die direkt mit dem Träger wechselwirkt (z.B. Trägeradapter, Zielstruktur, eine der DNA-Nanostrukturen oder ein Zwischenträgeradapter), entweder Biotin oder Streptavidin aufweisen. Entsprechend kann dann der Träger bzw. die Trägeroberfläche Streptavidin bzw. Biotin als Gegenstück aufweisen, so dass eine Streptavidin-Biotin Wechselwirkung möglich ist. Neben Streptavidin-Biotin Wechselwirkungen sind auch weitere Wechselwirkung wie z.B. Antikörper-Bindungen verwendbar. Ebenfalls ist denkbar, dass die die Komponente, die direkt mit dem Träger wechselwirkt (z.B. Trägeradapter, Zielstruktur, eine der DNA-Nanostrukturen oder ein Zwischenträgeradapter), mittels eines Trägeradapters über eine NHS-Reaktion auf einem Amin-modifizierten Träger, bzw. auf einer Amin-modifizierten Trägeroberfläche angebracht ist (wird). Auch die Anwendung von Click-Chemistry-Methoden (etwa beschrieben in H. C. Kolb; M. G. Finn; K. B. Sharpless (2001). "Click Chemistry: Diverse Chemical Function from a Few Good Reactions". Angewandte Chemie International Edition. 40 (11): 2004-2021) ist denkbar.

Mit dem erfindungsgemäßen Verfahren können auch mehrere Kopien der Zielstruktur nachgewiesen werden. Werden mehrere Kopien der Zielstruktur nachgewiesen (d.h. bildet sich die Identifizierungsstruktur mehrmals aus), sind/werden bevorzugt die Mehrheit der, bevorzugt alle, für diese Zielstruktur ausgebildeten Identifizierungsstrukturen an den Träger, bevorzugt an die erste Oberfläche des Trägers, gebunden. Bevorzugt sind/werden die Mehrheit der oder im Wesentlichen alle Identifizierungsstrukturen so am Träger angebracht, dass diese mit dem für das Messen des mindestens einen Fluoreszenzsignals in b) verwendeten Messverfahrens noch auflösbar sind. Das kann heißen, dass alle oder im Wesentlichen alle Identifizierungsstrukturen bzw. deren Messereignisse nicht räumlich überlappen (z.B. die beugungsbegrenzte Abbildungen der Identifizierungsstrukturen bei Fluoreszenzmikroskopie). Bevorzugt weisen alle oder im Wesentlichen alle Identifizierungsstrukturen einen Abstand von mindestens 250nm, bevorzugt mindestens 500nm und besonders bevorzugt mindestens 1µm auf. Dies kann heißen, dass mehrere Trägeradapter oder Trägerzwischenadapter in dem genannten Mindestabstand auf dem Träger bzw. der ersten Trägeroberfläche angebracht sind. Im Wesentlichen alle heißt dabei, dass mindestens 80%, bevorzugt mindestens 90% und besonders bevorzugt mindestens 95% der ausgebildeten Identifizierungsstrukturen diese Voraussetzungen erfüllen.

Vorzugsweise sind/werden mehrere Identifizierungsstrukturen an den Träger bzw. die Trägeroberfläche so gebunden/werden so gebunden, dass das Fluoreszenzsignal der einzelnen Identifizierungsstrukturen getrennt messbar ist. Über die Dichte bzw. den Abstand der Bindungsstellen auf der Oberfläche, also bspw. über die Dichte des Streptavidins bzw. Biotins bzw. der Trägeradapter oder Zwischenträgeradapter, welches an/in dem Träger bzw. dessen Oberfläche angebracht ist, kann reguliert werden in welchem räumlichen Abstand Identifizierungsstrukturen an den Träger bzw. die Trägeroberfläche binden. Bevorzugt sind die Bindungsstellen, also bspw. das Streptavidin, das Biotin, der Trägeradapter oder der Zwischenträgeradapter, auf dem Träger bzw. der Trägeroberfläche in folgendem Abstand/Muster/Dichte angeordnet.

Bevorzugt weisen alle oder im Wesentlichen alle Bindungsstellen einen Abstand von mindestens 250nm, bevorzugt mindestens 500nm und besonders bevorzugt mindestens 1µm auf. Bevorzugt sind die Bindungsstellen in einem hexagonalen Gitter mit oben genannter Kantenlänge angeordnet. Die Bindungsstellen können bevorzugt auch zufällig auf dem Träger bzw. dessen Oberfläche verteilt sein, mit einer Flächendichte von 1e-5/$\mu l^2$-1e3/$\mu m^2$, bevorzugt 1e-3$\mu m^2$-4/$\mu m^2$ und besonders bevorzugt 0,1/$\mu m^2$-1/$\mu m^2$. Diese Flächendichte stellt einen genügenden Abstand der Bindungsstellen sicher.

Zusätzlich oder alternativ kann die Regulierung des räumlichen Abstands der Identifizierungsstrukturen an dem Träger bzw. an dessen Oberfläche über die Konzentration der Identifizierungsstrukturen geregelt werden. Wird die Konzentration so gewählt, dass keine Sättigung der Bindestellen an dem Träger eintritt, bewirkt jede weitere Verminderung der Konzentration (also Verdünnung) eine Verringerung der Besetzungsdichte des Trägers. Gegebenenfalls wird zur Bestimmung der notwendigen Verdünnung das Verfahren ein oder mehrmals an einer Verdünnungsreihe durchgeführt, bis die geeignete Verdünnung/Konzentration ermittelt worden ist. Dementsprechend kann das erfindungsgemäße Verfahren nach dem Ausbilden der Identifizierungsstruktur(en) auch noch den Schritt des Verdünnens der Lösung, in der sich die Identifizierungsstruktur ausgebildet hat, vor dem Binden der Identifizierungsstrukturen an den Träger umfassen.

Wie oben erwähnt, kann die Zielstruktur im vorliegenden Verfahren ein teilweise einzelsträngiges Polynukleotid oder einzelsträngiges Polynukleotid sein oder umfassen. In diesem Fall ist es besonders bevorzugt, dass der Träger-Adapter ein Oligonukleotid aufweist oder ist, dessen Nukleinsäuresequenz so programmiert ist, dass es spezifisch an einen ersten einzelsträngigen Abschnitt der Nukleinsäuresequenz der Zielstruktur nach Anspruch 2 bindet. Besonders bevorzugt ist, dass die Zielstruktur eine m-RNA mit Poly-(A)-Schwanz ist, wobei der Träger-Adapter ein Oligonukleotid aufweist oder ist, dessen Nukleinsäuresequenz so programmiert ist, dass es spezifisch an den Poly-(A)-Schwanz der m-RNA Zielstruktur bindet. Das programmiert sein, um an einen Poly-(A)-Schwanz zu binden, kann das Aufweisen eines Poly-(T)-Sequenzabschnittes umfassen.

Wie oben erwähnt, kann die Zielstruktur im vorliegenden Verfahren auch ein Protein sein oder umfassen. In diesem Fall ist es besonders bevorzugt, dass der Träger-Adapter einen Antikörper oder eine antigenbindende Domäne eines Antikörpers aufweist oder ist, der/die (spezifisch) an die Zielstruktur bindet.

Das Verfahren zum Nachweis einer Zielstruktur kann ferner nach a) und vor b) das Waschen des Trägers bzw. der ersten Trägeroberfläche mit einer Pufferlösung umfassen. Ein solcher Waschschritt kann dazu dienen, nicht an die Oberfläche gebundene Komponenten zu entfernen. Insbesondere kann ein solcher Waschschritt sicherstellen, dass freie, nicht an der Identifizierungsstruktur gebundene 3D-DNA-Nanostrukturen vom Träger bzw. der ersten Trägeroberfläche gewaschen werden. Dies hat u.a. den Vorteil, dass ein ggf. störender Hintergrund an Fluoreszenzsignal durch freie 3D-Nanostrukturen verringert oder verhindert wird.

Der Waschschritt wird bevorzugt mit einer Pufferlösung durchgeführt. Die Zusammensetzung des Puffers ist bevorzugt wie bereits oben im Zusammenhang mit anderen Waschschritten definiert.

Eine 3D-DNA-Nanostruktur kann in einer Identifizierungsstruktur entweder direkt/unmittelbar oder mittels eines Ziel-Adapters an die Zielstruktur gebunden sein.

Also kann mindestens eine, bevorzugt alle der mindestens zwei 3D-DNA-Nanostrukturen für eine direkte Bindung an die Zielstruktur programmiert sein und direkt an das Zielmolekül gebunden sein. Bevorzugt ist mindestens eine, besonders bevorzugt sind alle der mindestens zwei 3D-DNA-Nanostrukturen so programmiert, dass sie direkt die jeweilige(n) Region(en) der Zielstruktur programmiert ist/sind und direkt an das Zielmolekül gebunden ist/sind. Eine direkte Bindung kann beispielsweise über Basenpaarung (z.B. bei einer teilweise einzelsträngigen oder einzelträngigen Polynukleotid als Zielstruktur) erfolgen. Folglich können mindestens eine, bevorzugt alle der mindestens zwei 3D-DNA-Nanostrukturen einen einzelsträngigen Sequenzabschnitt enthalten, der an die Zielstruktur (z.B. eine teilweise einzelsträngigen oder einzelträngigen Polynukleinsäure) spezifisch binden kann. Vorzugsweise ist dieser Sequenzabschnitt nach außen gerichtet bzw. an einer Außenoberfläche der 3D-DNA-Nanostruktur angeordnet. So wird sichergestellt, dass der Sequenzabschnitt möglichst gut zugänglich ist.

Alternativ kann die spezifische Bindung mindestens einer, bevorzugt aller 3D-DNA-Nanostrukturen durch einen jeweils zugeordneten Ziel-Adapter vermittelt sein. Der bzw. jeder der Ziel-Adapter ist dabei dazu programmiert, an die jeweilige DNA-Nanostruktur und an die jeweilige(n) Region(en) der Zielstruktur zu binden. Ein Ziel-Adapter kann somit einen ersten Abschnitt aufweisen, der dazu programmiert ist, an die jeweilige 3D-DNA-Nanostruktur (spezifisch) zu binden. Ein Ziel-Adapter kann ferner einen zweiten Abschnitt (auch Zielbindungsabschnitt genannt) aufweisen, der dazu programmiert ist, an die Zielstruktur (spezifisch) zu binden. Bevorzugt weist eine 3D-DNA-Nanostruktur, deren Bindung an die Zielstruktur durch einen Ziel-Adapter vermittelt ist, mindestens einen einzelsträngigen DNA-Abschnitt auf, der so programmiert ist, dass er den Ziel-Adapter spezifisch bindet. Dieser einzelsträngige DNA-Abschnitt ist vorzugsweise nach außen gerichtet, d.h. ist von außen zugänglich. Der oben genannte erste Abschnitt des Zieladapters ist vorzugsweise eine Polynukleinsäuresequenz (z.B. eine DNA, LNA oder RNA Sequenz), die so programmiert ist, das sie spezifisch an den einzelsträngigen DNA-Abschnitt der 3D-DNA-Nanostruktur durch Hybridisierung bindet (d.h. komplementär zu mindestens einem Teil des einzelsträngigen DNA-Abschnitt der 3D-DNA-Nanostruktur ist). Die Bindung erfolgt dabei über Basenpaarung. Bevorzugt hybridisieren mindestens 15, bevorzugt mindestens 18 und besonders bevorzugt 21 Basen der beiden einzelsträngigen Abschnitte.

Ein Zielbindungsadapter weist bevorzugt einen (bzw. mindestens einen) Zielbindungsabschnitt auf. Der Zielbindungsabschnitt ist bevorzugt so programmiert, dass er die spezifische Bindung an die Zielstruktur bzw. an die jeweilige Region der Zielstruktur, an die die jeweilige Identifizierungsstruktur bindet, vermittelt.

Ist oder umfasst die Zielstruktur ein teilweise einzelsträngiges Polynukleotid oder bevorzugt ein einzelsträngiges Polynukleotid (z.B. m-RNA), weist der Zielbindungsabschnitt vorzugsweise eine Nukleinsäuresequenz auf, die so programmiert ist, dass sie spezifisch an einen einzelsträngigen Abschnitt der Zielstruktur bindet. Bevorzugt ist der Zielbindungsabschnitt komplementär zu dem einzelsträngigen Abschnitt der Zielstruktur. Die Bindung zur Zielstruktur erfolgt dabei bevorzugt über Basenpaarung. Bevorzugt hybridisieren mindestens 14, bevorzugt mindestens 18 und besonders bevorzugt 21 Basen der beiden einzelsträngigen Abschnitte.

Ist oder umfasst die Zielstruktur ein Protein, kann der Zielbindungsabschnitt des Ziel-Adapters ein Peptid/Protein oder ein Antikörper (bzw. eine antigenbindendes Fragment eines Antikörpers) aufweisen oder sein, der an das Protein spezifisch bindet.

Das Verfahren zum Nachweis einer Zielstruktur kann ferner das Bereitstellen einer, bevorzugt wässrigen, Probenlösung, die die Zielstruktur enthält, und der mindestens zwei 3D-DNA-Nanostrukturen umfassen. Die Probenlösung kann bspw. Zell-Lysat, aus Zellsuspension und/oder Gewebe extrahierte Nukleinsäuren und/oder mRNA aufweisen. Optional kann das Verfahren ferner das Bereitstellen des Trägers, des/der Trägeradapter(s) und/oder des/der Zieladapter umfassen. Einer oder beide dieser Bereitstellungsschritte können vom Schritt des Ausbildens der Identifizierungsstruktur umfasst sein.

Das Verfahren kann ferner das Mischen einer, bevorzugt wässrigen, Probenlösung, die die Zielstruktur enthält, mit den mindestens zwei 3D-DNA-Nanostrukturen und optional dem Trägeradapter und/oder den Zieladaptern umfassen. Die Probenlösung kann bspw. Zell-Lysat, aus Zellsuspension und/oder Gewebe extrahierte Nukleinsäuren und/oder mRNA aufweisen. Ferner kann das Verfahren das in Kontakt bringen dieser Mischung mit dem Träger bzw. der ersten Träger Oberfläche umfassen. Das Mischen der Komponenten kann auch stufenweise erfolgen.

Eine Probenlösung, die die Zielstruktur enthält, kann ein Zell-Lysat, bevorzugt das Zell-Lysat einer einzelnen Zelle sein. Die Probenlösung kann auch eine Mischung von Nukleinsäuren, z.B. aufgereinigte Nukleinsäuren sein. Insbesondere kann die Probenlösung auch eine aufgereinigte Gesamt-RNA aus Zellen sein. Diese Gesamt-RNA kann mit kommerziell erhältlichen Kits aus Zellen gewonnen werden (z.B. TRIzol® Reagent, TRIzol® LS, PureLink™ Total RNA Blood Kit, RecoverAll™ Total Nucleic Acid Isolation Kit for FFPE von Thermo Scientific, oder RNeasy Kits, PAXgene Blood RNA Kit, oder RNAlater reagent von Qiagen GmbH).

Bevorzugt ist das erfindungsgemäße Verfahren zum Nachweis einer Zielstruktur ferner für den Nachweis weiterer, von der ersten Zielstruktur verschiedenen Zielstrukturen ausgelegt (wobei sich die verschiedenen Zielstrukturen paarweise unterscheiden). Folglich kann das Verfahren auch als Verfahren zum Nachweis von mindestens zwei verschiedenen Zielstrukturen, wobei die mindestens zwei verschiedenen Zielstrukturen paarweise von einander unterscheidbar sind, bezeichnet werden.

Die Erfindung richtet sich insbesondere in einem weiteren Aspekt auf ein Verfahren, das ferner zusätzlich für den Nachweis einer oder mehrerer weiterer, voneinander verschiedener Zielstrukturen geeignet ist, wobei sich die verschiedenen Zielstrukturen paarweise unterscheiden,
wobei für jede der Zielstrukturen die Gruppe von Wirtskörpern in Schritt p1) mindestens einen Wirtskörper mit der jeweiligen Zielstruktur aufweist;
und wobei das Verfahren ferner umfasst:

c) für jede der einen oder mehreren weiteren, voneinander verschiedenen Zielstrukturen: Ausbilden jeweils einer zugeordneten Identifizierungsstruktur, wobei jede der weiteren Identifizierungsstrukturen aufweist:

(i) die jeweils zugeordnete weitere Zielstruktur, und
(ii) mindestens zwei 3D-DNA-Nanostrukturen, wobei jede der mindestens zwei 3D-DNA-Nanostrukturen einen oder mehrere innenliegende Fluoreszenz-Farbstoffmoleküle aufweist und wobei jede der mindestens zwei 3D-DNA-Nanostrukturen spezifisch an die jeweilige weitere Zielstruktur gebunden ist, und wobei die mindestens zwei 3D-DNA-Nanostrukturen an paarweise unterschiedliche Regionen der jeweiligen Zielstruktur gebunden sind;

und wobei der Schritt a) ferner umfasst:
d) Nachweis der einen oder mehreren weiteren Zielstrukturen durch Messen des mindestens einen Fluoreszenzsignals,

wobei alle 3D-DNA-Nanostrukturen und die Parameter der Fluoreszenzmessung derart ausgewählt sind, dass das mindestens eine gemessene Fluoreszenzsignal der in a) und c) ausgebildeten Identifizierungsstrukturen von dem Fluoreszenzsignal aller isolierten 3D-DNA-Nanostrukturen unterscheidbar ist, wenn diese nicht in einer der Identifizierungsstrukturen gebunden sind, und dass die gemessenen Fluoreszenzsignale aller ausgebildeten Identifizierungsstrukturen paarweise voneinander unterscheidbar sind, wobei jede der verschiedenen Zielstrukturen mehrfach vorliegen kann und das Verfahren den mehrmaligen Nachweis einer oder mehrerer der verschiedenen Zielstrukturen umfassen kann.
Es handelt sich hierbei also um eine Verallgemeinerung des zuvor für den Nachweis bzw. die Quantifizierung einer oder mehrerer gleichartiger Zielstrukturen eines ersten Typs auf den Nachweis bzw. die Quantifizierung von jeweils einer oder mehreren gleichartigen Zielstrukturen eines oder mehrerer weiterer untereinander und zum ersten Typ paarweise verschiedener Typen. Hierbei erfolgt das Verfahren für jede der Zielstrukturen dann genauso wie oben für eine Zielstruktur beschrieben. Allerdings laufen die Verfahren für die mehreren nachzuweisenden bzw. zu quantifizierenden Zielstrukturen im Wesentlichen parallel ab und gleiche Verfahrensschritte werden jeweils bevorzugt zusammengefasst.
Bevorzugt wird jede der verschiedenen Zielstrukturen mehrfach nachgewiesen, d.h. jeder der Identifizierungsstrukturen wird bevorzugt mehrfach ausgebildet. Mit anderen Worten können beispielsweise N verschiedene Zielstrukturen nachgewiesen werden, die jeweils paarweise voneinander unterscheidbar sind, wobei aber eine oder mehrere der N verschiedenen Zielstrukturen jeweils mehrfach nachgewiesen werden. Dies impliziert, dass diejenigen Identifizierungsstrukturen, die den mehrfach nachgewiesenen Zielstrukturen zugeordnet sind, entsprechend mehrfach ausgebildet werden. Selbstverständlich müssen sich die Fluoreszenzsignale der für die jeweiligen gleichen Zielstrukturen ausgebildeten gleichen Identifizierungsstrukturen nicht voneinander unterscheiden. Vielmehr dient die wiederholte Messung desselben Fluoreszenzsignals der Quantifizierung der entsprechenden Zielstruktur. Beispielsweise kann das erfindungsgemäße Verfahren dazu dienen, die voneinander verschiedenen Zielstrukturen A, B und C in einer wässrigen Lösung nachzuweisen. Hierfür werden diesen zugeordnete 3D-DNA-Nanostrukturen a1, a2, b1, b2, c1 und c2 bereitgestellt, die zusammen mit den Zielstrukturen A, B und C die Identifizierungsstrukturen Aala2, Bb1b2 und Cc1c2 ausbilden. Die Fluoreszenzsignale der Identifizierungsstrukturen Aa1a2, Bb1b2 und Cc1c2 unterscheiden sich dabei sowohl paarweise voneinander als auch von den isolierten 3D-DNA-Nanostrukturen a1, a2, b1, b2, c1 und c2. Liegen die Zielstrukturen A, B und C jeweils mehrfach vor, müssen sich die Fluoreszenzsignale beispielsweise der gleichen Identifizierungsstrukturen Aala2 nicht voneinander unterscheiden. Jede Messung eines Fluoreszenzsignals der Identifizierungsstruktur Aa1a2 entspricht dann der Detektion einer Zielstruktur A, so dass aus der Anzahl der Messpunkte auf die Konzentration geschlossen werden kann.
Das oben für den Nachweis einer Zielstruktur Gesagte gilt *mutatis mutandis* für den Nachweis der weiteren bzw. jeder weiteren der verschiedenen Zielstrukturen. Insbesondere ist alles oben im Bezug auf die Zielstruktur, Identifizierungsstruktur, der Bindung oder das Binden der Identifizierungsstruktur an einen Träger, den Trägeradapter, die 3D-DNA Nanostruktur(en), den Ziel-Adapter und die weiteren optionalen Verfahrensschritte *mutatis mutandis* auf mindestens eine bevorzugt jede der weiteren Identifizierungsstrukturen anwendbar.
Mit Hinblick auf den bevorzugten Nachweis mehrerer verschiedener Zielstrukturen richtet sich die vorliegende Erfindung in einem Aspekt auch auf ein Verfahren zum Nachweis von mindestens zwei verschiedenen Zielstrukturen, wobei die mindestens zwei verschiedenen Zielstrukturen alle paarweise von einander unterscheidbar sind und das Verfahren umfasst:

a) Ausbilden jeweils einer Identifizierungsstruktur für jede der mindestens zwei Zielstrukturen, die aufweist:

(i) die jeweilige Zielstruktur, und
(ii) mindestens zwei 3D-DNA-Nanostrukturen, wobei jede der 3D-DNA-Nanostrukturen ein oder mehrere innenliegende Fluoreszenz-Farbstoffmoleküle aufweist und wobei jede der 3D-DNA-Nanostrukturen (spezifisch) an die jeweilige Zielstruktur gebunden ist,

b) Nachweis der mindestens zwei Zielstrukturen durch Messen mindestens eines Fluoreszenzsignals,

wobei alle 3D-DNA-Nanostrukturen und die Parameter der Fluoreszenzmessung derart ausgewählt sind, dass das mindestens eine gemessene Fluoreszenzsignal der in a) ausgebildeten Identifizierungsstrukturen von dem Fluoreszenzsignal aller isolierten 3D-DNA-Nanostrukturen unterscheidbar ist, wenn diese nicht in einer der jeweiligen Identifizierungsstruktur gebunden sind, und dass die gemessenen Fluoreszenzsignale der für die einzelnen verschiedenen Zielstrukturen ausgebildeten Identifizierungsstrukturen paarweise voneinander unterscheidbar sind.

Auch hier sind die oben für das Verfahren zum Nachweis einer Zielstruktur spezifizierten bevorzugten Merkmale *mutatis mutandis* anwendbar. Insbesondere ist alles oben im Bezug auf eine Zielstruktur, Identifizierungsstruktur, der Bindung oder das Binden der Identifizierungsstruktur an einen Träger, den Trägeradapter, die 3D-DNA Nanostruktur(en), den Ziel-Adapter und die weiteren optionalen Verfahrensschritte *mutatis mutandis* auf mindestens eine bevorzugt jede der mindestens zwei Identifizierungsstrukturen anwendbar. Insbesondere ist eine Kombination mit den Verfahrensschritten p1) und p2) bevorzugt.

Ist das Verfahren zum Nachweis einer Zielstruktur für den Nachweis weiterer von der von der ersten Zielstruktur verschiedenen Zielstrukturen ausgelegt, oder handelt es sich um ein Verfahren zum Nachweis mindestens zweier verschiedener Zielstrukturen, kann es sich bei den Zielstrukturen um Zielstrukturen gleicher Art (z.B. mehrere m-RNAs) mit paarweise unterschiedlicher Struktur/Sequenz handeln oder die Zielstrukturen umfassen mehrere verschiedene Zielstrukturen der gleichen Art. Alternativ sind auch Zielstrukturen unterschiedlicher Art (z.B. mindestens eine teilweise einzelsträngige DNA oder einzelsträngige DNA und mindestens eine teilweise einzelsträngige oder einzelsträngige RNA; oder mindestens eine teilweise einzelsträngige oder einzelsträngige RNA) denkbar. Bevorzugt ist jedoch, dass es sich um Zielstrukturen gleicher Art, mit paarweise unterscheidbarer Struktur oder Sequenz handelt. Es ist insbesondere auch angedacht, dass das erfindungsgemäße Verfahren ein Verfahren zur Genexpressionsanalyse ist. Bei einer Genexpressionsanalyse sind bevorzugt alle Zielstrukturen RNAs besonders bevorzugt m-RNAs, oder Proteine. Besonders bevorzugt ist im Falle der Genexpressionsanalyse, dass alle Zielstrukturen RNAs, besonders bevorzugt m-RNAs sind.

Sofern das Verfahren zum Nachweis von mehr als einer Zielstruktur ausgelegt ist, ist es besonders bevorzugt, dass auch mindestens eine weitere Identifizierungsstruktur, die eine der weiteren Zielstrukturen umfasst, bevorzugt alle weiteren Identifizierungsstrukturen, an den Träger gebunden werden/sind. Besonders bevorzugt werden alle Identifizierungsstrukturen, an den gleichen Träger gebunden. Das oben über "gebunden wird" und "gebunden ist" im Zusammenhang einer Identifizierungsstruktur Gesagte gilt *mutatis mutandis* auf das "gebunden werden" und des "gebunden sind" im Falle mehrerer/weiterer Identifizierungsstrukturen.

In den Ausführungsformen, in denen eine, mehrere oder alle Identifizierungsstrukturen an einen Träger bzw. einer ersten Trägeroberfläche angebracht sind/werden, ist bevorzugt, dass alle oder im Wesentlichen alle Identifizierungsstrukturen so am Träger angebracht werden/sind, dass diese mit dem für das Messen des mindestens einen Fluoreszenzsignals in b) verwendeten Messverfahren noch auflösbar sind. Das kann heißen, dass alle oder im Wesentlichen alle Identifizierungsstrukturen bzw. deren Messereignisse (z.B. (beugungsbegrenzte) Bilder im Falle von Fluoreszenzmikroskopie) nicht räumlich überlappen. Bevorzugt weisen alle Identifizierungsstrukturen (der gleichen Zielstruktur oder mehrerer Zielstrukturen) einen Abstand von mindestens 250 nm, bevorzugt mindestens 500 nm und besonders bevorzugt mindestens 1$\mu$m auf. Im Wesentlichen alle heißt dabei, mindestens 80%, bevorzugt mindestens 90% und besonders bevorzugt mindestens 95% der ausgebildeten Identifizierungsstrukturen diese Voraussetzungen erfüllen.

Die oben am Beispiel des Verfahrens zum Nachweis einer Zielstruktur erklärten Verfahren, wie eine solche Dichte der Identifizierungsstrukturen erreicht werden kann, finden hier entsprechend Anwendung. Insbesondere kann ein Träger bzw. die erste Trägeroberfläche daran (vor)gebundene Trägeradapter, die ein oder mehreren Zielstrukturen binden, aufweisen. Diese können in den oben genannten Abständen an den Träger bzw. die erste Trägeroberfläche gebunden sein.

Was in einem erfindungsgemäßen Verfahren zum Nachweis einer oder mehrerer Zielstrukturen unter "spezifisch binden" oder "spezifisch gebunden" zu verstehen ist, ist dem Fachmann bekannt. Insbesondere kann spezifisch binden im Kontext der Hybridisierung von Polynukelotid-Einzelsträngen heißen, dass mindestens 14, bevorzugt mindestens 18 und besonders bevorzugt mindestens 21 komplementäre Basen paaren. Spezifisch binden kann insbesondere auch heißen, dass mindestens 90 %, bevorzugt 95 % besonders bevorzugt 99 % der Basen der beiden komplementären Polynukleotid-Einzelstränge paaren (bevorzugt mit den im vorherigen Satz angegebenen Mindestzahl an ausgebildeten Basenpaaren). Im Kontext von Protein-Protein-Interaktionen (z.B. Antikörper-Antigen-Bindung) kann unter spezifisch binden bevorzugt eine Bindungsaffinität (-Δ ΔG) von mindestens 5 kcal/mol bevorzugt mindestens 8 kcal/mol und besonders bevorzugt mindestens 11 kcal/mol verstanden werden. Bindungsaffinitäten können beispielsweise mithilfe von thermophoretischen Messungen (M. Jerabek-Willemsen et al., "Molecular Interaction Studies Using Microscale Thermophoresis", Assay Drug Dev Technol, 9(4): 342-353 (2011) ermittelt werden. Eine weitere Möglichkeit sind Elektrodynamische Messungen wie sie beispielsweise von Dynamic Biosensors GmbH angeboten werden (A. Langer et al, "Protein analysis by time-resolved measurements with an electro-switchable DNA chip", Nat. Comm. 4:2099 (2013)).

Die eine oder mehreren verschiedenen Zielstrukturen können in den erfindungsgemäßen Verfahren in Zellen einer Zellsuspension, einer Droplet-Emulsion, einer wässrigen Lösung mit Bakterien, Vieren oder Exosomen, einer Vollblut-, Speichelprobe oder sonstiger Flüssigkeit tierischen oder menschlichen Ursprungs vorliegen. Bevorzugt liegen mehrere oder alle Zielstrukturen in Wirtskörpern in der gleichen Flüssigkeit vor. Bevorzugt ist die Konzentration der Wirtskörper in der Flüssigkeit so hoch, dass die Wahrscheinlichkeit zur Einzelbelegung von Mikro-Wells hoch ist. Bevorzugt liegt in der Flüssigkeit bei Befüllung des Mikro-Well Arrays 0,5-1,5 Wirtskörper pro Mikro-Well, stärker bevorzugt 0,8-1,2, stärker bevorzugt 0,9-1,1 und besonders bevorzugt 1 Wirtskörper pro Mikro-Well vor. Ist eine höhere Konzentration eines oder mehrerer Wirtskörper(s) in einer Lösung zu erwarten, kann das Verfahren ferner das Verdünnen der Probenlösung vor Ausbildung der Identifizierungsstruktur(en) umfassen. Bevorzugt liegt jede Zielstruktur in mindestens einem Wirtskörper in einer Anzahl von mindestens 1, bevorzugt mindestens 10 und besonders bevorzugt 100 vor.

Das Ausbilden der einen oder mehreren Identifizierungsstrukturen erfolgt in den erfindungsgemäßen Verfahren bevorzugt in einer Pufferlösung, die bevorzugt auch als Hybridisierungspuffer bezeichnet wird. Die Pufferlösung kann 1x bis 8x SSC, bevorzugt 3x bis 5x SSC, und besonders bevorzugt 4x SSC enthalten. Dabei bezeichnet SSC den sogenannten *saline sodium citrate*-Puffer, der aus einer wässrigen Lösung von 150 mM Natriumchlorid und 15 mM Trinatriumcitrat besteht, die mit HCl auf pH 7.0 gebracht wird. Als Pufferbasis sind alternativ zu einem Citratpuffer wie SSC auch Tris, oder PBS verwendbar. Der Puffer kann (bevorzugt alternativ zu SSC) auch NaCl und/oder $MgCl_2$ umfassen. Die Konzentration von NaCl ist bevorzugt 50 mM bis 1200 mM, besonders bevorzugt 200 mM bis 800 mM. Zum Beispiel kann die NaCl-Konzentration 600 mM, bevorzugt 500 mM und besonders bevorzugt 300 mM sein. Die Konzentration von $MgCl_2$ kann 2 mM bis 20 mM, bevorzugt 5 mM bis 15 mM, bevorzugt 8 mM bis 12 mM und besonders bevorzugt 10 mM betragen. Ferner kann die Pufferlösung 4% bis 6%, 2% bis 10%, 15%, oder 20% Dextran Sulfate umfassen. Bevorzugt umfasst der Puffer zum Beispiel 5% Dextran Sulfate. Die Pufferlösung kann auch Polyethylenglycol (PEG), z.B. PEG8000, PEG2000, PEG4000, PEG1000 umfassen. Auch kann der Puffer 0,01 bis 5% Tween-20 umfassen. Optional kann der Puffer auch EDTA, bevorzugt in einer Konzentration von 0,1 mM bis 5 mM, besonders bevorzugt 1 mM umfassen. Ein weiterer optionaler Bestandteil des Puffers ist "sheared salmon sperm" (kommerziell erhältlich), bevorzugt in einer Konzentration von 0,1 mg/ml. "Sheared salmon sperm" kann ggf. die Spezifität erhöhen. Ebenfalls kann der Puffer Denhardts Medium (bestehend aus einer wässrigen Lösung von 0,02% (w/v) BSA (Fraktion V), 0,02% Ficoll 400 (kommerziell erhältlich), sowie 0,02% Polyvinylpyrrolidon (PVP), siehe auch Cold Spring Harb Protoc 2008, doi:10.1101/pdb.recll538) umfassen, bevorzugt in 1-facher, 2-facher, 3-facher, 4-facher oder 5-facher Konzentration. Ein besonders bevorzugter Puffer umfasst oder hat die Zusammensetzung 4x SSC, 5% Dextran Sulfate und 0.1% Tween 20. Dieser Puffer wird besonders bevorzugt verwendet, wenn es sich bei mindestens einer der, bevorzugt bei allen, nachzuweisenden Zielstrukturen um eine Polynukleinsäure, z.B. eine m-RNA handelt.

Das Ausbilden der Identifizierungsstruktur(en) in den erfindungsgemäßen Verfahren erfolgt in den erfindungsgemäßen Verfahren bevorzugt bei einer Temperatur von 4° C bis 50°C, bevorzugt 18°C bis 40°C. Beispielsweise kann die Temperatur bei 40°C liegen. Besonders bevorzugt wird 30°C verwendet. Die angegebenen Temperaturen sind insbesondere bevorzugt, wenn eine oder mehrere teilweise einzelsträngige oder einzelsträngige Polynukleotide nachgewiesen werden sollen.

Die Ausbildung der Identifizierungsstruktur kann eine Inkubationszeit einer Mischung der darin gebundenen Komponenten umfassen. Bevorzugt kann die Inkubationszeit 5 min bis 20 h, bevorzugt 1 h bis 20 h (z.B 1 h, 2 h, 4 h, 8 h) und besonders bevorzugt 10h bis 20 h betragen.

In den erfindungsgemäßen Verfahren können die eine oder die mehreren Identifizierungsstrukturen statt sequenziell auch in einem Schritt ausgebildet (bevorzugt in der oben genannten Pufferlösung) und an den Träger gebunden werden.

Für das Ausbilden der Identifizierungsstruktur in einem Schritt werden die DNA Nanostrukturen (mindestens 2 pro Zielstruktur; bevorzugt in den oben angegebenen Konzentrationen) in die Flüssigkeit mit Wirtskörpern gegeben. Optional können auch noch ein oder mehr Trägeradapter mit in die Lösung gegeben werden. Trägeradapter können jedoch auch schon an den Träger vorgebunden sein. Nach dem Aufschluss der Wirtskörper in den Mikro-Wells sind dann schon alle Bestandteile der Identifizierungsstrukturen in den Mikro-Wells vorhanden und können sie ausbilden. Dadurch kann die Dauer von Beginn der Präparation bis zum Ergebnis der Analyse stark reduziert und der Aufwand reduziert werden. Das kann insbesondere bei der Einzelzell-Genexpressionsanalyse zeitkritischer klinischer Proben, wie beispielsweise bei der Bestimmung von Sepsiserregern, wichtig sein. Aber auch im allgemeinen Fall ist es ökonomisch vorteilhaft.

Z.B. kann der Trägeradapter in einer Konzentration von 1nM pro Zielstruktur, die durch den Trägeradapter gebunden wird, zugegeben werden. Bevorzugt werden Trägeradapter in der Gesamtkonzentration der 3D-DNA-Nansotrukturen pro Zielstruktur zugegeben (z.B. 3nM, bei 3D-DNA-Nansotrukturen für ein Ziel mit jeweils 1nM. Ein totales Volumen zur Ausbildung der Identifizierungsstruktur(en) kann zum Beispiel 1 µl bis 500 µl sein. Für das Binden der ausgebildeten Identifizierungsstruktur wird bevorzugt die oben beschriebene Mischung zur Ausbildung der Identifizierungsstruktur auf den Träger bzw. die erste Trägeroberfläche gegeben. Der Trägeradapter kann entweder bereits vorgebunden (falls nicht Teil der Lösung zur Ausbildung der Identifizierungsstruktur) oder kann in der Inkubation an die Trägeroberfläche gebunden werden. Die Bindung erfolgt entweder kovalent oder nicht kovalent (z.B. Biotin-Streptavidin Interaktion). Das Binden an den Träger kann eine Inkubationszeit von 1 min bis 2 h, 5 min bis 1 h, 8 min bis 25 min und besonders bevorzugt 10 min umfassen. Weitere Beispiele für Inkubationszeiten sind 2 min, 5 min, 20 min. Die Inkubation kann bei 4°C bis 30°C, bevorzugt Raumtemperatur (z.B. 20°C bis 25°C) ablaufen. Vor dem Aufbringen auf die Trägeroberfläche kann die Lösung nochmal verdünnt werden (z.B. mit PBS oder dem oben genannten Puffer, der für die Ausbildung der Identifizierungsstruktur verwendet wird), um z.B. den Dextran Anteil zu verringern, eine größere Fläche abzudecken oder die Konzentration anzupassen. Vor Durchführung der Messung kann dann die Oberfläche mit einem Puffer gewaschen werden. Vorzugsweise kann hier ein Puffer wie er oben für Waschschritte definiert ist verwendet werden.

Bevorzugt umfasst das Messen mindestens eines Fluoreszenzsignals in den erfindungsgemäßen Verfahren: q) Erstellen eines Datensatzes, der Daten betreffend von einem Ausschnitt einer Probe emittierte Fluoreszenzsignale enthält, mittels einem Fluoreszenzmikroskop, bevorzugt in Epifluoreszenz-, TIRF-, Lichtblatt- und/oder Konfokalmikroskopie. Mit anderen Worten kann das Messen mindestens eines Fluoreszenzsignals die Messung mehrerer Fluoreszenzsignale oder einer Vielzahl von Fluoreszenzsignalen umfassen, indem beispielsweise pixel- oder voxelweise die von einem zwei- oder dreidimensionalen Probenausschnitt emittierte Intensität gemessen und abgespeichert wird. Die Messung kann dabei auch eine gleichzeitige oder sequentielle Messung mit mehreren Anregungswellenlängen und/oder mehrerer Nachweiswellenlängen(bereichen) umfassen.

Bevorzugt umfasst der Nachweis der Zielstruktur bzw. der einen oder mehreren weiteren Zielstrukturen: w) Identifizieren eines oder mehrerer in dem Datensatz enthaltenen Datums, das bzw. die das Fluoreszenzsignal einer der Identifizierungsstrukturen repräsentiert.

Diese Identifikation kann beispielsweise den Abgleich der gemessenen Intensität eines Pixels oder Voxels bei einer bestimmten Anregungs- und/oder Detektionswellenlänge mit einem Set solcher vorbestimmter Kombinationen umfassen, so dass durch den Abgleich auf die Identifizierungsstruktur geschlossen werden kann, der diese Kombination zugeordnet ist.

Dementsprechend kann Schritt q) umfassen: Aufnehmen mindestens einer Bilddatei, die Fluoreszenzdaten des Ausschnitts der Probe enthalten. Ferner kann das Identifizieren in Schritt w) die folgenden Schritte umfassen:

w1) Auslesen einer Farb- und/oder einer Intensitätsinformation (und bevorzugt) eines Datums bzw. Bildelements; und
w2) Abgleichen der Farb- und/oder Intensitätsinformation (und bevorzugt) des Datums bzw. Bildelements mit einer Farb- und/oder Intensitätsinformation, die die Identifizierungsstruktur repräsentiert.

Die Proben werden bevorzugt mit einer Fluoreszenzmikroskopietechnik visualisiert. Für eine weitere Verarbeitung der Information kann das bereitgestellte Bild direkt ausgewertet werden. Bevorzugt wird es jedoch abgespeichert. Bilder werden bevorzugt digital gespeichert, es können jedoch auch analoge Bilder aufgenommen werden. In dem Bild enthaltene Information kann die Darstellung eines oder mehrerer Bildelemente enthalten. Ein Bildelement ist ein Teil eines Bildes. Ein Bildelement kann bspw. ein ausgedehnter Punkt oder eine andere Struktur einer ersten Intensität auf einem Hintergrund einer zweiten Intensität sein.

Je nach Wahl der Fluoreszenz-Farbstoffe auf den DNA-Nanostrukturen sind die Zielstrukturen anhand ihrer Farbe und/oder anhand ihrer Intensität unterscheidbar. Bevorzugt wird der Probenausschnitt in jede Farbe einzeln aufgenommen und in einem eigenen Bild abgespeichert. Der Probenausschnitt kann jedoch auch in mehreren Farben gleichzeitig aufgenommen werden, wobei die Aufnahmen unterschiedlicher Farbe in einem gemeinsamen und/oder in eigenen Bildern abgespeichert werden.

Diese Schritte können manuell, softwaregestützt manuell oder automatisch per Analysesoftware erfolgen.

In einer manuellen Analyse kann ein Anwender unter Kenntnis der Farb- und/oder Intensitätsinformation der nachzuweisenden Zielstruktur die entsprechenden Farb- und/oder Intensitätsinformationen in dem Bild suchen und identifizieren. Optional kann der Anwender die Zahl der solchermaßen identifizierten Zielstrukturen und/oder die zugehörige Ortsinformation auswerten. Liegen die Farb- und/oder Intensitätsinformationen bei mehrfarbigen Identifizierungsstrukturen in verschiedenen Bildern vor, können die Bildelemente für jede Farbe, d.h. jedes Bild, separat inklusive der Ortsinformation ausgewertet werden. Anschließend können die mehrfarbigen Elemente erkannt werden, indem verschiedenfarbige Bildelemente mit hinreichend ähnlichen Ortsinformationen identifiziert werden. Alternativ kann zuerst ein mehrfarbiges Bild durch übereinanderlegen der einzelnen einfarbigen Bilder erstellt werden. Eine mehrfarbige Identifizierungsstruktur kann dann als Bildelement mit der entsprechenden Mischfarbe identifiziert werden. Es ist zu beachten, dass die Bilder nicht in der echten Farbe abgespeichert sein müssen. Sie können selbstverständlich nachträglich mit einer Farbe, der echten oder einer Falschfarbe, versehen werden.

Die manuelle Analyse kann unter Zuhilfenahme einer Bildanalysesoftware, bspw. Fiji oder Ähnliches, auch softwaregestützt erfolgen. Der Übergang zu einer vollautomatischen Auswertung ist fließend. Grundsätzlich ist es möglich, beliebige Einzelschritte manuell durchzuführen oder durch eine Software vornehmen zu lassen.

Ein Aspekt der Erfindung betrifft die Auswertung mit einer Software, wobei die Auswertung auf der *density based spatial clustering of applications with noise* Methode (abgekürzt DBSCAN) basiert. Die Beschreibung erfolgt anhand der Anwendung für den Fall, dass das auszuwertende Bild mehrere Fluoreszenzelemente aufweist. Die Auswertung funktioniert selbstverständlich genauso, wenn nur ein einziges oder gar kein Fluoreszenzelement in dem Bild vorhanden ist.

Die dafür programmierte Auswertesoftware ist bevorzugt Python-basiert. Die Python-basierte Auswertesoftware lädt Bilddaten. Sie ermittelt bevorzugt lokale Maxima in Bildboxen. Die Größe der Bildboxen wird bevorzugt in Abhängigkeit von der Vergrößerung und der numerischer Apertur des verwendeten Objektivs eingestellt und beträgt beispielsweise 9-15 Pixel. Die Software errechnet bevorzugt den kummulativen Absolutwert des Gradienten als hintergrundunabhängige Messgröße des Signals. Um zwischen Bildboxen mit und ohne DNA-Nanostrukturen zu unterscheiden wird bevorzugt DBSCAN (*density based spatial clustering of applications with noise*) (publiziert in Ester, Martin; Kriegel, Hans-Peter; Sander, Jörg; Xu, Xiaowei "A density-based algorithm for discovering clusters in large spatial databases with noise", Proceedings of the Second International Conference on Knowledge Discovery and Data Mining (KDD-96). AAAI Press. pp. 226-231 (1996)) verwendet. Alternativ können aber auch andere Clustering-Algorythmen wie HDBSCAN (Ricardo J. G. B. Campello, Davoud Moulavi, Joerg Sander; "Density-Based Clustering Based on Hierarchical Density Estimates", Advances in Knowledge Discovery and Data Mining 2013, pp. 160-172), k-means oder hierarchisches Clustering (Rokach, Lior, and Oded Maimon. "Clustering methods." Data mining and knowledge discovery handbook. Springer US, 2005. 321-352) verwendet werden. Wahlweise werden die Bildboxen in Gruppen unterschiedlicher Werte des kummulativen Absolutwerts des Gradienten eingeteilt, was einer Einteilung nach unterschiedlichen Anzahlen von Fluorophoren entspricht. Dies kann insbesondere dann erfolgen, wenn unterschiedliche Zielstrukturen durch Identifizierungsstrukturen unterschiedlicher Intensität identifiziert werden. Die Bildboxen mit DNA-Nanostrukturen eines Farbkanals können basierend auf ihren transversalen (x, y)-Positionen mit allen Bildboxen mit DNA-Nanostrukturen anderer Farbkanäle verglichen werden, um mehrfarbige Fluoreszenzelemente, bspw. Punkte, im Bild zu erkennen. Dies kann selbstverständlich unterbleiben, wenn nur Identifizierungsstrukturen einer einzigen Farbe vorliegen. Die Software kann die ermittelten Werte für die Anzahl der einfarbigen Punkte sowie optional deren Orte im Bild und die Werte für die Anzahl der mehrfarbigen Punkte sowie optional deren Orte im Bild für die weitere Verwendung abspeichern. Die Software kann auch die Intensitätswerte der Punkte abspeichern. Beispielsweise kann die Software verwendet werden, um die Anzahl der einfarbigen im Vergleich zu mehrfarbigen Fluoreszenzelementen anzugeben. Beispielsweise kann die Software verwendet werden, um die Anzahl der detektierten Fluoreszenzelemente in Experimenten mit Proben mit und ohne Zielstruktur zu vergleichen. Dies ist in den angehängten Beispielen 1 bis 3 genauer erläutert.

Aufgrund der bei der Herstellung der 3D-Nanostrukturen manchmal auftretenden Variation der Anzahl von Farbstoffmolekülen pro 3D-Nanostruktur sowie aufgrund von Messfehlern hat man es hierbei jeweils mit bspw. Intensitätsverteilungen zu tun. Wenn zwei benachbarte Intensitätsverteilungen einen nicht vernachlässigbaren Überlapp aufweisen, so lässt sich ggf. alleine aus einer Intensitätsmessung nicht zweifelsfrei auf exakt eine Identifizierungsstruktur schließen. Um diesem Problem Rechnung zu tragen, ist es bevorzugt, statistische Methoden zu verwenden, mittels derer sich eine statistische Zuordnung erzielen lässt. Diese Verteilungen können ein- oder mehrdimensional entlang von Intensitäts- oder Farbverläufen erstrecken und mithilfe je eines oder mehrerer Messgrößen wie dem genannten kummulativen Gradienten, dem Mittelwert über die Box, dem maximalen Pixelwert innerhalb der Box oder anderer Messgrößen beschrieben werden. Die gemessene optional mehrdimensionale Verteilung ist eine Mischverteilung der einzelnen den Identifizierungsstrukturen mit denselben Zielstrukturen zugehörigen Verteilungen. Ziel der Analyse ist, den Anteil dieser einzelnen Verteilungen an der Mischverteilung zu errechnen und somit die relative Anzahl der unterschiedlichen Zielstrukturen zu erfahren. Diese einzelnen Verteilungen können entweder durch separate Messungen eruiert werden, oder durch Modelle (beispielsweise Gauß- und/oder Poissonverteilungen oder Mischungen daraus, möglicherweise unterschiedlich in verschiedenen Dimensionen) angenähert werden. Somit können die verschiedenen Anteile durch Entfaltung errechnet werden, oder durch statistische, bevorzugt auf Bayes'sche Inferenz basierende, Methoden (siehe D.S.Sivia, "Data Analysis, a bayesian tutorial", Oxford science publications, zweite Auflage 2006, ISBN: 0198568320 und/oder A.B. Downey, "Think Bayes", O'Reilly, Sebastopol CA, vierte Auflage 2013 ISBN: 9781449370787). Eine Möglichkeit ist hierbei, zunächst eine Gleichverteilung der Anzahlen von Zielstrukturen anzunehmen und ausgehend davon für jedes Messereignis die Wahrscheinlichkeit, von jeder der Verteilungen herzurühren, zu berechnen und damit die Annahme über die Verteilung der Anzahlen von Zielstrukturen zu aktualisieren. Bei einer genügend großen Zahl von Identifikationsstruktur-Messereignissen (über 50, bevorzugt über 100, besonders bevorzugt über 1000) ist diese Methode unabhängig von der Anfangsannahme über die Verteilung der Anzahlen von Zielstrukturen.

Wir lassen den Fall zu, dass die Intensitätsverteilungen verschiedenartiger Nanoreporter überlappen und nur statistisch, unter Betrachtung der Verteilungshistogramme eindeutig unterscheidbar sind, wie im Verfahrensschritt (h/ii) beschrieben. Dies macht die Analyse komplexer, da für jedes Messereignis die Wahrscheinlichkeit errechnet wird, von den diversen Nanoreportern zu stammen, und mit größerem Überlapp wird bei gleicher Anzahl von Messereignissen das Ergebnis unsicherer. Dafür kann aber die Anzahl unterschiedlicher Nanoreporterkombinationen und somit die Anzahl von Zielmolekülen erhöht werden, da die Intensitätsstufen dabei verringert werden. Dadurch ergibt sich eine Abwägung zwischen Größe des Überlapps der Intensitätsverteilungen und Anzahl simultan quantifizierbarer Zielmoleküle. Für jede Anwendung kann also die optimale Konfiguration gewählt werden.

Bevorzugt unterscheiden sich die Fluoreszenzsignale der für die einzelnen verschiedenen Zielstrukturen ausgebildeten Identifizierungsstrukturen von dem Fluoreszenzsignal aller isolierter 3D-DNA-Nanostrukturen, wenn diese nicht in einer der Identifizierungsstrukturen gebunden sind, und die Fluoreszenzsignale der für die einzelnen verschiedenen Zielstrukturen ausgebildeten Identifizierungsstrukturen paarweise voneinander dadurch, dass die entsprechenden Fluoreszenzsignale eine unterscheidbar andere Kombination aus Farb- und/oder Intensitätsinformation aufweist. Wie oben erläutert, kann "eine unterscheidbar andere Kombination" eine statistisch unterscheidbar andere Kombination sein, d.h. die Unterscheidbarkeit wird mittels statistischer Methoden sichergestellt.

Bevorzugt kommen in den 3D-DNA-Nanostrukturen k (statistisch) voneinander unterscheidbarer Intensitätslevel und/oder m (statistisch) voneinander unterscheidbarer Farblevel zum Einsatz, wobei bevorzugt jedes der k Intensitätslevel durch eine Intensitätsverteilung gebildet wird und wobei die k Intensitätsverteilungen, bevorzugt statistisch, voneinander unterscheidbar sind und/oder wobei bevorzugt jedes der m Farblevel durch eine Farbverteilung gebildet wird und wobei die m Farbverteilungen, bevorzugt statistisch, voneinander unterscheidbar sind. Hierfür ist es bevorzugt, dass jeweils der Überlapp benachbarter Verteilungen kleiner als 30 %, bevorzugt kleiner als 10 %, stärker bevorzugt kleiner als 5 %, besonders bevorzugt kleiner als 2 % und ganz besonders bevorzugt kleiner als 1 % ist.

Hierbei gilt bevorzugt: k>2, bevorzugt k>3, stärker bevorzugt k>4, noch stärker bevorzugt k>5, besonders bevorzugt k>6. Ferner gilt bevorzugt: m>2, bevorzugt m>3, stärker bevorzugt m>4, noch stärker bevorzugt m>5, besonders bevorzugt m>6.

Es ist ferner bevorzugt, dass der Schritt w1) einen oder eine Kombination der folgenden Schritte bzw. Techniken aufweist: Bestimmen eines Mittelwerts des Bildelements, Bestimmen eines Maximums des Bildelements, Berechnen eines kummulativen Gradienten, Berechnen eines oder mehrerer statistischer Momente wie z.B. Varianz der Pixel im Bildelement, Variationskoeffizient, Fano Faktor. Es ist ferner bevorzugt, dass der Schritt w2) basierend auf einer Clustering-Methode, bevorzugt einer DBSCAN (density based spatial clustering of applications with noise) Methode erfolgt. Es ist ferner bevorzugt, dass Schritt w1) und/oder w2) auf probabilistischer Betrachtung, bevorzugt unter Hinzunahme von Bayes' Theorem, basiert.

Insbesondere können die Intensitätsabstufungen der Nanoreporter bzw. 3D-DNA-Nanostrukturen jeder orthogonal messbaren Art beispielsweise durch Variation von Anzahl, Abstand oder Orientierung unterschiedlich gewählt werden. Die Abstufungen können so groß sein, dass die Intensitätsverteilungen, die aufgrund von Faktoren wie Poissonstatistik der Photonen oder unvollständiger Markermolekülbesetzung verbreitert sind, nicht überlappen. Dadurch ist die Analyse einfach und Messwerte können direkt mit Nanoreportern identifiziert werden. Zum Anderen können sie aber auch kleiner sein, so dass Intensitätsverteilungen benachbarter Intensitätsabstufungen zu 0,1-10 % und sogar 5-80% überlappen. Dann können nach der Messung die Vektoren aus Verteilungen der orthogonalen Messergebnisse verglichen werden mit den zu erwartenden, und mithilfe von Least-Squares- oder Maximum-Likelihood-basierten Methoden die relative Häufigkeit der Zielmoleküle bestimmt werden. Selbes kann auch mit Cluster-basierten Methoden des Machine Learning mit oder ohne Hinzuziehung der zu erwartenden Verteilungen erreicht werden.

Es ist auch bevorzugt, einen der orthogonal messbaren Nanoreporter in einer fixierten Amplitudengröße, deren Messwert von dem der doppelten Amplitudengröße eindeutig unterscheidbar ist, und an jedes Zielmolekül zu programmieren. Dadurch kann die Anzahl von Zielmolekülen in einem Messereignis direkt gemessen werden, was insbesondere die Erkennung und Ausschluss von Messereignissen, die von sehr nah aneinander am Träger gebundenen Zielstrukturen herrühren, erleichtert.

Für das oben diskutierte Verfahren ist es bevorzugt, ein Set aus mehreren 3D-DNA-Nanostrukturen (wobei es sich bevorzugt um 3D-DNA-Nanostrukturen wie in dieser Anmeldung beschrieben handelt) zu verwenden, wobei das Set N voneinander verschiedene 3D-DNA-Nanostrukturen aufweist und wobei sich die N voneinander verschiedenen 3D-DNA-Nanostrukturen des Sets durch die Fluoreszenzfarbstoffmoleküle paarweise voneinander unterscheiden. Bevorzugt enthalten die N voneinander verschiedenen 3D-DNA-Nanostrukturen eine unterschiedliche Anzahl von Fluoreszenzfarbstoffmolekülen und/oder unterschiedliche Fluoreszenzfarbstoffmoleküle, so dass mit den N voneinander verschiedenen 3D-DNA-Nanostrukturen des Sets k voneinander unterscheidbarer Intensitätslevel und/oder m voneinander unterscheidbarer Farblevel erzeugt werden können. Bevorzugt ist mindestens ein Teil der N voneinander verschiedenen 3D-DNA-Nanostrukturen mehrmals in dem Set enthalten ist, so dass jedes der k Intensitätslevel durch eine Intensitätsverteilung gebildet wird und wobei die k Intensitätsverteilungen, bevorzugt statistisch, voneinander unterscheidbar sind. Bevorzugt ist mindestens ein Teil der N voneinander verschiedenen 3D-DNA-Nanostrukturen mehrmals in dem Set enthalten, so dass jedes der m Farblevel durch eine Farbverteilung gebildet wird und wobei die m Farbverteilungen, bevorzugt statistisch, voneinander unterscheidbar sind. Dabei ist der Überlapp benachbarter Verteilungen kleiner als 30 %, bevorzugt kleiner als 10 %, stärker bevorzugt kleiner als 5 %, noch stärker bevorzugt kleiner als 2 % und besonders bevorzugt kleiner als 1 % ist.

Auch hier gilt bevorzugt: k>2, bevorzugt k>3, stärker bevorzugt k>4, noch stärker bevorzugt k>5, besonders bevorzugt k>6; und/oder m>2, bevorzugt m>3, stärker bevorzugt m>4, noch stärker bevorzugt m>5, besonders bevorzugt m>6.

Die vorliegende Erfindung richtet sich in einem Aspekt auf ein Kit, das aufweist:
ein Mikro-Well Array, vorzugsweise nach einer der bereits beschriebenen Ausführungsformen; mindestens zwei 3D-DNA-Nanostrukturen, wobei jede der 3D-DNA-Nanostrukturen mindestens ein innenliegendes Fluoreszenz-Farbstoffmolekül aufweist.

Bei den 3D-DNA-Nanostrukturen handelt es sich bevorzugt um vorstehend und/oder nachstehend beschriebene 3D-DNA-Nanostrukturen.

Insbesondere betrifft die vorliegende Erfindung ein Kit, bei dem mindestens eine der 3D-DNA-Nanostrukturen mindestens 2 innenliegende Fluoreszenz- Farbstoffmoleküle aufweist, und wobei der Abstand der mindestens zwei innenliegenden Fluoreszenz-Farbstoffmoleküle paarweise mindestens 2 nm, bevorzugt mindestens 5 nm und besonders bevorzugt mindestens 9 nm beträgt.

Ein Aspekt der Erfindung betrifft ein Kit, das für den Nachweis bzw. die Quantifizierung mehrerer verschiedener Zielstrukturen vorgesehen ist. Bevorzugt weist ein erfindungsgemäßes Kit demnach ein Set aus mehreren 3D-DNA-Nanostrukturen auf, wobei das Set N voneinander verschiedene 3D-DNA-Nanostrukturen aufweist und wobei sich die N voneinander verschiedenen 3D-DNA-Nanostrukturen des Sets durch die Fluoreszenzfarbstoffmoleküle paarweise voneinander unterscheiden.

In den erfindungsgemäßen Verfahren kann das Messen des mindestens einen Fluoreszenzsignals mittels Fluoreszenzmikroskopie, bevorzugt durch eine oder eine Kombination der folgenden Techniken erfolgen: Punktscannen, Weitfeldmikroskopie, (Spinning-Disc-)Konfokalmikroskopie, Zweiphotonenspektroskopie, Epifluoreszenz, Lichtblattmikroskopie, TIRF; wobei jeweils bevorzugt eine alternierende Anregung (ALEX) oder eine Anregung mittels gepulster Lichtquellen pulse-interleaved erfolgt.

Ein beispielhaftes Verfahren, um mit diesen DNA-Nanostrukturen oder anderen Nanoreportern das gewünschte Ziel zu erreichen, weist die folgenden Schritte auf oder besteht darin, dass

a. jeder Art von Zielstruktur eine eindeutige Kombination aus einem oder mehreren orthogonal messbaren Nanoreportern (z.B. erfindungsgemäße 3D-DNA-Nanostrukturen) so zugeordnet werden, dass jede Art von Zielstruktur eindeutig unterschieden und identifiziert werden kann
b. für jede Art Zielstruktur mindestens so viele Binderegionen wie zuvor Nanoreporter identifiziert und Adapter ausgewählt werden, die spezifisch und eindeutig an die Binderegionen binden können,
c. einer Art Zielstruktur zugeordnete Nanoreporter und Adapter eindeutig gepaart und gekoppelt werden,
d. optional die nicht Nanoreportern zugeordneten Adapter an Substrat, Träger oder Matrix gekoppelt werden,
e. die Komplexe aus Nanoreporter und Adapter mit den Zielstrukturen in Lösung inkubiert werden,
f. die resultierenden Komplexe aus Zielmolekülen, Adaptern und Nanoreportern mit Messapparaturen für die vorhandenen Marker so gemessen werden, dass nur mit einer akzeptabel geringen Wahrscheinlichkeit mehr als ein Komplex für ein Messereignis verantwortlich ist,
g. Messereignisse, die nicht über die Mindestanzahl an orthogonalen Signalen verfügen herausgefiltert werden,
h. Den übrigen Messereignissen entweder

  i. direkt durch die einzelnen Signale eine Kombination an Nanoreportern, oder
  ii. statistisch durch den Vergleich der gemessenen Verteilung an Messsignalen und den für jeden Nanoreporter erwarteten Verteilung eine Wahrscheinlichkeit von einer Kombination an Nanoreportern zu stammen zugeordnet werden, oder
  iii. durch clusterbasierte Algorithmen eine Wahrscheinlichkeit von einer Kombination an Nanoreportern zu stammen zugeordnet werden;

i. Die relative Anzahl der Zielstruktur durch die Gesamtheit der errechneten Kombinationen an Nanoreportern oder deren Wahrscheinlichkeiten ermittelt werden; und
j. Optional bei bekannter absoluter Konzentration mindestens einer der Zielstrukturen oder bekanntem Messvolumen die absolute Anzahl der Zielstrukturen ermittelt wird; und
k. Optional kann ein Nanoreporter, der orthogonal messbar zu allen anderen ist, auf alle Zielstrukturen programmiert werden, sodass sein Messsignal Auskunft über die Anzahl von Zielstrukturen, die einem Messereignis zugrunde liegen, gibt.

Es ist insbesondere angedacht, dass die erfindungsgemäßen Verfahren und/oder die 3D-DNA-Nanostrukturen zur Genexpressionsanalyse, bevorzugt zur Genexpressionsanalyse auf Einzelzellebene, verwendet werden.

Bei einer Genexpressionsanalyse auf Einzelzellebene wird bevorzugt eine Polymermatrix als Träger verwendet, die bevorzugt den Wirtskörper teilweise oder ganz umgibt. Das Ausbilden der Identifizierungsstruktur umfasst in diesem Fall bevorzugt:

1) Bereitstellen eines Wirtskörpers in einem Mikro-Well
2) Umgeben des Wirtskörpers mit einer Polymermatrix (d.h. dem Träger); Dabei ist darauf zu achten, dass die Polymermatrix so schnell herzustellen ist, dass der Wirtskörper seine Zielstruktur-Komposition vor dem Wirtskörper-Aufschluss nicht verändern kann, oder dass die Polymermatrix derart herzustellen ist, dass die Zielstruktur-Komposition des Wirtskörpers dadurch nicht beeinflusst wird. Letzteres kann beispielsweise durch Verwendung von Niedrigtemperatur-Agarose (Low melting point agarose) gewährleistet werden.
3) Aufschließen des Wirtskörpers, z.B. im Falle einer Zelle als Wirtskörper Lysieren (z.B. mechanisch durch Ultraschall, Osmose, oder Gefrieren; oder chemisch beispielsweise durch Veränderung des pH-Werts, Einbringen von EDTA, Enzymen wie beispielsweise Lysozym, Toluol, Triton-X oder Trizol, was jeweils die Zellwände oder -membranen destabilisiert oder Autolyse induziert), so dass die m-RNAs aus dem Wirtskörper, z.B. der Zelle, austreten.

Die Polymermatrix schränkt dabei bei entsprechender Wahl der Konzentration der Wirtskörper (z.B. Zellen) und Dichte der Polymermatrix die Diffusion der aus dem Wirtskörper entlassenen Zielstrukturen so ein, dass sie nicht aus den zugehörigen Mikro-Wells herausdiffundieren. Das Polymer-Netzwerk weist bevorzugt eine mittlere Maschengröße von 1 μm bis 50 μm, bevorzugt 2 μm bis 10 μm auf. Dies dient zur Einschränkung der Diffusion, sowie zur Bereitstellung von ausreichend vielen Bindestellen für Träger-Adapter bzw. Träger-Adapter-Bindestellen. Der paarweise Mindestabstand der Träger-Adapter bzw. Träger-Adapter-Bindestellen kann im Wesentlichen 200 nm bis 10 μm, bevorzugt 500 nm bis 5 μm, besonders bevorzugt 2 μm bis 3 μm betragen. Im Wesentlichen heißt in diesem Zusammenhang, dass mindestens 80%, bevorzugt mindestens 90% und besonders bevorzugt mindestens 99% der Träger-Adapter bzw. Träger-Adapter-Bindestellen mindestens die oben angegebene Entfernung von benachbarten Träger-Adapter bzw. Träger-Adapter-Bindestellen haben.

Zur Analyse der Zielstruktur-Komposition einzelner Wirtskörper kann bevorzugt

(a) ein Wirtskörper in einem Mikro-Well mit einer Polymermatrix teilweise oder ganz umgeben werden;
(b) die Polymermatrix mit Elementen dekoriert werden, die an die Zielmoleküle binden können, wie beispielsweise komplementären Oligonukleotiden;
(c) Der Wirtskörper aufgeschlossen werden, sodass die Zielstrukturen herausdiffundieren und an die dekorierenden Elemente binden;
(d) 3D-DNA Nanostrukturen oder andere Elemente hinzugefügt und nach einer angemessenen Inkubationszeit ausgewaschen werden;
(e) Die Probe beispielsweise auf einem Epifluoreszenz-, Lichtblatt- oder konfokalen Mikroskop abgebildet und wie oben beschrieben analysiert werden.

Dabei ist wichtig zu beachten dass die Dekorationsdichte der Zielstruktur bindenden Elemente an der Polymermatrix gering genug sein sollte, damit die meisten Komplexe in der Abbildung nicht überlappen. Als Ausgangsstoff für die Polymermatrix bietet sich hier insbesondere Agarose, aber auch Collagen, DNA oder andere Materialien an.

Der Begriff 'Zellen' umfasst hier sämtliche biologische Organismen, insbesondere humane Zellen, Zellen aus Gewebeproben, Tier-Zellen, Bakterien, Fungi, Algen. Insbesondere gelten alle für Zellen beschriebenen Merkmale *mutatis mutandis* für andere Wirtsköper.

Der Begriff 'Matrix' umfasst ein Gel aus Agarose oder anderen Stoffen, deren Ziel es ist:

◦ Die Diffusion von Zielmolekülen zu beschränken, um den Zielstruktur-Verlust aus den Mikro-Wells durch Diffusion zu minimieren.
◦ Und/oder mehr Ankerpunkte für Zielstrukturen bereitzustellen, sodass mehr Zielstrukturen pro Mikro-Well analysiert werden können

Eine Genexpressionsanalyse mit Einzelzellgenauigkeit kann aufweisen:

1. Deponieren der zu untersuchenden Wirtskörper in einem Mikro-Well Array.
2. Umgeben der Wirtskörper mit einer Polymer-Matrix.

3. Aufschluss der Wirtskörper, so dass die Zielstrukturen austreten können.
4. Ausbilden von Identifizierungsstrukturen am Träger (Polymermatrix und/oder Oberfläche der 1. und 2. Schicht des Mikro-Wells).
5. Detektion der Identifizierungsstrukturen mit entsprechender Instrumentation
6. Analyse und Zuordnung der detektierten Identifizierungsstrukturen und der entsprechenden Zielstrukturen zu ihrem ursprünglichen Wirtskörper.

Die vorliegende Erfindung richtet sich unter anderem auch auf die nachfolgenden Aspekte:

1. Verfahren zum Nachweis (bevorzugt Quantifizierung) einer Zielstruktur, umfassend:

p1) Einbringen einer Gruppe von Wirtskörpern inklusive eines Wirtskörpers mit der Zielstruktur in ein Mikro-Well Array derart, dass in mindestens einem Mikro-Well genau ein Wirtskörper vorhanden ist;
p2) Einbringen von mindestens zwei 3D-DNA-Nanostrukturen in das mindestens eine Mikro-Well, wobei jede der 3D-DNA-Nanostrukturen ein oder mehrere innenliegende Fluoreszenz-Farbstoffmoleküle aufweist;

a) Ausbilden einer Identifizierungsstruktur in dem mindestens einen Mikro-Well, die aufweist:

(i) die Zielstruktur, und
(ii) die mindestens zwei 3D-DNA-Nanostrukturen, wobei jede der 3D-DNA-Nanostrukturen spezifisch an die Zielstruktur gebunden ist, und wobei die 3D-DNA-Nanostrukturen an paarweise unterschiedliche Regionen der Zielstruktur gebunden sind;

b) Nachweis der Zielstruktur durch Messen mindestens eines Fluoreszenzsignals,

wobei die 3D-DNA-Nanostrukturen und die Parameter der Fluoreszenzmessung derart ausgewählt sind, dass das mindestens eine gemessene Fluoreszenzsignal der in a) ausgebildeten Identifizierungsstruktur von dem Fluoreszenzsignal jeder der mindestens zwei isolierten 3D-DNA-Nanostrukturen unterscheidbar ist, wenn diese nicht in der Identifizierungsstruktur gebunden sind, wobei die Identifizierungsstruktur an eine erste Oberfläche, bevorzugt den Boden des mindestens einen Mikro-Wells gebunden ist.

2. Verfahren nach Aspekt 1, wobei die Zielstruktur ein teilweise einzelsträngiges Polynukleotid, bevorzugt ein einzelsträngiges Polynukleotid und besonders bevorzugt eine m-RNA aufweist oder ist.

3. Verfahren nach einem der vorhergehenden Aspekte, wobei die Identifizierungsstruktur an einen Träger, bevorzugt an eine erste Oberfläche des Trägers, gebunden ist und/oder wobei das Verfahren ferner den Schritt des Bindens der ausgebildeten Identifizierungsstruktur an einen Träger, bevorzugt an eine erste Oberfläche des Trägers umfasst.

4. Verfahren nach Aspekt 3, wobei der Träger bzw. die erste Oberfläche des Trägers eine Glasoberfläche und/oder Polymeroberfläche aufweist bzw. daraus besteht und der Träger bzw. die erste Oberfläche des Trägers optional passiviert ist und/oder wobei der Träger ein Polymernetzwerk aufweist, welches bevorzugt eines oder eine Kombination der folgenden Materialien aufweist: Biopolymer, Agarose, Collagen.

5. Verfahren nach Aspekt 3 oder 4, wobei der Träger ein Mikroskopiechip oder ein Deckglässchen ist.

6. Verfahren nach Aspekt 3 bis 5, wobei die Bindung bzw. das Binden der Identifizierungsstruktur an den Träger bzw. die erste Oberfläche des Trägers über die Zielstruktur vermittelt ist bzw. wird.

7. Verfahren nach Aspekt 6, wobei die Zielstruktur vermittelt durch einen Träger-Adapter, der an die Zielstruktur spezifisch bindet, an den Träger bzw. die erste Oberfläche des Trägers gebunden ist bzw. wird.

8. Verfahren nach Aspekt 7, wobei der Träger-Adapter direkt an den Träger oder die erste Oberfläche des Trägers gebunden ist bzw. wird.

9. Verfahren nach Aspekt 7 oder 8, wobei der Träger-Adapter mittels einer kovalenten oder mittels einer nicht kovalenten Bindung an den Träger oder die erste Oberfläche des Trägers gebunden ist bzw. wird.

10. Verfahren nach einem der Aspekte 7 bis 9, wobei der Träger-Adapter mittels einer Biotin-Streptavidin Bindung an den Träger oder die erste Oberfläche des Trägers gebunden ist bzw. wird, wobei entweder der Träger-Adapter Biotin aufweist und der Träger bzw. die erste Oberfläche des Trägers Streptavidin aufweist, oder der Träger-Adapter Streptavidin aufweist und der Träger bzw. die erste Oberfläche des Trägers Biotin aufweist.

11. Verfahren nach einem der Aspekte 7 bis 10, wobei die Zielstruktur eine Zielstruktur nach Aspekt 2 ist, und wobei der Träger-Adapter ein Oligonukleotid oder ein Polynukleotid ist, dessen Nukleinsäuresequenz so programmiert ist, dass es spezifisch, bevorzugt durch Hybridisierung, an einen ersten einzelsträngigen Abschnitt der Nukleinsäuresequenz der Zielstruktur nach Aspekt 2 bindet.

12. Verfahren nach einem der Aspekte 7 bis 10, wobei die Zielstruktur eine m-RNA mit Poly-(A)-Schwanz ist, und wobei der Träger-Adapter ein Oligonukleotid ist, dessen Nukleinsäuresequenz so programmiert ist, dass es spezifisch an den Poly-(A)-Schwanz der m-RNA bindet.

13. Verfahren nach einem der Aspekte 3 bis 12, wobei das Verfahren nach a) und vor b) ferner das Waschen des Trägers bzw. der ersten Trägeroberfläche mit einer Pufferlösung umfasst.

14. Verfahren nach einem der vorhergehenden Aspekte, wobei eine, bevorzugt mehrere und besonders bevorzugt alle der 3D-DNA-Nanostrukturen für eine direkte Bindung an die jeweilige(n) Region(en) der Zielstruktur programmiert ist/sind und direkt an das Zielmolekül gebunden ist/sind.

15. Verfahren nach einem der vorhergehenden Aspekte, wobei die spezifische Bindung mindestens einer, bevorzugt aller 3D-DNA-Nanostrukturen durch einen jeweils zugeordneten Ziel-Adapter vermittelt wird, wobei der bzw. jeder der Ziel-Adapter dazu programmiert ist, an die jeweilige DNA-Nanostruktur und an die jeweilige(n) Region(en) der Zielstruktur zu binden, und/oder der bzw. jeder der Ziel-Adapter bevorzugt ein Oligo- oder Polynukleotid, besonders bevorzugt DNA, aufweist oder ist.

16. Verfahren nach Aspekt 15, wobei die 3D-DNA-Nanostruktur(en) mindestens einen an oder auf der Außenseite der Nanostruktur angeordneten einzelsträngigen DNA-Abschnitt aufweist/aufweisen, der so programmiert ist, dass er an den jeweiligen Ziel-Adapter spezifisch bindet.

17. Verfahren nach einem des Aspekts 15 oder 16, wobei die eine oder mehreren Ziel-Adapter jeweils einen einzelsträngigen DNA-Abschnitt aufweisen, der jeweils so programmiert ist, dass er die spezifische Bindung an die jeweilige 3D-DNA-Nanostruktur vermittelt.

18, Verfahren nach einem der Aspekte 15 bis 17, wobei die eine oder mehreren Ziel-Adapter jeweils einen Zielbindungsabschnitt aufweisen, der jeweils so programmiert ist, dass er die spezifische Bindung an die jeweilige(n) Region(en) der Zielstruktur vermittelt.

19. Verfahren nach Aspekt 18, wobei die Zielstruktur die in Aspekt 2 definierte Zielstruktur ist, und wobei der jeweilige Zielbindungsabschnitt eine Nukleotidsequenz aufweist, die spezifisch an den einen einzelsträngigen Abschnitt der Zielstruktur bindet.

20. Verfahren nach Aspekt 18, wobei die Zielstruktur ein Protein umfasst oder ist, und wobei der jeweilige Zielbindungsabschnitt ein Peptid, bevorzugt einen Antikörper, aufweist, das/der an das Protein spezifisch bindet.

21. Verfahren nach einem der vorherigen Aspekte, wobei das Verfahren, ferner das Bereitstellen einer, bevorzugt wässrigen, Probenlösung, die die Zielstruktur enthält, und der mindestens zwei 3D-DNA-Nanostrukturen umfasst und optional ferner das Bereitstellen des Trägers, des Trägeradapters und/oder der Zieladapter umfasst, wobei die Probenlösung bevorzugt Zell-Lysat, aus Zellsuspension und/oder Gewebe extrahierte Nukleinsäuren und/oder mRNA aufweist.

22. Verfahren nach einem der vorherigen Aspekte, wobei das Verfahren ferner das Mischen einer, bevorzugt wässrigen, Probenlösung, die die Zielstruktur enthält, mit den mindestens zwei 3D-DNA-Nanostrukturen und optional dem Trägeradapter und/oder den Zieladaptern umfasst, wobei die Probenlösung bevorzugt Zell-Lysat, aus Zellsuspension und/oder Gewebe extrahierte Nukleinsäuren und/oder mRNA aufweist.

23. Verfahren nach einem der vorherigen Aspekte, wobei das Verfahren ferner zusätzlich für den Nachweis einer oder mehrerer weiterer, voneinander verschiedener Zielstrukturen geeignet ist, wobei sich die verschiedenen Zielstrukturen paarweise unterscheiden, und wobei das Verfahren ferner umfasst:

c) für jede der einen oder mehreren weiteren, voneinander verschiedenen Zielstrukturen: Ausbilden jeweils einer zugeordneten Identifizierungsstruktur, wobei jede der weiteren Identifizierungsstrukturen aufweist:

(i) die jeweils zugeordnete weitere Zielstruktur, und
(ii) mindestens zwei 3D-DNA-Nanostrukturen, wobei jede der mindestens zwei 3D-DNA-Nanostrukturen einen oder mehrere innenliegende Fluoreszenz-Farbstoffmoleküle aufweist und wobei jede der mindestens zwei 3D-DNA-Nanostrukturen spezifisch an die jeweilige weitere Zielstruktur gebunden ist, und wobei die mindestens zwei 3D-DNA-Nanostrukturen an paarweise unterschiedliche Regionen der jeweiligen Zielstruktur gebunden sind;

und wobei der Schritt b) ferner umfasst:
d) Nachweis der einen oder mehreren weiteren Zielstrukturen durch Messen des mindestens einen Fluoreszenzsignals,

wobei alle 3D-DNA-Nanostrukturen und die Parameter der Fluoreszenzmessung derart ausgewählt sind, dass das mindestens eine gemessene Fluoreszenzsignal der in a) und c) ausgebildeten Identifizierungsstrukturen von dem Fluoreszenzsignal aller isolierten 3D-DNA-Nanostrukturen unterscheidbar ist, wenn diese nicht in einer der Identifizierungsstrukturen gebunden sind, und dass die gemessenen Fluoreszenzsignale aller ausgebildeten Identifizierungsstrukturen paarweise voneinander unterscheidbar sind, wobei jede der verschiedenen Zielstrukturen mehrfach vorliegen kann und das Verfahren den mehrmaligen Nachweis einer oder mehrerer der verschiedenen Zielstrukturen umfassen kann.

24. Verfahren nach Aspekt 23, wobei die weiteren Zielstrukturen jeweils ein teilweise einzelsträngiges Polynukleotid, bevorzugt jeweils ein einzelsträngiges Polynukleotid und besonders bevorzugt jeweils eine m-RNA aufweisen oder sind.

25. Verfahren nach einem der Aspekte 23 oder 24, wobei mindestens eine, bevorzugt alle Identifizierungsstrukturen an einen Träger, bevorzugt an eine erste Oberfläche des Trägers, gebunden sind und/oder wobei das Verfahren ferner den Schritt des Bindens mindestens einer, bevorzugt aller ausgebildeter Identifizierungsstrukturen an einen Träger, bevorzugt an eine erste Oberfläche des Trägers, umfasst.

26. Verfahren nach Aspekt 25, wobei die Oberfläche des Trägers eine Glasoberfläche und/oder eine Polymeroberfläche aufweist oder ist und optional passiviert ist und/oder wobei der Träger ein Polymernetzwerk aufweist, welches bevorzugt eines oder eine Kombination der folgenden Materialien aufweist: Biopolymer, Agarose, Collagen.

27. Verfahren nach Aspekt 25 oder 26, wobei der Träger ein Mikroskopiechip oder ein Deckglässchen ist.

28. Verfahren nach Aspekt 25 bis 27, wobei die Bindung bzw. das Binden mindestens einer, bevorzugt aller Identifizierungsstruktur(en), die an den Träger bzw. die erste Oberfläche des Trägers gebunden sind/werden, über die jeweilige Zielstruktur vermittelt ist bzw. wird.

29. Verfahren nach Aspekt 28, wobei die jeweilige Zielstruktur vermittelt durch einen jeweils zugeordneten Träger-Adapter, der an die jeweilige Zielstruktur spezifisch bindet, an den Träger bzw. die erste Oberfläche des Trägers gebunden ist bzw. wird.

30. Verfahren nach Aspekt 29, wobei der jeweilige Träger-Adapter direkt an den Träger oder die erste Oberfläche des Trägers gebunden ist bzw. wird.

31. Verfahren nach Aspekt 29 oder 30, wobei der jeweilige Träger-Adapter mittels einer kovalenten oder mittels einer nicht kovalenten Bindung an den Träger oder die erste Oberfläche des Trägers gebunden ist bzw. wird.

32. Verfahren nach einem der Aspekte 29 bis 31, wobei der jeweilige Träger-Adapter mittels einer Biotin-Streptavidin Bindung an den Träger oder die erste Oberfläche des Trägers gebunden ist bzw. wird, wobei entweder der jeweilige Träger-Adapter Biotin aufweist und der Träger bzw. die erste Oberfläche des Trägers Streptavidin aufweist, oder der jeweilige Träger-Adapter Streptavidin aufweist und der Träger bzw. die erste Oberfläche des Trägers Biotin aufweist.

33. Verfahren nach einem der Aspekte 29 bis 32, wobei die jeweilige Zielstruktur eine Zielstruktur nach Aspekt 2 ist, und wobei der jeweilige Träger-Adapter ein Poly- oder Oligonukleotid ist, dessen Nukleinsäuresequenz so programmiert ist, dass es spezifisch an einen ersten einzelsträngigen Abschnitt der Nukleinsäuresequenz der jeweiligen Zielstruktur nach Aspekt 2 bindet.

34. Verfahren nach einem der Aspekte 29 bis 33, wobei die an den Träger bzw. die erste Oberfläche des Trägers gebundene(n) Zielstruktur(en) eine m-RNA mit Poly-(A)-Schwanz ist/sind, und wobei der jeweilige Träger-Adapter für diese gebundene(n) Zielstruktur(en) ein Oligonukleotid ist, dessen Nukleinsäuresequenz so programmiert ist, dass es spezifisch an den Poly-(A)-Schwanz der m-RNA bindet.

35. Verfahren nach einem der Aspekte 23 bis 34, wobei a) und c) in einem Schritt, bevorzugt gleichzeitig, ablaufen.

36. Verfahren nach einem der Aspekte 25 bis 35, wobei das Verfahren ferner nach a) und c) und vor b) das Waschen des Träger bzw. der ersten Trägeroberfläche mit einer Pufferlösung umfasst.

37. Verfahren nach einem der Aspekte 23 bis 36, wobei eine, bevorzugt mehrere und besonders bevorzugt alle der 3D-DNA-Nanostrukturen für eine direkte Bindung an die jeweilige(n) Region(en) der jeweiligen Zielstruktur programmiert ist/sind und direkt an das Zielmolekül gebunden ist/sind.

38. Verfahren nach einem der Aspekte 23 bis 37, wobei die spezifische Bindung mindestens einer, bevorzugt aller 3D-DNA-Nanostruktur(en) durch einen jeweils zugeordneten Ziel-Adapter vermittelt wird, wobei der bzw. jeder der Ziel-Adapter dazu programmiert ist, an die jeweilige DNA-Nanostruktur und an die jeweilige(n) Region(en) der jeweiligen Zielstruktur zu binden.

39. Verfahren nach Aspekt 38, wobei die mindestens eine, bevorzugt alle 3D-DNA-Nanostruktur(en) mindestens einen an oder auf der Außenseite der Nanostruktur angeordneten einzelsträngigen DNA-Abschnitt aufweist/aufweisen, der so programmiert ist, dass er den jeweiligen Ziel-Adapter spezifisch bindet.

40. Verfahren nach einem des Aspekts 38 oder 39, wobei die ein oder mehreren Ziel-Adapter jeweils einen einzelsträngigen DNA-Abschnitt aufweisen, der jeweils so programmiert ist, dass er die spezifische Bindung an die jeweilige 3D-DNA-Nanostruktur vermittelt.

41. Verfahren nach einem der Aspekte 38 bis 40, wobei die ein oder mehreren Ziel-Adapter jeweils einen Zielbindungsabschnitt aufweisen, der jeweils so programmiert ist, dass er die spezifische Bindung an die jeweilige(n) Region(en) der Zielstruktur vermittelt.

42. Verfahren nach einem der Aspekte 23 bis 41, wobei alle Zielstrukturen in derselben Probe vorliegen und bevorzugt in einer Probenlösung bereitgestellt werden.

43. Verfahren nach einer der vorhergehenden Aspekte, wobei das Messen des mindestens einen Fluoreszenzsignals mittels Fluoreszenzmikroskopie, bevorzugt durch eine oder eine Kombination der folgenden Techniken erfolgt: Punktscannen, Weitfeldmikroskopie, (Spinning-Disc-)Konfokalmikroskopie; wobei jeweils bevorzugt eine alternierende Anregung (ALEX) oder eine Anregung mittels gepulster Lichtquellen pulse-interleaved erfolgt.

44. Verfahren nach einem der Aspekte 1, 2, 14 bis 24, 35, oder 37 bis 42, wobei das Messen des mindestens einen Fluoreszenzsignals mittels Durchflusszytometrie und/oder Fluoreszenzkorrelationsspektrometrie (FCS), bevorzugt durch eine oder eine Kombination der folgenden Techniken erfolgt: Konfokalmikroskopie, Zweiphotonenspektroskopie, Epifluoreszenz, Lichtblattmikroskopie, TIRF; wobei jeweils bevorzugt eine alternierende Anregung (ALEX) oder eine Anregung mittels gepulster Lichtquellen pulse-interleaved erfolgt.

45. Verfahren nach einem der vorhergehenden Aspekte, wobei die Identifizierungsstruktur bzw. mindestens eine oder alle der Identifizierungsstrukturen mehrfach ausgebildet wird.

46. Verfahren zum Nachweis von mindestens zwei verschiedenen Zielstrukturen, wobei die mindestens zwei verschiedenen Zielstrukturen paarweise von einander unterscheidbar sind und das Verfahren umfasst:

p1) Einbringen einer Gruppe von Wirtskörpern in ein Mikro-Well Array derart, dass in mindestens einem Mikro-Well genau ein Wirtskörper vorhanden ist; wobei für jede der Zielstrukturen die Gruppe von Wirtskörpern mindestens einen Wirtskörper mit der jeweiligen Zielstruktur aufweist;

p2) Einbringen von mindestens zwei 3D-DNA-Nanostrukturen in das mindestens eine Mikro-Well, wobei jede der 3D-DNA-Nanostrukturen ein oder mehrere innenliegende Fluoreszenz-Farbstoffmoleküle aufweist;

a) Ausbilden jeweils einer Identifizierungsstruktur für jede der mindestens zwei verschiedenen Zielstrukturen, die aufweist:

(i) die jeweilige Zielstruktur, und

(ii) mindestens zwei 3D-DNA-Nanostrukturen, wobei jede der mindestens zwei 3D-DNA-Nanostrukturen ein oder mehrere innenliegende Fluoreszenz-Farbstoffmoleküle aufweist und wobei jede der mindestens zwei 3D-DNA-Nanostrukturen spezifisch an die jeweilige Zielstruktur gebunden ist, und wobei die mindestens zwei 3D-DNA-Nanostrukturen an paarweise unterschiedliche Regionen der jeweiligen Zielstruktur gebunden sind;

b) Nachweis der mindestens zwei verschiedenen Zielstrukturen durch Messen mindestens eines Fluoreszenzsignals,

wobei alle 3D-DNA-Nanostrukturen und die Parameter der Fluoreszenzmessung derart ausgewählt sind, dass das mindestens eine gemessene Fluoreszenzsignal der in a) ausgebildeten Identifizierungsstrukturen von dem Fluoreszenzsignal aller isolierten 3D-DNA-Nanostrukturen unterscheidbar ist, wenn diese nicht in einer der jeweiligen Identifizierungsstruktur gebunden sind, und dass die gemessenen Fluoreszenzsignale der für die einzelnen Zielstrukturen ausgebildeten verschiedenen Identifizierungsstrukturen paarweise voneinander unterscheidbar sind, wobei jede der verschiedenen Zielstrukturen mehrfach vorliegen kann und das Verfahren den mehrmaligen Nachweis einer oder mehrerer der verschiedenen Zielstrukturen umfassen kann.

47. Verfahren nach Aspekt 46, wobei die mindestens zwei Zielstrukturen in derselben Probe vorliegen.

48. Verfahren nach Aspekt 1, wobei mindestens eine, bevorzugt jede, der Zielstrukturen ein teilweise einzelsträngiges Polynukleotid, bevorzugt ein einzelsträngiges Polynukleotid und besonders bevorzugt eine m-RNA aufweist oder ist.

49. Verfahren nach einem der Aspekte 46 bis 48, wobei mindestens eine, bevorzugt alle Identifizierungsstruktur(en) an einen Träger, bevorzugt an eine erste Oberfläche des Trägers, gebunden ist und/oder wobei das Verfahren ferner den Schritt des Bindens mindestens einer, bevorzugt aller ausgebildeten Identifizierungsstruktur(en) an einen Träger, bevorzugt eine erste Oberfläche des Trägers umfasst.

50. Verfahren nach Aspekt 49, wobei der Träger bzw. die erste Oberfläche des Trägers eine Glasoberfläche und/oder Polymeroberfläche aufweist bzw. daraus besteht und optional passiviert ist.

51. Verfahren nach Aspekt 49 oder 50, wobei der Träger ein Mikroskopiechip oder ein Deckglässchen ist.

52. Verfahren nach Aspekt 49 bis 51, wobei die Bindung bzw. das Binden mindestens einer, bevorzugt aller Identifizierungsstruktur(en), die an den Träger bzw. die erste Oberfläche des Trägers gebunden sind/werden, über die jeweilige Zielstruktur vermittelt ist bzw. wird.

53. Verfahren nach Aspekt 52, wobei die jeweilige Zielstruktur vermittelt durch einen jeweils zugeordneten Träger-Adapter, der die jeweilige Zielstruktur spezifisch bindet, an den Träger bzw. die erste Oberfläche des Trägers gebunden ist bzw. wird.

54. Verfahren nach Aspekt 53, wobei der jeweilige Träger-Adapter direkt an den Träger oder die erste Oberfläche des Trägers gebunden ist bzw. wird.

55. Verfahren nach Aspekt 53 oder 54, wobei der jeweilige Träger-Adapter mittels einer kovalenten oder mittels einer nicht kovalenten Bindung an den Träger oder die erste Oberfläche des Trägers gebunden ist bzw. wird.

56. Verfahren nach einem der Aspekte 53 bis 55, wobei der jeweilige Träger-Adapter mittels einer Biotin-Streptavidin Bindung an den Träger oder die erste Oberfläche des Trägers gebunden ist bzw. wird, wobei entweder der jeweilige Träger-Adapter Biotin aufweist und der Träger bzw. die erste Oberfläche des Trägers Streptavidin aufweist, oder der jeweilige Träger-Adapter Streptavidin aufweist und der Träger bzw. die erste Oberfläche des Trägers Biotin aufweist.

57. Verfahren nach einem der Aspekte 53 bis 56, wobei die jeweilige Zielstruktur eine Zielstruktur nach Aspekt 2 ist, und wobei der jeweilige Träger-Adapter ein Oligonukleotid ist, dessen Nukleinsäuresequenz so programmiert ist, dass es spezifisch an einen ersten einzelsträngigen Abschnitt der Nukleinsäuresequenz der jeweiligen Zielstruktur nach Aspekt 2 bindet.

58. Verfahren nach einem der Aspekte 53 bis 57, wobei die an den Träger bzw. die erste Oberfläche des Trägers gebundene(n) Zielstruktur(en) eine m-RNA mit Poly-(A)-Schwanz ist/sind, und wobei der jeweilige Träger-Adapter für diese gebundene(n) Zielstruktur(en) ein Oligonukleotid ist, dessen Nukleinsäuresequenz so programmiert ist, dass es spezifisch an den Poly-(A)-Schwanz der m-RNA bindet.

59. Verfahren nach einem der Aspekte 49 bis 58, wobei das Verfahren ferner nach a) und vor b) das Waschen des Träger bzw. der ersten Trägeroberfläche mit einer Pufferlösung umfasst.

60. Verfahren nach einem der Aspekte 46 bis 59, wobei eine, bevorzugt mehrere und besonders bevorzugt alle der 3D-DNA-Nanostrukturen für eine direkte Bindung an die jeweilige(n) Region(en) der jeweiligen Zielstruktur programmiert ist/sind und direkt an das Zielmolekül gebunden ist/sind.

61. Verfahren nach einem der Aspekte 46 bis 60, wobei die spezifische Bindung mindestens einer, bevorzugt aller 3D-DNA-Nanostruktur(en) durch einen jeweils zugeordneten Ziel-Adapter vermittelt wird, wobei der bzw. jeder der Ziel-Adapter dazu programmiert ist an die jeweilige DNA-Nanostruktur und an die jeweilige(n) Region(en) der jeweiligen Zielstruktur zu binden.

62. Verfahren nach Aspekt 61, wobei die mindestens eine, bevorzugt alle 3D-DNA-Nanostruktur(en) mindestens einen nach außen gerichtete einzelsträngigen DNA-Abschnitt aufweist/aufweisen, der so programmiert ist, dass er den jeweiligen Ziel-Adapter spezifisch bindet.

63. Verfahren nach einem des Aspekts 61 oder 62, wobei die ein oder mehreren Ziel-Adapter jeweils einen einzelsträngigen DNA-Abschnitt aufweisen, der jeweils so programmiert ist, dass er die spezifische Bindung an die jeweilige 3D-DNA-Nanostruktur vermittelt.

64. Verfahren nach einem der Aspekte 61 bis 63, wobei die ein oder mehreren Ziel-Adapter jeweils einen Zielbindungsabschnitt aufweisen, der jeweils so programmiert ist, dass er die spezifische Bindung an die jeweilige(n) Region(en) der Zielstruktur vermittelt.

65. Verfahren nach Aspekt 64, wobei eine erste der mindestens zwei Zielstrukturen die in Aspekt 48 definierte Zielstruktur ist, und wobei der Zielbindungsabschnitt des an die erste Zielstruktur bindenden Zielbindungsadapters eine Nukleotidsequenz aufweist, die spezifisch an den einen einzelsträngigen Abschnitt der ersten Zielstruktur bindet.

66. Verfahren nach Aspekt 65, wobei das Merkmal nach Aspekt 63 auch für alle weiteren Zielstrukturen und die jeweiligen Zielbindungsabschnitte der jeweiligen Zielbindungsadapter zutrifft.

67. Verfahren nach Aspekt 64, wobei eine erste der mindestens zwei Zielstrukturen ein Protein umfasst oder ist, und wobei der Zielbindungsabschnitt des an die erste Zielstruktur bindenden Zielbindungsadapters ein Peptid, bevorzugt einen Antikörper, aufweist, das/der an die erste Zielstruktur spezifisch bindet.

68. Verfahren nach Aspekt 67, wobei das Merkmal nach Aspekt 63 auch für alle weiteren Zielstrukturen und die jeweiligen Zielbindungsabschnitte der jeweiligen Zielbindungsadapter zutrifft.

69. Verfahren nach einem der Aspekte 46 bis 68, wobei das Verfahren ferner das Bereitstellen einer Probenlösung, die die mindestens zwei Zielstrukturen enthält, und der mindestens zwei 3D-DNA-Nanostrukturen umfasst und optional ferner das Bereitstellen des Trägers, des/der Trägeradapter(s) und/oder des/der Zieladapter(s) umfasst.

70. Verfahren nach einem der Aspekte 46 bis 69, wobei das Verfahren ferner das Mischen einer Probenlösung, die die mindestens zwei Zielstrukturen enthält, mit den mindestens zwei 3D-DNA-Nanostrukturen und optional dem/den Trägeradapter(n) und/oder dem/den Zieladapter(n) umfasst.

71. Verfahren nach einem der vorhergehenden Aspekte, wobei das Messen mindestens eines Fluoreszenzsignals umfasst:
q) Erstellen eines Datensatzes, der Daten betreffend von einem Ausschnitt einer Probe emittierte Fluoreszenzsignale enthält, mittels einem Fluoreszenzmikroskop, bevorzugt in Epifluoreszenz-, TIRF-, Lichtblatt- und/oder Konfokalmikroskopie;
und wobei der Nachweis der Zielstruktur bzw. der einen oder mehreren weiteren Zielstrukturen umfasst:
w) Identifizieren eines oder mehrerer in dem Datensatz enthaltenen Datums, das bzw. die das Fluoreszenzsignal einer der Identifizierungsstrukturen repräsentiert.

72. Verfahren nach Aspekt 71, wobei Schritt q) umfasst: Aufnehmen mindestens einer Bilddatei, die Fluoreszenzdaten des Ausschnitts der Probe enthalten.

73. Verfahren nach Aspekt 71 oder 72, wobei das Identifizieren in Schritt w) die folgenden Schritte umfasst:

w1) Auslesen einer Farb- und/oder einer Intensitätsinformation eines Datums bzw. Bildelements; und
w2) Abgleichen der Farb- und/oder Intensitätsinformation des Datums bzw. Bildelements mit einer Farb- und/oder Intensitätsinformation, die die Identifizierungsstruktur repräsentiert.

74. Verfahren nach Aspekt 73, wobei das Identifizieren in Schritt w) die folgenden Schritte umfasst:

w1) Auslesen einer Farb- und einer Intensitätsinformation eines Datums bzw. Bildelements; und
w2) Abgleichen der Farb- und Intensitätsinformation des Datums bzw. Bildelements mit einer Farb- und Intensitätsinformation, die die Identifizierungsstruktur repräsentiert.

75. Verfahren nach Aspekt 73 oder 74, wobei sich die Fluoreszenzsignale der für die einzelnen verschiedenen Zielstrukturen ausgebildeten Identifizierungsstrukturen von dem Fluoreszenzsignal aller isolierter 3D-DNA-Nanostrukturen, wenn diese nicht in einer der Identifizierungsstrukturen gebunden sind, und die Fluoreszenzsignale der für die einzelnen verschiedenen Zielstrukturen ausgebildeten Identifizierungsstrukturen paarweise voneinander dadurch unterscheiden, dass die entsprechenden Fluoreszenzsignale eine unterscheidbar andere Kombination aus Farb- und/oder Intensitätsinformation aufweist.

76. Verfahren nach Aspekt 75, wobei in den 3D-DNA-Nanostrukturen k voneinander unterscheidbarer Intensitätslevel und/oder m voneinander unterscheidbarer Farblevel zum Einsatz kommen, wobei bevorzugt jedes der k Intensitätslevel durch eine Intensitätsverteilung gebildet wird und wobei die k Intensitätsverteilungen, bevorzugt statistisch, voneinander unterscheidbar sind und/oder wobei bevorzugt jedes der m Farblevel durch eine Farbverteilung gebildet wird und wobei die m Farbverteilungen, bevorzugt statistisch, voneinander unterscheidbar sind, wobei jeweils der Überlapp benachbarter Verteilungen bevorzugt kleiner als 30 %, stärker bevorzugt kleiner als 10 %, noch stärker bevorzugt kleiner als 5 %, besonders bevorzugt kleiner als 2 % und ganz besonders bevorzugt kleiner als 1 % ist.

77. Verfahren nach Aspekt 76, wobei $k>2$, bevorzugt $k>3$, stärker bevorzugt $k>4$, noch stärker bevorzugt $k>5$, besonders bevorzugt $k>6$.

78. Verfahren nach Aspekt 76 oder 77, wobei $m>2$, bevorzugt $m>3$, stärker bevorzugt $m>4$, noch stärker bevorzugt $m>5$, besonders bevorzugt $m>6$.

79. Verfahren nach einem der Aspekte 73 bis 78, wobei der Schritt w1) einen oder eine Kombination der folgenden Schritte bzw. Techniken aufweist: Bestimmen eines Mittelwerts des Bildelements, Bestimmen eines Maximums des Bildelements, Berechnen eines kummulativen Gradienten, Berechnen eines oder mehrere statistischer Momente wie z.B. Varianz der Pixel im Bildelement, Varianzkoeffizient, Fano Faktor; und/oder wobei der Schritt w2) basierend auf einer Clustering-Methode, bevorzugt einer DBSCAN (density based spatial clustering of applications with noise) Methode erfolgt; und/oder wobei schritt w1) und/oder w2) auf probabilistischer Betrachtung, bevorzugt unter Hinzunahme von Bayes' Theorem, basiert.

80. 3D-DNA-Nanostruktur, auf der mindestens ein Fluoreszenz-Farbstoffmolekül angebracht ist, wobei die Form der 3D-DNA-Nanostruktur verhindert, dass bei Annäherung an eine zweite 3D-DNA-Nanostruktur, auf der mindestens ein Fluoreszenz-Farbstoffmolekül angebracht ist, die Fluoreszenz-Farbstoffmoleküle der beiden 3D-DNA-Nanostrukturen signifikant wechselwirken.

81. 3D-DNA-Nanostruktur nach Aspekt 80, wobei das Wechselwirken Fluoreszenzauslöschung und/oder FRET umfasst.

82. 3D-DNA-Nanostruktur nach Aspekt 80 oder 81, wobei auf der 3D-DNA-Nanostruktur mindestens zwei Fluoreszenz-Farbstoffmoleküle angebracht sind, und wobei der Abstand zwischen den mindestens zwei Fluoreszenz-Farbstoffmolekülen paarweise größer als derjenige ist, bei dem sie signifikant wechselwirken.

83. 3D-DNA-Nanostruktur nach einem der Aspekte 80 bis 82, wobei die 3D-DNA-Nanostruktur im Wesentlichen als Hohlzylinder ausgebildet ist, und wobei das mindestens eine Fluoreszenz-Farbstoffmolekül oder die mindestens zwei Fluoreszenz-Farbstoffmoleküle im Inneren der zylinderförmigen DNA-Nanostruktur angebracht sind.

84. 3D-DNA-Nanostruktur mit einem Hohlraum und mindestens einem innenliegenden Fluoreszenz-Farbstoffmolekül, wobei der Abstand des mindestens einen innenliegenden Fluoreszenz-Farbstoffmoleküls zum Rand der 3D-DNA-Nanostruktur mindestens 2 nm, bevorzugt mindestens 3 nm und besonders bevorzugt mindestens 5 nm beträgt.

85. 3D-DNA-Nanostruktur nach Aspekt 84, wobei die 3D-DNA-Nanostruktur mindestens zwei innenliegende Fluoreszenz-Farbstoffmoleküle aufweist, und wobei der Abstand der mindestens zwei innenliegenden Fluoreszenz-Farbstoffmoleküle mindestens 2 nm, bevorzugt mindestens 5 nm und besonders bevorzugt mindestens 9 nm beträgt.

86. 3D-DNA-Nanostruktur nach einem der Aspekte 80 bis 85, wobei die 3D-DNA-Nanostruktur im Wesentlichen als elliptischer Hohlzylinder, bevorzugt als Kreishohlzylinder ausgebildet ist.

87. 3D-DNA-Nanostruktur nach Aspekt 86, wobei der Hohlzylinder ein Kreiszylinder ist und einen Innenradius von mindestens 5 nm, bevorzugt einen Innenradius von 30 nm bis 60 nm, besonders bevorzugt einen Innenradius von 60 nm und/oder eine Höhe von maximal 200 nm, bevorzugt eine Höhe von 30 nm bis 60 nm, besonders bevorzugt eine Höhe von 30 nm aufweist.

88. 3D-DNA-Nanostruktur nach einem der Aspekte 86 bis 87, wobei der Hohlzylinder eine Wandstärke von mindestens 2 nm, bevorzugt 2 nm bis 7 nm, besonders bevorzugt 5 nm aufweist und/oder wobei der Hohlzylinder DNA-Helices mit einem Helixdurchmesser aufweist und wobei die Wandstärke mindestens einen, bevorzugt mindestens zwei Helixdurchmesser misst und/oder wobei die Wandstärke höchstens vier, bevorzugt höchstens drei Helixdurchmesser misst.

89. 3D-DNA-Nanostruktur nach einem der Aspekte 80 bis 88, wobei die 3D-DNA-Nanostruktur einen einzelsträngigen DNA-Gerüststrang aus mindestens 3000 Basen, bevorzugt 5000 - 50000 Basen, besonders bevorzugt 10000 - 11000 Basen aufweist, wobei der DNA-Gerüststrang bevorzugt zirkulär ist.

90. 3D-DNA-Nanostruktur nach Aspekt 89, wobei die 3D-DNA-Nanostruktur ferner eine Vielzahl von Klammersträngen aufweist, bevorzugt 100 bis 500 Klammerstränge mit bevorzugt je 30 bis 100 Basen umfasst.

91. 3D-DNA-Nanostruktur nach Aspekt 90, wobei eines der innenliegenden Fluoreszenz-Farbstoffmoleküle an einen innen liegender Abschnitt eines Klammerstrangs gebunden ist, bevorzugt wobei jeder der innenliegenden Fluoreszenz-Farbstoffmoleküle an jeweils einen innen liegender Abschnitt eines Klammerstrangs gebunden ist,

92. 3D-DNA-Nanostruktur nach einem der Aspekte 89 bis 91, ferner mindestens ein innenliegendes einzelsträngiges Farbstoffadapter-DNA-Oligomer aufweisend, das so programmiert ist, dass es spezifisch an einen innenliegenden einzelsträngigen Sequenzabschnitt des Gerüststrangs oder eines der Klammerstränge gebunden ist, und wobei das mindestens eine Farbstoffadapter-DNA-Oligomer eines der innenliegenden Farbstoffmoleküle aufweist.

93. 3D-DNA-Nanostruktur nach Aspekt 92, wobei der innenliegende einzelsträngige Sequenzabschnitt des Klammerstrangs ein Überhang ist, der nach innen ausgerichtet ist und der nicht zur Ausbildung der 3D-DNA-Nanostruktur nötig ist.

94. 3D-DNA-Nanostruktur nach einem der Aspekte 80 bis 93, wobei die 3D-DNA-Nanostruktur einen einzelsträngigen DNA-Abschnitt aufweist, der spezifisch an eine Zielstruktur bevorzugt eine Polynukleoid-Zielstruktur und besonders bevorzugt an eine einzelsträngiges Polynukleotid-Zielstruktur binden kann.

95. 3D-DNA-Nanostruktur nach einem der Aspekte 80 bis 94, wobei die 3D-DNA-Nanostruktur ferner mindestens einen Ziel-Adapter aufweist, wobei der Zieladapter die spezifische Bindung an einen Zieladapter vermitteln kann.

96. 3D-DNA-Nanostruktur nach Aspekt 95, wobei der Ziel-Adapter ein erstes einzelsträngiges Polynukleotidsequenz aufweist, und wobei die erste einzelsträngige Polynukleotidsequenz an einen einzelsträngigen Sequenzabschnitt der 3D-DNA-Nanostruktur, bevorzugt an einen einzelsträngigen Sequenzabschnitt des Gerüststrangs oder eines der Klammerstränge und besonders bevorzugt an einem einzelsträngigen Sequenzabschnitt eines der Klammerstränge gebunden ist

97. 3D-DNA-Nanostruktur nach Aspekt 95 oder 96, wobei der Ziel-Adapter ferner einen Zielbindungsabschnitt aufweist, der eine Zielstruktur spezifisch binden kann.

98. 3D-DNA-Nanostruktur nach Aspekt 97, wobei der Zielbindungsabschnitt eine zweite einzelsträngige Polynukleotidsequenz aufweist oder ist, und wobei die Zielstruktur ein einzelsträngiges Polynukleotid, bevorzugt eine m-RNA aufweist..

99. 3D-DNA-Nanostruktur nach Aspekt 97, wobei der Zielbindungsabschnitt ein Protein, das spezifisch an die Zielstruktur bindet, bevorzugt einen Antikörper oder eine antikörperbindendes Fragment eines Antikörpers aufweist.

100. 3D-DNA-Nanostruktur nach einem der Aspekte 95 bis 99, wobei der Zieladapter, bevorzugt mindestens der Zielbindungsabschnitt nach außen weist.

101. 3D-DNA-Nanostruktur nach einem der Aspekte 80 bis 100, wobei die 3D-Nanostruktur keine nach außenliegenden Fluoreszenz-Farbstoffmoleküle aufweist.

102. Verwendung der 3D-DNA-Nanostruktur nach einem der Aspekte 80 bis 101 für ein Verfahren nach einem der Aspekte 1-79.

103. Set aus mehreren 3D-DNA-Nanostrukturen nach einem der Aspekte 80 bis 101, wobei das Set N voneinander verschiedene 3D-DNA-Nanostrukturen aufweist und wobei sich die N voneinander verschiedenen 3D-DNA-Nanostrukturen des Sets durch die Fluoreszenzfarbstoffmoleküle paarweise voneinander unterscheiden.

104. Set nach Aspekt 103, wobei die N voneinander verschiedenen 3D-DNA-Nanostrukturen eine unterschiedliche Anzahl von Fluoreszenzfarbstoffmolekülen und/oder unterschiedliche Fluoreszenzfarbstoffmoleküle enthalten, so dass mit den N voneinander verschiedenen 3D-DNA-Nanostrukturen des Sets k voneinander unterscheidbarer Intensitätslevel und/oder m voneinander unterscheidbarer Farblevel erzeugt werden können.

105. Set nach Aspekt 104, wobei mindestens ein Teil der N voneinander verschiedenen 3D-DNA-Nanostrukturen mehrmals in dem Set enthalten ist, so dass jedes der k Intensitätslevel durch eine Intensitätsverteilung gebildet wird und wobei die k Intensitätsverteilungen, bevorzugt statistisch, voneinander unterscheidbar sind.

106. Set nach Aspekt 105 oder 106, wobei mindestens ein Teil der N voneinander verschiedenen 3D-DNA-Nanostrukturen mehrmals in dem Set enthalten ist, so dass jedes der m Farblevel durch eine Farbverteilung gebildet wird und wobei die m Farbverteilungen, bevorzugt statistisch, voneinander unterscheidbar sind.

107. Set nach Aspekt 105 oder 106, wobei der Überlapp benachbarter Verteilungen kleiner als 30 %, bevorzugt kleiner als 10 %, stärker bevorzugt kleiner als 5 %, noch stärker bevorzugt kleiner als 2 % und besonders bevorzugt kleiner als 1 % ist.

108. Set nach einem der Aspekte 104 bis 107, wobei $k>2$, bevorzugt $k>3$, stärker bevorzugt $k>4$, noch stärker bevorzugt $k>5$, besonders bevorzugt $k>6$.

109. Set nach einem der Aspekte 104 bis 108, wobei $m>2$, bevorzugt $m>3$, stärker bevorzugt $m>4$, noch stärker bevorzugt $m>5$, besonders bevorzugt $m>6$.

110. Verwendung des Sets nach einem der Aspekte 103 bis 109 für ein Verfahren nach einem der Aspekte 1-79.

Bevorzugte Ausführungsformen der Erfindung werden nachfolgend unter Bezugnahme auf die Figuren näher erläutert. Es zeigen:

Fig. 1 schematisch das der Erfindung zugrundeliegende Prinzip für den exemplarischen Fall der Genexpressionsanalyse;
Fig. 2 schematisch den Ablauf einer Einzelzell-Genexpressionsanalyse gemäß einer bevorzugten Ausführungsform des erfindungsgemäßen Verfahrens;
Fig. 3 die Ergebnisse eines Experiments;
Fig. 4 eine 3D-DNA-Nanostruktur gemäß einer bevorzugten Ausführungsform,
Fig. 5 schematisch ein Mikro-Well Array gemäß einer bevorzugten Ausführungsform;
Fig. 6 schematisch eine Mikro-Well Kammer gemäß einer bevorzugten Ausführungsform; und
Fig. 7 schematisch Verfahrensschritte zur Einzelzell-Genexpressionsanalyse.

Fig. 1 zeigt schematisch das der Erfindung zugrundeliegende Prinzip für den exemplarischen Fall der Genexpressionsanalyse. Die im dargestellten Fall zylinderförmige 3D-DNA-Nanostruktur 1d (vgl. Fig. 1 oben) mit innenliegenden Fluroeszenz-Farbstoffmolekülen 1b (in einem Abstand 1c) wird, wie schematisch angedeutet, durch Krümmen bzw. Rollen einer die Zylinderwand bildenden 2D-DNA-Nanostruktur 1a geformt und der Zylinder mittels entsprechender Klammerstränge in seiner Form stabilisiert. Der Zylinder weist einen an diesen gekoppelten Adapter 1f auf, mittels dessen die 3D-DNA-Nanostruktur 1d an eine Zielstruktur 1g binden kann (vgl. Fig. 1 unten), hier an mRNA mittels Hybridisierung. Das Bezugszeichen 1h soll beispielhaft eine Watson-Crick-Basenpaarung zur spezifischen Bindung von 3D-DNA-Nanostruktur und Zielstruktur andeuten. Die angegebene Sequenz soll nur beispielhaft den Mechanismus verdeutlichen und spiegelt nicht die tatsächliche Sequenz wider. In eckigen Klammern 1i ist die optionale Situation dargestellt, dass die Zielstruktur über den Adapter an ein Substrat 1j gekoppelt ist.
In Fig. 1 unten sind beispielhaft drei 3D-DNA-Nanostrukturen 1e an die Zielstruktur 1g gebunden, wobei sich die unter Fluoreszenzbedingungen gemessenen Intensitäten der drei 3D-DNA-Nanostrukturen 1e voneinander unterscheiden, was durch "HELL", "SCHWACH" und "MITTEL" angedeutet und durch einer verschiedenen Anzahl in den 3D-DNA-Nanostrukturen vorhandenen Fluoreszenzmolekülen verursacht ist.

Fig. 2 zeigt schematisch den Ablauf einer Einzelzell-Genexpressionsanalyse gemäß einer bevorzugten Ausführungsform des erfindungsgemäßen Verfahrens. Eine in ein durch eine 2. Schicht 2j begrenztes Mikro-Well 2a vereinzelte Zelle 2c (hier ist der Wirtskörper also eine Zelle) befinden sich unter und/oder in einer Polymermatrix 2b. Ein Zoom in die Polymermatrix (vgl. untere Abbildung) zeigt ein Polymerfilament 2g dieser Matrix, das mit Träger-Adaptern 2h besetzt ist. Von Schritt 1 zu Schritt 2 werden die Zellen lysiert, sodass Zielstrukturen, z.B. mRNAs (2d), sowie andere Zellbestandteile (2e) sich frei bewegen können. Zielstrukturen binden an die Träger-Adapter und bilden Zielstruktur-Träger-Adapter-Komplexe 2i. In Schritt 3 werden einige der anderen Zellbestandteile weggespült. Schritt 4 zeigt die hinzugefügten 3D-DNA-Nanostrukturen 2f mit Ziel-Adaptern, die an die Zielstrukturen binden. Durch die hohe örtliche Dichte der Träger-Adapter können die Zielstrukturen einzelner Zellen besonders gut abgefangen werden. Insbesondere behindert die Polymermatrix eine Diffusion der Zielstrukturen aus ihren Mikro-Wells heraus und/oder in andere Mikro-Wells hinein. Aufgrund der Dreidimensionalität der Polymermatrix können dicht angeordnete Identifizierungsstrukturen dennoch, beispielsweise mittels Konfokalmikroskopie, gut separat voneinander abgebildet werden.

Fig. 4A zeigt schematisch eine 3D-DNA-Nanostruktur gemäß einer bevorzugten Ausführungsform in Form eines Hohlzylinders mit einem Durchmesser d von 60 nm und einer Höhe h von 30 nm. Der Zylinder wird im dargestellten Beispiel durch 22 DNA-Doppelhelices mit einem Durchmesser von etwa 2 nm gebildet, wobei jeder Kreis den Querschnitt durch eine Helix symbolisieren soll. Wie gut zu erkennen ist, hat der Hohlzylinder keine glatte (oder gar mathematisch perfekte) Außenwand. Vielmehr weist die Außenwand Vorsprünge und Vertiefungen in Umfangsrichtung auf. Selbiges gilt für die Innenwand. Dabei sind die Helices auf einer gitterartigen oder honigwabenförmigen Struktur angeordnet, so dass die Wandstärke b etwa 2,5 Helixdurchmessern (oder etwa 5 nm) entspricht.

Figur 5.1 zeigt eine schematische Darstellung eines Mikro-Well Arrays nach der vorliegenden Erfindung in einer Ansicht von oben. Figur 5.2 zeigt einen Schnitt durch das Mikro-Well Array aus Figur 5.1 entlang der Markierungen S. Das Mikro-Well Array weist zuunterst eine 1. Schicht 5a auf. Die 1. Schicht ist bevorzugt ein Mikroskopie-Deckgläschen, bevorzugt ein für die Fluoreszenzmikroskopie geeignetes Deckgläschen. Das Material der 1. Schicht weist bevorzugt Glas auf, kann aber auch Kunststoff aufweisen.

Auf der 1. Schicht ist eine 2. Schicht 5b angebracht, die die Umfangswände 6e der Mikro-Wells 6c bildet. Die Mikro-Wells 5c sind also als Ausnehmungen in der 2. Schicht 5b ausgebildet. Die Mikro-Wells 5c sind hier mit rechteckigem Grundriss, also rechteckigen Böden 5f und senkrecht dazu verlaufenden Umfangswänden 5e dargestellt. Die Böden 5f der Mikro-Wells 5c werden durch die 1. Schicht 5a, hier ein Mikroskopie-Deckgläschen, gebildet. Nach oben hin, das heißt oberhalb der 2. Schicht 5b, sind die Mikro-Wells 5c offen. Die Tiefe 5h ist ebenfalls in Figur 5.2 eingezeichnet. Die Mikro-Wells 5c können jedoch wie bereits beschrieben auch gänzlich andere Formen annehmen. Insbesondere für Mikro-Wells mit unregelmäßig geformtem Grundriss kann die Größe der Mikro-Wells mithilfe des Durchmessers eines einbeschriebenen Kreises 5d oder auch unter Angabe des Volumens des Mikro-Wells 5c angegeben werden. Die Umfangswand 5b eines Mikro-Wells muss nicht senkrecht zu seinem Boden 5f verlaufen. Die Umfangswand 5e kann auch in einem Winkel ≠ 90° zum Boden 5f verlaufen. Wichtig ist hierbei, dass die Steigung der Umfangswand 5b relativ zum Boden 5f ausreicht, um einen Wirtskörper, der unter Einfluss der Schwerkraft Richtung Boden 5f des Mikro-Wells 5c sinkt, zum Boden 5f des Mikro-Wells 5c weiterzuleiten. Bevorzugt weicht der Winkel ± 15° oder weniger, stärker bevorzugt ± 10° oder weniger, stärker bevorzugt ± 5° oder weniger, besonders bevorzugt ± 2° oder weniger von der Senkrechten zum Boden 5f ab.

Figur 6.1 zeigt eine schematische Darstellung einer Mikro-Well Kammer gemäß der vorliegenden Erfindung in einer Ansicht von oben. Figur 6.2 zeigt eine schematische Darstellung eines Schnitts durch die Mikro-Well Kammer aus Figur 6.1 gemäß der Markierungen S. In der dargestellten Ausführungsform weist die Mikro-Well Kammer eine 1. Schicht 6a aus einem Deckgläschen, bevorzugt einem Deckgläschen, das für die Mikroskopie, insbesondere die Fluoreszenzmikroskopie geeignet ist, auf. Die 1. Schicht besteht bevorzugt aus Glas, kann jedoch auch aus Kunststoff bestehen. Auf der 1. Schicht 6a ist eine 2. Schicht 6b angebracht. Die 2. Schicht 6b weist Ausnehmungen 6g auf. Die Ausnehmungen 6g werden auf ihrer Unterseite durch die 1. Schicht begrenzt. Dadurch werden Mikro-Wells gebildet, die jeweils einen Boden, der durch die 1. Schicht 6a gebildet wird, und eine Umfangswand, die durch die 2. Schicht 6b gebildet wird, aufweisen. Die Mikro-Wells sind nach oben hin offen. Die 1. und 2. Schicht bilden zusammen ein Mikro-Well Array. Auf der Oberseite der 2. Schicht 6b bzw. des Mikro-Well Arrays ist in der in Figur 6 dargestellten Ausführungsform eine 3. Schicht 6c angebracht. Die 3. Schicht 6c weist einen Hohlraum 6f auf. Der Hohlraum 6f erstreckt sich angrenzend an und über die Mikro-Wells. Es liegt also eine direkte Verbindung zwischen dem Hohlraum 6f der 3. Schicht 6c und den nach oben offenen Mikro-Wells des Mikro-Well Arrays vor. Dadurch ergibt sich ein Arbeits-Hohlraum, der den Hohlraum 6f der 3. Schicht 6c und die Mikro-Wells beinhaltet. Der Arbeitshohlraum ist von der Umgebung durch die 1., 2. und 3. Schicht abgeschlossen. Eine beabsichtigte Verbindung zwischen dem Arbeitshohlraum und der Umgebung wird in Form eines Einlasses 6d und eines Auslasses 6e bereitgestellt. Über den Einlass 6d können für das Verfahren benötigte Flüssigkeiten in das Arbeitsvolumen zugeführt und somit auf das Mikro-Well Array aufgebracht werden. Überschüssiges Flüssigkeitsvolumen, und insbesondere auch Luft, kann durch den Auslass 6e abfließen. Dadurch wird ein sequenzielles Einbringen und Ausspülen von Flüssigkeiten gewährleistet.

Figur 7 zeigt eine schematische Darstellung einer Serie von Verfahrensschritten gemäß der vorliegenden Erfindung. In Figur 7.1 wird eine Gruppe von Wirtskörpern 7a in ein Mikro-Well Array 7b eingebracht. Vorzugsweise geschieht dies, indem die Wirtskörper 7a in einer geeigneten Flüssigkeit vorliegen und die Wirtskörper-Flüssigkeit auf die Oberseite des Mikro-Well Arrays aufgebracht wird. Die Wirtskörper 7a werden in diesem Schritt bevorzugt in einer Form verwendet, in der die einzelnen Wirtskörper 7a nicht aneinanderhaften. Die Wirtskörper 7a sind hier exemplarisch als Zellen dargestellt. In Figur 7.2 fallen die auf das Mikro-Well Array aufgebrachten Wirtskörper 7a unter Einwirkung der Schwerkraft in die einzelnen Mikro-Wells 7c und sinken dort zu Boden. Eine hierfür benötigte Inkubationszeit hängt von der Tiefe der Mikro-Wells sowie von den verwendeten Wirtskörpern 7a und der Viskosität der verwendeten Flüssigkeit ab. Der Fachmann wird hier ohne weiteres eine geeignete Inkubationszeit finden. Hierbei kann jeder Mikro-Well mit keinem, genau einem oder mit mehr als einen Wirtskörper 7a besetzt werden. Für die spätere Auswertung sind die Mikro-Wells jeweils vorzugsweise mit genau einem Wirtskörper 7a besetzt. Um diesen Zustand zu gewährleisten, werden die Mikro-Wells auf ihren Inhalt untersucht. Dies kann beispielsweise mit Hilfe von Hellfeld-Mikroskopie und/oder jedweder anderen geeigneten bildgebenden Technik erfolgen. Diejenigen Mikro-Wells 7d, die mehrere oder keine Zellen aufweisen, sind in der Figur mit einem X gekennzeichnet und werden von der späteren Analyse ausgeschlossen. Diejenigen Mikro-Wells 7c, die genau einen Wirtskörper 7a aufweisen, werden in der späteren Analyse berücksichtigt (keine zusätzliche Kennzeichnung in der Figur).

In Figur 7.3 werden die Wirtskörper 7a (hier Zellen) durch Zugabe von Aufschluss-Puffer (hier Lyse-Puffer) 7e aufgeschlossen. Auf diese Weise werden in den Wirtskörpern 7a vorliegende Zielstrukturen 7f freigesetzt. Die freigesetzten Zielstrukturen 7f binden an den Boden der Mikro-Wells 7c. Diese Bindung kann durch Träger-Adapter, die jeweils sowohl an den Boden als auch an eine Zielstruktur binden, vermittelt werden.

Anschließend werden in Figur 7.4 3D-DNA-Nanostrukturen 7g hinzu gegeben. Die 3D-DNA-Nanostrukturen sind auf die eine oder mehreren zu untersuchenden Zielstrukturen abgestimmt. Die 3D-DNA-Nanostrukturen sind derart ausgewählt, dass für jede der einen oder mehreren voneinander verschiedenen Zielstrukturen jeweils eine zugeordnete Identifizierungsstruktur ausgebildet werden kann, wobei jede der Identifizierungsstrukturen die jeweils zugeordnete Zielstruktur und mindestens zwei 3D-DNA-Nanostrukturen aufweist, wobei jede der mindestens zwei 3D-DNA-Nanostrukturen einen oder mehrere innenliegendes Fluoreszenz-Farbstoffmoleküle aufweist und wobei jede der mindestens zwei 3D-DNA-Nanostrukturen spezifisch an die jeweilige Zielstruktur bindet, und wobei die mindestens zwei 3D-DNA-Nanostrukturen an paarweise unterschiedliche Regionen der jeweiligen Zielstruktur gebunden sind.

Figur 7.5 zeigt schematisch die Analyse mit einem Mikroskop, wobei nur das Objektiv 7i dargestellt ist. Hierbei werden lediglich die zuvor in Figur 7.2 zur Analyse freigegebenen Mikro-Wells 7c analysiert. Die Analyse erfolgt mittels Fluoreszenzmikroskopie. Die 3D-DNA-Nanostrukturen 7g und die Parameter der Fluoreszenzmessung sind derart ausgewählt, dass das mindestens eine gemessene Fluoreszenzsignal der Identifizierungsstrukturen von dem Fluoreszenzsignal aller isolierten 3D-DNA-Nanostrukturen unterscheidbar ist, wenn diese nicht in einer der Identifizierungsstrukturen gebunden sind, und dass die gemessenen Fluoreszenzsignale aller paarweise verschiedenen Identifizierungsstrukturen paarweise voneinander unterscheidbar sind. Selbstverständlich kann jede der verschiedenen Zielstrukturen mehrfach vorliegen, sodass in der Fluoreszenzmessung das Signal jeder der verschiedenen Zielstrukturen mehrfach vorliegen kann.

Die nachfolgenden, nicht-limitierenden Beispiele dienen der Veranschaulichung der vorliegenden Erfindung.

## Beispiel 1: Herstellung von erfindungsgemäßen 3D-Nanostrukturen

### 1. Beschreibung der hergestellten 3D-DNA Nanostrukturen

3D-DNA-Nanostrukturen mit Hohlzylinder-Form mit jeweils 60 innenliegenden Farbstoffmolekülen, die paarweise Abstände von jeweils mindestens 9 nm zu den jeweils nächsten Farbstoffmolekülen innerhalb ein und derselben 3D-DNA-Nanostruktur aufweisen, wurden in drei verschiedenen Farbstoff-Varianten hergestellt, nämlich in den Varianten 3D_1, 3D_2 und 3D_3:

- 3D-DNA-Nanostruktur der Variante 3D_1: rot fluoreszierend:
  Die 3D_1-DNA-Nanostruktur weist 60 innenliegende DNA-Klammerstränge mit zusätzlichen einzelsträngigen Komplementärsequenzen zu Fluorophor-Adaptern für rote Farbstoffe (die mit Sequenz S1 bezeichnet wird), sowie 60 zugehörige Fluorophor-Adapter, die jeweils mit einem Atto647N-Farbstoff-Molekül bestückt sind, auf (Oligomere SEQ_ID_NO 1259). Die für Fluorophor-Adapter-Bindung zu verlängernden Klammerstränge wurden im Design-Programm CaDNAno ausgewählt mit den Kriterien, dass sie innen im Hohlzylinder liegen, weiter als 2 nm vom Rand des Hohlzylinders entfernt sind, ein freies Ende an der Innenoberfläche des Hohlzylinders haben, sowie mehr als 5 nm voneinander entfernt sind (letzteres trifft aufgrund des Designs und der Sequenzlängen auf alle Klammerstränge zu, die mit den vorigen Bedingungen in Einklang sind). Die Farbstoff-Adapter sind am 3'-Ende Farbstoff-modifiziert. Dieses Ende ist ausgewählt damit sich die Farbstoffe möglichst nahe an der inneren Hohlzylinderobefläche befinden und wenig Bewegungsspielraum haben und einander somit möglichst wenig nahe kommen können. Mehrere Modifikationen pro Farbstoff-Adapter wären auch denkbar, bedeuten aber höhere Kosten und einen gewissen Kontrollverlust in der Positionierung. Die 3D_1-Nanostruktur wurde zudem mit einem T1-Ziel-Adapter ausgestattet (Oligomer SEQ_ID_NO 1263). Die SEQ ID NOs aller für die 3D_1-Nanostruktur verwendeten Klammerstränge sind in der Definition oligopool3D_S1 weiter unten zusammengefasst. Als Gerüst-Strang wurde der Strang p7308 (SEQ ID NO 1258) verwendet.
- 3D-DNA-Nanostruktur der Variante 3D_2: grün fluoreszierend:
  Eine 3D_2-DNA-Nanostruktur weist 60 innenliegende DNA-Klammerstränge mit zusätzlichen einzelsträngigen Komplementärsequenzen zu Fluorophor-Adaptern für grüne Farbstoffe (die mit Sequenz S2 bezeichnet wird), sowie 60 zugehörige Fluorophor-Adapter, die jeweils mit einem Atto565-Farbstoff-Molekül bestückt sind, auf (Oligomere SEQ_ID_NO 1260). Die für Fluorophor-Adapter-Bindung zu verlängernden Klammerstränge wurden im Design-Programm CaDNAno ausgewählt mit den Kriterien, dass sie innen im Hohlzylinder liegen, nicht mehr als 2 nm vom Rand des Hohlzylinders entfernt sind, ein freies Ende an der Innenoberfläche des Hohlzylinders haben, sowie mehr als 5 nm voneinander entfernt sind (letzteres trifft aufgrund des Designs und der Sequenzlängen auf alle Klammerstränge zu, die mit den vorigen Bedingungen in Einklang sind). Die Farbstoff-Adapter sind am 3'-Ende Farbstoff-modifiziert. Dieses Ende ist ausgewählt damit sich die Farbstoffe möglichst nahe an der inneren Hohlzylinderobefläche befinden und wenig Bewegungsspielraum haben, einander somit möglichst wenig nahe kommen können. Mehrere Modifikationen pro Farbstoff-Adapter wären auch denkbar, bedeuten aber höhere Kosten und einen gewissen Kontrollverlust in der Positionierung. Die 3D_2-Nanostruktur wurde mit einem T2-Ziel-Adapter ausgestattet (Oligomer SEQ_ID_NO 1264). Die SEQ ID NOs aller für die 3D_2-Nanostruktur verwendeten Klammerstränge sind in der Definition oligopool3D_S2 weiter unten zusammengefasst. Als Gerüst- an welcher Position der Farbstoff angeordnet wird. Als Gerüst-Strang wurde der Strang p7308 (SEQ ID NO 1258) verwendet.
- 3D-DNA-Nanostruktur der Variante 3D_3: blau fluoreszierend:
  Eine 3D_3-DNA-Nanostruktur weist 60 innenliegende DNA-Klammerstränge mit zusätzlichen einzelsträngigen Komplementärsequenzen zu Fluorophor-Adaptern für blaue Farbstoffe (die mit Sequenz S3 bezeichnet wird), sowie 60 zugehörige Fluorophor-Adapter, die jeweils mit einem Atto488-Farbstoff bestückt sind, auf (Oligomere SEQ_ID_NO 1261). Die für Fluorophor-Adapter-Bindung zu verlängernden Klammerstränge wurden im Design-Programm CaDNAno ausgewählt mit den Kriterien, dass sie innen im Hohlzylinder liegen, nicht mehr als 2 nm vom Rand des Hohlzylinders entfernt sind, ein freies Ende an der Innenoberfläche des Hohlzylinders haben, sowie mehr als 5 nm voneinander entfernt sind (letzteres trifft aufgrund des Designs und der Sequenzlängen auf alle Klammerstränge zu, die mit den vorigen Bedingungen in Einklang sind). Die Farbstoff-Adapter sind am 3'-Ende Farbstoff-modifiziert. Dieses Ende ist ausgewählt damit sich die Farbstoffe möglichst nahe an der inneren Hohlzylinderobefläche befinden und wenig Bewegungsspielraum haben, einander somit möglichst wenig nahe kommen können. Mehrere Modifikationen pro Farbstoff-Adapter wären auch denkbar, bedeuten aber höhere Kosten und einen gewissen Kontrollverlust in der Positionierung. Die 3D_3-Nanostruktur wurde mit einem T3-Ziel-Adapter ausgestattet (Oligomer SEQ_ID_NO 1265). Die SEQ ID NOs aller für die 3D_3-Nanostruktur verwendeten Klammerstränge sind in der Definition oligopool3D_S3 weiter unten zusammengefasst. Als Gerüst-Strang wurde der Strang p7308 (SEQ ID NO 1258) verwendet.

Alle DNA-Oligomere (auch die Fluoreszenz-Farbstoff bestückten DNA-Oligomere) wurden bei Eurofins Genomics GmbH gekauft.

Eine Veranschaulichung einer 3D-DNA-Nanostruktur des Beispiels 1 ist in der oben bereits diskutierten Fig. 5 gezeigt. Hierbei zeigt Fig. 5A eine schematische Darstellung der 3D-DNA-Nanostrukturen 3D_1, 3D_2 und 3D-3, die sich nur durch die Art der angebrachten Fluoreszenz-Farbstoffmoleküle unterscheiden. Fig. 5B zeigt den Hohlzylinder aus Fig. 5A (für Anschauungszwecke) aufgeschnitten und abgerollt, wobei die Innenseite des Hohlzylinders aus Fig. 5A in Draufsicht dargestellt ist. Jedes x repräsentiert ein Fluoreszenz-Farbstoffmolekül. Unter nächsten Nachbarn betragen die paarweisen Abstände a der Farbstoff-Moleküle 9 nm. Zwischen den sonstigen Farbstoff-Molekülen sind die paarweisen Abstände, beispielsweise der Abstand g, größer. Die Farbstoff-Moleküle befinden sich dabei jeweils an der in Fig. 5A innersten Helix, so dass der Abstand der Farbstoff-Moleküle vom Außenrand des Hohlzylinders in etwa der Wandstärke der Zylinderwand, d.h. etwa 2,5 Helixdurchmesser (entsprechend ungefähr 5 nm), entspricht.

**2. Herstellungsprotokoll:**

Im vorliegenden Beispiel wurden die 3D-DNA Nanostrukturen 3D_1-3 nach dem im Folgenden beschriebenen Herstellungsprotokoll hergestellt.
Zur Herstellung der 3D-DNA Nanostrukturen 3D_1, 3D_2 und 3D_3wurden zunächst folgende Bestandteile zu einem finalen Volumen von 200 µl gemischt:

- 10 nM p7308 Gerüst-Strang NO 1258)
- 100 nM von jedem der Klammer-Stränge (SEQ ID NOs aus oligopool3D_S1 für 3D_1-DNA-Nanostruktur, SEQ ID NOs aus oligopool3D_S2 für 3D_2-DNA-Nanostruktur bzw. SEQ ID NOs aus oligopool3D_S3 für 3D_3-DNA-Nanostruktur)
- 120 nM Fluoreszenz-Farbstoff-bestückte DNA-Oligos (SEQ ID NO 1259 für 3D_1-DNA-Nanostruktur, SEQ ID NO 1260 für 3D_2-DNA-Nanostruktur bzw. SEQ ID NO 1261 für 3D_3-DNA-Nanostruktur)
- 400 nM Ziel bindende DNA-Oligos (SEQ ID NO 1263 für 3D_1-DNA-Nanostruktur, SEQ ID NO 1264 für 3D_2-DNA-Nanostruktur bzw. SEQ ID NO 1265 für 3D_3-DNA-Nanostruktur)
- 1x Puffer FB02 (Pufferzusammensetzung siehe weiter unten)

Um die DNA-Nanostrukturen in ihre Form falten zu lassen wurden die oben genannten Mischungen zunächst erhitzt, um etwaige vorhandene Sekundärstrukturen zu schmelzen und dann langsam abgekühlt, damit die Basenpaarungen ins thermische Gleichgewicht, und entsprechend in die geplante Konformation, finden konnten. Dazu diente folgendes Programm im Thermocycler (Mastercycler Nexus X2 von Eppendorf GmbH, Hamburg, Deutschland):

- 15 Minuten bei 65°C halten und innerhalb einer Minute auf 50°C abkühlen..
- Absenken von 50°C auf 40°C in 66 Stunden bei konstanter Rate
- Mit Ablauf der 66h (ggf. kann auch noch für einige weitere Stunden bei 40°C inkubiert werden) innerhalb einer Minute auf 4°C kühlen und bei 2-8°C aufbewahren

Anschließend wurden korrekt gefaltete 3D-DNA-Nanostrukturen von einzelnen DNA-Oligomeren oder kleineren DNA-Oligomer-Komplexen getrennt. Dazu wurde folgende PEG-Aufreinigung (Aufreinigung mit Polyethylenglykol) verwendet:

- Flüssigkeit aus dem Thermocycler in gleichem Volumen mit PEGX01 (Pufferzusammensetzung siehe weiter unten) mischen.
- 35 min zentrifugieren bei 13000 - 16000 rfc bei Raumtemperatur (20-25°C)
- Überstand abpipettieren
- in 200 µl FB02 (Pufferzusammensetzung siehe weiter unten) und 200 µl PEGX01 (Pufferzusammensetzung siehe weiter unten) resuspendieren.
- 35 min zentrifugieren bei 13000 - 16000 rfc bei Raumtemperatur (20-25°C)
- in 100 µl FB01 (Pufferzusammensetzung siehe weiter unten) resuspendieren
- 8 bis 16, bevorzugt 10 Stunden (hier über Nacht) bei Dunkelheit, Raumtemperatur und 600 rpm auf Shaker inkubieren um Pellet vollständig zu lösen.

- bei 4°C für die weitere Verwendung lagern (bis zu 12 Monate möglich, vorliegend Lagerung aber nur wenige Tage)

Während vorliegend eine PEG-Aufreinigung der DNA-Nanostrukturen verwendet wurde, könnte prinzipiell auch alternativ eine Aufreinigung mittels Agarose-Gelelektrophorese und anschließender Extraktion der DNA-Nanostrukturen aus dem Gel erfolgen. Eine Agarose-Gelelektrophorese basierte Aufreinigung könnte z.B. wie folgt durchgeführt werden:

- 1,5% w/v Agarose-Gel vorbereiten
- Probe mit 5x Orange G loading buffer versetzen
- Elektrophorese bei 4,5 V/cm Spannung für 1,5 h mit Eiskühlung
- Gelstücke mit DNA-Origami-Bande ausschneiden. Diese ist etwa 2 mm breit und kann optional durch Beladen einer nebenliegenden Bahn mit Gerüst-Strang leichter identifiziert werden, in Freeze 'N Squeeze™ DNA Gel Extraction Spin Columns (BioRad Laboratories GmbH) stecken und 4,5 min bei Raumtemperatur und 1050 rcf zentrifugieren.

**Verwendete Pufferlösungen**

**PEGX01:**

- 15% PEG 8000
- 1x TAE (Tris-Acetat-EDTA-Puffer: 40 mM Tris, 20 mM Essigsäure, 1 mM EDTA)
- 12,5mM MgCl2
- 500mM NaCl

**FB01:**

- 10mM Tris pH8.0
- 1mM EDTA
- 12,5mM MgCl2

**FB02:**

- 10mM Tris pH8.0
- 1mM EDTA
- 10mM MgCl2

**DNA-Oligo-Pools:**

Für die Herstellung der jeweiligen 3D-DNA-Nanostrukturen wurden die Oligomere anhand ihrer SEQ_ID_NOs (Nummern in den eckigen Klammern geben die jeweils SEQ ID NOs an) zu folgenden Pools zusammengefasst:

**oligopool_3D-S1:** [1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 277, 286, 287, 288, 278, 279, 280, 281, 282, 283, 284, 285, 286, 287, 288, 469, 478, 479, 480, 470, 471, 472, 473, 474, 475, 476, 477, 478, 479, 480, 265, 274, 275, 276, 266, 267, 268, 269, 270, 271, 272, 273, 274, 275, 276, 301, 310, 311, 312, 302, 303, 304, 305, 306, 307, 308, 309, 310, 311, 312, 253, 262, 263, 264, 254, 255, 256, 257, 258, 259, 260, 261, 262, 263, 264, 289, 298, 299, 300, 290, 291, 292, 293, 294, 295, 296, 297, 298, 299, 300, 241, 250, 251, 252, 242, 243, 244, 245, 246, 247, 248, 249, 250, 251, 252]

**oligopool_3D-S2:** [1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 277, 286, 287, 288, 278, 279, 280, 281, 282, 283, 284, 285, 286, 287, 288, 481, 490, 491, 492, 482, 483, 484, 485, 486, 487, 488, 489, 490, 491, 492, 337, 346, 347, 348, 338, 339, 340, 341, 342, 343, 344, 345, 346, 347, 348, 361, 370, 371, 372, 362, 363, 364, 365, 366, 367, 368, 369, 370, 371, 372, 325, 334, 335, 336, 326, 327, 328, 329, 330, 331, 332, 333, 334, 335, 336, 349, 358, 359, 360, 350, 351, 352, 353, 354, 355, 356, 357, 358, 359, 360, 241, 250, 251, 252, 242, 243, 244, 245, 246, 247, 248, 249, 250, 251, 252]

**oligopool_3D-S3:** [1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 277, 286, 287, 288, 278, 279, 280, 281, 282, 283, 284, 285, 286, 287, 288, 493, 502, 503, 504, 494, 495, 496, 497, 498, 499, 500, 501, 502, 503, 504, 385, 394, 395, 396, 386, 387, 388, 389, 390, 391, 392, 393, 394, 395, 396, 409, 418, 419, 420, 410, 411, 412, 413, 414, 415, 416, 417, 418, 419, 420, 373, 382, 383, 384, 374, 375, 376, 377, 378, 379, 380, 381, 382, 383, 384, 397, 406, 407, 408, 398, 399, 400, 401, 402, 403, 404, 405, 406, 407, 408, 241, 250, 251, 252, 242, 243, 244, 245, 246, 247, 248, 249, 250, 251, 252]

## Beispiel 2: Herstellung von 2D-DNA Nanostrukturen zum Vergleich

### 1. Beschreibung der hergestellten 2D-DNA Nanostrukturen

Rechteckige 2D-DNA Nanostrukturen mit jeweils 48 Farbstoffmolekülen mit einem paarweisen Abstand von 5 nm zu den jeweils nächsten Farbstoffmolekülen innerhalb ein und derselben 2D-DNA-Nanostruktur (eine derartige 2D-DNA-Nanostruktur ist zum Beispiel in Figur 1A der WO 002016140727 abgebildet) wurden in drei Varianten hergestellt, nämlich in den Varianten 2D_1, 2D_2 und 2D_3:

- 2D-DNA-Nanostruktur der Variante 2D_1: rot fluoreszierend:
  Eine 2D_1-DNA-Nanostruktur weist 48 DNA-Klammerstränge mit zusätzlichen einzelsträngigen Komplementärsequenzen zu Fluorophor-Adaptern für rote Farbstoffe (die mit Sequenz S1 bezeichnet wird), sowie 48 zugehörige Fluorophor-Adapter, die mit jeweils einem Atto647N-Farbstoff bestückt sind, auf (Oligomere SEQ_ID_NO 1259). Die für Fluorophor-Adapter-Bindung zu verlängernden Klammerstränge wurden im Design-Programm CaDNAno ausgewählt mit den Kriterien, dass sie alle an der selben Seite des Rechtecks liegen und weit genug auseinander liegen um nicht signifikant wechselzuwirken. Die Farbstoff-Adapter sind am 3'-Ende Farbstoff-modifiziert. Dieses Ende ist ausgewählt damit sich die Farbstoffe möglichst nahe an der Reckteckoberfläche befinden und wenig Bewegungsspielraum haben, einander somit möglichst wenig nahe kommen können. Mehrere Modifikationen pro Farbstoff-Adapter wären auch denkbar, bedeuten aber höhere Kosten und einen gewissen Kontrollverlust in der Positionierung. Die 2D_1-DNA-Nanostruktur wurde mit einem T1-Ziel-Adapter ausgestattet (Oligomer SEQ_ID_NO 1266), der sich laut Design entlang des Rands der Struktur befindet. Die SEQ ID NOs aller für die 2D_1-DNA-Nanostruktur verwendeten Klammerstränge sind in der Definition oligopool2D_S1 weiter unten zusammengefasst. Als Gerüst-Strang wurde der Strang p7249 (SEQ ID NO 1257) verwendet.
- 2D-DNA-Nanostruktur der Variante 2D_2: grün fluoreszierend:

Eine 2D_2-DNA-Nanostruktur weist 48 DNA-Klammerstränge mit zusätzlichen einzelsträngigen Komplementärsequenzen zu Fluorophor-Adaptern für grüne Farbstoffe (die mit Sequenz S2 bezeichnet wird), sowie 48 zugehörige Fluorophor-Adapter, die mit jeweils einem Atto565-Farbstoff bestückt sind. auf (Oligomere SEQ_ID_NO 1260). Die für Fluorophor-Adapter-Bindung zu verlängernden Klammerstränge wurden im Design-Programm CaDNAno ausgewählt mit den Kriterien, dass sie alle an der selben Seite des Rechtecks liegen und weit genug auseinander liegen um nicht signifikant wechselzuwirken. Die Farbstoff-Adapter sind am 3'-Ende Farbstoff-modifiziert. Dieses Ende ist ausgewählt damit sich die Farbstoffe möglichst nahe an der inneren Reckteckoberfläche befinden und wenig Bewegungsspielraum haben, einander somit möglichst wenig nahe kommen können. Mehrere Modifikationen pro Farbstoff-Adapter wären auch denkbar, bedeuten aber höhere Kosten und einen gewissen Kontrollverlust in der Positionierung. Die 2D_2-DNA-Nanostruktur wurde mit einem T2-Ziel-Adapterausgestattet (Oligomer SEQ_ID_NO 1267), der sich laut Design entlang des Rands der Struktur befindet. SEQ ID NOs aller verwendeten Klammerstränge sind in der Definition oligopool2D_S2 weiter unten zusammengefasst. Als Gerüst-Strang wurde der Strang p7249 (SEQ ID NO 1257) verwendet.

- 2D-DNA-Nanostruktur der Variante 2D_3: blau fluoreszierend:
  Eine 2D_3-DNA-Nanostruktur weist 48 DNA-Klammerstränge mit zusätzlichen einzelsträngigen Komplementärsequenzen zu Fluorophor-Adaptern für blaue Farbstoffe (die mit Sequenz S3 bezeichnet wird), sowie 48 zugehörige Fluorophor-Adapter, die mit jeweils einem Atto488-Farbstoff bestückt sind, auf (Oligomere SEQ_ID_NO 1261). Die für Fluorophor-Adapter-Bindung zu verlängernden Klammerstränge wurden im Design-Programm CaDNAno ausgewählt mit den Kriterien, dass sie alle an der selben Seite des Rechtecks liegen und weit genug auseinander liegen um nicht signifikant wechselzuwirken. Die Farbstoff-Adapter sind am 3'-Ende Farbstoff-modifiziert. Dieses Ende ist ausgewählt damit sich die Farbstoffe möglichst nahe an der inneren Reckteckoberfläche befinden und wenig Bewegungsspielraum haben, einander somit möglichst wenig nahe kommen können. Mehrere Modifikationen pro Farbstoff-Adapter wären auch denkbar, bedeuten aber höhere Kosten und einen gewissen Kontrollverlust in der Positionierung. Die 2D_3-DNA-Nanostruktur wurde mit einem T3-Ziel-Adapterausgestattet (Oligomer SEQ_ID_NO 1268), der sich laut Design entlang des Rands der Struktur befindet. SEQ ID NOs aller verwendeten Klammerstränge sind in der Definition oligopool2D_S3 weiter unten zusammengefasst. Als Gerüst-Strang wurde der Strang p7249 (SEQ ID NO 1257) verwendet.

**2. Herstellungsprotokoll:**

Im vorliegenden Beispiel wurden die 2D-DNA Nanostrukturen 2D_1-3 nach dem im Folgenden beschriebenen Herstellungsprotokoll hergestellt.

Zur Herstellung der 2D-DNA Nanostrukturen 2D_1-3 wurden zunächst die folgenden Bestandteile bei einem finalen Volumen von 200 μl gemischt:

- 10 nM p7249 Gerüst-Strang [SEQ ID NO 1257]
- 100 nM von jedem der Klammer-Stränge (SEQ ID NOs aus oligopool2D_S1 für 2D_1-DNA-Nanostruktur, SEQ ID NOs aus oligopool2D_S2 für 2D_2-DNA-Nanostruktur bzw. SEQ ID NOs aus oligopool2D_S3 für 2D_3-DNA-Nanostruktur)
- 120 nM Farbstoff-bestückte DNA-Oligos (SEQ ID NO 1259 für 2D_1-DNA-Nanostruktur, SEQ ID NO 1260 für 2D_2-DNA-Nanostruktur bzw. SEQ ID NO 1261 für 2D_3-DNA-Nanostruktur)
- 400 nM Ziel bindende DNA-Oligos (SEQ ID NO 1266 für 2D_1-DNA-Nanostruktur, SEQ ID NO 1267 für 2D_2-DNA-Nanostruktur bzw. SEQ ID NO 1268 für 2D_3-DNA-Nanostruktur)
- 1x Puffer FB01 (Pufferzusammensetzung s. Beispiel 1)

Um die DNA-Nanostrukturen in ihre Form falten zu lassen wurden sie zunächst erhitzt, um etwaige vorhandene Sekundärstrukturen zu schmelzen und dann langsam abgekühlt, damit die Basenpaarungen ins Thermische Gleichgewicht, und entsprechend in die geplante Konformation, finden konnten. Dazu diente folgendes Thermocycler-Programm (im Mastercycler Nexus X2 von Eppendorf GmbH, Hamburg, Deutschland):

- 15 Minuten bei 65°C halten und innerhalb einer Minute auf 60°C abkühlen.
- Absenken von 60°C auf 20°C in 1 Stunde bei konstanter Rate
- Innerhalb einer Minute auf 4°C abkühlen und bei 4°C halten

Anschließend wurden korrekt gefaltete 2D-DNA-Nanostrukturen von einzelnen DNA-Oligomeren oder kleineren DNA-Oligomer-Komplexen getrennt. Dazu wurde folgende PEG-Aufreinigung (Aufreinigung mit Polyethylenglykol) verwendet: Flüssigkeit aus dem Thermocycler in gleichem Volumen PEGX01 (Pufferzusammensetzung siehe weiter unten) mischen.

- 35 min zentrifugieren bei 13000 - 16000 rfc bei Raumtemperatur (20-25°C)
- in 200 µl FB01 und 200 µl FB02 (Pufferzusammensetzung siehe Beispiel 1) resuspendieren.
- 35 min zentrifugieren bei 13000 - 16000 rfc bei Raumtemperatur (20-25°C)
- in 100 µl FB01 resuspendieren
- Über Nacht bei Dunkelheit, Raumtemperatur (20-25°C) und 600rpm auf Shaker inkubieren.
- bei -20°C (z.B. bis zu 3 Jahre) oder bei 4°C (z.B. bis zu 12 Monate) für die weitere Verwendung lagern (vorliegend für einige Tage gelagert)

Auch hier könnte prinzipiell alternativ die in Beispiel 1 erwähnte Agarosegelelektrophorese basierte Aufreinigung anstatt der PEG-basierten Aufreinigung verwendet werden.

**Verwendete DNA-Oligo-Pools:**

für die Herstellung der jeweiligen 2D-DNA-Nanostrukturen wurden die Oligomere anhand ihrer SEQ_ID_NOs (Nummern in den eckigen Klammern geben die jeweils SEQ ID NOs an) zu folgenden Pools zusammengefasst:

**oligopool_2D-SI:** [529, 538, 539, 540, 530, 531, 532, 533, 534, 535, 536, 537, 538, 539, 540, 563, 572, 573, 574, 564, 565, 566, 567, 568, 569, 570, 571, 572, 573, 574, 623, 632, 633, 634, 624, 625, 626, 627, 628, 629, 630, 631, 632, 633, 634, 657, 666, 667, 668, 658, 659, 660, 661, 662, 663, 664, 665, 666, 667, 668, 693, 702, 703, 704, 694, 695, 696, 697, 698, 699, 700, 701, 702, 703, 704, 705, 714, 715, 716, 706, 707, 708, 709, 710, 711, 712, 713, 714, 715, 716, 787, 796, 797, 798, 788, 789, 790, 791, 792, 793, 794, 795, 796, 797, 798, 799, 808, 809, 810, 800, 801, 802, 803, 804, 805, 806, 807, 808, 809, 810, 575, 584, 585, 586, 576, 577, 578, 579, 580, 581, 582, 583, 584, 585, 586, 587, 596, 597, 598, 588, 589, 590, 591, 592, 593, 594, 595, 596, 597, 598, 669, 678, 679, 680, 670, 671, 672, 673, 674, 675, 676, 677, 678, 679, 680, 681, 690, 691, 692, 682, 683, 684, 685, 686, 687, 688, 689, 690, 691, 541, 548, 549, 550, 542, 543, 544, 545, 546, 547, 548, 549, 550, 551, 560, 561, 562, 552, 553, 554, 555, 556, 557, 558, 559, 560, 561, 562, 635, 642, 643, 644, 636, 637, 638, 639, 640, 641, 642, 643, 644, 645, 654, 655, 656, 646, 647, 648, 649, 650, 651, 652, 653, 654, 655, 656]

**oligopool_2D-S2:** [529, 538, 539, 540, 530, 531, 532, 533, 534, 535, 536, 537, 538, 539, 540, 563, 572, 573, 574, 564, 565, 566, 567, 568, 569, 570, 571, 572, 573, 574, 623, 632, 633, 634, 624, 625, 626, 627, 628, 629, 630, 631, 632, 633, 634, 657, 666, 667, 668, 658, 659, 660, 661, 662, 663, 664, 665, 666, 667, 668, 505, 514, 515, 516, 506, 507, 508, 510, 512, 514, 515, 516, 517, 526, 527, 528, 518, 519, 520, 522, 524, 526, 527, 528, 599, 608, 609, 610, 600, 601, 602, 604, 606, 608, 609, 610, 611, 620, 621, 622, 612, 613, 614, 616, 618, 620, 621, 622, 697, 699, 701, 709, 711, 713, 791, 793, 795, 803, 805, 807, 951, 960, 961, 962, 952, 953, 954, 955, 956, 957, 958, 959, 960, 961, 962, 963, 972, 973, 974, 964, 965, 966, 967, 968, 969, 970, 971, 972, 973, 974, 1045, 1054, 1055, 1056, 1046, 1047, 1048, 1049, 1050, 1051, 1052, 1053, 1054, 1055, 1056, 1057, 1066, 1067, 1068, 1058, 1059, 1060, 1061, 1062, 1063, 1064, 1065, 1066, 1067, 1068, 541, 548, 549, 550, 542, 543, 544, 545, 546, 547, 548, 549, 550, 551, 560, 561, 562, 552, 553, 554, 555, 556, 557, 558, 559, 560, 561, 562, 635, 642, 643, 644, 636, 637, 638, 639, 640, 641, 642, 643, 644, 645, 654, 655, 656, 646, 647, 648, 649, 650, 651, 652, 653, 654, 655, 656]

**oligopool_2D-S3:** [529, 538, 539, 540, 530, 531, 532, 533, 534, 535, 536, 537, 538, 539, 540, 563, 572, 573, 574, 564, 565, 566, 567, 568, 569, 570, 571, 572, 573, 574, 623, 632, 633, 634, 624, 625, 626, 627, 628, 629, 630, 631, 632, 633, 634, 657, 666, 667, 668, 658, 659, 660, 661, 662, 663, 664, 665, 666, 667, 668, 505, 514, 515, 516, 506, 507, 508, 510, 512, 514, 515, 516, 517, 526, 527, 528, 518, 519, 520, 522, 524, 526, 527, 528, 599, 608, 609, 610, 600, 601, 602, 604, 606, 608, 609, 610, 611, 620, 621, 622, 612, 613, 614, 616, 618, 620, 621, 622, 697, 699, 701, 709, 711, 713, 791, 793, 795, 803, 805, 807, 575, 584, 585, 586, 576, 577, 578, 579, 580, 581, 582, 583, 584, 585, 586, 587, 596, 597, 598, 588, 589, 590, 591, 592, 593, 594, 595, 596, 597, 598, 669, 678, 679, 680, 670, 671, 672, 673, 674, 675, 676, 677, 678, 679, 680, 681, 690, 691, 692, 682, 683, 684, 685, 686, 687, 688, 689, 690, 691, 1105, 1112, 1113, 1114, 1106, 1107, 1108, 1109, 1110, 1111, 1112, 1113, 1114, 1115, 1124, 1125, 1126, 1116, 1117, 1118, 1119, 1120, 1121, 1122, 1123, 1124, 1125, 1126, 1199, 1206, 1207, 1208, 1200, 1201, 1202, 1203, 1204, 1205, 1206, 1207, 1208, 1209, 1218, 1219, 1220, 1210, 1211, 1212, 1213, 1214, 1215, 1216, 1217, 1218, 1219, 1220]

**Beispiel 3: Vergleich des Nachweises einer Zielstruktur mit den 3D-Nanostrukturen aus Beispiel 1 und den 2D-DNA Nanostrukturen aus Beispiel 2**

**1. Überblick**

Zum Vergleich, wie gut sich Zielstrukturen mit den 2D-DNA-Nanostrukturen bzw. den erfindungsgemäßen 3D-DNA-Nanostrukturen über das erfindungsgemäße Verfahren in seiner Ausführungsform, in der die Identifizierungstruktur mit Zielstrukturen und den jeweiligen gebundenen DNA-Nanostrukturen an eine Trägeroberfläche (hier die Oberfläche eines Deckglässchens) gebunden ist/wird nachweisen lassen, wurden im hier beschriebenen Beispiel die gleichen Ziel-DNA-Oligomere als Zielstruktur verwendet und zum Einen durch das Binden von roten, grünen, und blauen 2D-DNA Nanostrukturen der Varianten 2D_1, 2D_2 bzw. 2D_3 wie in Beispiel 2 beschrieben (2D-Probe), und zum Anderen durch das Binden von roten, grünen und blauen 3D-DNA Nanostrukturen der Varianten 3D_1, 3D_2 bzw. 3D_3 wie in Beispiel 1 beschrieben (3D-Probe) nachgewiesen. Zudem wurde für beide Experimente jeweils ein Kontrollexperiment durchgeführt, in dem durch die Abwesenheit einer Zielstruktur überprüft wurde, ob und in welchem Ausmaß eine falsch positive Detektion auf der Deckglässchenoberfläche erfolgt. Der Vergleich zeigt, dass die 3D-DNA Nanostrukturen durch die optimierte und durchdachte Positionierung der Farbstoffmoleküle deutlich weniger an der Oberfläche des Mikroskopie-Deckgläschens haften.

**2. Material und Methoden:**

Für den Nachweis von Zielstrukturen (hier Ziel-DNA-Oligomere, kurz Zieloligomere) wurden diese zunächst in einer Hybridisierungsreaktion mit den jeweiligen DNA-Nanostrukturen (d.h. entweder 2D_1, 2D_2 und 2D_3 oder 3D_1, 3D_2 bzw. 3D_3) und mit Capture-Strang in einem geeigneten Puffer inkubiert, sodass all diese Bestandteile aneinanderbinden und die Identifizierungsstruktur bilden (siehe Fig. 1, 1k) konnten. Dies wurde separat für 2D- und 3D-DNA Nanostrukturen durchgeführt.
Die Hybridisierung erfolgte für den Fall der Probe mit den 3D-DNA-Nanostrukturen in einer Lösung mit folgenden Bestandteilen:

- 4 µl Mischung von DNA-Nanopartikel 3D_1-3, mit 200 pM je DNA-Nanopartikel-Set nach Beispiel 1
- 10 µl 2x Puffer RX07 (Zusammensetzung siehe unten)
- 1µl 20 nM Träger-Adapter (hier auch Capture-Strang genannt) (SEQ ID NO 1262) mit Biotin-Adapter, wobei der Capture-Stang mit Biotin-Adapter (am 5'-Ende mit Biotin modifiziert) von dem Unternehmen Eurofins geliefert wurden
- 1 µl 750 pM einzelsträngige sZiel-DNA-Oligomer (SEQ ID NO 1269). Dieses hat komplementäre Regionen für T1-, T2 und T3, so dass die 3D-DNA-Nanostrukturen 3D_1, 3D_2 und 3D-3 jeweils mittels ihrer T1, 2 bzw.3-Regionen an die entsprechenden komplementären Regionen des Ziel-DNA-Oligomers binden können, sowie eine komplementäre Region für den Capture-Strang. Die Zielstruktur und die jeweiligen Binderegionen waren so ausgelegt, dass alle drei 3D-DNA-Oligomere sowie der Capture-Strang gleichzeitig an ein Ziel-DNA-Oligomer binden konnten. Für das Kontrollexperiment wurde dieses Volumen mit $H_2O$ ersetzt.
- 4 µl H2O

Dies wurde bei 30°C für 16 Stunden inkubiert, um die Capture-Stränge, Ziel-Oligomere, und 3D-DNA-Nanostrukturen aneinander binden zu lassen.

Die Hybridisierung erfolgte für den Fall der Probe mit den 2D-DNA-Nanostrukturen in einer Lösung mit den gleichen Bestandteilen, allerdings wurden 4 µl Mischung von DNA-Nanopartikel 2D_1-3, mit 200 pM je DNA-Nanopartikel-Set nach Beispiel 2 statt 4 µl Mischung von DNA-Nanopartikel 3D_1-3, mit 200 pM je DNA-Nanopartikel-Set nach Beispiel 1 verwendet. Auch hier war gewährleistet, dass alle drei 2D-DNA-Nanostrukturen sowie der Capture-Strang an das Ziel-DNA-Oligomer binden konnten. Das verwendete Ziel-DNA-Oligomer wies ebenfalls die Sequenz mit SEQ ID NO 1269 auf. Auch für die 2D-DNA-Nanostrukturen wurde eine Kontrollreaktion durchgeführt, in der kein Ziel-DNA-Oligomer enthalten war.
Insgesamt wurden somit vier Experimente angesetzt.

Für die Messung wurden die Capture-Stränge jedes Experiments jeweils an eine Mikroskopie-Oberfläche eines Mikroskopie-Probenträgers gebracht. Als Mikroskopie-Probenträger wurde ein µ-Slide vom Typ $VI^{0,1}$ der ibidi GmbH (Martinsried, Deutschland) verwendet. Dazu wurde dieses behandelt gemäß dem folgenden Protokoll für die Präparation eines Mikroskop-Slides als Probe:

- µ-Slides $VI^{0,1}$ (ibidi GmbH, Martinsried) wurden mit 100 µl Puffer A (Zusammensetzung, siehe unten) gespült
- Einpipettieren von 40 µl 1mg/ml biotinyliertes BSA (Sigma-Aldrich GmbH) in einen ersten Anschluss des entsprechenden Kanals des µ-Slides, 2 min Inkubation, Auspipettieren der Flüssigkeit aus dem anderen, zweiten Anschluss des entsprechenden Kanals des μ-Slides, Einpipettieren von 100 μl Puffer A in obigen ersten Anschluss, auspipettieren der Flüssigkeit aus obigem zweiten Anschluss.

- Einpipettieren von 40 μl 0,5 mg/ml Streptavidin (Thermo Scientific) in obigen ersten Anschluss, 2 min Inkubation auspipettieren aus obigem zweiten Anschluss, Einpipettieren von 100 μl Puffer A in obigen ersten Anschluss und auspipettieren der Flüssigkeit aus obigem zweiten Anschluss.
- Einpipettieren von 100 μl Puffer B (Zusammensetzung siehe unten) in obigen ersten Anschluss und auspipettieren der Flüssigkeit aus obigem zweiten Anschluss
- Einpipettieren von 40 μl der hybridisierten Lösung aus vorigem Schritt (Lösung mit 2D-Nanostrukturen für die 2D-Probe oder Lösung mit 3D-DNA-Nanostrukturen für die 3D-Probe bzw. entsprechende Kontrollexperimente ohne Ziel-DNA-Oligomer) in obigen ersten Anschluss. 15 min Inkubation. Während dieser Zeit ist das Biotin der Capture-Stränge an das oberflächengebundene Streptavidin. Zusätzlich haften einige DNA-Nanostrukturen unspezifisch an der Oberfläche. Dies ist ungewollt und passiert bei 2D-DNA Nanostrukturen deutlich gehäufter als bei 3D-DNA Nanostrukturen. Auspipettieren aus obigem zweiten Anschluss
- Auswaschen der ungebundenen DNA-Nanostrukturen durch Einpipettieren von 200 μl Puffer RX07 (Zusammensetzung, siehe unten) in obigen ersten Anschluss und auspipettieren aus obigem zweiten Anschluss
- Einpipettieren von 50 μl Puffer RX07 (Zusammensetzung siehe unten) in obigen ersten Anschluss

Mit Abschluss der vorgenannten Schritte lagen zwei Proben vor, eine 2D-Probe und eine 3D-Probe. Zudem lag jeweils eine Kontrolle (ohne Ziel-DNA) für die 2D- und 3D-Nanostrukturen vor. Die Kombination der Wahl von Konzentrationen von biotin-BSA, Streptavidin, und Zielstrukturen in dem oben beschriebenen Verfahren waren so gewählt, dass die einzelnen Identifizierungsstrukturen in der folgenden Analyse gut aufgelöst werden konnten.

Die Identifizierungsstrukturen in den beiden Proben wurden anschließend mit einem Epifluoreszenz-Mikroskop abgebildet. Dazu wurde ein Mikroskop vom "Elite" (DeltaVision) verwendet. Denkbar wäre auch die Verwendung eines Typ "Ti Eclipse" (Nikon) Mikropskos. Die verwendete Kamera war eine sCMOS (edge 4.2 von PCO) mit einer Pixelgröße von 6.5 μm. Bei einer Objektiv-Vergrößerung von 100x und einer numerischen Apertur von 1,4 lag somit eine Pixel-Auflösung von 60 nm vor, d.h. ein Pixel im Bild entspricht 60 nm in der Realität. Somit wurden beugungslimitierte Bilder der DNA-Nanostrukturen aufgenommen. Die Filter-Sets waren Chroma 49914 für rote, Chroma 49008 für grüne, Chroma 49020 für blaue Anregung.

Sowohl für die 2D-Probe als auch die 3D-Probe, sowie für beide Kontrollen ohne Ziel-DNA wurden je 10 Bilder pro Farbe aufgenommen. Die Farben wurden sequentiell aufgenommen. Jedes dieser Bilder wurde an einer eigenen Stelle der Probe, die sich mit den Stellen der übrigen Bilder nicht überschnitt und sich in der mittleren Hälfte entlang der Kanalbreite befand, aufgenommen.

Für jede Probe wurden die zugehörigen 10 Bilder ausgewertet. Die hier präsentierte Auswertung zielt darauf ab, die Anzahl der detektierten Punkte in der jeweiligen Probe mit Ziel-DNA und der jeweiligen Probe ohne Ziel-DNA zu vergleichen, bzw. die Anzahl der einfarbigen im Vergleich zu mehrfarbigen Punkten anzugeben. Die Auswertung erfolgte manuell. Dazu wurden die Bilder in dem Bildbearbeitungsprogramm FIJI (www.fiji.sc) geöffnet und einfarbige, dreifarbige, sowie die Gesamtzahl der fluoreszierenden Punkte gezählt. Für 2D-DNA-Nanostrukturen und 3D-DNA-Nanostrukturen wurden Mittelwert und Standardabweichung verschiedener Kennwerte der je 10 zugehörigen Bilder ermittelt und in Fig. 3 aufgetragen.

Wenn auch im vorliegenden Beispiel nicht angewendet, kann statt der manuellen Auswertung eine Auswertung mittels einer eigens programmierten Auswertesoftware erfolgen. Die dafür programmierte Auswertesoftware ist im vorliegenden Beispiel Python-basiert. Die Python-basierte Auswertesoftware lädt die Messdaten, ermittelt lokale Maxima in Bildboxen von 9-15 pixel Größe (je nach Vergrößerung und numerischer Apertur des Objektivs), und errechnet den kummulativen Absolutwert des Gradienten als hintergrundunabhängige Messgröße des Signals. Um zwischen Bildboxen mit und ohne DNA-Nanostrukturen zu unterscheiden wird DBSCAN (density based spatial clustering of applications with noise) (publiziert in Ester, Martin; Kriegel, Hans-Peter; Sander, Jörg; Xu, Xiaowei "A density-based algorithm for discovering clusters in large spatial databases with noise", Proceedings of the Second International Conference on Knowledge Discovery and Data Mining (KDD-96). AAAI Press. pp. 226-231 (1996)) verwendet. Wahlweise werden die Bildboxen in Gruppen unterschiedlicher Werte des kummulativen Absolutwerts des Gradienten eingeteilt, was einer Einteilung in unterschiedlichen Anzahlen von Fluorophoren entspricht. Die Bildboxen mit DNA-Nanostrukturen jedes Farbkanals werden basierend auf ihren transversalen (x, y)-Positionen mit allen Bildboxen mit DNA-Nanostrukturen anderer Farbkanäle verglichen um mehrfarbige Punkte im Bild zu erkennen. Auch die hier präsentierte Auswertung mittels der Python-basierten Software zielt darauf ab die Anzahl der detektierten Punkte in Experimenten mit und ohne Ziel-DNA-Oligomeren zu vergleichen, bzw. die Anzahl der einfarbigen im Vergleich zu mehrfarbigen Punkten anzugeben. Die Software speichert die ermittelten Werte für die Anzahl der einfarbigen Punkte sowie deren Orte im Bild und die Werte für die Anzahl der mehrfarbigen Punkte sowie deren Orte im Bild für die weitere Verwendung ab.

**3. Ergebnisse und Diskussion:**

Um die Eignung der DNA-Nanostrukturen für das beschriebene Verfahren zu evaluieren, wurde gemessen, wieviele DNA-Nanostrukturen in der jeweiligen Kontrolle (ohne Ziel-DNA-Oligomer) im Vergleich zur jeweiligen Probe mit Ziel-DNA-Oligomer zu finden waren (Fig. 3 oben). Damit konnte eine Aussage darüber gemacht werden, welcher Anteil der in Anwesenheit der Zielstruktur gemessenen DNA-Nanostrukturen in der Probe auf unspezifische an die Oberfläche gebundene Nanostrukturen zurückzuführen war. Es zeigte sich, dass die 3D-DNA-Nanostrukturen deutlich weniger (1,2 % bei 3D gegenüber 4,0 % bei 2D) unspezifisch an die Mikroskopie-Oberfläche des Mikroskopie-Probenträgers haften. Der Fehlerbalken (Standardabweichung) für die 3D-DNA Nanostrukturen schneidet die Null-Linie und zeigt damit an, dass bei den 3D-DNA Nanostrukturen die Rate unspezifischer Bindung unter der Auflösungsgrenze der Messreihe liegt.

Des Weiteren wurde gemessen, wieviele dreifarbige Punkte, also als Zielmoleküle interpretierte Datenpunkte, in der Kontrolle im Vergleich zur Probe mit Ziel-DNA-Oligomer zu finden sind, und zwar wieder für die 2D-Probe im Vergleich zur 2D-Kontrolle und für die 3D-Probe im Vergleich zur 3D-Kontrolle. Dies gibt Aufschluss über die Rate der falsch-positiv erkannten Zielstrukturen im Falle der 2D-DNA-Nanostrukturen und im Falle der 3D-DNA-Nanostrukturen. Grund für das Vorhandensein von dreifarbigen Messpunkten in der Kontrolle können beispielsweise die Wechselwirkung nach außen exponierter Farbstoffe unterschiedlicher DNA-Nanostrukturen sein. Dies wurde durch das Design der 3D-DNA-Nanostrukturen mit innenliegenden Farbstoffen vermieden. Fig. 3 (Mitte) zeigt das Ergebnis mit 0,7 % für die 2D-DNA-Nanostrukturen und 0,0% für die 3D-DNA Nanostrukturen. Im 3D-Fall wurde keine einzige falsch-positive Messung gemacht. Das Ergebnis zeigt somit, dass durch die Verwendung mehrerer DNA-Nanostrukturen, die an eine Zielstruktur binden, eine sehr niedrige Rate an falsch positiven Signalen gemessen wird. Ferner zeigt das Ergebnis, das die erfindungsgemäßen 3D-DNA-Nanostrukturen mit innenliegenden Fluoreszenzfarbstoffmolekülen weniger mit der im Beispiel verwendeten Trägeroberfläche interagieren. Dies ist auf deren Lage im Inneren der 3D-Struktur zurückzuführen.

Eine weitere Kennzahl ist der Anteil dreifarbiger Messpunkte an allen Messpunkten in der Probe mit Ziel-DNA-Oligomeren. Sie wurde jeweils für die 2D-Probe und die 3D-Probe aus den Ergebnissen der Bildauswertung berechnet und in Fig. 3 (unten) dargestellt. Aufgrund der Reaktionskinetik hat man unter bestimmten Umständen mit nur teilweise hybridisierten Identifizierungsstrukturen zu rechnen. Dies kann z.B. der Fall sein, wenn die Konzentration der DNA-Nanostrukturen nicht hoch genug für eine Sättigung der Reaktion innerhalb der Dauer der Hybridisierungsreaktion ist. Wenn die Reaktion bei der Messung noch nicht in Sättigung ist, kann ein interner Standard oder auf empirische Daten basierend absolut quantifiziert werden. Der interne Standard gibt bevorzugt einen Vergleichswert mit bekannter Konzentration an, während empirische Daten bevorzugt einen Vergleich von ungesättigter zu gesättigter Messung herstellen können. Für relative Messungen (wie hier durchgeführt) muss dies allerdings nicht beachtet werden, da das Verhältnis von teilweise hybridisierten Identifizierungsstrukturen zu Zielstrukturen unabhängig von Zielstruktur und Ausführungsformen der 3D-DNA-Nanostrukturen ist, und somit die relativen Häufigkeiten der vollständig hybridisierten Identifizierungsstrukturen untereinander die selben wie die relativen Häufigkeiten der Zielstrukturen untereinander sind. Dies verhält sich jedoch aufgrund der ähnlichen Größe vergleichbar für 2D- und 3D-DNA-Nanostrukturen. Allerdings können sowohl aneinander gebundene, als auch unspezifisch an die Oberfläche gebundene DNA-Nanostrukturen das Ergebnis drastisch verschieben. Dies zeigt sich bei Fig. 3 (unten), wo für 2D-DNA Nanostrukturen nur 6,8 % der Messpunkte auf der Oberfläche als zu erkennende Struktur infrage kommen, während es bei den 3D-DNA Nanostrukturen 25,7% sind. Somit kann die Mikroskopieoberfläche im Fall der 3D-DNA-Nanostrukturen effizienter genutzt werden als im Fall der 2D-DNA Nanostrukturen.

Zusammenfassend lässt sich also sagen, dass die erfindungsgemäßen 3D-DNA-Nanostrukturen eine mehr als dreimal geringere Tendenz dazu haben an die Mikroskopieoberfläche des Mikroskopie-Probenträgers unspezifisch zu haften, und eine drastisch und unbezifferbar geringere Tendenz aneinander zu haften, als die 2D-DNA-Nanostrukturen. Die Flächennutzungseffizienz der 3D-DNA-Nanostrukturen ist knapp viermal größer.

Das erfindungsgemäße Verfahren nutzt Mehrfachbindung von DNA-Nanostrukturen um die Spezifität zu erhöhen, verringert also die falsch-positive Identifizierungsrate. Diese wäre im Messverfahren mit nur einer DNA-Nanostruktur-Bindung so hoch wie in Fig. 3 (oben) gezeigt, also bei 4,0 % (2D) und 1,2 % (3D), während sie im erfindungsgemäßen Verfahren bei 0.7 % (2D) und 0 % (3D) liegt.

**Verwendete Puffer**

RX07-Puffer

- 4x SSC (*saline sodium citrate*-Puffer, der aus einer wässrigen Lösung von 150 mM Natriumchlorid und 15 mM Trinatriumcitrat besteht, die mit HCl auf pH 7.0 gebracht wird)

- 5% Dextran Sulfate
- 0,1% Tween20
- 5x Denhardts

Puffer A

- 10 mM Tris-HCl auf pH 7,5
- 100 mM NaCl
- 0,05 % Tween 20

Puffer B

- 10 mM Tris-HCl auf pH 8,0
- 10 mM$MgCl_2$
- 1 mM EDTA
- 0,05 % Tween 20

**Beispiel 4: Genexpressions-Analyse aus Gewebeproben**

Hierbei soll eine Gewebeprobe, z.B. eines Brusttumors auf die Expression einer Anzahl von Markergenen, z.B. 100 Gene wie Her2/neu, Estrogen Rezeptor, Progesteron Rezeptor, TFRC, GAPDH, usw. untersucht werden.
Die Gewebeprobe kann zu Beginn in eine Suspension einzelner Zellen, z.B. enzymatisch und/oder durch Scherkräfte, aufgelöst werden.
Anschließend können die Zellen aufgebrochen werden, z.B. mechanisch, durch Lyse-Puffer, enzymatisch und/oder chemisch oder durch Licht.
Das Lysat kann weiter verarbeitet werden, z.B. kann RNA extrahiert werden, Komponenten können gefiltert werden, Nukleinsäuren können z.B. durch Ethanol-Präzipitation isoliert werden. Alternativ kann das Lysat direkt weiter genutzt werden.
Lysat, die mindestens zwei Nanoreporter (programmiert auf die entsprechenden mRNA Sequenzen welche den zu untersuchenden Genen entsprechen), in manchen Anwendungen auch Substrat-Bindende Adapter (Ziel-Adpater) und Reaktions-Puffer werden zusammengegeben und für eine ausreichende Zeit, z.B. 12h inkubiert, wobei Komplexe (Identifizierungsstrukturen) gebildet werden.
Die Komplexe werden anschließend detektiert, in manchen Anwendungen auf einer Oberfläche, oder in Lösung.
Durch Zählen der einzelnen detektierten und identifizierten mRNA Sequenzen kann eine relative Genexpression bestimmt werden, z.B. wird mRNA 1 200 Mal detektiert und mRNA 2 300 Mal, ist die Genexpression von Gen 2 3/2 höher als von Gen 1.
Zusätzlich können Nukleinsäuren bekannten Konzentration als Referenz im Inkubationsschritt zugegeben werden, welche an definierte Nanoreporter binden (genau vergleichbar zu einer mRNA Sequenz). Damit können die übrigen detektierten mRNA Sequenzen normiert und absolut qualifiziert werden. Wird diese Referenz z.B. 100 mal identifiziert, bei einer initialen Konzentration von 100 pM, kann die Konzentration von mRNA 1 auf 200 pM bestimmt werden.

**Beispiel 5: Proteindetektion**

Eine Detektion und Identifikation von Protein-Zielmolekülen kann in ähnlicher Weise wie die von Nukleinsäure-Zielmolekülen erfolgen.
Voraussetzung hierfür ist, dass das Protein-Zielmolekül mindestens zwei unterscheidbare Epitope besitzt, welche spezifisch durch entsprechende Binder (Antikörper, Aptamere, Nanobodies, Adhirons) gebunden werden können.
Diese Adapter können spezifisch an Nanoreporter gebunden werden, die sie identifizieren. Proteinbasierte Binder (Antikörper, Adhirons, Nanobodies), können dazu mit einer spezifischen, kurzen (15 -35 nt) DNA Sequenz (über SNAP-Tags, HALO-Tags, Click-Chemistry, SMCC-Linker, NHS/Amino-Reaktionen oder Ähnliches) modifiziert werden, deren reverses Komplement an den entsprechenden DNA Nanostrukturen (Nanoreporter) befestigt ist und somit zum Adapter wird. Nukleinsäure-basierte Binder (RNA- oder DNA-Aptamere) können durch eine entsprechende Sequenz zum Adapter verlängert werden, wobei deren reverses Komplement an den entsprechenden DNA Nanostrukturen befestigt ist. Durch Zusammengabe von Protein-Zielmolekülen und Adaptern an DNA Nanostrukturen und durch ausreichende Reaktionszeit, bilden sich detektierbare Komplexe aus Ziel-Protein und mindestens zwei Adaptern welche an jeweils eine DNA Nanostruktur gekoppelt sind.
Die Komplexe lassen sich durch simultane Detektion der mindestens zwei DNA Nanostrukturen identifizieren.
Beispiel zur Detektion von zwei Protein-Zielen:

Protein-Ziel 1 mit Epitop A und Epitop B, an welche jeweils Antikörper A und Antikörper B binden.
Protein-Ziel 2 mit Epitop C und Epitop D, an welche jeweils Antikörper C und Antikörper D binden.
Antikörper A wird an eine DNA Nanostruktur gekoppelt, welche mit roten Fluoreszenzfarbstoffen markiert ist.
Antikörper B wird an eine DNA Nanostruktur gekoppelt, welche mit grünen Fluoreszenzfarbstoffen markiert ist.
Antikörper C wird an eine DNA Nanostruktur gekoppelt, welche mit gelben Fluoreszenzfarbstoffen markiert ist.
Antikörper D wird an eine DNA Nanostruktur gekoppelt, welche mit blauen Fluoreszenzfarbstoffen markiert ist.

Zusammengabe der Protein-Ziele und der gekoppelten Antikörper/DNA Nanostrukturen und Reaktion für eine ausreichende Zeit, z.B. 12 Stunden.

Messung der Komplexe in einer Fluss-Detektion. Die Reaktionslösung wird so verdünnt, dass nur in extrem seltenen Fällen mehr als ein Komplex detektiert wird. Simultane Detektion von rot/grün identifiziert ein einziges Protein-Ziel 1, simultane Detektion von gelb/blau identifiziert ein einziges Protein-Ziel 2. Durch Zählen der unterschiedlichen Detektionen können nach vorheriger Kalibration die Protein-Ziele 1 und 2 quantifiziert werden.

Mit gleichem Verfahren lassen sich auch Cluster von Proteinen identifizieren, wobei hier unterschiedliche Antikörper unterschiedliche Proteine (statt unterschiedlicher Epitope) im Ziel-Cluster identifizieren.

## Beispiel 6: Genexpressionsanalyse dreier Gene

Um eine besonders bevorzugten Ausführungsform des Verfahrens zu demonstrieren, wird eine Untersuchung der Genexpression der Gene GAPDH, TFRC und ACTB von HeLa-Zellen näher beschrieben. Die mRNAs der Gene GAPDH, TFRC und ACTB sind also die Zielstrukturen und die HeLa-Zellen sind die Wirtskörper.

Dazu wird ein geeignetes Set von 3D-DNA-Nanostrukturen vorbereitet. Es besteht aus den 3D-DNA-Nanostrukturen 3D_1_1, 3D_1_2, 3D_2_1, 3D_2_2, 3D_3_1 und 3D_3_2. Diese sind ähnlich zu den 3D-DNA-Nanostrukturen 3D_1, 3D_2 und 3D_3 wie sie in Beispiel 1 beschrieben worden sind, jedoch mit folgendem Unterschied: im Falle von 3D_1_1 ist die Zusammensetzung wie für 3D_1 mit dem Unterschied, dass die verwendeten zielbindenden DNA-Oligos partiell komplementär zu einem Bereich einer mRNA von GAPDH (SEQ_ID_NO 1270 statt SEQ_ID_NO 1263) sind, im Falle von 3D_1_2 ist die Zusammensetzung wie für 3D_2 mit dem Unterschied, dass die verwendeten zielbindenden DNA-Oligos partiell komplementär zu einem anderen Bereich einer mRNA von GAPDH sind (SEQ_ID_NO 1276 statt SEQ_ID_NO 1264) sind, im Falle von 3D_2_1 ist die Zusammensetzung wie für 3D_1 mit dem Unterschied, dass die verwendeten zielbindenden DNA-Oligos partiell komplementär zu einem Bereich einer mRNA von TFRC (SEQ_ID_NO 1271 statt SEQ_ID_NO 1263) sind, im Falle von 3D_2_2 ist die Zusammensetzung wie für 3D_3 mit dem Unterschied, dass die verwendeten zielbindenden DNA-Oligos partiell komplementär zu einem Teil einem anderen Bereich einer mRNA von TFRC (SEQ_ID_NO 1277 statt SEQ_ID_NO 1265) sind, im Falle von 3D_3_1 ist die Zusammensetzung wie für 3D_2 mit dem Unterschied, dass die verwendeten zielbindenden DNA-Oligos partiell komplementär zu einem Bereich einer mRNA von ACTB (SEQ_ID NO 1272 statt SEQ_ID_NO 1264) und im Falle von 3D_3_2 ist die Zusammensetzung wie für 3D_3 mit dem Unterschied, dass die verwendeten zielbindenden DNA-Oligos partiell komplementär zu zu einem anderen Bereich einer mRNA von ACTB (SEQ_1D_NO 1278 statt SEQ_ID_NO 1265).

Für dieses Beispiel wird eine Mikro-Well Kammer verwendet. Diese weist ein Mikro-Well Array mit einer 1. Schicht aus einem 170 μm dicken Quarz-Waver und einer 2. Schicht aus Liquid Glass auf. Eine generell für alle Ausführungsformen der Erfindung mögliche Komposition des Liquid Glass lautet wie folgt: 49 Vol% HEMA (Monomer, Hydroxyethylmethacrylat); 5 vol% TEGDA (Polymer, Tetra(ethylenglycol)diacrylat); 18 vol% POE (Lysemittel, phenoxyethanol); 28 vol% Aerosl OX50 (Silica Nanopulver); 0,5 wt% DMPAP (Photoinitiator, 2,2Dimethoxy-2-phenylacetophenon (siehe bspw. F. Kotz et al, "Three-dimensional printing of transparent fused silica glass", Nature 2017, 544 (7650), 337-339 sowie F.Kotz et al., "Liquid Glass: A Facile Soft Replication Method for Structuring Glass", Advanced Materials 2016, 28, 4646-4650). Die 3. Schicht der Mikro-Well Kammer wird hier durch ein ibidi sticky-slide VI 0.4 (ibidi GmbH) gebildet, das auf die 2. Schicht geklebt ist. Alternativ können andere Kleber oder andere Anbringungstechniken verwendet werden. Die 3. Schicht weist eine Vertiefung zur Bildung eines Hohlraums auf. Die 1., 2. und 3. Schicht sind dabei so angeordnet, dass sie einen Arbeitshohlraum mit Mikro-Wells nach bereits beschriebener Art bilden. Das Volumen des Arbeitshohlraum inklusive der Mikro-Wells beträgt hier bspw. 40 Mikroliter.

Als nächster Schritt wird eine Lösung von 1 mg/ml biotinyliertes BSA in Puffer A (biotin-BSA: Produkt A8549, Sigma-Aldrich GmbH) in den Arbeitshohlraum gespült und für 10 Minuten inkubiert. Anschließend folgt ein Spül-Schritt mit 0,5 ml Puffer A (Rezept siehe unten). Anschließend wird eine Lösung von Streptavidin in Puffer A mit einer Konzentration von 0,5 mg/ml eingespülten und für 10 Minuten inkubiert. Während der Inkubationszeit bindet mindestens ein Teil des Streptavidins an die Oberflächen der Mikro-Wells.

Anschließend folgt ein Spül-Schritt mit 0,5 ml Puffer A und ein weiterer Spül-Schritt mit 0,5 ml Puffer RX07 (Rezept siehe unten). Danach wird eine Lösung von 1 nM Biotin-Adapter-Oligo (SEQ_ID_NO 1262) in RX07 eingespülten und für 10 Minuten inkubiert. Während der Inkubationszeit bindet mindestens ein Teil der Biotin-Adapter-Oligos vermittelst Biotin- Streptavidin-Bindung an die an den Oberflächen gebundenen Streptavidine und somit selbst an die Oberflächen.

Anschließend folgt ein Spül-Schritt mit 0,5 ml Puffer RX 07. Danach wird eine Lösung mit SEQ_ID_NO 1273, 1274 und 1275 (siehe unten), jeweils mit einer Konzentration von 1 nM, in Puffer RX07 eingespült und 10 Minuten lang inkubiert, sodass SEQ_ID_NO 1273, 1274 und 1275 an Biotin-Adapter-Oligos SEQ_ID_NO 1262 binden.

Anschließend wird nach einem Spül-Schritt mit 0,5 ml Puffer RX 07 eine Zellsuspension mit einer Dichte von 1 Million Zellen pro Milliliter eingespült. Bei dem Puffer der Zellsuspension handelt es sich um PBS (Phosphate Buffered Saline, Rezept siehe unten). Den Zellen wird 10 Minuten Zeit gegeben, um in die Mikro-Wells zu sinken.

Anschließend wird die Mikro-Well Kammer für die weitere Untersuchung und die Auswahl der mit genau einer Zelle besetzten Mikro-Wells auf ein Mikroskop verbracht. Das Anordnen der Mikro-Well Kammer auf dem Mikroskop auch zu einem früheren Zeitpunkt, beispielsweise ganz am Anfang der geschilderten Schritte stattfinden kann. Bevorzugt ist das Mikroskop für alle nachfolgenden Mikroskopie-Analyseschritte geeignet. Alternativ kann auch zwischen verschiedenen Mikroskopen für verschiedene Mikroskopietechniken gewechselt werden. Sodann werden die einzelnen Mikro-Wells danach kategorisiert, ob sie mehrere Zellen, genau eine Zelle oder keine Zellen aufweisen. Nur diejenigen Mikro-Wells, die genau eine Zelle aufweisen, werden in der späteren Analyse berücksichtigt. Diese Kategorisierung kann mit jedweder geeigneten Mikroskopietechnik erfolgen, bevorzugt mit Hellfeld, Phasenkontrast, Dunkelfeld, oder DIC (differential interference contrast)-Mikroskope oder Fluoreszenzmikroskopie, darunter bevorzugt Epifluoreszenzmikroskopie, TIRF-Mikroskopie, Punktraster-Konfokalmikroskopie, Spinning-Disc Konfokalmikroskopie, Lichtblattmikroskopie.

Anschließend wird ein Aufschluss-Puffer, genauer ein Lyse-Puffer (Rezept siehe unten) in die Mikro-Well Kammer eingespült und für 30 Minuten inkubiert. Dadurch werden die Zellen lysiert und etwaige darin enthaltene Zielstrukturen freigesetzt. Die Zielstrukturen binden nun an die Träger-Adapter an den Böden der einzelnen Mikro-Wells, in denen die Zielstrukturen freigesetzt wurden.

Nach einem Spül-Schritt mit 0,5 ml RX 07, bei dem die Zielstrukturen aufgrund ihrer Bindung an die Böden in den Mikro-Wells verbleiben, wird eine Lösung mit jeweils 50 nM 3D_1_1, 3D_1_2, 3D_2_1, 3D_2_2, 3D_3_1 und 3D_3_2 in RX 07 eingespült und 60 Minuten lang inkubiert. Während der Inkubation binden viele der 3D-DNA-Nanostrukturen an eine korrespondierende Zielstruktur.

Es folgen 3 Spül-Schritte mit jeweils 0,5 ml RX 07.

Anschließend werden Fluoreszenz-Aufnahmen des bodennahen Bereichs der zuvor für die Analyse ausgewählten Mikro-Wells einschließlich der in den 3D-DNA-Nanostrukturen verwendeten Wellenlängen gemacht. Da im vorliegenden Beispiel lediglich 3 Gene untersucht werden, konnten die 3D-DNA-Nanostrukturen so ausgewählt werden, dass zwei Typen von 3D-DNA-Nanostrukturen, hier 3D_1_1 und 3D_2_1, eine 1. Farbe, bevorzugt Rot, aufweisen, zwei 2. Typen von 3D-DNA-Nanostrukturen, hier 3D_1_2 und 3D_3_1, eine 2. Farbe, bevorzugt Grün, aufweisen und zwei Dritte Typen von 3D-DNA-Nanostrukturen, hier 3D_2_2 und 3D_3_2 eine 3. Farbe, bevorzugt Blau, aufweisen.

Dann erfolgt die Evaluation, d.h. der Nachweis bzw. die Quantifizierung, der Zielstrukturen, indem Punkte der jeweiligen Farbe in jedem der zur Analyse ausgewählten Mikro-Wells gesucht werden. Ein rot-grüner Punkt steht für GAPDH-mRNA, ein rot-blauer Punkt steht für TFRC-mRNA und ein grünblauer Punkt steht für ACTB-mRNA.

Eine Auftragung der Anzahl an gefundenen farbigen Punkten je Mikro-Well in zwei Scatter-Plots ermöglicht bei Bedarf die weitere Auswertung. Im 1. Scatter-Plot wird die Anzahl der gefundenen Punkte je Mikro-Well für die Farben Rot versus Grün aufgetragen, während im 2. Scatter-Plot die Anzahl der gefundenen Punkte je Mikro-Well für die Farben Grün versus Blau (oder alternativ Rot versus Blau) aufgetragen wird. Aus den Scatter Plots lassen sich z.B. die Genexpressionslevel für die drei untersuchten Gene sowie verschiedene Subpopulationen von Zellen ablesen.

**Rezepte zum Beispiel 6:**

| | |
|---|---|
| *Puffer A:* | Siehe Puffer A in Beispiel 3 |
| *RX07-Puffer:* | Siehe RX07 Puffer in Beispiel 3 |

*Lyse Puffer*

- 20 mM Tris pH 7.5
- 150 mMNaCl
- 1 mM EDTA
- 1 mM EGTA

- 1 % protease inhibitor cocktail, P-8340 for mammalian cells, sigma-aldrich

*PBS Puffer (Phosphate Buffered Saline)*

- 137 mM NaCl
- 2,7 mM KCl
- 10 mM $Na_2HPO_4$
- 1,8 mM $KH_2PO_4$

**SEQ IDs**

SEQ_ID_NO: 1270 T4_gapdh
TTGTGTCGTGACGAGAAACACCAAATTTCAACTTTTTTTCCATTGATGACAAG
CTTCCCGTTCTCAGCCTT

SEQ_ID_NO: 1271 T4_tfrc
TTGTGTCGTGACGAGAAACACCAAATTTCAACTTTTTCGAGTTTTGAGCGCTGT
CTTTGACCTGAATCTTAAC

SEQ_ID_NO: 1272 T4_actb
TTGTGTCGTGACGAGAAACACCAAATTTCAACTTTTTAATGATCTTGATCTTCA
TTGTGCTGGGTGCCAG

SEQ_ID_NO: 1273 Trägeradapter_gapdh

GCCATGGGTGGAATCATATTGGAACATGTAAACCTTCGGTTGTACTGTGACCG
ATTC

SEQ_ID_NO: 1274 Trägeradapter_tfrc
CACGCCAGACTTTGCTGAGTTTAAATTCACGTTCGGTTGTACTGTGACCGATTC

SEQ_ID_NO: 1275 Trägeradapter_actb
ATGATGGAGTTGAAGGTAGTTTCGTGGATGCCACATTCGGTTGTACTGTGACCG
ATTC

SEQ_ID_NO 1276 T5_gapdh
TTGTGTCGTGACGAGAAACACCAAATTTCAACTTTTctgggtggcagtgatggcatggactgtg
gtcatgagtcct

SEQ_ID_NO 1277 T5_TFRC

TTGTGTCGTGACGAGAAACACCAAATTTCAACTTTTTacccatcttttaagaccatatctgagaac atctgggc

SEQ_ID_NO 1278 T5_ACTB

TTGTGTCGTGACGAGAAACACCAAATTTCAACTTTTTcagtgtacaggtaagccctggctgcct ccaccc

Die folgenden Aspekte sind bevorzugte Ausführungsformen der vorliegenden Erfindung.

1. Verfahren zum Nachweis einer Zielstruktur, umfassend:

p1) Einbringen einer Gruppe von Wirtskörpern inklusive eines Wirtskörpers mit der Zielstruktur in ein Mikro-Well Array derart, dass in mindestens einem Mikro-Well genau ein Wirtskörper vorhanden ist;
p2) Einbringen von mindestens zwei 3D-DNA-Nanostrukturen in das mindestens eine Mikro-Well, wobei jede der 3D-DNA-Nanostrukturen ein oder mehrere innenliegende Fluoreszenz-Farbstoffmoleküle aufweist;

a) Ausbilden einer Identifizierungsstruktur in dem mindestens einen Mikro-Well, die aufweist:

(i) die Zielstruktur, und
(ii) die mindestens zwei 3D-DNA-Nanostrukturen, wobei jede der 3D-DNA-Nanostrukturen spezifisch an die Zielstruktur gebunden ist, und wobei die 3D-DNA-Nanostrukturen an paarweise unterschiedliche Regionen der Zielstruktur gebunden sind;

b) Nachweis der Zielstruktur durch Messen mindestens eines Fluoreszenzsignals,

wobei die 3D-DNA-Nanostrukturen und die Parameter der Fluoreszenzmessung derart ausgewählt sind, dass das mindestens eine gemessene Fluoreszenzsignal der in a) ausgebildeten Identifizierungsstruktur von dem Fluoreszenzsignal jeder der mindestens zwei isolierten 3D-DNA-Nanostrukturen unterscheidbar ist, wenn diese nicht in der Identifizierungsstruktur gebunden sind, wobei die Identifizierungsstruktur an eine erste Oberfläche, bevorzugt den Boden des mindestens einen Mikro-Wells gebunden ist.

2. Verfahren nach Aspekt 1, wobei der Wirtskörper eine Zelle, ein Virus, ein Exosom, ein Vesikel und/oder ein Droplet ist.

3. Verfahren nach Aspekt 1 oder 2, wobei die Zielstruktur in einem Wirtskörper vorliegt, wobei das Verfahren ferner umfasst:
e) Aufschließen der Wirtskörper, vorzugsweise durch Flüssigkeitsaustausch mit einem Aufschluss-Puffer, um die Zielstruktur aus dem Wirtskörper zu befreien.

4. Verfahren nach einem der Aspekte 1 - 3, wobei die Zielstruktur ein Polynukleotid, bevorzugt ein teilweise einzelsträngiges Polynukleotid, bevorzugt ein einzelsträngiges Polynukleotid und besonders bevorzugt eine m-RNA aufweist oder ist.

5. Mikro-Well Array für die Fluoreszenzmikroskopie, das mehrere Mikro-Wells aufweist, wobei jedes Mikro-Well einen Boden und eine Umfangswand aufweist, wobei die Böden für Fluoreszenzmikroskopie geeignet sind und wobei jedes der Mikro-Wells eine bevorzugt offene Oberseite aufweist,
wobei das Mikro-Well Array ferner aufweist:

eine 1. Schicht, die die Böden der Mikro-Wells bildet, wobei die 1. Schicht für Fluoreszenzmikroskopie geeignet ist und vorzugsweise ein Deckgläschen ist;
eine 2. Schicht, die an der 1. Schicht angebracht ist, wobei die 2. Schicht die Wände der Mikro-Wells bildet.

6. Mikro-Well Array nach Aspekt 5, wobei ein oder mehrere Träger-Adapter am Boden mindestens eines der Mikro-Wells gebunden ist.

7. Mikro-Well Array nach einem der Aspekte 5 - 6 wobei die 2. Schicht SU-8, bevorzugt PEG-DA, besonders bevorzugt PDMS, stärker bevorzugt Cytop, und besonders bevorzugt Liquid Glass aufweist und/oder daraus besteht.

8. Verfahren nach einem der Aspekte 1 - 4, wobei das Mikro-Well Array ein Mikro-Well Array nach einem der Aspekte 5 - 7 ist und wobei die 1. Oberfläche der Boden des mindestens einen Mikro-Wells ist, wobei bevorzugt die Zielstruktur vermittelt durch einen Träger-Adapter, der an die Zielstruktur spezifisch bindet, an den Boden des Mikro-Wells gebunden ist bzw. wird.

9. Verfahren nachAspekt 8, ferner umfassend:
f) Schließen der Oberseite der Mikro-Wells durch Aufbringen einer Ölschicht auf die 2. Schicht;
wobei optional das Öl mit Lipiden versetzt ist und das Verfahren optional ferner umfasst:
g) Abziehen des Öls durch Überschütten des Mikro-Well Arrays mit wässriger Lösung. wobei optional die wässrige Lösung mit Membrankanälen versetzt ist.

10. Verfahren nach einem der Aspekte 1 - 4 oder 8 - 9, wobei das Verfahren ferner zusätzlich für den Nachweis einer oder mehrerer weiterer, voneinander verschiedener Zielstrukturen geeignet ist, wobei sich die verschiedenen Zielstrukturen paarweise unterscheiden,
wobei für jede der Zielstrukturen die Gruppe von Wirtskörpern in Schritt p1) mindestens einen Wirtskörper mit der jeweiligen Zielstruktur aufweist;
und wobei das Verfahren ferner umfasst:

c) für jede der einen oder mehreren weiteren, voneinander verschiedenen Zielstrukturen: Ausbilden jeweils einer zugeordneten Identifizierungsstruktur, wobei jede der weiteren Identifizierungsstrukturen aufweist:

(i) die jeweils zugeordnete weitere Zielstruktur, und
(ii) mindestens zwei 3D-DNA-Nanostrukturen, wobei jede der mindestens zwei 3D-DNA-Nanostrukturen einen oder mehrere innenliegende Fluoreszenz-Farbstoffmoleküle aufweist und wobei jede der mindestens zwei 3D-DNA-Nanostrukturen spezifisch an die jeweilige weitere Zielstruktur gebunden ist, und wobei die mindestens zwei 3D-DNA-Nanostrukturen an paarweise unterschiedliche Regionen der jeweiligen Zielstruktur gebunden sind;

und wobei der Schritt a) ferner umfasst:
d) Nachweis der einen oder mehreren weiteren Zielstrukturen durch Messen des mindestens einen Fluoreszenzsignals,

wobei alle 3D-DNA-Nanostrukturen und die Parameter der Fluoreszenzmessung derart ausgewählt sind, dass das mindestens eine gemessene Fluoreszenzsignal der in a) und c) ausgebildeten Identifizierungsstrukturen von dem Fluoreszenzsignal aller isolierten 3D-DNA-Nanostrukturen unterscheidbar ist, wenn diese nicht in einer der Identifizierungsstrukturen gebunden sind, und dass die gemessenen Fluoreszenzsignale aller ausgebildeten Identifizierungsstrukturen paarweise voneinander unterscheidbar sind, wobei jede der verschiedenen Zielstrukturen mehrfach vorliegen kann und das Verfahren den mehrmaligen Nachweis einer oder mehrerer der verschiedenen Zielstrukturen umfassen kann.

11. Verfahren nach einem der Aspekte 8 - 10, wobei in Schritt p1) die Wirtskörper vereinzelt werden, indem ein Volumen einer Flüssigkeit, die die Wirtskörper enthält, auf die offene Seite des Mikro-Well Arrays aufgebracht wird und den Wirtskörpern Zeit gegeben wird, durch Wirkung der Schwerkraft auf den Boden der Mikro-Wells zu sinken.

12. Verfahren nach einem der Aspekte 1 - 4 oder 8 - 11, wobei das Verfahren ferner umfasst:

aa) Herstellen einer Träger-Matrix in dem mindestens einen Mikro-Well; und optional:
bb) Befestigen von Träger-Adaptern an der Träger-Matrix.

13. Verfahren nach einem der Aspekte 1 - 4 oder 9 - 12
h) Entfernen der Umfangswände der Mikro-Wells, vorzugsweise der 2. Schicht, von den Böden zwischen den Schritten p1) und a) oder a) und b).

14. Ein Kit, das aufweist:

ein Mikro-Well Array, vorzugsweise nach einem der Aspekte 5 - 7;
mindestens zwei 3D-DNA-Nanostrukturen, wobei jede der 3D-DNA-Nanostrukturen mindestens ein innenliegendes Fluoreszenz-Farbstoffmolekül aufweist.

15. Kit nach Aspekt 14, wobei für jede der mindestens zwei 3D-DNA-Nanostrukturen ein Abstand des mindestens einen innenliegenden Fluoreszenz-Farbstoffmoleküls zum Rand der 3D-DNA-Nanostruktur mindestens 2 nm, bevorzugt mindestens 3 nm und besonders bevorzugt mindestens 5 nm beträgt, wobei optional mindestens eine der 3D-DNA-Nanostrukturen mindestens 2 innenliegende Fluoreszenz- Farbstoffmoleküle aufweist, und wobei der Abstand der mindestens zwei innenliegenden Fluoreszenz-Farbstoffmoleküle paarweise mindestens 2 nm, bevorzugt mindestens 5 nm und besonders bevorzugt mindestens 9 nm beträgt.

## Patentansprüche

1. Verfahren zum Nachweis einer Zielstruktur, umfassend:

   a) Ausbilden einer Identifizierungsstruktur, die aufweist:

      (i) die Zielstruktur, und
      (ii) mindestens zwei 3D-DNA-Nanostrukturen, wobei jede der 3D-DNA-Nanostrukturen ein oder mehrere innenliegende Fluoreszenz-Farbstoffmoleküle aufweist und wobei jede der 3D-DNA-Nanostrukturen (spezifisch) an die Zielstruktur gebunden ist,

   b) Nachweis der Zielstruktur durch Messen mindestens eines Fluoreszenzsignals,

   wobei die 3D-DNA-Nanostrukturen und die Parameter der Fluoreszenzmessung derart ausgewählt sind, dass das mindestens eine gemessene Fluoreszenzsignal der in a) ausgebildeten Identifizierungsstruktur von dem Fluoreszenzsignal jeder der mindestens zwei isolierten 3D-DNA-Nanostrukturen unterscheidbar ist, wenn diese nicht in der Identifizierungsstruktur gebunden sind.

2. Verfahren nach Anspruch 1, wobei die Identifizierungsstruktur in Schritt b) an einen Träger gebunden ist.

3. Verfahren nach Anspruch 2, wobei der Träger ein Polymernetzwerk aufweist, wobei das Polymernetzwerk bevorzugt eines oder eine Kombination der folgenden Materialien aufweist: Biopolymer, Agarose, Collagen.

4. Verfahren nach Anspruch 3, wobei die Identifizierungsstruktur in das Polymernetzwerk eingebettet ist/wird und/oder an eine Oberfläche des Polymernetzwerks gebunden ist/wird.

5. Verfahren nach Anspruch 4, ferner umfassend die Schritte:
   p1) Einbringen einer Gruppe von Wirtskörpern inklusive eines Wirtskörpers mit der Zielstruktur in ein Mikro-Well Array derart, dass in mindestens einem Mikro-Well genau ein Wirtskörper vorhanden ist;
   aa) Einbringen eines Polymernetzwerks in das mindestens einen Mikro-Well. p2)
   Einbringen von mindestens zwei 3D-DNA-Nanostrukturen in das mindestens eine Mikro-Well, wobei jede der 3D-DNA-Nanostrukturen ein oder mehrere innenliegende Fluoreszenz-Farbstoffmoleküle aufweist;
   wobei die Identifizierungsstruktur in Schritt b) an das Polymernetzwerkgebunden ist und/oder in das Polymernetzwerk eingebettet ist.

6. Verfahren nach Anspruch 4 oder 5, wobei die Identifizierungsstruktur in Schritt b) an eine Oberfläche des Polymernetzwerks gebunden ist.

7. Verfahren nach einem der Ansprüche 5 oder 6, wobei das Einbringen des Polymernetzwerks in Schritt aa) aufweist: Herstellen des Polymernetzwerks in dem mindestens einen Micro-Well.

8. Verfahren nach einem der Ansprüche 3 bis 7, wobei das Polymernetzwerk eine mittlere Maschengröße von 1 $\mu$m bis 50 $\mu$m, bevorzugt 2 $\mu$m bis 10 $\mu$m aufweist.

9. Verfahren nach einem der Ansprüche 4bis 8, wobei das Verfahren ferner den Schritt des Bindens der ausgebildeten Identifizierungsstruktur an das Polymernetzwerk, bzw. an die Oberfläche des Polymernetzwerks umfasst.

**10.** Verfahren nach einem der vorhergehenden Ansprüche, wobei die Zielstruktur ein teilweise einzelsträngiges Polynukleotid, bevorzugt ein einzelsträngiges Polynukleotid und besonders bevorzugt eine m-RNA aufweist oder ist.

**11.** Verfahren nach einem der Ansprüche 4 bis 10, wobei die Bindung bzw. das Binden der Identifizierungsstruktur an das Polymernetzwerk bzw. die Oberfläche des Polymernetzwerks über die Zielstruktur vermittelt ist bzw. wird.

**12.** Verfahren nach einem der Ansprüche 4 bis 11, wobei im Wesentlichen alle Bindungsstellen für die Identifizierungsstruktur an/in dem Polymernetzwerk bzw. dessen Oberfläche einen Abstand von mindestens 250nm, bevorzugt mindestens 500nm und besonders bevorzugt mindestens 1μm aufweisen.

**13.** Verfahren nach einem der Ansprüche 4 bis 12, wobei die Identifizierungsstruktur vermittelt durch einen Träger-Adapter, der an die Identifizierungsstruktur, bevorzugt die Zielstruktur, spezifisch bindet, an das Polymernetzwerk bzw. die Oberfläche des Polymernetzwerks gebunden ist bzw. wird.

**14.** Verfahren nach Anspruch 13, wobei das Verfahren ferner umfasst:
bb) Befestigen von Träger-Adaptern an dem Polymernetzwerk,
wobei der Schritt bb) vorzugsweise nach Schritt aa) und vor Schritt p2) erfolgt.

**15.** Verfahren nach einem der Ansprüche 13 oder 14, wobei der paarweise Mindestabstand der Träger-Adapter bzw. Träger-Adapterbindestellen im Wesentlichen 200 nm bis 10 μm, bevorzugt 500 nm bis 5 μm, besonders bevorzugt 2 μm bis 3 μm beträgt.

Fig. 1

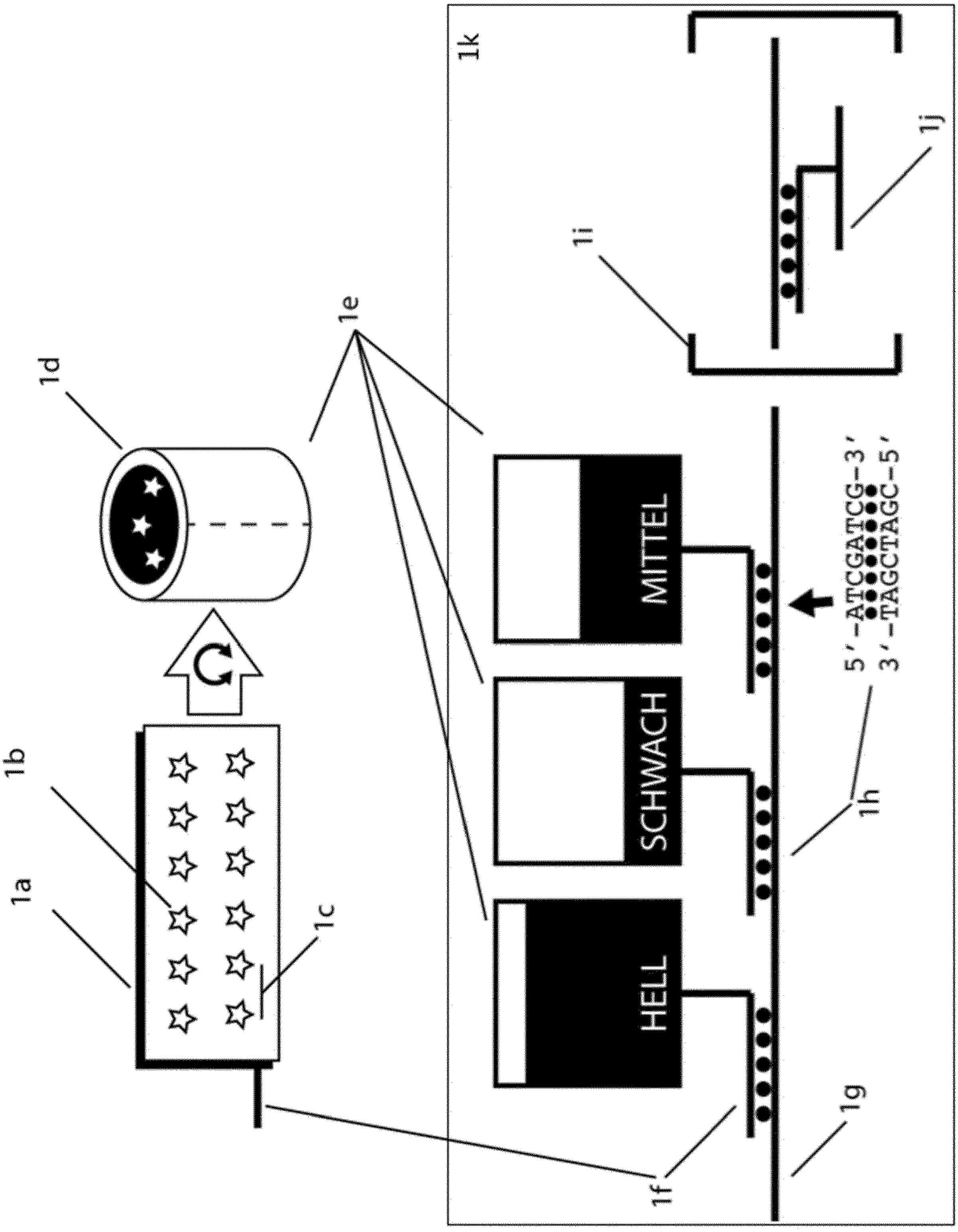
1a
1b
1c
1d
1e
1f
1g
1h
1i
1j
1k
HELL
SCHWACH
MITTEL
5'-ATCGATCG-3'
3'-TAGCTAGC-5'

Fig. 2

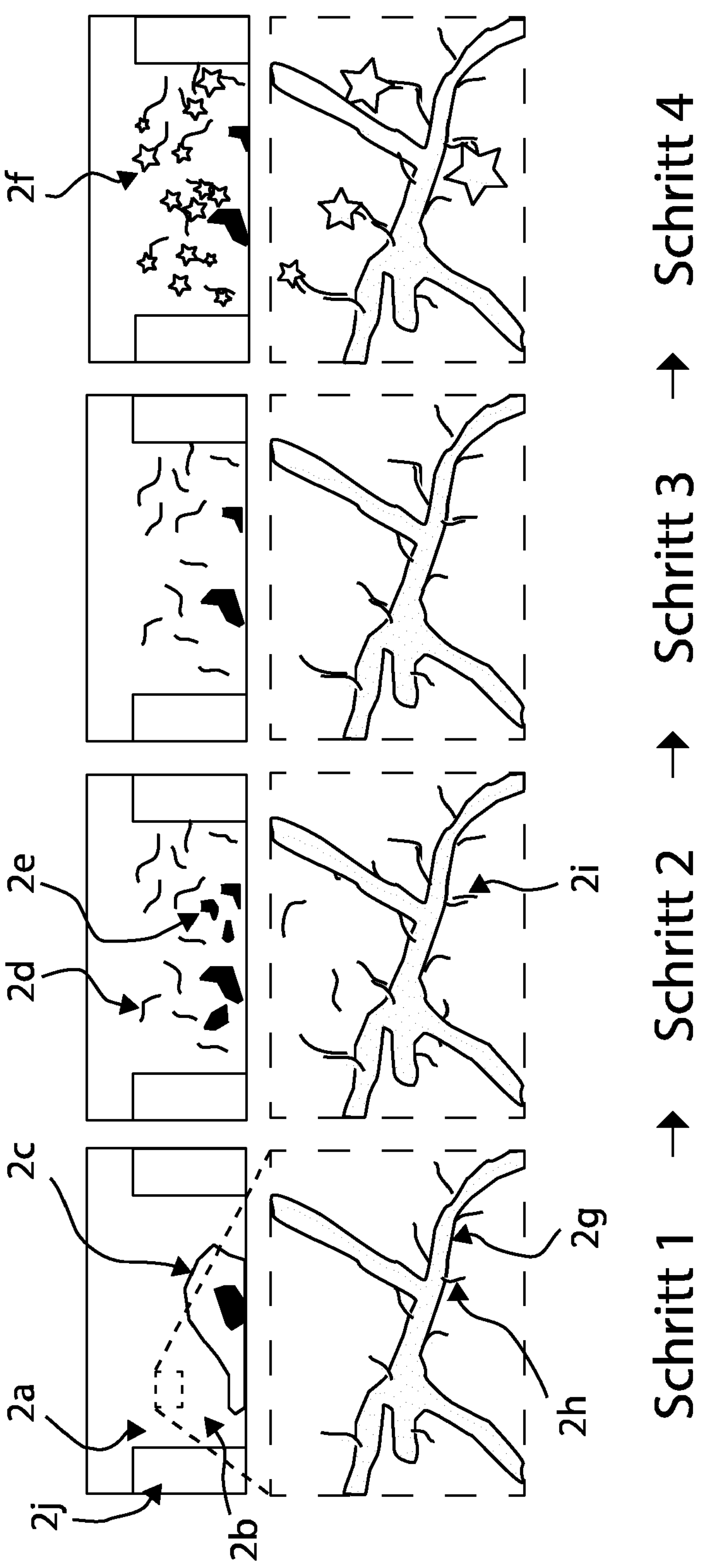
2a
2b
2c
2d
2e
2f
2g
2h
2i
2j
Schritt 1
Schritt 2
Schritt 3
Schritt 4

Fig. 3

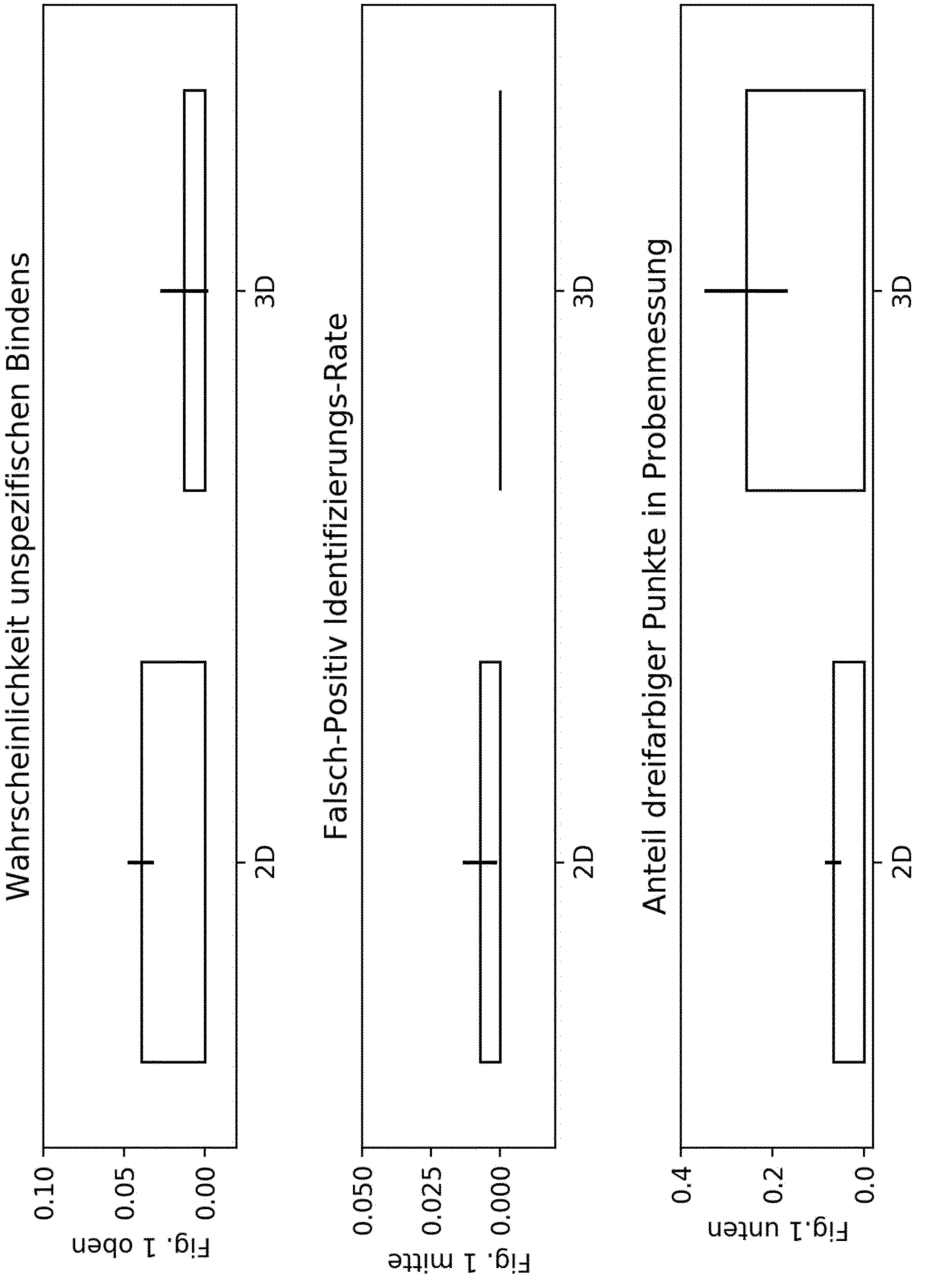
Wahrscheinlichkeit unspezifischen Bindens
Fig. 1 oben
0.10
0.05
0.00
2D
3D
Falsch-Positiv Identifizierungs-Rate
Fig. 1 mitte
0.050
0.025
0.000
2D
3D
Anteil dreifarbiger Punkte in Probenmessung
Fig.1 unten
0.4
0.2
0.0
2D
3D

Fig. 4

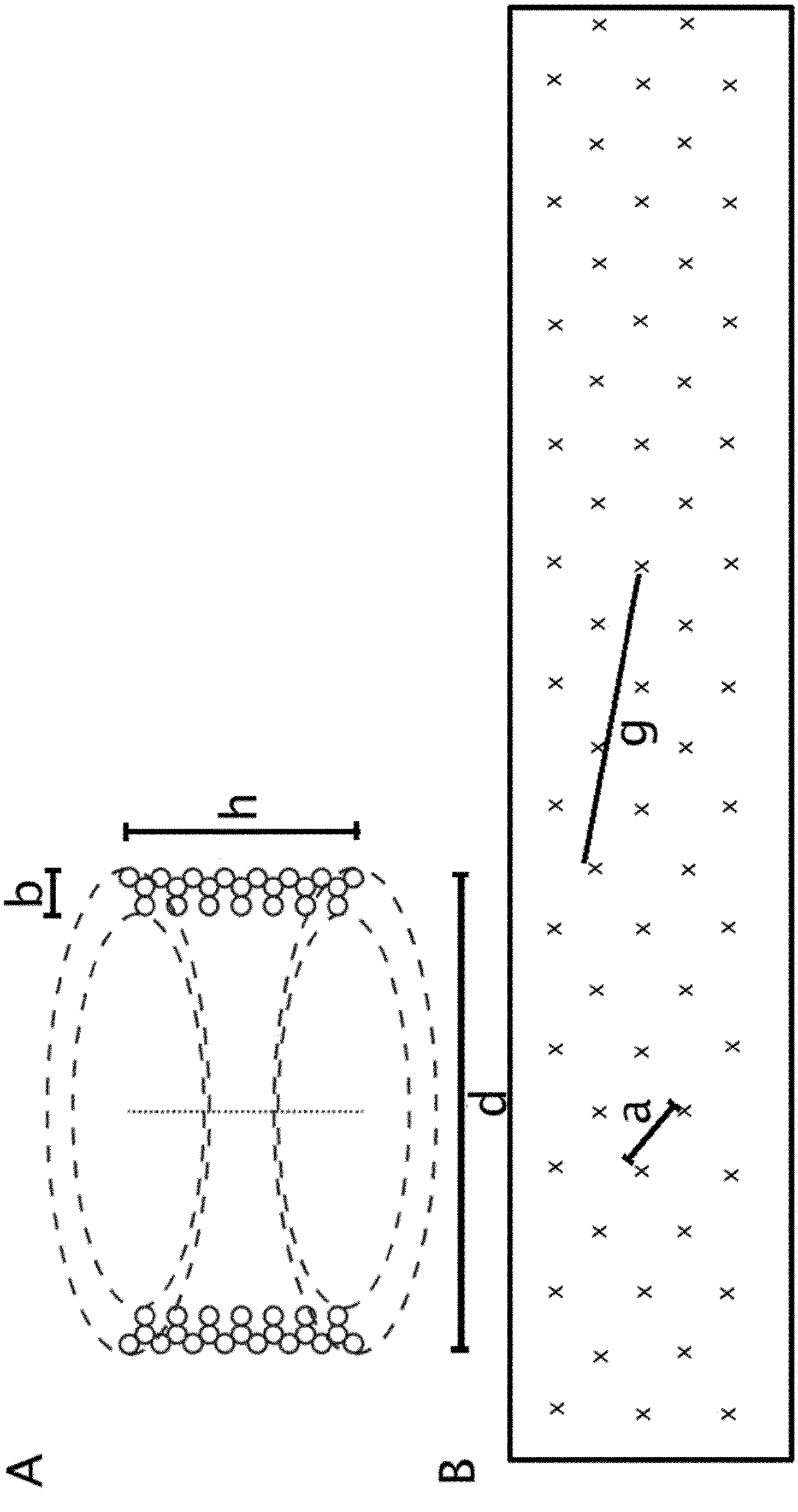

Fig. 5

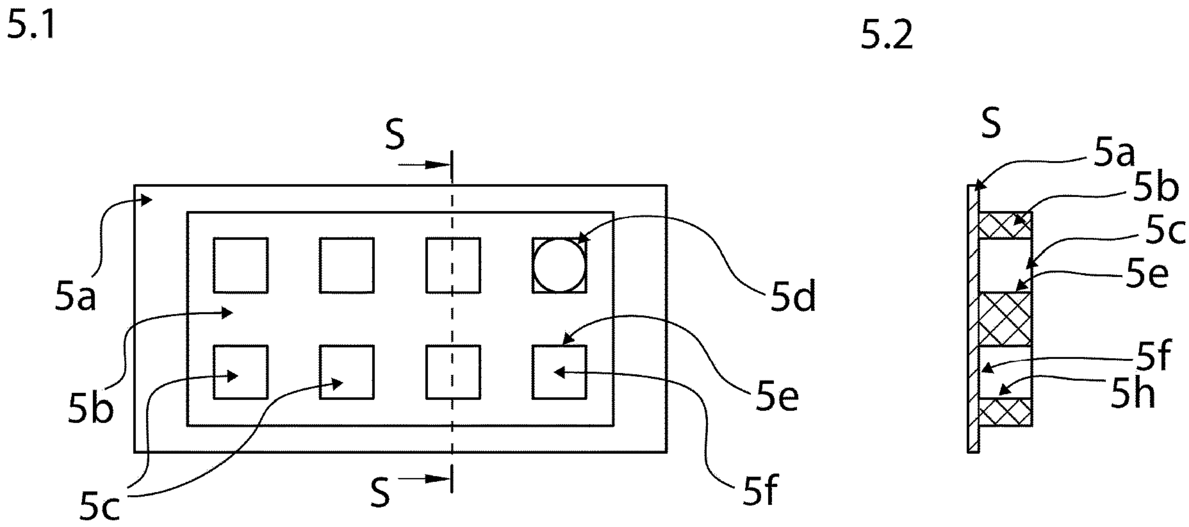
5.1
5.2
S
S
S
5a
5b
5c
5d
5e
5f
5a
5b
5c
5e
5f
5h

Fig. 6

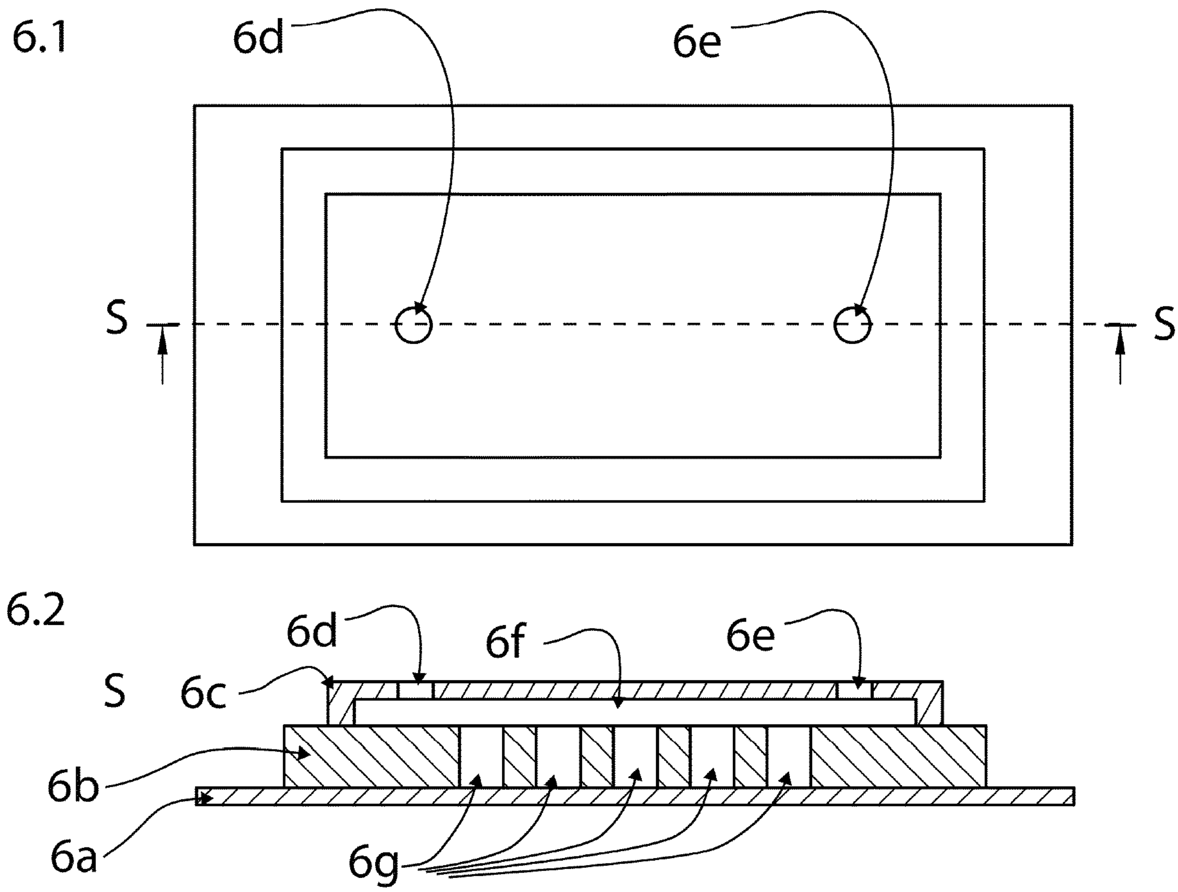
6.1
6d
6e
S
S
6.2
6d
6f
6e
S
6c
6b
6a
6g

Fig. 7

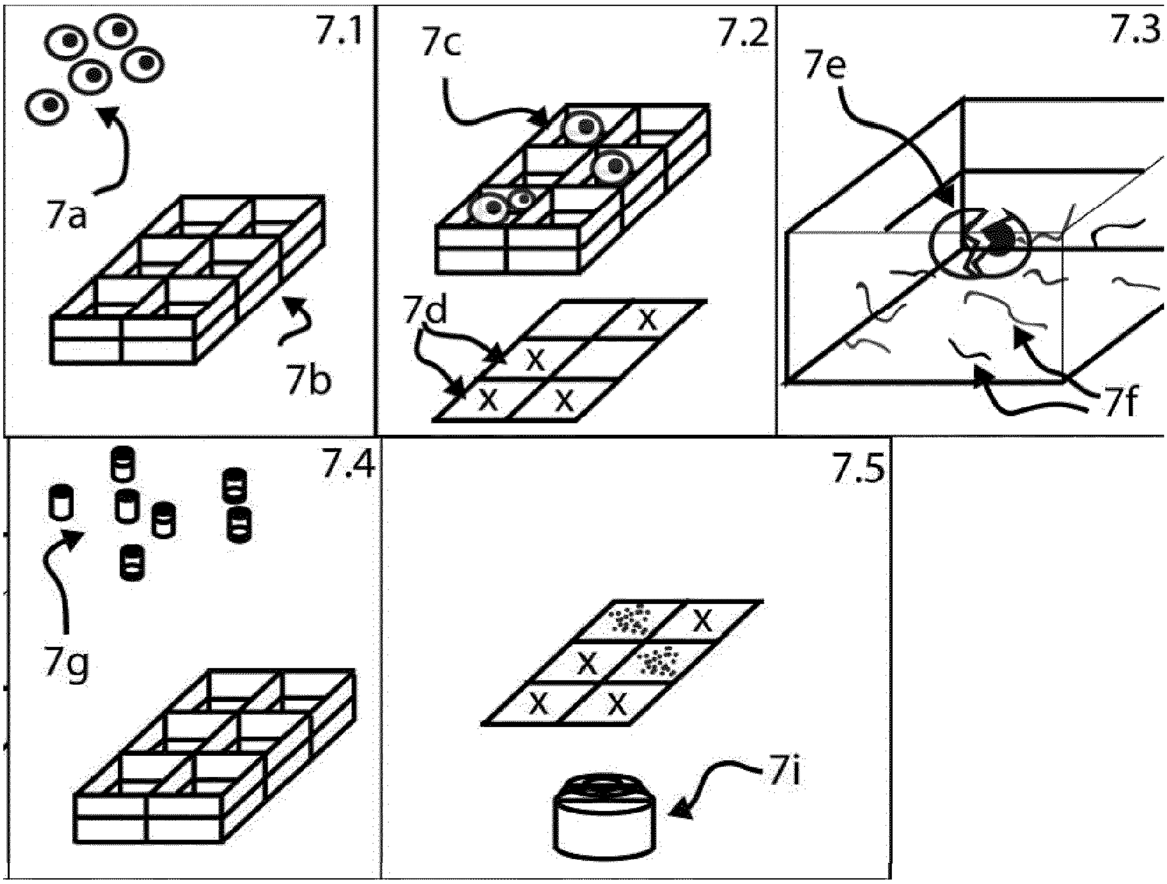

Europäisches Patentamt
European Patent Office
Office européen des brevets

# EUROPÄISCHER RECHERCHENBERICHT

Nummer der Anmeldung
EP 20 20 0076

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | YONGGANG KE ET AL: "Regulation at a distance of biomolecular interactions using a DNA origami nanoactuator", NATURE COMMUNICATIONS, Bd. 7, 18. März 2016 (2016-03-18), Seite 10935, XP055474810, DOI: 10.1038/ncomms10935 * das ganze Dokument * ----- | 1-15 | INV. C12Q1/682 B01L3/00 G01N33/58 |
| A | FAN HONG ET AL: "DNA Origami: Scaffolds for Creating Higher Order Structures", CHEMICAL REVIEWS, Bd. 117, Nr. 20, 25. Oktober 2017 (2017-10-25), Seiten 12584-12640, XP055474939, US ISSN: 0009-2665, DOI: 10.1021/acs.chemrev.6b00825 * das ganze Dokument * ----- | 1-15 | |
| A | WO 2012/151328 A2 (HARVARD COLLEGE [US]; SCHAUS THOMAS E [US]; ZHANG DAVID YU [US]; SUN W) 8. November 2012 (2012-11-08) * p. 9, line 9 - p. 1, line 8, p. 15, line 3 - p. 17, line 26, p. 32, line 26 - p. 41, line 13 * ----- | 1-15 | RECHERCHIERTE SACHGEBIETE (IPC): C12Q B01L G01N |
| A | EBBE S. ANDERSEN ET AL: "Self-assembly of a nanoscale DNA box with a controllable lid", NATURE, Bd. 459, Nr. 7243, 7. Mai 2009 (2009-05-07), Seiten 73-76, XP055246572, GB ISSN: 0028-0836, DOI: 10.1038/nature07971 * das ganze Dokument * ----- -/-- | 1-15 | |

2

Der vorliegende Recherchenbericht wurde für alle Patentansprüche erstellt

| Recherchenort | Abschlußdatum der Recherche | Prüfer |
|---|---|---|
| München | 2. März 2021 | R. von Eggelkraut-G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 20 20 0076

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | US 2016/083783 A1 (BLAINEY PAUL [US] ET AL) 24. März 2016 (2016-03-24)<br>* p. 3, paragraph [0032] - p. 9, paragraph [0078], [0085], pp. 12-18, Ex. 1 *<br>----- | 1-15 | |
| A | EIJI NAKATA ET AL: "Zinc-Finger Proteins for Site-Specific Protein Positioning on DNA-Origami Structures",<br>ANGEWANDTE CHEMIE INTERNATIONAL EDITION,<br>Bd. 51, Nr. 10,<br>27. Januar 2012 (2012-01-27), Seiten 2421-2424, XP055411232,<br>ISSN: 1433-7851, DOI: 10.1002/anie.201108199<br>* das ganze Dokument *<br>----- | 1-15 | |
| A | OCHSNER M ET AL: "MICRO-WELL ARRAYS FOR 3D SHAPE CONTROL AND HIGH RESOLUTION ANALYSIS OF SINGLE CELLS",<br>LAB ON A CHIP, ROYAL SOCIETY OF CHEMISTRY,<br>Bd. 7, 21. Juni 2007 (2007-06-21), Seiten 1074-1077, XP002535450,<br>ISSN: 1473-0197, DOI: 10.1039/B704449F<br>[gefunden am 2007-06-21]<br>* das ganze Dokument *<br>----- | 1-15 | RECHERCHIERTE SACHGEBIETE (IPC) |
| A | WO 2012/120270 A1 (UNIV CARDIFF [GB]; ALLENDER CHRIS JOHN [GB]; SAYERS EDWARD JOHN [GB];)<br>13. September 2012 (2012-09-13)<br>* pp. 10-15 *<br>-----<br>-/-- | 1-15 | |

Der vorliegende Recherchenbericht wurde für alle Patentansprüche erstellt

2

| Recherchenort | Abschlußdatum der Recherche | Prüfer |
|---|---|---|
| München | 2. März 2021 | R. von Eggelkraut-G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt
European Patent Office
Office européen des brevets

# EUROPÄISCHER RECHERCHENBERICHT

Nummer der Anmeldung
EP 20 20 0076

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | YULI WANG ET AL: "Trapping cells on a stretchable microwell array for single-cell analysis", ANALYTICAL AND BIOANALYTICAL CHEMISTRY, SPRINGER, BERLIN, DE, Bd. 402, Nr. 3, 17. November 2011 (2011-11-17), Seiten 1065-1072, XP019993327, ISSN: 1618-2650, DOI: 10.1007/S00216-011-5535-9 * das ganze Dokument * ----- | 1-15 | |
| A | David K. Wood ET AL: "Single cell trapping and DNA damage analysis using microwell arrays", Proceedings of the National Academy of Sciences of the United States of America, 1. Juni 2010 (2010-06-01), Seiten 10008-10013, XP055134085, United States DOI: 10.1073/pnas.1004056107 Gefunden im Internet: URL:http://www.pnas.org/content/107/22/10008.abstract [gefunden am 2018-07-04] * das ganze Dokument * ----- | 1-15 | RECHERCHIERTE SACHGEBIETE (IPC) |
| A | SARA LINDSTRÃM ET AL: "Miniaturization of biological assays Overview on microwell devices for single-cell analyses", BIOCHIMICA ET BIOPHYSICA ACTA (BBA) - GENERAL SUBJECTS, ELSEVIER, AMSTERDAM, NL, Bd. 1810, Nr. 3, 16. April 2010 (2010-04-16), Seiten 308-316, XP028140859, ISSN: 0304-4165, DOI: 10.1016/J.BBAGEN.2010.04.009 [gefunden am 2010-05-06] * pp. 311-314, paragraphs 4 and 5 * ----- | 1-15 | |

Der vorliegende Recherchenbericht wurde für alle Patentansprüche erstellt

2

| Recherchenort | Abschlußdatum der Recherche | Prüfer |
|---|---|---|
| München | 2. März 2021 | R. von Eggelkraut-G. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 20 20 0076

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-03-2021

| Im Recherchenbericht angeführtes Patentdokument | Datum der Veröffentlichung | Mitglied(er) der Patentfamilie | Datum der Veröffentlichung |
|---|---|---|---|
| WO 2012151328 A2 | 08-11-2012 | US 2014066610 A1 | 06-03-2014 |
| | | WO 2012151328 A2 | 08-11-2012 |
| US 2016083783 A1 | 24-03-2016 | US 2016083783 A1 | 24-03-2016 |
| | | WO 2014145620 A2 | 18-09-2014 |
| WO 2012120270 A1 | 13-09-2012 | EP 2680972 A1 | 08-01-2014 |
| | | GB 2502902 A | 11-12-2013 |
| | | US 2014030755 A1 | 30-01-2014 |
| | | WO 2012120270 A1 | 13-09-2012 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2016140727 A2
- WO 2016140726 A2
- WO 20161407726 A2
- US 2005321352 A
- WO 002016140727 A


### In der Beschreibung aufgeführte Nicht-Patentliteratur


- **STADLER, Z. K. et al.** *Critical Reviews in Oncology Hematology,* 2009, vol. 69, 1-11
- **TREVINO, V. et al.** *Mol Med,* 2007, vol. 13, 527-541
- **VANGUILDER, H. D. et al.** *Biotechniques,* 2008, vol. 44, 619-626
- **BAKER, M.** *Nature Methods,* 2012, vol. 9, 541-544
- **WANG, Z. et al.** *Nat Rev Genet,* 2009, vol. 10, 57-63
- **GEISS, G. K. et al.** *Nat Biotechnol,* 2008, vol. 26, 317-325
- **JOO, C. et al.** *Annu Rev Biochem,* 2008, vol. 77, 51-76
- Thermo Scientific Pierce Cell Lysis Technical Handbook. Thermo Scientific
- **D. LIU ; L. HUANG.** Trypsin-induced lysis of lipid vesicles: effect of surface charge and lipid composition. *Anal Biochem,* 1992, vol. 202 (1), 1-5
- **L TURNBULL et al.** Explosive cell lysis as a mechanism for the biogenesis of bacterial membrane vesicles and biofilms. *Nat Commun,* 14. April 2016, vol. 7, 11220
- **C. LÄSSER et al.** Isolation and Characterization of RNA-Containing Exosomes. *J Vis Exp,* 2012, vol. 59, 3037
- **PIN LI et al.** Progress in Exosome Isolation Techniques. *Theranostics,* 2017, vol. 7 (3), 789-804
- **MD. DHAWAN et al.** Development of a laser-induced cell lysis system. *Anal Bioanal Chem,* 2002, vol. 373 (3), 421-6
- **K. RAU et al.** Pulsed Laser Microbeam-induced Cell Lysis: Time-Resolved Imaging and Analysis of Hydrodynamic Effects. *Biophys J.,* 2006, vol. 91 (1), 317-329
- **M. CHAVEZ-PAEZ et al.** Coalescence in double emulsions. *Langmuir,* 2012, vol. 28, 5934-5939
- Extraction and purification of nucleic acids from viruses. **G. F. STEWARD ; A. I. CULLEY.** Manual of aquatic viral ecology. American Society of Limnology and Oceanography, 2010, 154-165
- **DEMTRÖDER.** Experimentalphysik 2 - Elektrizität und Optik. Springer, 1995, 212
- **P. ATKINS.** Physikalische Chemie. Wiley-VCH, 2013
- **DEMTRÖDER.** Experimentalphysik 2 - Elektrizität und Optik. Springer, 1995, 218
- Quanta-phi F-3029 Integrating Sphere Operation Manual. Horiba Scientific, 2010
- FluoroMax-4 & FluoroMax-4P with USB Operation Manual. Horiba Scientific, 2012
- **Y. SOSKIND.** Field Guide to Diffractive Optics. SPIE Press, 2011, 14
- **F. KOTZ et al.** Three-dimensional printing of transparent fused silica glass. *Nature,* 2017, vol. 544 (7650), 337-339
- **F.KOTZ et al.** Liquid Glass: A Facile Soft Replication Method for Structuring Glass. *Advanced Materials,* 2016, vol. 28, 4646-4650
- **B. FANG et al.** Surfaces for Competitive Selective Bacterial Capture from Protein Solutions. *ACS Appl. Mater. Interfaces,* 2015, vol. 7 (19), 10275-10282
- **Y. WANG et al.** Trapping cells on a stretchable microwell array for single-cell analysis. *Anal Bioanal Chem,* 2012, vol. 402 (3), 1065-72
- **C.PROBST et al.** *Polydimethylsiloxane (PDMS) Sub-Micron Traps for Single-Cell Analysis of Bacteria*
- **R WATANABE et al.** Arrayed lipid bilayer chambers allow single-molecule analysis of membrane transporter activity. *Nat. Commun.,* 2014, vol. 5
- **C. J. SCHRÖTER et al.** A rapid method to separate endosomes from lysosomal contents using differential centrifugation and hypotonic lysis of lysosomes. *Journal of immunological methods,* 1999, vol. 227 (1-2), 161-168

- **X. ZHANG et al.** Natural channel protein inserts and functions in a completely artificial, solid-supported bilayer membrane. *Scientific Reports,* 2013, vol. 3, 2196
- **E. ZAKHARIAN.** Recording of Ion Channel Activity in Planar Lipid Bilayer Experiments. *Methods Mol Biol.,* 2013, vol. 998, 109-118
- **ROTHEMUND.** Folding DNA to create nano-scale shapes and patterns. *Nature,* Marz 2006, vol. 440, 297-302
- **DOUGLAS et al.** *Nature,* 2009, vol. 459, 414-418
- **SEEMAN.** Nanomaterials based on DNA. *An. Rev. Biochem.,* 2010, vol. 79, 65-87
- **PJ CRANFILL et al.** Quantitative assessment of fluorescent proteins. *Nature Methods,* 2016, vol. 13, 557-562
- **Q. ZHENG ; L LAVIS.** Development of photostable fluorophores for molecular imaging. *Curr. Op. Chem. Biol.,* 2017, vol. 39, 32-38
- **E. PETRYAYEVA et al.** Quantum dots in Bioanalysis: a review of applications across various platforms for fluorescence spectroscopy and imaging. *Appl. spectroscopy,* 2013, vol. 67
- Optical Interactions. **NOVOTNY, LUKAS.** Principles of nano-optics. Cambridge Univ. Press, 2013, 224
- Wechselwirkungen zwischen Molekülen. **PETER ATKINS.** Physikalische Chemie. Wiley-VCH, 2013, 657
- **FÖRSTER T.** Zwischenmolekulare Energiewanderung und Fluoreszenz. *Ann. Physik.,* 1948, vol. 437, 55
- **SAUER S ; LECHNER D ; BERLIN K et al.** Full flexibility genotyping of single nucleotide polymorphisms by the GOOD assay. *Nucleic Acids Res,* 2000, vol. 28, E100
- **HAFF LA ; SMIRNOV IP.** Single-nucleotide polymorphism identification assays using a thermostable DNA poly- merase and delayed extraction MALDI-TOF mass spectrome- try. *Genome Res,* 1997, vol. 7, 378-388
- **WENZEL T ; ELSSNER T ; FAHR K et al.** Genosnip: SNP genotyping by MALDI-TOF MS using photocleavable oligonucleotides. *Nucleosides Nucleotides Nucleic Acids,* 2003, vol. 22, 1579-1581
- **BRAUN A ; LITTLE DP ; KOSTER H.** Detecting CFTR gene mutations by using primer oligo base extension and mass spectrometry. *Clin Chem,* 1997, vol. 43, 1151-1158
- **SUN X ; DING H ; HUNG K et al.** A new MALDI-TOF based mini-sequencing assay for genotyping of SNPS. *Nucleic Acids Res,* 2000, vol. 28, E68
- **S.M. DOUGLAS et al.** Rapid prototyping of 3D DNA-origami shapes with caDNAno. *Nucleic Acids Res.,* 2009, vol. 37 (15
- **D SELNIHIN ; ES ANDERSEN.** Computer-aided design of DNA origami structures. *Comp. Meth. Synth. Biol.,* 2014, vol. 1244, 23-44
- **KNUDSEN J. et al.** *Nature Nanotechnology,* 2015, vol. 10, 892-898
- **S CHANDRADOSS et al.** Surface Passivation for Single-molecule Protein Studies. *Jove,* 2014
- **J PIEHLER et al.** A high-density poly(ethylene glycol) polymer brush for immobilization on glass-type surfaces. *Biosensors & Bioelectronics,* 2000
- **R SCHLAPAK et al.** Glass Surfaces Grafted with High-Density Poly(ethylene glycol) as Substrates for DNA Oligonucleotide Microarrays. *Langmuir,* 2006
- **B HUA ; TJ HA et al.** An improved surface passivation method for single-molecule studies. *Nature Methods,* 2014
- **A. KUMAR et al.** Silanized nucleic acids: a general platform for DNA immobilization. *Nuc Ac Research,* 2000
- **H LABIT et al.** A simple and optimized method of producing silanized surfaces for FISH and replication mapping on combed DNA fibers. *BioTechniques,* 2008
- **H. C. KOLB ; M. G. FINN ; K. B. SHARPLESS.** Click Chemistry: Diverse Chemical Function from a Few Good Reactions. *Angewandte Chemie International Edition,* 2001, vol. 40 (11), 2004-2021
- **M. JERABEK-WILLEMSEN et al.** Molecular Interaction Studies Using Microscale Thermophoresis. *Assay Drug Dev Technol,* 2011, vol. 9 (4), 342-353
- **A. LANGER et al.** Protein analysis by time-resolved measurements with an electro-switchable DNA chip. *Nat. Comm.,* 2013, vol. 4, 2099
- *Cold Spring Harb Protoc,* 2008
- A density-based algorithm for discovering clusters in large spatial databases with noise. **ESTER, MARTIN ; KRIEGEL, HANS-PETER ; SANDER, JÖRG ; XU, XIAOWEI.** Proceedings of the Second International Conference on Knowledge Discovery and Data Mining (KDD-96). AAAI Press, 1996, 226-231
- **RICARDO J. G. B. CAMPELLO ; DAVOUD MOULAVI ; JOERG SANDER.** Density-Based Clustering Based on Hierarchical Density Estimates. *Advances in Knowledge Discovery and Data Mining,* 2013, 160-172
- **D.S.SIVIA.** Data Analysis, a bayesian tutorial. Oxford science publications, 2006
- **A.B. DOWNEY.** Think Bayes. O'Reilly, 2013

SEQUENCE LISTING

<110> GRABMAYR Heinrich
WOEHRSTEIN Johannes Benedikt

<120> Einzelmolekülnachweis bzw. -quantifizierung durch DNA-Nanotechnologie in Mikro-Wells

<130> AA3126 EP S5

<160> 1278

<170> BiSSAP 1.3.6

<210> 1
<211> 45
<212> DNA
<213> Artificial Sequence

<220>
<223> Klammerstrang_0000 zugehoerig zu 3D-DNA-Nanostruktur.

<400> 1
agagtccact attttaatga acgctttcca gtcgggggtc gacgt 45

<210> 2
<211> 45
<212> DNA
<213> Artificial Sequence

<220>
<223> Klammerstrang_0001 zugehoerig zu 3D-DNA-Nanostruktur.

<400> 2
tatctaaaat atcttttgaa tacctgaaag cgtaagagca ataca 45

<210> 3
<211> 45
<212> DNA
<213> Artificial Sequence

<220>
<223> Klammerstrang_0002 zugehoerig zu 3D-DNA-Nanostruktur.

<400> 3
cagtacataa atcgcacgta aatggaaggg ttagaaagat taggt 45

<210> 4
<211> 45
<212> DNA
<213> Artificial Sequence

<220>
<223> Klammerstrang_0003 zugehoerig zu 3D-DNA-Nanostruktur.

<400> 4

aacatgttca gctccgtgtg aatcttctga cctaaatgct tctaa 45

<210> 5
<211> 45
<212> DNA
<213> Artificial Sequence

<220>
<223> Klammerstrang_0004 zugehoerig zu 3D-DNA-Nanostruktur.

<400> 5
gggtaattga gcgttaaatc atagcgaacc tcccgacaac aatac 45

<210> 6
<211> 45
<212> DNA
<213> Artificial Sequence

<220>
<223> Klammerstrang_0005 zugehoerig zu 3D-DNA-Nanostruktur.

<400> 6
cgtcagactg tagaatagaa aaaagacacc acggaacaag aatga 45

<210> 7
<211> 45
<212> DNA
<213> Artificial Sequence

<220>
<223> Klammerstrang_0006 zugehoerig zu 3D-DNA-Nanostruktur.

<400> 7
accgtactca ggagcgtcat atggaaagcg cagtctgtca tagag 45

<210> 8
<211> 45
<212> DNA
<213> Artificial Sequence

<220>
<223> Klammerstrang_0007 zugehoerig zu 3D-DNA-Nanostruktur.

<400> 8
aacggctaca gagcgaataa tcggagtgag aatagaaacc gcctc 45

<210> 9
<211> 45
<212> DNA
<213> Artificial Sequence

<220>
<223> Klammerstrang_0008 zugehoerig zu 3D-DNA-Nanostruktur.

<400> 9
aataaaacga actgtacaga cgaactgacc aacttttcat gaggc 45

<210> 10
<211> 45
<212> DNA
<213> Artificial Sequence

<220>
<223> Klammerstrang_0009 zugehoerig zu 3D-DNA-Nanostruktur.

<400> 10
tgttttaaat atgatgacca tccccctcaa atgctttaga aagtt 45

<210> 11
<211> 45
<212> DNA
<213> Artificial Sequence

<220>
<223> Klammerstrang_0010 zugehoerig zu 3D-DNA-Nanostruktur.

<400> 11
gtaatcgtaa aacgggagaa gtgtaccaaa aacattagtt tcaca 45

<210> 12
<211> 45
<212> DNA
<213> Artificial Sequence

<220>
<223> Klammerstrang_0011 zugehoerig zu 3D-DNA-Nanostruktur.

<400> 12
aaaacgacgg ccacatcgta accgtaatgg gataggccgg ttgcg 45

<210> 13
<211> 45
<212> DNA
<213> Artificial Sequence

<220>
<223> Klammerstrang_0012 zugehoerig zu 3D-DNA-Nanostruktur.

<400> 13
tcccttataa atccggtttg caagcctggg gtgcctcaag tccgg 45

<210> 14
<211> 45
<212> DNA
<213> Artificial Sequence

<220>
<223> Klammerstrang_0013 zugehoerig zu 3D-DNA-Nanostruktur.

<400> 14
aaccctcaat caaaactgat acagtcacac gaccagtcac ttgaa 45

<210> 15
<211> 45
<212> DNA
<213> Artificial Sequence

<220>
<223> Klammerstrang_0014 zugehoerig zu 3D-DNA-Nanostruktur.

<400> 15
caagaaaaca aaaagatttt catcagatga tggcaattga ggaca 45

<210> 16
<211> 45
<212> DNA
<213> Artificial Sequence

<220>
<223> Klammerstrang_0015 zugehoerig zu 3D-DNA-Nanostruktur.

<400> 16
aagagaatat aaataaacac ctccaatcgc aagacaattt tccat 45

<210> 17
<211> 45
<212> DNA
<213> Artificial Sequence

<220>
<223> Klammerstrang_0016 zugehoerig zu 3D-DNA-Nanostruktur.

<400> 17
ataaaaacag ggacagctac aatagcaagc aaatcaaata ataat 45

<210> 18
<211> 45
<212> DNA
<213> Artificial Sequence

<220>
<223> Klammerstrang_0017 zugehoerig zu 3D-DNA-Nanostruktur.

<400> 18
caatgaaacc atcaaagaca agtatgttag caaacgagca agaac 45

<210> 19
<211> 45
<212> DNA
<213> Artificial Sequence

<220>
<223> Klammerstrang_0018 zugehoerig zu 3D-DNA-Nanostruktur.

<400> 19
accaggcgga taagtgtact gcaggtcaga cgattgtctt ttcac 45

<210> 20
<211> 45
<212> DNA
<213> Artificial Sequence

<220>
<223> Klammerstrang_0019 zugehoerig zu 3D-DNA-Nanostruktur.

<400> 20
ggccgctttt gcgccaaaaa aacgttagta aatgaaagag ccagt 45

<210> 21
<211> 45
<212> DNA
<213> Artificial Sequence

<220>
<223> Klammerstrang_0020 zugehoerig zu 3D-DNA-Nanostruktur.

<400> 21
tgcgatttta agattcatca agacctgctc catgttaaat acgaa 45

<210> 22
<211> 45
<212> DNA
<213> Artificial Sequence

<220>
<223> Klammerstrang_0021 zugehoerig zu 3D-DNA-Nanostruktur.

<400> 22
gaggtcattt ttgcctgact aaaatgttta gactggaata ccata 45

<210> 23
<211> 45
<212> DNA
<213> Artificial Sequence

<220>
<223> Klammerstrang_0022 zugehoerig zu 3D-DNA-Nanostruktur.

<400> 23
ctacaaaggc tataaaattt taaggcaaag aattagcaat tctaa 45

<210> 24
<211> 45

<212> DNA
<213> Artificial Sequence

<220>
<223> Klammerstrang_0023 zugehoerig zu 3D-DNA-Nanostruktur.

<400> 24
ggatgtgctg caaggacgac gttaaatgtg agcgagacag gaaat 45

<210> 25
<211> 45
<212> DNA
<213> Artificial Sequence

<220>
<223> Klammerstrang_0024 zugehoerig zu 3D-DNA-Nanostruktur.

<400> 25
acgctggttt gccttctttt ctgaaattgt tatccggcta gtagc 45

<210> 26
<211> 45
<212> DNA
<213> Artificial Sequence

<220>
<223> Klammerstrang_0025 zugehoerig zu 3D-DNA-Nanostruktur.

<400> 26
gctgagagcc agcaataccg aatacctaca ttttgatatc ggccc 45

<210> 27
<211> 45
<212> DNA
<213> Artificial Sequence

<220>
<223> Klammerstrang_0026 zugehoerig zu 3D-DNA-Nanostruktur.

<400> 27
gcgcagaggc gaaacagtaa ccattttgcg gaacaaaaac aatac 45

<210> 28
<211> 45
<212> DNA
<213> Artificial Sequence

<220>
<223> Klammerstrang_0027 zugehoerig zu 3D-DNA-Nanostruktur.

<400> 28
atcgccatat ttagcctgtt tcctttttaa cctccggcga tagtc 45

<210> 29
<211> 45
<212> DNA
<213> Artificial Sequence

<220>
<223> Klammerstrang_0028 zugehoerig zu 3D-DNA-Nanostruktur.

<400> 29
agaaacgatt tttcgctaac gctcatcgag aacaagtgta gaaga 45

<210> 30
<211> 45
<212> DNA
<213> Artificial Sequence

<220>
<223> Klammerstrang_0029 zugehoerig zu 3D-DNA-Nanostruktur.

<400> 30
aattagagcc agcagggagg gacgcaataa taacggatct tacta 45

<210> 31
<211> 45
<212> DNA
<213> Artificial Sequence

<220>
<223> Klammerstrang_0030 zugehoerig zu 3D-DNA-Nanostruktur.

<400> 31
agtattaaga ggcgtgcctt gcagagccgc caccagagag ccagg 45

<210> 32
<211> 45
<212> DNA
<213> Artificial Sequence

<220>
<223> Klammerstrang_0031 zugehoerig zu 3D-DNA-Nanostruktur.

<400> 32
aacaaccatc gccattgtat cattccacag acagccgcaa gccaa 45

<210> 33
<211> 45
<212> DNA
<213> Artificial Sequence

<220>
<223> Klammerstrang_0032 zugehoerig zu 3D-DNA-Nanostruktur.

<400> 33
gagtagtaaa ttgatattca tcgaaacaaa gtacaaaacc taaac 45

<210> 34
<211> 45
<212> DNA
<213> Artificial Sequence

<220>
<223> Klammerstrang_0033 zugehoerig zu 3D-DNA-Nanostruktur.

<400> 34
ccggaagcaa actttgcatc aaaaaccaaa atagcgtaac gccac 45

<210> 35
<211> 45
<212> DNA
<213> Artificial Sequence

<220>
<223> Klammerstrang_0034 zugehoerig zu 3D-DNA-Nanostruktur.

<400> 35
gatattcaac cgttgcaatg caaggtggca tcaattgacc attga 45

<210> 36
<211> 45
<212> DNA
<213> Artificial Sequence

<220>
<223> Klammerstrang_0035 zugehoerig zu 3D-DNA-Nanostruktur.

<400> 36
aactgttggg aagcgcactc caggaacgcc atcaaaaatt gtaat 45

<210> 37
<211> 45
<212> DNA
<213> Artificial Sequence

<220>
<223> Klammerstrang_0036 zugehoerig zu 3D-DNA-Nanostruktur.

<400> 37
ctggccaaca gaggattagt gatagggttg agtgtacgcg cggct 45

<210> 38
<211> 45
<212> DNA
<213> Artificial Sequence

<220>
<223> Klammerstrang_0037 zugehoerig zu 3D-DNA-Nanostruktur.

<400> 38
tgattgtttg gataatagat caaatcaaca gttgaagtct ttatt 45

<210> 39
<211> 45
<212> DNA
<213> Artificial Sequence

<220>
<223> Klammerstrang_0038 zugehoerig zu 3D-DNA-Nanostruktur.

<400> 39
tttttcaaat atagtcgcta aatttcattt gaattataaa gaacc 45

<210> 40
<211> 45
<212> DNA
<213> Artificial Sequence

<220>
<223> Klammerstrang_0039 zugehoerig zu 3D-DNA-Nanostruktur.

<400> 40
ccggtattct aagtcctgaa aaagtaattc tgtccggcgt taaac 45

<210> 41
<211> 45
<212> DNA
<213> Artificial Sequence

<220>
<223> Klammerstrang_0040 zugehoerig zu 3D-DNA-Nanostruktur.

<400> 41
aaaggtggca acaagcccaa agaattaact gaacattgct attat 45

<210> 42
<211> 45
<212> DNA
<213> Artificial Sequence

<220>
<223> Klammerstrang_0041 zugehoerig zu 3D-DNA-Nanostruktur.

<400> 42
acaaataaat cctattagcg atcagtagcg acagatggtt tacat 45

<210> 43
<211> 45
<212> DNA
<213> Artificial Sequence

<220>

<223> Klammerstrang_0042 zugehoerig zu 3D-DNA-Nanostruktur.

<400> 43
ttgctaaaca actgaaccgc ttgatataag tatagtttga tgaaa 45

<210> 44
<211> 45
<212> DNA
<213> Artificial Sequence

<220>
<223> Klammerstrang_0043 zugehoerig zu 3D-DNA-Nanostruktur.

<400> 44
gcgcagacgg tcaccattaa gcagcgaaag acagctcacg ttgtt 45

<210> 45
<211> 45
<212> DNA
<213> Artificial Sequence

<220>
<223> Klammerstrang_0044 zugehoerig zu 3D-DNA-Nanostruktur.

<400> 45
cggaatcgtc ataagttgag cagtcaggac gttggatagg ctgag 45

<210> 46
<211> 45
<212> DNA
<213> Artificial Sequence

<220>
<223> Klammerstrang_0045 zugehoerig zu 3D-DNA-Nanostruktur.

<400> 46
agcctcagag cattaacagt ttaattgctg aatataaatc aggtg 45

<210> 47
<211> 45
<212> DNA
<213> Artificial Sequence

<220>
<223> Klammerstrang_0046 zugehoerig zu 3D-DNA-Nanostruktur.

<400> 47
tctccgtggg aacgaaaagc gagtctggag caaacttcaa cgcaa 45

<210> 48
<211> 45
<212> DNA
<213> Artificial Sequence

<220>
<223> Klammerstrang_0047 zugehoerig zu 3D-DNA-Nanostruktur.

<400> 48
cacattaatt gcgctttgat taacgccagg gtttttctgc cagat 45

<210> 49
<211> 45
<212> DNA
<213> Artificial Sequence

<220>
<223> Klammerstrang_0048 zugehoerig zu 3D-DNA-Nanostruktur.

<400> 49
atggattatt tacagaagaa cctgtttgat ggtgggcgcc aggca 45

<210> 50
<211> 45
<212> DNA
<213> Artificial Sequence

<220>
<223> Klammerstrang_0049 zugehoerig zu 3D-DNA-Nanostruktur.

<400> 50
agcggaatta tcagtattag taaagcatca ccttgaacat cgcaa 45

<210> 51
<211> 45
<212> DNA
<213> Artificial Sequence

<220>
<223> Klammerstrang_0050 zugehoerig zu 3D-DNA-Nanostruktur.

<400> 51
ataactatat gtatccttga acctgagcaa aagaaacgtc agagg 45

<210> 52
<211> 45
<212> DNA
<213> Artificial Sequence

<220>
<223> Klammerstrang_0051 zugehoerig zu 3D-DNA-Nanostruktur.

<400> 52
tcatcgtagg aatctaattt aatttaggca gaggctaatt actat 45

<210> 53
<211> 45
<212> DNA

<213> Artificial Sequence

<220>
<223> Klammerstrang_0052 zugehoerig zu 3D-DNA-Nanostruktur.

<400> 53
ggcatgatta agaaaatagc atgaaaatag cagcccctga atctt 45

<210> 54
<211> 45
<212> DNA
<213> Artificial Sequence

<220>
<223> Klammerstrang_0053 zugehoerig zu 3D-DNA-Nanostruktur.

<400> 54
gccgccagca ttgaaatcac accattacca ttagcacatt caact 45

<210> 55
<211> 45
<212> DNA
<213> Artificial Sequence

<220>
<223> Klammerstrang_0054 zugehoerig zu 3D-DNA-Nanostruktur.

<400> 55
cgatctaaag tttcattttc gaaggattag gattagtttt aaccc 45

<210> 56
<211> 45
<212> DNA
<213> Artificial Sequence

<220>
<223> Klammerstrang_0055 zugehoerig zu 3D-DNA-Nanostruktur.

<400> 56
cgcctgataa attactacga tattcggtcg ctgagcaaaa ggaaa 45

<210> 57
<211> 45
<212> DNA
<213> Artificial Sequence

<220>
<223> Klammerstrang_0056 zugehoerig zu 3D-DNA-Nanostruktur.

<400> 57
gaagttttgc cagctaatgc aatttcaact ttaatcttga caaat 45

<210> 58

<211> 45
<212> DNA
<213> Artificial Sequence

<220>
<223> Klammerstrang_0057 zugehoerig zu 3D-DNA-Nanostruktur.

<400> 58
cattaacatc caaagtagat tagagagtac ctttacagaa gcaaa 45

<210> 59
<211> 45
<212> DNA
<213> Artificial Sequence

<220>
<223> Klammerstrang_0058 zugehoerig zu 3D-DNA-Nanostruktur.

<400> 59
ccttcctgta gcctaagcaa aatgccggag agggtctcat ataag 45

<210> 60
<211> 45
<212> DNA
<213> Artificial Sequence

<220>
<223> Klammerstrang_0059 zugehoerig zu 3D-DNA-Nanostruktur.

<400> 60
acatacgagc cggaccgtat cctcttcgct attaccggcc tcagg 45

<210> 61
<211> 45
<212> DNA
<213> Artificial Sequence

<220>
<223> Klammerstrang_0060 zugehoerig zu 3D-DNA-Nanostruktur.

<400> 61
attgcaacag gaagtaatat cctggccctg agagaagacg ggccg 45

<210> 62
<211> 45
<212> DNA
<213> Artificial Sequence

<220>
<223> Klammerstrang_0061 zugehoerig zu 3D-DNA-Nanostruktur.

<400> 62
taattttaaa agtctcgtat gtcagtatta acaccaccag cagcc 45

<210> 63
<211> 45
<212> DNA
<213> Artificial Sequence

<220>
<223> Klammerstrang_0062 zugehoerig zu 3D-DNA-Nanostruktur.

<400> 63
tatcaaaatc atataagacg cctgattgct ttgaatttac atcat 45

<210> 64
<211> 45
<212> DNA
<213> Artificial Sequence

<220>
<223> Klammerstrang_0063 zugehoerig zu 3D-DNA-Nanostruktur.

<400> 64
agaacgggta ttaaataatc agccaacgct caacatatgc gtttt 45

<210> 65
<211> 45
<212> DNA
<213> Artificial Sequence

<220>
<223> Klammerstrang_0064 zugehoerig zu 3D-DNA-Nanostruktur.

<400> 65
caaagttacc agacttttta aaacagccat attatttcca gagca 45

<210> 66
<211> 45
<212> DNA
<213> Artificial Sequence

<220>
<223> Klammerstrang_0065 zugehoerig zu 3D-DNA-Nanostruktur.

<400> 66
aaccgccacc ctcaaccgcc attatcaccg tcaccatatt gacaa 45

<210> 67
<211> 45
<212> DNA
<213> Artificial Sequence

<220>
<223> Klammerstrang_0066 zugehoerig zu 3D-DNA-Nanostruktur.

<400> 67

accagtacaa actaacccat ctatttcgga acctagtgcc cgtag 45

<210> 68
<211> 45
<212> DNA
<213> Artificial Sequence

<220>
<223> Klammerstrang_0067 zugehoerig zu 3D-DNA-Nanostruktur.

<400> 68
tttgaccccc agcgaggcaa agcttgatac cgatacagct tgctc 45

<210> 69
<211> 45
<212> DNA
<213> Artificial Sequence

<220>
<223> Klammerstrang_0068 zugehoerig zu 3D-DNA-Nanostruktur.

<400> 69
ataaccctcg tttattacga ataaggcttg ccctgatcaa cgttc 45

<210> 70
<211> 45
<212> DNA
<213> Artificial Sequence

<220>
<223> Klammerstrang_0069 zugehoerig zu 3D-DNA-Nanostruktur.

<400> 70
atattttcat ttgtttcgca gcgttttaat tcgagtaaga ggatc 45

<210> 71
<211> 45
<212> DNA
<213> Artificial Sequence

<220>
<223> Klammerstrang_0070 zugehoerig zu 3D-DNA-Nanostruktur.

<400> 71
gttaaatcag ctcatatttt aaggccggag acagtatgtg tagct 45

<210> 72
<211> 45
<212> DNA
<213> Artificial Sequence

<220>
<223> Klammerstrang_0071 zugehoerig zu 3D-DNA-Nanostruktur.

<400> 72
taatcatggt cataaggatc aggcaaagcg ccatttttcc ggctt 45

<210> 73
<211> 42
<212> DNA
<213> Artificial Sequence

<220>
<223> Klammerstrang_0072 zugehoerig zu 3D-DNA-Nanostruktur.

<400> 73
acgtggacgg gactgcgcga gggtaggacc agctgcaaaa ga 42

<210> 74
<211> 40
<212> DNA
<213> Artificial Sequence

<220>
<223> Klammerstrang_0073 zugehoerig zu 3D-DNA-Nanostruktur.

<400> 74
gtggcacagt gggcacactc gagaactccc gttgtaatac 40

<210> 75
<211> 42
<212> DNA
<213> Artificial Sequence

<220>
<223> Klammerstrang_0074 zugehoerig zu 3D-DNA-Nanostruktur.

<400> 75
gagcactaac gggcccgaat tgagggcaag acaatatttt ag 42

<210> 76
<211> 40
<212> DNA
<213> Artificial Sequence

<220>
<223> Klammerstrang_0075 zugehoerig zu 3D-DNA-Nanostruktur.

<400> 76
catatcactg aggatatgag acgatgacaa ctaatcctac 40

<210> 77
<211> 42
<212> DNA
<213> Artificial Sequence

<220>
<223> Klammerstrang_0076 zugehoerig zu 3D-DNA-Nanostruktur.

<400> 77
atgtgagtcc agtcgccccc cgcagttgaa attatttaat at 42

<210> 78
<211> 40
<212> DNA
<213> Artificial Sequence

<220>
<223> Klammerstrang_0077 zugehoerig zu 3D-DNA-Nanostruktur.

<400> 78
tggtttgatc tgggtagcag cggtaggaat aaccttttaa 40

<210> 79
<211> 42
<212> DNA
<213> Artificial Sequence

<220>
<223> Klammerstrang_0078 zugehoerig zu 3D-DNA-Nanostruktur.

<400> 79
agaacgcgtt gcccacgagt gcggtgataa ataccgaaat gc 42

<210> 80
<211> 40
<212> DNA
<213> Artificial Sequence

<220>
<223> Klammerstrang_0079 zugehoerig zu 3D-DNA-Nanostruktur.

<400> 80
gggaggtatc ccggaccaca tatatccctg tttatcttgc 40

<210> 81
<211> 42
<212> DNA
<213> Artificial Sequence

<220>
<223> Klammerstrang_0080 zugehoerig zu 3D-DNA-Nanostruktur.

<400> 81
atcagagact gtgggccgcg caccgccctt tgaagcccta at 42

<210> 82
<211> 40
<212> DNA
<213> Artificial Sequence

<220>
<223> Klammerstrang_0081 zugehoerig zu 3D-DNA-Nanostruktur.

<400> 82
ttattttgag gcgacggcta ttgaccgata acccataagt 40

<210> 83
<211> 42
<212> DNA
<213> Artificial Sequence

<220>
<223> Klammerstrang_0082 zugehoerig zu 3D-DNA-Nanostruktur.

<400> 83
tttcatcgct agcggttcta agtcgggtgt cacaatccgc gt 42

<210> 84
<211> 40
<212> DNA
<213> Artificial Sequence

<220>
<223> Klammerstrang_0083 zugehoerig zu 3D-DNA-Nanostruktur.

<400> 84
tttaccgtgt aaatgcgcgg ccgagggcat tttcgctgaa 40

<210> 85
<211> 42
<212> DNA
<213> Artificial Sequence

<220>
<223> Klammerstrang_0084 zugehoerig zu 3D-DNA-Nanostruktur.

<400> 85
agtaccgctg ctggaccgtg gaccaaggtt ccagtaaggt tt 42

<210> 86
<211> 40
<212> DNA
<213> Artificial Sequence

<220>
<223> Klammerstrang_0085 zugehoerig zu 3D-DNA-Nanostruktur.

<400> 86
acaactatga gggtacggga ccatggcacc ctcagaagga 40

<210> 87
<211> 42

<212> DNA
<213> Artificial Sequence

<220>
<223> Klammerstrang_0086 zugehoerig zu 3D-DNA-Nanostruktur.

<400> 87
gaggactagg agttgtgcca atggcgagaa ggaattggct tt 42

<210> 88
<211> 40
<212> DNA
<213> Artificial Sequence

<220>
<223> Klammerstrang_0087 zugehoerig zu 3D-DNA-Nanostruktur.

<400> 88
aggacaggca ctgggcgcct tgttcaaaga cttttgaaag 40

<210> 89
<211> 42
<212> DNA
<213> Artificial Sequence

<220>
<223> Klammerstrang_0088 zugehoerig zu 3D-DNA-Nanostruktur.

<400> 89
aacaacatgc tctagtcgga cgtcgactat gaacggtaac gg 42

<210> 90
<211> 40
<212> DNA
<213> Artificial Sequence

<220>
<223> Klammerstrang_0089 zugehoerig zu 3D-DNA-Nanostruktur.

<400> 90
agttcagcgc aacgggacgc acagcatatt acaggtaaac 40

<210> 91
<211> 42
<212> DNA
<213> Artificial Sequence

<220>
<223> Klammerstrang_0090 zugehoerig zu 3D-DNA-Nanostruktur.

<400> 91
aaagtacgcg agggtagggc ctaactcaaa aacgagacaa ct 42

<210> 92
<211> 40
<212> DNA
<213> Artificial Sequence

<220>
<223> Klammerstrang_0091 zugehoerig zu 3D-DNA-Nanostruktur.

<400> 92
cctgtaagcg tctagggact cggcacgtgt ctggaatgac 40

<210> 93
<211> 42
<212> DNA
<213> Artificial Sequence

<220>
<223> Klammerstrang_0092 zugehoerig zu 3D-DNA-Nanostruktur.

<400> 93
tgtcaatcgc ggtggccgta ctggcccata cttttgctag ca 42

<210> 94
<211> 40
<212> DNA
<213> Artificial Sequence

<220>
<223> Klammerstrang_0093 zugehoerig zu 3D-DNA-Nanostruktur.

<400> 94
ttggtgttaa tctgtggata atccgaatat gtacctcacg 40

<210> 95
<211> 42
<212> DNA
<213> Artificial Sequence

<220>
<223> Klammerstrang_0094 zugehoerig zu 3D-DNA-Nanostruktur.

<400> 95
aagcttgccg gctcaaggcc tcgcgccaag atgggcggtg cc 42

<210> 96
<211> 40
<212> DNA
<213> Artificial Sequence

<220>
<223> Klammerstrang_0095 zugehoerig zu 3D-DNA-Nanostruktur.

<400> 96
tgtcgtgggc caagacgtag cgcatgatgc ctgcaaaacc 40

<210> 97
<211> 42
<212> DNA
<213> Artificial Sequence

<220>
<223> Klammerstrang_0096 zugehoerig zu 3D-DNA-Nanostruktur.

<400> 97
cagtttggcc ggagacccag ggataagccc tcggccatgt tc 42

<210> 98
<211> 40
<212> DNA
<213> Artificial Sequence

<220>
<223> Klammerstrang_0097 zugehoerig zu 3D-DNA-Nanostruktur.

<400> 98
acccttcgag gttgagggcg cagatgaact tctttataga 40

<210> 99
<211> 42
<212> DNA
<213> Artificial Sequence

<220>
<223> Klammerstrang_0098 zugehoerig zu 3D-DNA-Nanostruktur.

<400> 99
attgaggagc ggctgcgtac gttcgagctg ggctattaag ga 42

<210> 100
<211> 40
<212> DNA
<213> Artificial Sequence

<220>
<223> Klammerstrang_0099 zugehoerig zu 3D-DNA-Nanostruktur.

<400> 100
ttctgaaatg ccgcttctcc agtgttagag ccgtctatac 40

<210> 101
<211> 42
<212> DNA
<213> Artificial Sequence

<220>
<223> Klammerstrang_0100 zugehoerig zu 3D-DNA-Nanostruktur.

<400> 101
ttttaatgta ctacccgagg acggagctta aacagaaacc tt 42

<210> 102
<211> 40
<212> DNA
<213> Artificial Sequence

<220>
<223> Klammerstrang_0101 zugehoerig zu 3D-DNA-Nanostruktur.

<400> 102
gttaattcgc tgttccctgc gcgcttgagt aaatctttta 40

<210> 103
<211> 42
<212> DNA
<213> Artificial Sequence

<220>
<223> Klammerstrang_0102 zugehoerig zu 3D-DNA-Nanostruktur.

<400> 103
acgacaatca cacacacggc gaattatgtc taaataaaga cg 42

<210> 104
<211> 40
<212> DNA
<213> Artificial Sequence

<220>
<223> Klammerstrang_0103 zugehoerig zu 3D-DNA-Nanostruktur.

<400> 104
gaggcgttgt ggcgtcgtgc gatggcaaag ataagaacgc 40

<210> 105
<211> 42
<212> DNA
<213> Artificial Sequence

<220>
<223> Klammerstrang_0104 zugehoerig zu 3D-DNA-Nanostruktur.

<400> 105
aacaaagtgt ttcgaggagg ccgttcgttt agattagccc tg 42

<210> 106
<211> 40
<212> DNA
<213> Artificial Sequence

<220>

<223> Klammerstrang_0105 zugehoerig zu 3D-DNA-Nanostruktur.

<400> 106
aagaaacgtt acatcccccct cacaagcatg agttatataa 40

<210> 107
<211> 42
<212> DNA
<213> Artificial Sequence

<220>
<223> Klammerstrang_0106 zugehoerig zu 3D-DNA-Nanostruktur.

<400> 107
gtttgcctca gtgcgtgcgc cacgtggtgc attcataatc aa 42

<210> 108
<211> 40
<212> DNA
<213> Artificial Sequence

<220>
<223> Klammerstrang_0107 zugehoerig zu 3D-DNA-Nanostruktur.

<400> 108
aagccagtac ctcccaggct cctacattcc cccttcatta 40

<210> 109
<211> 42
<212> DNA
<213> Artificial Sequence

<220>
<223> Klammerstrang_0108 zugehoerig zu 3D-DNA-Nanostruktur.

<400> 109
aataggtgga ggccggtcac cctagctgaa catggctccc gg 42

<210> 110
<211> 40
<212> DNA
<213> Artificial Sequence

<220>
<223> Klammerstrang_0109 zugehoerig zu 3D-DNA-Nanostruktur.

<400> 110
cagtttctgg caagggctga gggcagtaac cctcattcaa 40

<210> 111
<211> 42
<212> DNA
<213> Artificial Sequence

<220>
<223> Klammerstrang_0110 zugehoerig zu 3D-DNA-Nanostruktur.

<400> 111
aacgagggga actcgctgtg cgggtctgag aatttttatc gg 42

<210> 112
<211> 40
<212> DNA
<213> Artificial Sequence

<220>
<223> Klammerstrang_0111 zugehoerig zu 3D-DNA-Nanostruktur.

<400> 112
aagggaacac gttgcgcatg aagccataga agtttatcat 40

<210> 113
<211> 42
<212> DNA
<213> Artificial Sequence

<220>
<223> Klammerstrang_0112 zugehoerig zu 3D-DNA-Nanostruktur.

<400> 113
aaaatctagg tgaccgttgg cgtaccgtcc caggcgcgaa ga 42

<210> 114
<211> 40
<212> DNA
<213> Artificial Sequence

<220>
<223> Klammerstrang_0113 zugehoerig zu 3D-DNA-Nanostruktur.

<400> 114
tcattgaagg ttggccgctg acttctcgat tcatcaatat 40

<210> 115
<211> 42
<212> DNA
<213> Artificial Sequence

<220>
<223> Klammerstrang_0114 zugehoerig zu 3D-DNA-Nanostruktur.

<400> 115
tgtagctcat tcgccgtgga cgcctttgat aaatcaaaat gc 42

<210> 116
<211> 40
<212> DNA

<213> Artificial Sequence

<220>
<223> Klammerstrang_0115 zugehoerig zu 3D-DNA-Nanostruktur.

<400> 116
taaatcgcct atgcgcgtag acgcagaatt ccataaaagc 40

<210> 117
<211> 42
<212> DNA
<213> Artificial Sequence

<220>
<223> Klammerstrang_0116 zugehoerig zu 3D-DNA-Nanostruktur.

<400> 117
aatcgatgcg cagcccgcca gcacactcgt cctttataag ag 42

<210> 118
<211> 40
<212> DNA
<213> Artificial Sequence

<220>
<223> Klammerstrang_0117 zugehoerig zu 3D-DNA-Nanostruktur.

<400> 118
gcggattgct tcctgagatt cttactaaat aatcaaaacg 40

<210> 119
<211> 42
<212> DNA
<213> Artificial Sequence

<220>
<223> Klammerstrang_0118 zugehoerig zu 3D-DNA-Nanostruktur.

<400> 119
tcacgacgcg gccaaggatt tactgctcga ccgtgcaccc ag 42

<210> 120
<211> 40
<212> DNA
<213> Artificial Sequence

<220>
<223> Klammerstrang_0119 zugehoerig zu 3D-DNA-Nanostruktur.

<400> 120
ctcactgtgc tctgctccgc tgcggttttc tagacttgcg 40

<210> 121

<211> 42
<212> DNA
<213> Artificial Sequence

<220>
<223> Klammerstrang_0120 zugehoerig zu 3D-DNA-Nanostruktur.

<400> 121
aatagcccaa acgacgaggg gcacgaaaaa ggagaggaaa ag 42

<210> 122
<211> 40
<212> DNA
<213> Artificial Sequence

<220>
<223> Klammerstrang_0121 zugehoerig zu 3D-DNA-Nanostruktur.

<400> 122
aaagggaggc agcactgcaa gaacgggaaa taacataata 40

<210> 123
<211> 42
<212> DNA
<213> Artificial Sequence

<220>
<223> Klammerstrang_0122 zugehoerig zu 3D-DNA-Nanostruktur.

<400> 123
ggtcagttat cagggagtca ccgcctctca atgcgcgtat ct 42

<210> 124
<211> 40
<212> DNA
<213> Artificial Sequence

<220>
<223> Klammerstrang_0123 zugehoerig zu 3D-DNA-Nanostruktur.

<400> 124
caatatagcc cgcctgaaag acgcctggaa tacatttcat 40

<210> 125
<211> 42
<212> DNA
<213> Artificial Sequence

<220>
<223> Klammerstrang_0124 zugehoerig zu 3D-DNA-Nanostruktur.

<400> 125
tacatttact ggcgtccccc tgagtcccat attgcgttta at 42

<210> 126
<211> 40
<212> DNA
<213> Artificial Sequence

<220>
<223> Klammerstrang_0125 zugehoerig zu 3D-DNA-Nanostruktur.

<400> 126
cgcgagatat cactcgtgag cccagtactt aattaaagaa 40

<210> 127
<211> 42
<212> DNA
<213> Artificial Sequence

<220>
<223> Klammerstrang_0126 zugehoerig zu 3D-DNA-Nanostruktur.

<400> 127
gacaaaagct aagccgaagc ggaaagccaa ataagaagta cc 42

<210> 128
<211> 40
<212> DNA
<213> Artificial Sequence

<220>
<223> Klammerstrang_0127 zugehoerig zu 3D-DNA-Nanostruktur.

<400> 128
agaaggcggc accacacgag aaggacgtca agaaagatat 40

<210> 129
<211> 42
<212> DNA
<213> Artificial Sequence

<220>
<223> Klammerstrang_0128 zugehoerig zu 3D-DNA-Nanostruktur.

<400> 129
attagacgcg tggctcgcaa gggcagcgtt ttgcaccagc gc 42

<210> 130
<211> 40
<212> DNA
<213> Artificial Sequence

<220>
<223> Klammerstrang_0129 zugehoerig zu 3D-DNA-Nanostruktur.

<400> 130

aatacattgc tgttggttac acgccgggta ataagtagaa 40

<210> 131
<211> 42
<212> DNA
<213> Artificial Sequence

<220>
<223> Klammerstrang_0130 zugehoerig zu 3D-DNA-Nanostruktur.

<400> 131
cagcaccgtg ctgccgcaag cgagaaaaac cagcgccgat ag 42

<210> 132
<211> 40
<212> DNA
<213> Artificial Sequence

<220>
<223> Klammerstrang_0131 zugehoerig zu 3D-DNA-Nanostruktur.

<400> 132
gatattccaa gatggtaggt ccttgatatt tgccagcctt 40

<210> 133
<211> 42
<212> DNA
<213> Artificial Sequence

<220>
<223> Klammerstrang_0132 zugehoerig zu 3D-DNA-Nanostruktur.

<400> 133
gtcgagagca actggagtgg cgaccggtac tacaggagtg cc 42

<210> 134
<211> 40
<212> DNA
<213> Artificial Sequence

<220>
<223> Klammerstrang_0133 zugehoerig zu 3D-DNA-Nanostruktur.

<400> 134
tgtatgggtg cgcgagaccg ttctagggca ccctcttttc 40

<210> 135
<211> 42
<212> DNA
<213> Artificial Sequence

<220>
<223> Klammerstrang_0134 zugehoerig zu 3D-DNA-Nanostruktur.

<400> 135
gtcacccttc aaagcacgcc caccgtgtga aaaatctgga tc 42

<210> 136
<211> 40
<212> DNA
<213> Artificial Sequence

<220>
<223> Klammerstrang_0135 zugehoerig zu 3D-DNA-Nanostruktur.

<400> 136
gccggaacca cgcagtgtca gtcggacaac gggtaactta 40

<210> 137
<211> 42
<212> DNA
<213> Artificial Sequence

<220>
<223> Klammerstrang_0136 zugehoerig zu 3D-DNA-Nanostruktur.

<400> 137
ctcattatca acgggcacag cccatcggcg gctgaccact gg 42

<210> 138
<211> 40
<212> DNA
<213> Artificial Sequence

<220>
<223> Klammerstrang_0137 zugehoerig zu 3D-DNA-Nanostruktur.

<400> 138
gtccaatcgc aaggttctcc gactcaacat ttaggatagc 40

<210> 139
<211> 42
<212> DNA
<213> Artificial Sequence

<220>
<223> Klammerstrang_0138 zugehoerig zu 3D-DNA-Nanostruktur.

<400> 139
ggcttagacg tccctgcgca ataggccgac tctttaccgg at 42

<210> 140
<211> 40
<212> DNA
<213> Artificial Sequence

<220>
<223> Klammerstrang_0139 zugehoerig zu 3D-DNA-Nanostruktur.

<400> 140
ttaagcagag tgctgtgttt cgacgggctg attcccaaaa 40

<210> 141
<211> 42
<212> DNA
<213> Artificial Sequence

<220>
<223> Klammerstrang_0140 zugehoerig zu 3D-DNA-Nanostruktur.

<400> 141
cattgcctga gagtcgtggt acgatgtgat aaggatacag gt 42

<210> 142
<211> 40
<212> DNA
<213> Artificial Sequence

<220>
<223> Klammerstrang_0141 zugehoerig zu 3D-DNA-Nanostruktur.

<400> 142
acccgtcaga taggctggct ccgtgtgacc caaaataaca 40

<210> 143
<211> 42
<212> DNA
<213> Artificial Sequence

<220>
<223> Klammerstrang_0142 zugehoerig zu 3D-DNA-Nanostruktur.

<400> 143
ttaagttgca aggctgttcc gcaacggggg tttgaggggc ga 42

<210> 144
<211> 40
<212> DNA
<213> Artificial Sequence

<220>
<223> Klammerstrang_0143 zugehoerig zu 3D-DNA-Nanostruktur.

<400> 144
gtgagctgga ctgtgagcga ccggtcggag cgaggaatga 40

<210> 145
<211> 42
<212> DNA
<213> Artificial Sequence

<220>
<223> Klammerstrang_0144 zugehoerig zu 3D-DNA-Nanostruktur.

<400> 145
aaatcggcca gcattcccta gcctcccgta gtattggttc cg 42

<210> 146
<211> 40
<212> DNA
<213> Artificial Sequence

<220>
<223> Klammerstrang_0145 zugehoerig zu 3D-DNA-Nanostruktur.

<400> 146
cagattcggg aacaccacca ctgatgaacc tgagtattgg 40

<210> 147
<211> 42
<212> DNA
<213> Artificial Sequence

<220>
<223> Klammerstrang_0146 zugehoerig zu 3D-DNA-Nanostruktur.

<400> 147
cctcaaatgg acttgcaagg cggcgaaaac gccctaactg aa 42

<210> 148
<211> 40
<212> DNA
<213> Artificial Sequence

<220>
<223> Klammerstrang_0147 zugehoerig zu 3D-DNA-Nanostruktur.

<400> 148
ttcctgaggc ttcttgcggc atgatgattt tagaatcata 40

<210> 149
<211> 42
<212> DNA
<213> Artificial Sequence

<220>
<223> Klammerstrang_0148 zugehoerig zu 3D-DNA-Nanostruktur.

<400> 149
tgaaacaagg tcccgacagc ttcgcgtatt aggtttagat ga 42

<210> 150
<211> 40

<212> DNA
<213> Artificial Sequence

<220>
<223> Klammerstrang_0149 zugehoerig zu 3D-DNA-Nanostruktur.

<400> 150
tgatgcaaag cacggagcat tgccgtacct tagaaaatgc 40

<210> 151
<211> 42
<212> DNA
<213> Artificial Sequence

<220>
<223> Klammerstrang_0150 zugehoerig zu 3D-DNA-Nanostruktur.

<400> 151
cgagccagtg ccgcgactcg aaggtaacaa ggaatcaatt tt 42

<210> 152
<211> 40
<212> DNA
<213> Artificial Sequence

<220>
<223> Klammerstrang_0151 zugehoerig zu 3D-DNA-Nanostruktur.

<400> 152
ccgcgcccgg gagcagacgt gtggtgtatc ccatccatta 40

<210> 153
<211> 42
<212> DNA
<213> Artificial Sequence

<220>
<223> Klammerstrang_0152 zugehoerig zu 3D-DNA-Nanostruktur.

<400> 153
agagagaaga acgaacgagc gggattcaca attttatttt ac 42

<210> 154
<211> 40
<212> DNA
<213> Artificial Sequence

<220>
<223> Klammerstrang_0153 zugehoerig zu 3D-DNA-Nanostruktur.

<400> 154
tattacgaca ttcccattag ccgggataaa caatgctcct 40

<210> 155
<211> 42
<212> DNA
<213> Artificial Sequence

<220>
<223> Klammerstrang_0154 zugehoerig zu 3D-DNA-Nanostruktur.

<400> 155
cggaaacgcg acgatttgca gcgcttctca aagggcgaag gc 42

<210> 156
<211> 40
<212> DNA
<213> Artificial Sequence

<220>
<223> Klammerstrang_0155 zugehoerig zu 3D-DNA-Nanostruktur.

<400> 156
aggttgaacg agcagaccgg cacgggtcat aatcaacagg 40

<210> 157
<211> 42
<212> DNA
<213> Artificial Sequence

<220>
<223> Klammerstrang_0156 zugehoerig zu 3D-DNA-Nanostruktur.

<400> 157
gttttgctgt gcgtcgaggc gactaactgg gtaataagcg gg 42

<210> 158
<211> 40
<212> DNA
<213> Artificial Sequence

<220>
<223> Klammerstrang_0157 zugehoerig zu 3D-DNA-Nanostruktur.

<400> 158
tctttcctag ttgggccggt cccgtccacc acccttgtcg 40

<210> 159
<211> 42
<212> DNA
<213> Artificial Sequence

<220>
<223> Klammerstrang_0158 zugehoerig zu 3D-DNA-Nanostruktur.

<400> 159
cagggagtcg ggatgccttc tgcttctcag aaggctcgct tg 42

<210> 160
<211> 40
<212> DNA
<213> Artificial Sequence

<220>
<223> Klammerstrang_0159 zugehoerig zu 3D-DNA-Nanostruktur.

<400> 160
gaaatccggt gcgaactgtg gccgactata atgccgtgtc 40

<210> 161
<211> 42
<212> DNA
<213> Artificial Sequence

<220>
<223> Klammerstrang_0160 zugehoerig zu 3D-DNA-Nanostruktur.

<400> 161
tgaattacgg gagtctgccg agaatggcgc gagtaatcat tg 42

<210> 162
<211> 40
<212> DNA
<213> Artificial Sequence

<220>
<223> Klammerstrang_0161 zugehoerig zu 3D-DNA-Nanostruktur.

<400> 162
gtaatagcgt gcttttccat gtcggtctca ttcaaagggg 40

<210> 163
<211> 42
<212> DNA
<213> Artificial Sequence

<220>
<223> Klammerstrang_0162 zugehoerig zu 3D-DNA-Nanostruktur.

<400> 163
tccttttgct acctgggcca ctgcacgcta ttatagtatt gc 42

<210> 164
<211> 40
<212> DNA
<213> Artificial Sequence

<220>
<223> Klammerstrang_0163 zugehoerig zu 3D-DNA-Nanostruktur.

<400> 164
catacagacg cattacctgg aacgacatgc gaacgtaaat 40

<210> 165
<211> 42
<212> DNA
<213> Artificial Sequence

<220>
<223> Klammerstrang_0164 zugehoerig zu 3D-DNA-Nanostruktur.

<400> 165
tttttgagct cacaccgcag tagcccgagc tagaaccagc ta 42

<210> 166
<211> 40
<212> DNA
<213> Artificial Sequence

<220>
<223> Klammerstrang_0165 zugehoerig zu 3D-DNA-Nanostruktur.

<400> 166
tcatcaacct acgtagaccg cacgatagga ttgtaagctt 40

<210> 167
<211> 42
<212> DNA
<213> Artificial Sequence

<220>
<223> Klammerstrang_0166 zugehoerig zu 3D-DNA-Nanostruktur.

<400> 167
ctggcgaagt agtgccgcga cgctgatgca acagtatgcc ag 42

<210> 168
<211> 40
<212> DNA
<213> Artificial Sequence

<220>
<223> Klammerstrang_0167 zugehoerig zu 3D-DNA-Nanostruktur.

<400> 168
taaagtgcgt cccttcggac ccacgtaggc tagcgaagca 40

<210> 169
<211> 42
<212> DNA
<213> Artificial Sequence

<220>

<223> Klammerstrang_0168 zugehoerig zu 3D-DNA-Nanostruktur.

<400> 169
aggcgaaaac ggtggtcacc ggtgcgtgca gtggtttcca gc 42

<210> 170
<211> 40
<212> DNA
<213> Artificial Sequence

<220>
<223> Klammerstrang_0169 zugehoerig zu 3D-DNA-Nanostruktur.

<400> 170
aatcgtcccg actgccggat ggttcgatct caaaccgctc 40

<210> 171
<211> 42
<212> DNA
<213> Artificial Sequence

<220>
<223> Klammerstrang_0170 zugehoerig zu 3D-DNA-Nanostruktur.

<400> 171
atgaaaaact ttccttctag cttatggctg cattaaaagc aa 42

<210> 172
<211> 40
<212> DNA
<213> Artificial Sequence

<220>
<223> Klammerstrang_0171 zugehoerig zu 3D-DNA-Nanostruktur.

<400> 172
ccaccagcct agggcgcccg gtgagatcac tttacagaaa 40

<210> 173
<211> 42
<212> DNA
<213> Artificial Sequence

<220>
<223> Klammerstrang_0172 zugehoerig zu 3D-DNA-Nanostruktur.

<400> 173
catttcaagc attgaggccc tcggaaggaa tgaatattta tt 42

<210> 174
<211> 40
<212> DNA
<213> Artificial Sequence

<220>
<223> Klammerstrang_0173 zugehoerig zu 3D-DNA-Nanostruktur.

<400> 174
ggttggggca tggacccagg tgaagattaa acatagctta 40

<210> 175
<211> 42
<212> DNA
<213> Artificial Sequence

<220>
<223> Klammerstrang_0174 zugehoerig zu 3D-DNA-Nanostruktur.

<400> 175
gccaacatgt cagctgctct ccgtccggtt agaaaaaaca ac 42

<210> 176
<211> 40
<212> DNA
<213> Artificial Sequence

<220>
<223> Klammerstrang_0175 zugehoerig zu 3D-DNA-Nanostruktur.

<400> 176
cgtttttcga ggcacacgtc taggttgtac gagcacaagc 40

<210> 177
<211> 42
<212> DNA
<213> Artificial Sequence

<220>
<223> Klammerstrang_0176 zugehoerig zu 3D-DNA-Nanostruktur.

<400> 177
aacgtcaacg ctctgttcgc gcactaccat ttaccaatgt tt 42

<210> 178
<211> 40
<212> DNA
<213> Artificial Sequence

<220>
<223> Klammerstrang_0177 zugehoerig zu 3D-DNA-Nanostruktur.

<400> 178
ccaaaagccg cgttcggcga aacgggaaaa tagctaatac 40

<210> 179
<211> 42
<212> DNA

<213> Artificial Sequence

<220>
<223> Klammerstrang_0178 zugehoerig zu 3D-DNA-Nanostruktur.

<400> 179
caccagtagg ccggaagttg tttatccaaa ccgattgaaa at 42

<210> 180
<211> 40
<212> DNA
<213> Artificial Sequence

<220>
<223> Klammerstrang_0179 zugehoerig zu 3D-DNA-Nanostruktur.

<400> 180
ccaccagtac aggcatgagt gcggcggccg gaaccaacca 40

<210> 181
<211> 42
<212> DNA
<213> Artificial Sequence

<220>
<223> Klammerstrang_0180 zugehoerig zu 3D-DNA-Nanostruktur.

<400> 181
ctcctcaaga cgtaaatgac taaacccgag gggtcatga ga 42

<210> 182
<211> 40
<212> DNA
<213> Artificial Sequence

<220>
<223> Klammerstrang_0181 zugehoerig zu 3D-DNA-Nanostruktur.

<400> 182
agttagcatg gataccggcg gctggcgaag ggatactcat 40

<210> 183
<211> 42
<212> DNA
<213> Artificial Sequence

<220>
<223> Klammerstrang_0182 zugehoerig zu 3D-DNA-Nanostruktur.

<400> 183
ataaccgatg ccgctgctgt gctcgcaggt gcctttacac gc 42

<210> 184

<211> 40
<212> DNA
<213> Artificial Sequence

<220>
<223> Klammerstrang_0183 zugehoerig zu 3D-DNA-Nanostruktur.

<400> 184
atttgtacct ggcccaaaac tggagataag gcacccggag 40

<210> 185
<211> 42
<212> DNA
<213> Artificial Sequence

<220>
<223> Klammerstrang_0184 zugehoerig zu 3D-DNA-Nanostruktur.

<400> 185
gagatggttc gcatatgcgg atcccaactc gaaccggggc tt 42

<210> 186
<211> 40
<212> DNA
<213> Artificial Sequence

<220>
<223> Klammerstrang_0185 zugehoerig zu 3D-DNA-Nanostruktur.

<400> 186
cttttgcttc gcgcgttgga gtcgatttag atacaagagg 40

<210> 187
<211> 42
<212> DNA
<213> Artificial Sequence

<220>
<223> Klammerstrang_0186 zugehoerig zu 3D-DNA-Nanostruktur.

<400> 187
aggtcagggg ctagtgccag cccgtcctaa aagcggacca ac 42

<210> 188
<211> 40
<212> DNA
<213> Artificial Sequence

<220>
<223> Klammerstrang_0187 zugehoerig zu 3D-DNA-Nanostruktur.

<400> 188
aatagtagcc tacagtttgt ggccgaattt agtttctact 40

<210> 189
<211> 42
<212> DNA
<213> Artificial Sequence

<220>
<223> Klammerstrang_0188 zugehoerig zu 3D-DNA-Nanostruktur.

<400> 189
ctgataaagc gcgagcagtc gatagcgcgt ttttaaatct ag 42

<210> 190
<211> 40
<212> DNA
<213> Artificial Sequence

<220>
<223> Klammerstrang_0189 zugehoerig zu 3D-DNA-Nanostruktur.

<400> 190
ttcgcgtact cgaggctagg ttcaacttat atttaaataa 40

<210> 191
<211> 42
<212> DNA
<213> Artificial Sequence

<220>
<223> Klammerstrang_0190 zugehoerig zu 3D-DNA-Nanostruktur.

<400> 191
tcggtgcggc ggaattgacg tcaatgccct ggaagatggc ga 42

<210> 192
<211> 40
<212> DNA
<213> Artificial Sequence

<220>
<223> Klammerstrang_0191 zugehoerig zu 3D-DNA-Nanostruktur.

<400> 192
aattccagtt gttgttctct ctccgcggac gcatgctcac 40

<210> 193
<211> 42
<212> DNA
<213> Artificial Sequence

<220>
<223> Klammerstrang_0192 zugehoerig zu 3D-DNA-Nanostruktur.

<400> 193

agcaagcgct tcacgttaag cactgcgctg accagtggtt gc 42

<210> 194
<211> 40
<212> DNA
<213> Artificial Sequence

<220>
<223> Klammerstrang_0193 zugehoerig zu 3D-DNA-Nanostruktur.

<400> 194
ctcatggacc ctgtaggcct ccctcggtct tgctgaaacg 40

<210> 195
<211> 42
<212> DNA
<213> Artificial Sequence

<220>
<223> Klammerstrang_0194 zugehoerig zu 3D-DNA-Nanostruktur.

<400> 195
caacagtgcg gacccggctg tggagctaaa acgaaccgcc tg 42

<210> 196
<211> 40
<212> DNA
<213> Artificial Sequence

<220>
<223> Klammerstrang_0195 zugehoerig zu 3D-DNA-Nanostruktur.

<400> 196
taacattgga ggcctctgca tctgcacctc gacaattgag 40

<210> 197
<211> 42
<212> DNA
<213> Artificial Sequence

<220>
<223> Klammerstrang_0196 zugehoerig zu 3D-DNA-Nanostruktur.

<400> 197
agttacaaag accgcgtagg accgctgaat agtaccttac ca 42

<210> 198
<211> 40
<212> DNA
<213> Artificial Sequence

<220>
<223> Klammerstrang_0197 zugehoerig zu 3D-DNA-Nanostruktur.

<400> 198
gagagacgat gagtacgcat cgtccaaact tagatggtct 40

<210> 199
<211> 42
<212> DNA
<213> Artificial Sequence

<220>
<223> Klammerstrang_0198 zugehoerig zu 3D-DNA-Nanostruktur.

<400> 199
gcttaattgt aggccggcac tggcacggta agtatcagta gg 42

<210> 200
<211> 40
<212> DNA
<213> Artificial Sequence

<220>
<223> Klammerstrang_0199 zugehoerig zu 3D-DNA-Nanostruktur.

<400> 200
agtaccgtgt cgcaggccac aggtgagaac caatcaacca 40

<210> 201
<211> 42
<212> DNA
<213> Artificial Sequence

<220>
<223> Klammerstrang_0200 zugehoerig zu 3D-DNA-Nanostruktur.

<400> 201
ccaatccaca aaccgttcca gcccacatca agcgtcttta tc 42

<210> 202
<211> 40
<212> DNA
<213> Artificial Sequence

<220>
<223> Klammerstrang_0201 zugehoerig zu 3D-DNA-Nanostruktur.

<400> 202
accgaggaca tcgggtggtg ccctagaacg aagccaggaa 40

<210> 203
<211> 42
<212> DNA
<213> Artificial Sequence

<220>
<223> Klammerstrang_0202 zugehoerig zu 3D-DNA-Nanostruktur.

<400> 203
gagccattag ggcgacgcac gtcataacaa aaggtaagac tt 42

<210> 204
<211> 40
<212> DNA
<213> Artificial Sequence

<220>
<223> Klammerstrang_0203 zugehoerig zu 3D-DNA-Nanostruktur.

<400> 204
caccacctct gtatgctagg tgctcgtgcc accggagagc 40

<210> 205
<211> 42
<212> DNA
<213> Artificial Sequence

<220>
<223> Klammerstrang_0204 zugehoerig zu 3D-DNA-Nanostruktur.

<400> 205
ctgaaacagc ggccctgcca agctgcccct agtaacatta tt 42

<210> 206
<211> 40
<212> DNA
<213> Artificial Sequence

<220>
<223> Klammerstrang_0205 zugehoerig zu 3D-DNA-Nanostruktur.

<400> 206
gcctgtaatc gggcgtccct aacttctgca ataggacaac 40

<210> 207
<211> 42
<212> DNA
<213> Artificial Sequence

<220>
<223> Klammerstrang_0206 zugehoerig zu 3D-DNA-Nanostruktur.

<400> 207
gccgacaacg gcatggcgaa caaggacagc ggtttatgtt gc 42

<210> 208
<211> 40
<212> DNA
<213> Artificial Sequence

<220>
<223> Klammerstrang_0207 zugehoerig zu 3D-DNA-Nanostruktur.

<400> 208
taccaagtct cgccaccctc accttgtgaa cgaaagatta 40

<210> 209
<211> 42
<212> DNA
<213> Artificial Sequence

<220>
<223> Klammerstrang_0208 zugehoerig zu 3D-DNA-Nanostruktur.

<400> 209
aaacaccaca ccttctgcac tgtcgtcacg tacccaaacg ag 42

<210> 210
<211> 40
<212> DNA
<213> Artificial Sequence

<220>
<223> Klammerstrang_0209 zugehoerig zu 3D-DNA-Nanostruktur.

<400> 210
acgacgagct tcacagaccc taccgcgaaa aaggaaccag 40

<210> 211
<211> 42
<212> DNA
<213> Artificial Sequence

<220>
<223> Klammerstrang_0210 zugehoerig zu 3D-DNA-Nanostruktur.

<400> 211
aagcgaacaa cacctccgta ctccacccta aaaagatctt ca 42

<210> 212
<211> 40
<212> DNA
<213> Artificial Sequence

<220>
<223> Klammerstrang_0211 zugehoerig zu 3D-DNA-Nanostruktur.

<400> 212
cgagctgttg cgcaaaaggc gcgaagcaag atacagggcg 40

<210> 213
<211> 42

<212> DNA
<213> Artificial Sequence

<220>
<223> Klammerstrang_0212 zugehoerig zu 3D-DNA-Nanostruktur.

<400> 213
caccatcatg ttgagtccgc tgccatgaaa ctgagtacaa at 42

<210> 214
<211> 40
<212> DNA
<213> Artificial Sequence

<220>
<223> Klammerstrang_0213 zugehoerig zu 3D-DNA-Nanostruktur.

<400> 214
ttaaccactc cgaggtcgcc ggagggataa cgttaatttt 40

<210> 215
<211> 42
<212> DNA
<213> Artificial Sequence

<220>
<223> Klammerstrang_0214 zugehoerig zu 3D-DNA-Nanostruktur.

<400> 215
ttcaggctca cgcaacactc ccgtctagat agccagccgc ca 42

<210> 216
<211> 40
<212> DNA
<213> Artificial Sequence

<220>
<223> Klammerstrang_0215 zugehoerig zu 3D-DNA-Nanostruktur.

<400> 216
tttcctgact cgaaccgcac cctgcagccc cgtatagctg 40

<210> 217
<211> 42
<212> DNA
<213> Artificial Sequence

<220>
<223> Klammerstrang_0216 zugehoerig zu 3D-DNA-Nanostruktur.

<400> 217
cccttcactg tgcaccgccc ggatggtgtt aacagctgat tg 42

<210> 218
<211> 40
<212> DNA
<213> Artificial Sequence

<220>
<223> Klammerstrang_0217 zugehoerig zu 3D-DNA-Nanostruktur.

<400> 218
taccgccaag acaacagcgc caggctcgcc agaacaatat 40

<210> 219
<211> 42
<212> DNA
<213> Artificial Sequence

<220>
<223> Klammerstrang_0218 zugehoerig zu 3D-DNA-Nanostruktur.

<400> 219
aggtgagggt atagcgccat cgggcataag aagataaaac ag 42

<210> 220
<211> 40
<212> DNA
<213> Artificial Sequence

<220>
<223> Klammerstrang_0219 zugehoerig zu 3D-DNA-Nanostruktur.

<400> 220
ccgaacggga cccagccgag agccgacgta aatcctttgc 40

<210> 221
<211> 42
<212> DNA
<213> Artificial Sequence

<220>
<223> Klammerstrang_0220 zugehoerig zu 3D-DNA-Nanostruktur.

<400> 221
aacggattaa ctgcgtgagt gaaggaactt gggagaaaca at 42

<210> 222
<211> 40
<212> DNA
<213> Artificial Sequence

<220>
<223> Klammerstrang_0221 zugehoerig zu 3D-DNA-Nanostruktur.

<400> 222
aatagtgaca ggtgtaggat cgggcgcgct gagaagagtc 40

<210> 223
<211> 42
<212> DNA
<213> Artificial Sequence

<220>
<223> Klammerstrang_0222 zugehoerig zu 3D-DNA-Nanostruktur.

<400> 223
accagtatgg catcaactgt ggtgacggaa atacaaattc tt 42

<210> 224
<211> 40
<212> DNA
<213> Artificial Sequence

<220>
<223> Klammerstrang_0223 zugehoerig zu 3D-DNA-Nanostruktur.

<400> 224
ttatcataga cctctgcttg tgccacaagg ctgtctttcc 40

<210> 225
<211> 42
<212> DNA
<213> Artificial Sequence

<220>
<223> Klammerstrang_0224 zugehoerig zu 3D-DNA-Nanostruktur.

<400> 225
gttacaaagc gccagcgact gggcgtcctc cctaatttgc ca 42

<210> 226
<211> 40
<212> DNA
<213> Artificial Sequence

<220>
<223> Klammerstrang_0225 zugehoerig zu 3D-DNA-Nanostruktur.

<400> 226
agatagctct gctcaacact caccgcatag aaaagtaagc 40

<210> 227
<211> 42
<212> DNA
<213> Artificial Sequence

<220>
<223> Klammerstrang_0226 zugehoerig zu 3D-DNA-Nanostruktur.

<400> 227
ttaaaggtag tccgccctac agaagttccg ggaaattatt ca 42

<210> 228
<211> 40
<212> DNA
<213> Artificial Sequence

<220>
<223> Klammerstrang_0227 zugehoerig zu 3D-DNA-Nanostruktur.

<400> 228
gccacccccc ggtcgaaaga gcaaacgatc cctcagagcc 40

<210> 229
<211> 42
<212> DNA
<213> Artificial Sequence

<220>
<223> Klammerstrang_0228 zugehoerig zu 3D-DNA-Nanostruktur.

<400> 229
tgccccctag cagcccgtcc gtgatgagtc ataaacagtt aa 42

<210> 230
<211> 40
<212> DNA
<213> Artificial Sequence

<220>
<223> Klammerstrang_0229 zugehoerig zu 3D-DNA-Nanostruktur.

<400> 230
tgagtttagg ccagagtcga gtgcatgcgt accgtaacac 40

<210> 231
<211> 42
<212> DNA
<213> Artificial Sequence

<220>
<223> Klammerstrang_0230 zugehoerig zu 3D-DNA-Nanostruktur.

<400> 231
tttcttaaat ccgggcattt gggaagcccg tttcgaggtg aa 42

<210> 232
<211> 40
<212> DNA
<213> Artificial Sequence

<220>

<223> Klammerstrang_0231 zugehoerig zu 3D-DNA-Nanostruktur.

<400> 232
aaacactggc cggcgtctta gttacgacaa gaatacacta 40

<210> 233
<211> 42
<212> DNA
<213> Artificial Sequence

<220>
<223> Klammerstrang_0232 zugehoerig zu 3D-DNA-Nanostruktur.

<400> 233
cattcagtcc gggccgcgca tacggaccca aacaaagctg ct 42

<210> 234
<211> 40
<212> DNA
<213> Artificial Sequence

<220>
<223> Klammerstrang_0233 zugehoerig zu 3D-DNA-Nanostruktur.

<400> 234
gcaacactga ctgggcagtg gttgctgagg catagtaaga 40

<210> 235
<211> 42
<212> DNA
<213> Artificial Sequence

<220>
<223> Klammerstrang_0234 zugehoerig zu 3D-DNA-Nanostruktur.

<400> 235
ttcaaatatc gtcgcgcgtg tagcatatta agcccgaaag ac 42

<210> 236
<211> 40
<212> DNA
<213> Artificial Sequence

<220>
<223> Klammerstrang_0235 zugehoerig zu 3D-DNA-Nanostruktur.

<400> 236
cctgtttgtt cgttcgatga ttgtcgtcaa tggtcaataa 40

<210> 237
<211> 42
<212> DNA
<213> Artificial Sequence

<220>
<223> Klammerstrang_0236 zugehoerig zu 3D-DNA-Nanostruktur.

<400> 237
aagggtgaac cctgtcgggc cacatggtag gtaaagattc aa 42

<210> 238
<211> 40
<212> DNA
<213> Artificial Sequence

<220>
<223> Klammerstrang_0237 zugehoerig zu 3D-DNA-Nanostruktur.

<400> 238
attaaatctt acgtgggcgt cgagaagagt taaaattcgc 40

<210> 239
<211> 42
<212> DNA
<213> Artificial Sequence

<220>
<223> Klammerstrang_0238 zugehoerig zu 3D-DNA-Nanostruktur.

<400> 239
gccggaaact tgttgtgtca cctcggagtt accgcttctg gt 42

<210> 240
<211> 40
<212> DNA
<213> Artificial Sequence

<220>
<223> Klammerstrang_0239 zugehoerig zu 3D-DNA-Nanostruktur.

<400> 240
gctcgaagcc cgaagaggac gtgtgccccc cgggtaccga 40

<210> 241
<211> 39
<212> DNA
<213> Artificial Sequence

<220>
<223> Klammerstrang_0240 zugehoerig zu 3D-DNA-Nanostruktur.

<400> 241
tcgggcccgt catcgtctca tatcctcagc aactgcggg 39

<210> 242
<211> 39
<212> DNA

<213> Artificial Sequence

<220>
<223> Klammerstrang_0241 zugehoerig zu 3D-DNA-Nanostruktur.

<400> 242
gggcgactgg ctaccgctgc tacccagata tcaccgcac 39

<210> 243
<211> 39
<212> DNA
<213> Artificial Sequence

<220>
<223> Klammerstrang_0242 zugehoerig zu 3D-DNA-Nanostruktur.

<400> 243
tcgtgggcaa gatatatgtg gtccgggatg ggcggtgcg 39

<210> 244
<211> 39
<212> DNA
<213> Artificial Sequence

<220>
<223> Klammerstrang_0243 zugehoerig zu 3D-DNA-Nanostruktur.

<400> 244
cggcccacag ggtcaatagc cgtcgcctca cccgactta 39

<210> 245
<211> 39
<212> DNA
<213> Artificial Sequence

<220>
<223> Klammerstrang_0244 zugehoerig zu 3D-DNA-Nanostruktur.

<400> 245
gaaccgctag cctcggccgc gcatttacac cttggtcca 39

<210> 246
<211> 39
<212> DNA
<213> Artificial Sequence

<220>
<223> Klammerstrang_0245 zugehoerig zu 3D-DNA-Nanostruktur.

<400> 246
cggtccagca ccatggtccc gtaccctcac tcgccattg 39

<210> 247

<211> 39
<212> DNA
<213> Artificial Sequence

<220>
<223> Klammerstrang_0246 zugehoerig zu 3D-DNA-Nanostruktur.

<400> 247
gcacaactcc tgaacaaggc gcccagtgca gtcgacgtc 39

<210> 248
<211> 39
<212> DNA
<213> Artificial Sequence

<220>
<223> Klammerstrang_0247 zugehoerig zu 3D-DNA-Nanostruktur.

<400> 248
cgactagagc tgctgtgcgt cccgttgcgt gagttaggc 39

<210> 249
<211> 39
<212> DNA
<213> Artificial Sequence

<220>
<223> Klammerstrang_0248 zugehoerig zu 3D-DNA-Nanostruktur.

<400> 249
cctaccctcg gtgccgagtc cctagacgct gggccagta 39

<210> 250
<211> 39
<212> DNA
<213> Artificial Sequence

<220>
<223> Klammerstrang_0249 zugehoerig zu 3D-DNA-Nanostruktur.

<400> 250
cggccaccgc tcggattatc cacagattat ggcgcgagg 39

<210> 251
<211> 39
<212> DNA
<213> Artificial Sequence

<220>
<223> Klammerstrang_0250 zugehoerig zu 3D-DNA-Nanostruktur.

<400> 251
ccttgagccg catgcgctac gtcttggcct cctaccctc 39

<210> 252
<211> 39
<212> DNA
<213> Artificial Sequence

<220>
<223> Klammerstrang_0251 zugehoerig zu 3D-DNA-Nanostruktur.

<400> 252
gcgcagtccc gttctcgagt gtgcccactt gccctcaat 39

<210> 253
<211> 60
<212> DNA
<213> Artificial Sequence

<220>
<223> Klammerstrang_0252 zugehoerig zu 3D-DNA-Nanostruktur.

<400> 253
actccctgat aggcgtcttt caggcgggcg ggactcaggt tcctctacca cctacatcac 60

<210> 254
<211> 60
<212> DNA
<213> Artificial Sequence

<220>
<223> Klammerstrang_0253 zugehoerig zu 3D-DNA-Nanostruktur.

<400> 254
gggacgccag actgggctca cgagtgatag gctttccgct tcctctacca cctacatcac 60

<210> 255
<211> 60
<212> DNA
<213> Artificial Sequence

<220>
<223> Klammerstrang_0254 zugehoerig zu 3D-DNA-Nanostruktur.

<400> 255
ttcggcttag gtccttctcg tgtggtgccc gctgcccttt tcctctacca cctacatcac 60

<210> 256
<211> 60
<212> DNA
<213> Artificial Sequence

<220>
<223> Klammerstrang_0255 zugehoerig zu 3D-DNA-Nanostruktur.

<400> 256

gcgagccacg cggcgtgtaa ccaacagcat tttctcgctt tcctctacca cctacatcac 60

<210> 257
<211> 60
<212> DNA
<213> Artificial Sequence

<220>
<223> Klammerstrang_0256 zugehoerig zu 3D-DNA-Nanostruktur.

<400> 257
tgcggcagca tcaaggacct accatcttga ccggtcgcct tcctctacca cctacatcac 60

<210> 258
<211> 60
<212> DNA
<213> Artificial Sequence

<220>
<223> Klammerstrang_0257 zugehoerig zu 3D-DNA-Nanostruktur.

<400> 258
actccagttg ctagaacggt ctcgcgcaca cacggtgggt tcctctacca cctacatcac 60

<210> 259
<211> 60
<212> DNA
<213> Artificial Sequence

<220>
<223> Klammerstrang_0258 zugehoerig zu 3D-DNA-Nanostruktur.

<400> 259
cgtgctttga tccgactgac actgcgtggc cgatgggctt tcctctacca cctacatcac 60

<210> 260
<211> 60
<212> DNA
<213> Artificial Sequence

<220>
<223> Klammerstrang_0259 zugehoerig zu 3D-DNA-Nanostruktur.

<400> 260
gtgcccgttg tgagtcggag aaccttgcgc ggcctattgt tcctctacca cctacatcac 60

<210> 261
<211> 60
<212> DNA
<213> Artificial Sequence

<220>
<223> Klammerstrang_0260 zugehoerig zu 3D-DNA-Nanostruktur.

<400> 261
cgcagggacg ccgtcgaaac acagcactcc acatcgtact tcctctacca cctacatcac 60

<210> 262
<211> 60
<212> DNA
<213> Artificial Sequence

<220>
<223> Klammerstrang_0261 zugehoerig zu 3D-DNA-Nanostruktur.

<400> 262
cacgactctc acacggagcc agcctatctc ccgttgcggt tcctctacca cctacatcac 60

<210> 263
<211> 60
<212> DNA
<213> Artificial Sequence

<220>
<223> Klammerstrang_0262 zugehoerig zu 3D-DNA-Nanostruktur.

<400> 263
aacagccttg gaccggtcgc tcacagtcct ttcgtgccct tcctctacca cctacatcac 60

<210> 264
<211> 60
<212> DNA
<213> Artificial Sequence

<220>
<223> Klammerstrang_0263 zugehoerig zu 3D-DNA-Nanostruktur.

<400> 264
ctcgtcgttt ccgttcttgc agtgctgcca gaggcggtgt tcctctacca cctacatcac 60

<210> 265
<211> 60
<212> DNA
<213> Artificial Sequence

<220>
<223> Klammerstrang_0264 zugehoerig zu 3D-DNA-Nanostruktur.

<400> 265
agaaggaaag tctcaccggg cgccctaggc cttccgaggt tcctctacca cctacatcac 60

<210> 266
<211> 60
<212> DNA
<213> Artificial Sequence

<220>
<223> Klammerstrang_0265 zugehoerig zu 3D-DNA-Nanostruktur.

<400> 266
gcctcaatgc tcttcacctg ggtccatgcc cggacggagt tcctctacca cctacatcac 60

<210> 267
<211> 60
<212> DNA
<213> Artificial Sequence

<220>
<223> Klammerstrang_0266 zugehoerig zu 3D-DNA-Nanostruktur.

<400> 267
agcagctgac aacctagacg tgtgcctcgg gtagtgcgct tcctctacca cctacatcac 60

<210> 268
<211> 60
<212> DNA
<213> Artificial Sequence

<220>
<223> Klammerstrang_0267 zugehoerig zu 3D-DNA-Nanostruktur.

<400> 268
gaacagagcg cccgtttcgc cgaacgcggt ggataaacat tcctctacca cctacatcac 60

<210> 269
<211> 60
<212> DNA
<213> Artificial Sequence

<220>
<223> Klammerstrang_0268 zugehoerig zu 3D-DNA-Nanostruktur.

<400> 269
acttccggcc cgccgcactc atgcctgtac gggtttagtt tcctctacca cctacatcac 60

<210> 270
<211> 60
<212> DNA
<213> Artificial Sequence

<220>
<223> Klammerstrang_0269 zugehoerig zu 3D-DNA-Nanostruktur.

<400> 270
catttacgtc gccagccgcc ggtatccatc tgcgagcact tcctctacca cctacatcac 60

<210> 271
<211> 60
<212> DNA
<213> Artificial Sequence

<220>
<223> Klammerstrang_0270 zugehoerig zu 3D-DNA-Nanostruktur.

<400> 271
agcagcggca tctccagttt tgggccaggg ttgggatcct tcctctacca cctacatcac 60

<210> 272
<211> 60
<212> DNA
<213> Artificial Sequence

<220>
<223> Klammerstrang_0271 zugehoerig zu 3D-DNA-Nanostruktur.

<400> 272
gcatatgcga atcgactcca acgcgcgaaa ggacgggctt tcctctacca cctacatcac 60

<210> 273
<211> 60
<212> DNA
<213> Artificial Sequence

<220>
<223> Klammerstrang_0272 zugehoerig zu 3D-DNA-Nanostruktur.

<400> 273
ggcactagcc tcggccacaa actgtaggcg cgctatcgat tcctctacca cctacatcac 60

<210> 274
<211> 60
<212> DNA
<213> Artificial Sequence

<220>
<223> Klammerstrang_0273 zugehoerig zu 3D-DNA-Nanostruktur.

<400> 274
ctgctcgcgc gttgaaccta gcctcgagtg gcattgacgt tcctctacca cctacatcac 60

<210> 275
<211> 60
<212> DNA
<213> Artificial Sequence

<220>
<223> Klammerstrang_0274 zugehoerig zu 3D-DNA-Nanostruktur.

<400> 275
tcaattccgc gcggagagag aacaacaacc acgcaccggt tcctctacca cctacatcac 60

<210> 276
<211> 60

<212> DNA
<213> Artificial Sequence

<220>
<223> Klammerstrang_0275 zugehoerig zu 3D-DNA-Nanostruktur.

<400> 276
tgaccaccgt cgaaccatcc ggcagtcggg ccataagctt tcctctacca cctacatcac 60

<210> 277
<211> 39
<212> DNA
<213> Artificial Sequence

<220>
<223> Klammerstrang_0276 zugehoerig zu 3D-DNA-Nanostruktur.

<400> 277
ggcgctatac tcggctctcg gctgggtccg ttccttcac 39

<210> 278
<211> 39
<212> DNA
<213> Artificial Sequence

<220>
<223> Klammerstrang_0277 zugehoerig zu 3D-DNA-Nanostruktur.

<400> 278
tcacgcagtt cgcccgatcc tacacctgtc cgtcaccac 39

<210> 279
<211> 39
<212> DNA
<213> Artificial Sequence

<220>
<223> Klammerstrang_0278 zugehoerig zu 3D-DNA-Nanostruktur.

<400> 279
agttgatgcc gtggcacaag cagaggtctg gacgcccag 39

<210> 280
<211> 39
<212> DNA
<213> Artificial Sequence

<220>
<223> Klammerstrang_0279 zugehoerig zu 3D-DNA-Nanostruktur.

<400> 280
tcgctggcgc gcggtgagtg ttgagcagag aacttctgt 39

<210> 281
<211> 39
<212> DNA
<213> Artificial Sequence

<220>
<223> Klammerstrang_0280 zugehoerig zu 3D-DNA-Nanostruktur.

<400> 281
agggcggact gtttgctctt tcgaccgggc tcatcacgg 39

<210> 282
<211> 39
<212> DNA
<213> Artificial Sequence

<220>
<223> Klammerstrang_0281 zugehoerig zu 3D-DNA-Nanostruktur.

<400> 282
acgggctgct atgcactcga ctctggcctg gcttcccaa 39

<210> 283
<211> 39
<212> DNA
<213> Artificial Sequence

<220>
<223> Klammerstrang_0282 zugehoerig zu 3D-DNA-Nanostruktur.

<400> 283
atgcccggat cgtaactaag acgccggccg gtccgtatg 39

<210> 284
<211> 39
<212> DNA
<213> Artificial Sequence

<220>
<223> Klammerstrang_0283 zugehoerig zu 3D-DNA-Nanostruktur.

<400> 284
cgcggcccgg agcaaccact gcccagtcaa tatgctaca 39

<210> 285
<211> 39
<212> DNA
<213> Artificial Sequence

<220>
<223> Klammerstrang_0284 zugehoerig zu 3D-DNA-Nanostruktur.

<400> 285
cgcgcgacga cgacaatcat cgaacgaaca ccatgtggc 39

<210> 286
<211> 39
<212> DNA
<213> Artificial Sequence

<220>
<223> Klammerstrang_0285 zugehoerig zu 3D-DNA-Nanostruktur.

<400> 286
ccgacagggt ttctcgacgc ccacgtaagc tccgaggtg 39

<210> 287
<211> 39
<212> DNA
<213> Artificial Sequence

<220>
<223> Klammerstrang_0286 zugehoerig zu 3D-DNA-Nanostruktur.

<400> 287
acacaacaag gcacacgtcc tcttcgggcc accatccgg 39

<210> 288
<211> 39
<212> DNA
<213> Artificial Sequence

<220>
<223> Klammerstrang_0287 zugehoerig zu 3D-DNA-Nanostruktur.

<400> 288
gcggtgcaca agcctggcgc tgttgtcttt atgcccgat 39

<210> 289
<211> 60
<212> DNA
<213> Artificial Sequence

<220>
<223> Klammerstrang_0288 zugehoerig zu 3D-DNA-Nanostruktur.

<400> 289
cctcaacctc gctcgaacgt acgcagccgc aacactggat tcctctacca cctacatcac 60

<210> 290
<211> 60
<212> DNA
<213> Artificial Sequence

<220>
<223> Klammerstrang_0289 zugehoerig zu 3D-DNA-Nanostruktur.

<400> 290
gaagcggcat agctccgtcc tcgggtagta aagcgcgcat tcctctacca cctacatcac 60

<210> 291
<211> 60
<212> DNA
<213> Artificial Sequence

<220>
<223> Klammerstrang_0290 zugehoerig zu 3D-DNA-Nanostruktur.

<400> 291
gggaacagcg cataattcgc cgtgtgtgtg gccatcgcat tcctctacca cctacatcac 60

<210> 292
<211> 60
<212> DNA
<213> Artificial Sequence

<220>
<223> Klammerstrang_0291 zugehoerig zu 3D-DNA-Nanostruktur.

<400> 292
cgacgccaca acgaacggcc tcctcgaaac cttgtgaggt tcctctacca cctacatcac 60

<210> 293
<211> 60
<212> DNA
<213> Artificial Sequence

<220>
<223> Klammerstrang_0292 zugehoerig zu 3D-DNA-Nanostruktur.

<400> 293
gggatgtaac accacgtggc gcacgcactg tgtaggagct tcctctacca cctacatcac 60

<210> 294
<211> 60
<212> DNA
<213> Artificial Sequence

<220>
<223> Klammerstrang_0293 zugehoerig zu 3D-DNA-Nanostruktur.

<400> 294
ctgggaggta cagctagggt gaccggcctc ctgccctcat tcctctacca cctacatcac 60

<210> 295
<211> 60
<212> DNA
<213> Artificial Sequence

<220>

<223> Klammerstrang_0294 zugehoerig zu 3D-DNA-Nanostruktur.

<400> 295
gcccttgcca cagacccgca cagcgagttc tggcttcatt tcctctacca cctacatcac 60

<210> 296
<211> 60
<212> DNA
<213> Artificial Sequence

<220>
<223> Klammerstrang_0295 zugehoerig zu 3D-DNA-Nanostruktur.

<400> 296
gcgcaacgtg acggtacgcc aacggtcacc agaagtcagt tcctctacca cctacatcac 60

<210> 297
<211> 60
<212> DNA
<213> Artificial Sequence

<220>
<223> Klammerstrang_0296 zugehoerig zu 3D-DNA-Nanostruktur.

<400> 297
cggccaacct caaaggcgtc cacggcgaat ctgcgtctat tcctctacca cctacatcac 60

<210> 298
<211> 60
<212> DNA
<213> Artificial Sequence

<220>
<223> Klammerstrang_0297 zugehoerig zu 3D-DNA-Nanostruktur.

<400> 298
cgcgcatagg gagtgtgctg gcgggctgcg agtaagaatt tcctctacca cctacatcac 60

<210> 299
<211> 60
<212> DNA
<213> Artificial Sequence

<220>
<223> Klammerstrang_0298 zugehoerig zu 3D-DNA-Nanostruktur.

<400> 299
ctcaggaagc gagcagtaaa tccttggccg accgcagcgt tcctctacca cctacatcac 60

<210> 300
<211> 60
<212> DNA
<213> Artificial Sequence

<220>
<223> Klammerstrang_0299 zugehoerig zu 3D-DNA-Nanostruktur.

<400> 300
gagcagagca gcttatccct gggtctccgg catctgcgct tcctctacca cctacatcac 60

<210> 301
<211> 60
<212> DNA
<213> Artificial Sequence

<220>
<223> Klammerstrang_0300 zugehoerig zu 3D-DNA-Nanostruktur.

<400> 301
tggtgttccc tttcgccgcc ttgcaagtcc catcatgcct tcctctacca cctacatcac 60

<210> 302
<211> 60
<212> DNA
<213> Artificial Sequence

<220>
<223> Klammerstrang_0301 zugehoerig zu 3D-DNA-Nanostruktur.

<400> 302
gcaagaagcc tacgcgaagc tgtcgggacc acggcaatgt tcctctacca cctacatcac 60

<210> 303
<211> 60
<212> DNA
<213> Artificial Sequence

<220>
<223> Klammerstrang_0302 zugehoerig zu 3D-DNA-Nanostruktur.

<400> 303
ctccgtgctt gttaccttcg agtcgcggca caccacacgt tcctctacca cctacatcac 60

<210> 304
<211> 60
<212> DNA
<213> Artificial Sequence

<220>
<223> Klammerstrang_0303 zugehoerig zu 3D-DNA-Nanostruktur.

<400> 304
tctgctcccg tgaatcccgc tcgttcgttc tcccggctat tcctctacca cctacatcac 60

<210> 305
<211> 60
<212> DNA

<213> Artificial Sequence

<220>
<223> Klammerstrang_0304 zugehoerig zu 3D-DNA-Nanostruktur.

<400> 305
atgggaatgt agaagcgctg caaatcgtcg cccgtgccgt tcctctacca cctacatcac 60

<210> 306
<211> 60
<212> DNA
<213> Artificial Sequence

<220>
<223> Klammerstrang_0305 zugehoerig zu 3D-DNA-Nanostruktur.

<400> 306
gtctgctcgt agttagtcgc ctcgacgcac gacgggacct tcctctacca cctacatcac 60

<210> 307
<211> 60
<212> DNA
<213> Artificial Sequence

<220>
<223> Klammerstrang_0306 zugehoerig zu 3D-DNA-Nanostruktur.

<400> 307
ggcccaacta gagaagcaga aggcatcccg gtcggccact tcctctacca cctacatcac 60

<210> 308
<211> 60
<212> DNA
<213> Artificial Sequence

<220>
<223> Klammerstrang_0307 zugehoerig zu 3D-DNA-Nanostruktur.

<400> 308
agttcgcacc gccattctcg gcagactccc accgacatgt tcctctacca cctacatcac 60

<210> 309
<211> 60
<212> DNA
<213> Artificial Sequence

<220>
<223> Klammerstrang_0308 zugehoerig zu 3D-DNA-Nanostruktur.

<400> 309
gaaaagcacg gcgtgcagtg gcccaggtag gtcgttccat tcctctacca cctacatcac 60

<210> 310

<211> 60
<212> DNA
<213> Artificial Sequence

<220>
<223> Klammerstrang_0309 zugehoerig zu 3D-DNA-Nanostruktur.

<400> 310
ggtaatgcgt tcgggctact gcggtgtgag atcgtgcggt tcctctacca cctacatcac 60

<210> 311
<211> 60
<212> DNA
<213> Artificial Sequence

<220>
<223> Klammerstrang_0310 zugehoerig zu 3D-DNA-Nanostruktur.

<400> 311
tctacgtagg catcagcgtc gcggcactac acgtgggtct tcctctacca cctacatcac 60

<210> 312
<211> 60
<212> DNA
<213> Artificial Sequence

<220>
<223> Klammerstrang_0311 zugehoerig zu 3D-DNA-Nanostruktur.

<400> 312
cgaagggacg cgggaggcta gggaatgctg catcagtggt tcctctacca cctacatcac 60

<210> 313
<211> 39
<212> DNA
<213> Artificial Sequence

<220>
<223> Klammerstrang_0312 zugehoerig zu 3D-DNA-Nanostruktur.

<400> 313
cctacagggt tagctccaca gccgggtccg tgcagatgc 39

<210> 314
<211> 39
<212> DNA
<213> Artificial Sequence

<220>
<223> Klammerstrang_0313 zugehoerig zu 3D-DNA-Nanostruktur.

<400> 314
agaggcctcc tcagcggtcc tacgcggtct tggacgatg 39

<210> 315
<211> 39
<212> DNA
<213> Artificial Sequence

<220>
<223> Klammerstrang_0314 zugehoerig zu 3D-DNA-Nanostruktur.

<400> 315
cgtactcatc ccgtgccagt gccggcctac tcacctgtg 39

<210> 316
<211> 39
<212> DNA
<213> Artificial Sequence

<220>
<223> Klammerstrang_0315 zugehoerig zu 3D-DNA-Nanostruktur.

<400> 316
gcctgcgaca atgtgggctg gaacggtttg ctagggcac 39

<210> 317
<211> 39
<212> DNA
<213> Artificial Sequence

<220>
<223> Klammerstrang_0316 zugehoerig zu 3D-DNA-Nanostruktur.

<400> 317
cacccgatgt gttatgacgt gcgtcgccct cgagcacct 39

<210> 318
<211> 39
<212> DNA
<213> Artificial Sequence

<220>
<223> Klammerstrang_0317 zugehoerig zu 3D-DNA-Nanostruktur.

<400> 318
agcatacaga gggcagcttg gcagggccgc gaagttagg 39

<210> 319
<211> 39
<212> DNA
<213> Artificial Sequence

<220>
<223> Klammerstrang_0318 zugehoerig zu 3D-DNA-Nanostruktur.

<400> 319

gacgcccgat tgtccttgtt cgccatgccg caaggtgag 39

<210> 320
<211> 39
<212> DNA
<213> Artificial Sequence

<220>
<223> Klammerstrang_0319 zugehoerig zu 3D-DNA-Nanostruktur.

<400> 320
ggtggcgaga tgacgacagt gcagaaggtg gcggtaggg 39

<210> 321
<211> 39
<212> DNA
<213> Artificial Sequence

<220>
<223> Klammerstrang_0320 zugehoerig zu 3D-DNA-Nanostruktur.

<400> 321
tctgtgaagc gggtggagta cggaggtgtt cttcgcgcc 39

<210> 322
<211> 39
<212> DNA
<213> Artificial Sequence

<220>
<223> Klammerstrang_0321 zugehoerig zu 3D-DNA-Nanostruktur.

<400> 322
ttttgcgcaa tcatggcagc ggactcaaca ccctccggc 39

<210> 323
<211> 39
<212> DNA
<213> Artificial Sequence

<220>
<223> Klammerstrang_0322 zugehoerig zu 3D-DNA-Nanostruktur.

<400> 323
gacctcggag ctagacggga gtgttgcgtg tgcagggtg 39

<210> 324
<211> 39
<212> DNA
<213> Artificial Sequence

<220>
<223> Klammerstrang_0323 zugehoerig zu 3D-DNA-Nanostruktur.

<400> 324
cggttcgagt gcgcagtgct taacgtgaag cgagggagg 39

<210> 325
<211> 60
<212> DNA
<213> Artificial Sequence

<220>
<223> Klammerstrang_0324 zugehoerig zu 3D-DNA-Nanostruktur.

<400> 325
actccctgat aggcgtcttt caggcgggcg ggactcaggt aacattccta acttctcata 60

<210> 326
<211> 60
<212> DNA
<213> Artificial Sequence

<220>
<223> Klammerstrang_0325 zugehoerig zu 3D-DNA-Nanostruktur.

<400> 326
gggacgccag actgggctca cgagtgatag gctttccgct aacattccta acttctcata 60

<210> 327
<211> 60
<212> DNA
<213> Artificial Sequence

<220>
<223> Klammerstrang_0326 zugehoerig zu 3D-DNA-Nanostruktur.

<400> 327
ttcggcttag gtccttctcg tgtggtgccc gctgcccttt aacattccta acttctcata 60

<210> 328
<211> 60
<212> DNA
<213> Artificial Sequence

<220>
<223> Klammerstrang_0327 zugehoerig zu 3D-DNA-Nanostruktur.

<400> 328
gcgagccacg cggcgtgtaa ccaacagcat tttctcgctt aacattccta acttctcata 60

<210> 329
<211> 60
<212> DNA
<213> Artificial Sequence

<220>
<223> Klammerstrang_0328 zugehoerig zu 3D-DNA-Nanostruktur.

<400> 329
tgcggcagca tcaaggacct accatcttga ccggtcgcct aacattccta acttctcata 60

<210> 330
<211> 60
<212> DNA
<213> Artificial Sequence

<220>
<223> Klammerstrang_0329 zugehoerig zu 3D-DNA-Nanostruktur.

<400> 330
actccagttg ctagaacggt ctcgcgcaca cacggtgggt aacattccta acttctcata 60

<210> 331
<211> 60
<212> DNA
<213> Artificial Sequence

<220>
<223> Klammerstrang_0330 zugehoerig zu 3D-DNA-Nanostruktur.

<400> 331
cgtgctttga tccgactgac actgcgtggc cgatgggctt aacattccta acttctcata 60

<210> 332
<211> 60
<212> DNA
<213> Artificial Sequence

<220>
<223> Klammerstrang_0331 zugehoerig zu 3D-DNA-Nanostruktur.

<400> 332
gtgcccgttg tgagtcggag aaccttgcgc ggcctattgt aacattccta acttctcata 60

<210> 333
<211> 60
<212> DNA
<213> Artificial Sequence

<220>
<223> Klammerstrang_0332 zugehoerig zu 3D-DNA-Nanostruktur.

<400> 333
cgcagggacg ccgtcgaaac acagcactcc acatcgtact aacattccta acttctcata 60

<210> 334
<211> 60
<212> DNA
<213> Artificial Sequence

<220>
<223> Klammerstrang_0333 zugehoerig zu 3D-DNA-Nanostruktur.

<400> 334
cacgactctc acacggagcc agcctatctc ccgttgcggt aacattccta acttctcata 60

<210> 335
<211> 60
<212> DNA
<213> Artificial Sequence

<220>
<223> Klammerstrang_0334 zugehoerig zu 3D-DNA-Nanostruktur.

<400> 335
aacagccttg gaccggtcgc tcacagtcct ttcgtgccct aacattccta acttctcata 60

<210> 336
<211> 60
<212> DNA
<213> Artificial Sequence

<220>
<223> Klammerstrang_0335 zugehoerig zu 3D-DNA-Nanostruktur.

<400> 336
ctcgtcgttt ccgttcttgc agtgctgcca gaggcggtgt aacattccta acttctcata 60

<210> 337
<211> 60
<212> DNA
<213> Artificial Sequence

<220>
<223> Klammerstrang_0336 zugehoerig zu 3D-DNA-Nanostruktur.

<400> 337
agaaggaaag tctcaccggg cgccctaggc cttccgaggt aacattccta acttctcata 60

<210> 338
<211> 60
<212> DNA
<213> Artificial Sequence

<220>
<223> Klammerstrang_0337 zugehoerig zu 3D-DNA-Nanostruktur.

<400> 338
gcctcaatgc tcttcacctg ggtccatgcc cggacggagt aacattccta acttctcata 60

<210> 339
<211> 60

<212> DNA
<213> Artificial Sequence

<220>
<223> Klammerstrang_0338 zugehoerig zu 3D-DNA-Nanostruktur.

<400> 339
agcagctgac aacctagacg tgtgcctcgg gtagtgcgct aacattccta acttctcata 60

<210> 340
<211> 60
<212> DNA
<213> Artificial Sequence

<220>
<223> Klammerstrang_0339 zugehoerig zu 3D-DNA-Nanostruktur.

<400> 340
gaacagagcg cccgtttcgc cgaacgcggt ggataaacat aacattccta acttctcata 60

<210> 341
<211> 60
<212> DNA
<213> Artificial Sequence

<220>
<223> Klammerstrang_0340 zugehoerig zu 3D-DNA-Nanostruktur.

<400> 341
acttccggcc cgccgcactc atgcctgtac gggtttagtt aacattccta acttctcata 60

<210> 342
<211> 60
<212> DNA
<213> Artificial Sequence

<220>
<223> Klammerstrang_0341 zugehoerig zu 3D-DNA-Nanostruktur.

<400> 342
catttacgtc gccagccgcc ggtatccatc tgcgagcact aacattccta acttctcata 60

<210> 343
<211> 60
<212> DNA
<213> Artificial Sequence

<220>
<223> Klammerstrang_0342 zugehoerig zu 3D-DNA-Nanostruktur.

<400> 343
agcagcggca tctccagttt tgggccaggg ttgggatcct aacattccta acttctcata 60

<210> 344
<211> 60
<212> DNA
<213> Artificial Sequence

<220>
<223> Klammerstrang_0343 zugehoerig zu 3D-DNA-Nanostruktur.

<400> 344
gcatatgcga atcgactcca acgcgcgaaa ggacgggctt aacattccta acttctcata 60

<210> 345
<211> 60
<212> DNA
<213> Artificial Sequence

<220>
<223> Klammerstrang_0344 zugehoerig zu 3D-DNA-Nanostruktur.

<400> 345
ggcactagcc tcggccacaa actgtaggcg cgctatcgat aacattccta acttctcata 60

<210> 346
<211> 60
<212> DNA
<213> Artificial Sequence

<220>
<223> Klammerstrang_0345 zugehoerig zu 3D-DNA-Nanostruktur.

<400> 346
ctgctcgcgc gttgaaccta gcctcgagtg gcattgacgt aacattccta acttctcata 60

<210> 347
<211> 60
<212> DNA
<213> Artificial Sequence

<220>
<223> Klammerstrang_0346 zugehoerig zu 3D-DNA-Nanostruktur.

<400> 347
tcaattccgc gcggagagag aacaacaacc acgcaccggt aacattccta acttctcata 60

<210> 348
<211> 60
<212> DNA
<213> Artificial Sequence

<220>
<223> Klammerstrang_0347 zugehoerig zu 3D-DNA-Nanostruktur.

<400> 348
tgaccaccgt cgaaccatcc ggcagtcggg ccataagctt aacattccta acttctcata 60

<210> 349
<211> 60
<212> DNA
<213> Artificial Sequence

<220>
<223> Klammerstrang_0348 zugehoerig zu 3D-DNA-Nanostruktur.

<400> 349
cctcaacctc gctcgaacgt acgcagccgc aacactggat aacattccta acttctcata 60

<210> 350
<211> 60
<212> DNA
<213> Artificial Sequence

<220>
<223> Klammerstrang_0349 zugehoerig zu 3D-DNA-Nanostruktur.

<400> 350
gaagcggcat agctccgtcc tcgggtagta aagcgcgcat aacattccta acttctcata 60

<210> 351
<211> 60
<212> DNA
<213> Artificial Sequence

<220>
<223> Klammerstrang_0350 zugehoerig zu 3D-DNA-Nanostruktur.

<400> 351
gggaacagcg cataattcgc cgtgtgtgtg gccatcgcat aacattccta acttctcata 60

<210> 352
<211> 60
<212> DNA
<213> Artificial Sequence

<220>
<223> Klammerstrang_0351 zugehoerig zu 3D-DNA-Nanostruktur.

<400> 352
cgacgccaca acgaacggcc tcctcgaaac cttgtgaggt aacattccta acttctcata 60

<210> 353
<211> 60
<212> DNA
<213> Artificial Sequence

<220>
<223> Klammerstrang_0352 zugehoerig zu 3D-DNA-Nanostruktur.

<400> 353
gggatgtaac accacgtggc gcacgcactg tgtaggagct aacattccta acttctcata 60

<210> 354
<211> 60
<212> DNA
<213> Artificial Sequence

<220>
<223> Klammerstrang_0353 zugehoerig zu 3D-DNA-Nanostruktur.

<400> 354
ctgggaggta cagctagggt gaccggcctc ctgccctcat aacattccta acttctcata 60

<210> 355
<211> 60
<212> DNA
<213> Artificial Sequence

<220>
<223> Klammerstrang_0354 zugehoerig zu 3D-DNA-Nanostruktur.

<400> 355
gcccttgcca cagacccgca cagcgagttc tggcttcatt aacattccta acttctcata 60

<210> 356
<211> 60
<212> DNA
<213> Artificial Sequence

<220>
<223> Klammerstrang_0355 zugehoerig zu 3D-DNA-Nanostruktur.

<400> 356
gcgcaacgtg acggtacgcc aacggtcacc agaagtcagt aacattccta acttctcata 60

<210> 357
<211> 60
<212> DNA
<213> Artificial Sequence

<220>
<223> Klammerstrang_0356 zugehoerig zu 3D-DNA-Nanostruktur.

<400> 357
cggccaacct caaaggcgtc cacggcgaat ctgcgtctat aacattccta acttctcata 60

<210> 358
<211> 60
<212> DNA
<213> Artificial Sequence

<220>

<223> Klammerstrang_0357 zugehoerig zu 3D-DNA-Nanostruktur.

<400> 358
cgcgcatagg gagtgtgctg gcgggctgcg agtaagaatt aacattccta acttctcata 60

<210> 359
<211> 60
<212> DNA
<213> Artificial Sequence

<220>
<223> Klammerstrang_0358 zugehoerig zu 3D-DNA-Nanostruktur.

<400> 359
ctcaggaagc gagcagtaaa tccttggccg accgcagcgt aacattccta acttctcata 60

<210> 360
<211> 60
<212> DNA
<213> Artificial Sequence

<220>
<223> Klammerstrang_0359 zugehoerig zu 3D-DNA-Nanostruktur.

<400> 360
gagcagagca gcttatccct gggtctccgg catctgcgct aacattccta acttctcata 60

<210> 361
<211> 60
<212> DNA
<213> Artificial Sequence

<220>
<223> Klammerstrang_0360 zugehoerig zu 3D-DNA-Nanostruktur.

<400> 361
tggtgttccc tttcgccgcc ttgcaagtcc catcatgcct aacattccta acttctcata 60

<210> 362
<211> 60
<212> DNA
<213> Artificial Sequence

<220>
<223> Klammerstrang_0361 zugehoerig zu 3D-DNA-Nanostruktur.

<400> 362
gcaagaagcc tacgcgaagc tgtcgggacc acggcaatgt aacattccta acttctcata 60

<210> 363
<211> 60
<212> DNA
<213> Artificial Sequence

<220>
<223> Klammerstrang_0362 zugehoerig zu 3D-DNA-Nanostruktur.

<400> 363
ctccgtgctt gttaccttcg agtcgcggca caccacacgt aacattccta acttctcata 60

<210> 364
<211> 60
<212> DNA
<213> Artificial Sequence

<220>
<223> Klammerstrang_0363 zugehoerig zu 3D-DNA-Nanostruktur.

<400> 364
tctgctcccg tgaatcccgc tcgttcgttc tcccggctat aacattccta acttctcata 60

<210> 365
<211> 60
<212> DNA
<213> Artificial Sequence

<220>
<223> Klammerstrang_0364 zugehoerig zu 3D-DNA-Nanostruktur.

<400> 365
atgggaatgt agaagcgctg caaatcgtcg cccgtgccgt aacattccta acttctcata 60

<210> 366
<211> 60
<212> DNA
<213> Artificial Sequence

<220>
<223> Klammerstrang_0365 zugehoerig zu 3D-DNA-Nanostruktur.

<400> 366
gtctgctcgt agttagtcgc ctcgacgcac gacgggacct aacattccta acttctcata 60

<210> 367
<211> 60
<212> DNA
<213> Artificial Sequence

<220>
<223> Klammerstrang_0366 zugehoerig zu 3D-DNA-Nanostruktur.

<400> 367
ggcccaacta gagaagcaga aggcatcccg gtcggccact aacattccta acttctcata 60

<210> 368
<211> 60
<212> DNA

<213> Artificial Sequence

<220>
<223> Klammerstrang_0367 zugehoerig zu 3D-DNA-Nanostruktur.

<400> 368
agttcgcacc gccattctcg gcagactccc accgacatgt aacattccta acttctcata 60

<210> 369
<211> 60
<212> DNA
<213> Artificial Sequence

<220>
<223> Klammerstrang_0368 zugehoerig zu 3D-DNA-Nanostruktur.

<400> 369
gaaaagcacg gcgtgcagtg gcccaggtag gtcgttccat aacattccta acttctcata 60

<210> 370
<211> 60
<212> DNA
<213> Artificial Sequence

<220>
<223> Klammerstrang_0369 zugehoerig zu 3D-DNA-Nanostruktur.

<400> 370
ggtaatgcgt tcgggctact gcggtgtgag atcgtgcggt aacattccta acttctcata 60

<210> 371
<211> 60
<212> DNA
<213> Artificial Sequence

<220>
<223> Klammerstrang_0370 zugehoerig zu 3D-DNA-Nanostruktur.

<400> 371
tctacgtagg catcagcgtc gcggcactac acgtgggtct aacattccta acttctcata 60

<210> 372
<211> 60
<212> DNA
<213> Artificial Sequence

<220>
<223> Klammerstrang_0371 zugehoerig zu 3D-DNA-Nanostruktur.

<400> 372
cgaagggacg cgggaggcta gggaatgctg catcagtggt aacattccta acttctcata 60

<210> 373

<211> 60
<212> DNA
<213> Artificial Sequence

<220>
<223> Klammerstrang_0372 zugehoerig zu 3D-DNA-Nanostruktur.

<400> 373
actccctgat aggcgtcttt caggcgggcg ggactcaggt taccatctct cctaaactcg 60

<210> 374
<211> 60
<212> DNA
<213> Artificial Sequence

<220>
<223> Klammerstrang_0373 zugehoerig zu 3D-DNA-Nanostruktur.

<400> 374
gggacgccag actgggctca cgagtgatag gctttccgct taccatctct cctaaactcg 60

<210> 375
<211> 60
<212> DNA
<213> Artificial Sequence

<220>
<223> Klammerstrang_0374 zugehoerig zu 3D-DNA-Nanostruktur.

<400> 375
ttcggcttag gtccttctcg tgtggtgccc gctgcccttt taccatctct cctaaactcg 60

<210> 376
<211> 60
<212> DNA
<213> Artificial Sequence

<220>
<223> Klammerstrang_0375 zugehoerig zu 3D-DNA-Nanostruktur.

<400> 376
gcgagccacg cggcgtgtaa ccaacagcat tttctcgctt taccatctct cctaaactcg 60

<210> 377
<211> 60
<212> DNA
<213> Artificial Sequence

<220>
<223> Klammerstrang_0376 zugehoerig zu 3D-DNA-Nanostruktur.

<400> 377
tgcggcagca tcaaggacct accatcttga ccggtcgcct taccatctct cctaaactcg 60

<210> 378
<211> 60
<212> DNA
<213> Artificial Sequence

<220>
<223> Klammerstrang_0377 zugehoerig zu 3D-DNA-Nanostruktur.

<400> 378
actccagttg ctagaacggt ctcgcgcaca cacggtgggt taccatctct cctaaactcg 60

<210> 379
<211> 60
<212> DNA
<213> Artificial Sequence

<220>
<223> Klammerstrang_0378 zugehoerig zu 3D-DNA-Nanostruktur.

<400> 379
cgtgctttga tccgactgac actgcgtggc cgatgggctt taccatctct cctaaactcg 60

<210> 380
<211> 60
<212> DNA
<213> Artificial Sequence

<220>
<223> Klammerstrang_0379 zugehoerig zu 3D-DNA-Nanostruktur.

<400> 380
gtgcccgttg tgagtcggag aaccttgcgc ggcctattgt taccatctct cctaaactcg 60

<210> 381
<211> 60
<212> DNA
<213> Artificial Sequence

<220>
<223> Klammerstrang_0380 zugehoerig zu 3D-DNA-Nanostruktur.

<400> 381
cgcagggacg ccgtcgaaac acagcactcc acatcgtact taccatctct cctaaactcg 60

<210> 382
<211> 60
<212> DNA
<213> Artificial Sequence

<220>
<223> Klammerstrang_0381 zugehoerig zu 3D-DNA-Nanostruktur.

<400> 382

cacgactctc acacggagcc agcctatctc ccgttgcggt taccatctct cctaaactcg 60

<210> 383
<211> 60
<212> DNA
<213> Artificial Sequence

<220>
<223> Klammerstrang_0382 zugehoerig zu 3D-DNA-Nanostruktur.

<400> 383
aacagccttg gaccggtcgc tcacagtcct ttcgtgccct taccatctct cctaaactcg 60

<210> 384
<211> 60
<212> DNA
<213> Artificial Sequence

<220>
<223> Klammerstrang_0383 zugehoerig zu 3D-DNA-Nanostruktur.

<400> 384
ctcgtcgttt ccgttcttgc agtgctgcca gaggcggtgt taccatctct cctaaactcg 60

<210> 385
<211> 60
<212> DNA
<213> Artificial Sequence

<220>
<223> Klammerstrang_0384 zugehoerig zu 3D-DNA-Nanostruktur.

<400> 385
agaaggaaag tctcaccggg cgccctaggc cttccgaggt taccatctct cctaaactcg 60

<210> 386
<211> 60
<212> DNA
<213> Artificial Sequence

<220>
<223> Klammerstrang_0385 zugehoerig zu 3D-DNA-Nanostruktur.

<400> 386
gcctcaatgc tcttcacctg ggtccatgcc cggacggagt taccatctct cctaaactcg 60

<210> 387
<211> 60
<212> DNA
<213> Artificial Sequence

<220>
<223> Klammerstrang_0386 zugehoerig zu 3D-DNA-Nanostruktur.

<400> 387
agcagctgac aacctagacg tgtgcctcgg gtagtgcgct taccatctct cctaaactcg 60

<210> 388
<211> 60
<212> DNA
<213> Artificial Sequence

<220>
<223> Klammerstrang_0387 zugehoerig zu 3D-DNA-Nanostruktur.

<400> 388
gaacagagcg cccgtttcgc cgaacgcggt ggataaacat taccatctct cctaaactcg 60

<210> 389
<211> 60
<212> DNA
<213> Artificial Sequence

<220>
<223> Klammerstrang_0388 zugehoerig zu 3D-DNA-Nanostruktur.

<400> 389
acttccggcc cgccgcactc atgcctgtac gggtttagtt taccatctct cctaaactcg 60

<210> 390
<211> 60
<212> DNA
<213> Artificial Sequence

<220>
<223> Klammerstrang_0389 zugehoerig zu 3D-DNA-Nanostruktur.

<400> 390
catttacgtc gccagccgcc ggtatccatc tgcgagcact taccatctct cctaaactcg 60

<210> 391
<211> 60
<212> DNA
<213> Artificial Sequence

<220>
<223> Klammerstrang_0390 zugehoerig zu 3D-DNA-Nanostruktur.

<400> 391
agcagcggca tctccagttt tgggccaggg ttgggatcct taccatctct cctaaactcg 60

<210> 392
<211> 60
<212> DNA
<213> Artificial Sequence

<220>
<223> Klammerstrang_0391 zugehoerig zu 3D-DNA-Nanostruktur.

<400> 392
gcatatgcga atcgactcca acgcgcgaaa ggacgggctt taccatctct cctaaactcg 60

<210> 393
<211> 60
<212> DNA
<213> Artificial Sequence

<220>
<223> Klammerstrang_0392 zugehoerig zu 3D-DNA-Nanostruktur.

<400> 393
ggcactagcc tcggccacaa actgtaggcg cgctatcgat taccatctct cctaaactcg 60

<210> 394
<211> 60
<212> DNA
<213> Artificial Sequence

<220>
<223> Klammerstrang_0393 zugehoerig zu 3D-DNA-Nanostruktur.

<400> 394
ctgctcgcgc gttgaaccta gcctcgagtg gcattgacgt taccatctct cctaaactcg 60

<210> 395
<211> 60
<212> DNA
<213> Artificial Sequence

<220>
<223> Klammerstrang_0394 zugehoerig zu 3D-DNA-Nanostruktur.

<400> 395
tcaattccgc gcggagagag aacaacaacc acgcaccggt taccatctct cctaaactcg 60

<210> 396
<211> 60
<212> DNA
<213> Artificial Sequence

<220>
<223> Klammerstrang_0395 zugehoerig zu 3D-DNA-Nanostruktur.

<400> 396
tgaccaccgt cgaaccatcc ggcagtcggg ccataagctt taccatctct cctaaactcg 60

<210> 397
<211> 60
<212> DNA
<213> Artificial Sequence

<220>
<223> Klammerstrang_0396 zugehoerig zu 3D-DNA-Nanostruktur.

<400> 397
cctcaacctc gctcgaacgt acgcagccgc aacactggat taccatctct cctaaactcg 60

<210> 398
<211> 60
<212> DNA
<213> Artificial Sequence

<220>
<223> Klammerstrang_0397 zugehoerig zu 3D-DNA-Nanostruktur.

<400> 398
gaagcggcat agctccgtcc tcgggtagta aagcgcgcat taccatctct cctaaactcg 60

<210> 399
<211> 60
<212> DNA
<213> Artificial Sequence

<220>
<223> Klammerstrang_0398 zugehoerig zu 3D-DNA-Nanostruktur.

<400> 399
gggaacagcg cataattcgc cgtgtgtgtg gccatcgcat taccatctct cctaaactcg 60

<210> 400
<211> 60
<212> DNA
<213> Artificial Sequence

<220>
<223> Klammerstrang_0399 zugehoerig zu 3D-DNA-Nanostruktur.

<400> 400
cgacgccaca acgaacggcc tcctcgaaac cttgtgaggt taccatctct cctaaactcg 60

<210> 401
<211> 60
<212> DNA
<213> Artificial Sequence

<220>
<223> Klammerstrang_0400 zugehoerig zu 3D-DNA-Nanostruktur.

<400> 401
gggatgtaac accacgtggc gcacgcactg tgtaggagct taccatctct cctaaactcg 60

<210> 402
<211> 60

<212> DNA
<213> Artificial Sequence

<220>
<223> Klammerstrang_0401 zugehoerig zu 3D-DNA-Nanostruktur.

<400> 402
ctgggaggta cagctagggt gaccggcctc ctgccctcat taccatctct cctaaactcg 60

<210> 403
<211> 60
<212> DNA
<213> Artificial Sequence

<220>
<223> Klammerstrang_0402 zugehoerig zu 3D-DNA-Nanostruktur.

<400> 403
gcccttgcca cagacccgca cagcgagttc tggcttcatt taccatctct cctaaactcg 60

<210> 404
<211> 60
<212> DNA
<213> Artificial Sequence

<220>
<223> Klammerstrang_0403 zugehoerig zu 3D-DNA-Nanostruktur.

<400> 404
gcgcaacgtg acggtacgcc aacggtcacc agaagtcagt taccatctct cctaaactcg 60

<210> 405
<211> 60
<212> DNA
<213> Artificial Sequence

<220>
<223> Klammerstrang_0404 zugehoerig zu 3D-DNA-Nanostruktur.

<400> 405
cggccaacct caaaggcgtc cacggcgaat ctgcgtctat taccatctct cctaaactcg 60

<210> 406
<211> 60
<212> DNA
<213> Artificial Sequence

<220>
<223> Klammerstrang_0405 zugehoerig zu 3D-DNA-Nanostruktur.

<400> 406
cgcgcatagg gagtgtgctg gcgggctgcg agtaagaatt taccatctct cctaaactcg 60

<210> 407
<211> 60
<212> DNA
<213> Artificial Sequence

<220>
<223> Klammerstrang_0406 zugehoerig zu 3D-DNA-Nanostruktur.

<400> 407
ctcaggaagc gagcagtaaa tccttggccg accgcagcgt taccatctct cctaaactcg 60

<210> 408
<211> 60
<212> DNA
<213> Artificial Sequence

<220>
<223> Klammerstrang_0407 zugehoerig zu 3D-DNA-Nanostruktur.

<400> 408
gagcagagca gcttatccct gggtctccgg catctgcgct taccatctct cctaaactcg 60

<210> 409
<211> 60
<212> DNA
<213> Artificial Sequence

<220>
<223> Klammerstrang_0408 zugehoerig zu 3D-DNA-Nanostruktur.

<400> 409
tggtgttccc tttcgccgcc ttgcaagtcc catcatgcct taccatctct cctaaactcg 60

<210> 410
<211> 60
<212> DNA
<213> Artificial Sequence

<220>
<223> Klammerstrang_0409 zugehoerig zu 3D-DNA-Nanostruktur.

<400> 410
gcaagaagcc tacgcgaagc tgtcgggacc acggcaatgt taccatctct cctaaactcg 60

<210> 411
<211> 60
<212> DNA
<213> Artificial Sequence

<220>
<223> Klammerstrang_0410 zugehoerig zu 3D-DNA-Nanostruktur.

<400> 411
ctccgtgctt gttaccttcg agtcgcggca caccacacgt taccatctct cctaaactcg 60

<210> 412
<211> 60
<212> DNA
<213> Artificial Sequence

<220>
<223> Klammerstrang_0411 zugehoerig zu 3D-DNA-Nanostruktur.

<400> 412
tctgctcccg tgaatcccgc tcgttcgttc tcccggctat taccatctct cctaaactcg 60

<210> 413
<211> 60
<212> DNA
<213> Artificial Sequence

<220>
<223> Klammerstrang_0412 zugehoerig zu 3D-DNA-Nanostruktur.

<400> 413
atgggaatgt agaagcgctg caaatcgtcg cccgtgccgt taccatctct cctaaactcg 60

<210> 414
<211> 60
<212> DNA
<213> Artificial Sequence

<220>
<223> Klammerstrang_0413 zugehoerig zu 3D-DNA-Nanostruktur.

<400> 414
gtctgctcgt agttagtcgc ctcgacgcac gacgggacct taccatctct cctaaactcg 60

<210> 415
<211> 60
<212> DNA
<213> Artificial Sequence

<220>
<223> Klammerstrang_0414 zugehoerig zu 3D-DNA-Nanostruktur.

<400> 415
ggcccaacta gagaagcaga aggcatcccg gtcggccact taccatctct cctaaactcg 60

<210> 416
<211> 60
<212> DNA
<213> Artificial Sequence

<220>
<223> Klammerstrang_0415 zugehoerig zu 3D-DNA-Nanostruktur.

<400> 416
agttcgcacc gccattctcg gcagactccc accgacatgt taccatctct cctaaactcg 60

<210> 417
<211> 60
<212> DNA
<213> Artificial Sequence

<220>
<223> Klammerstrang_0416 zugehoerig zu 3D-DNA-Nanostruktur.

<400> 417
gaaaagcacg gcgtgcagtg gcccaggtag gtcgttccat taccatctct cctaaactcg 60

<210> 418
<211> 60
<212> DNA
<213> Artificial Sequence

<220>
<223> Klammerstrang_0417 zugehoerig zu 3D-DNA-Nanostruktur.

<400> 418
ggtaatgcgt tcgggctact gcggtgtgag atcgtgcggt taccatctct cctaaactcg 60

<210> 419
<211> 60
<212> DNA
<213> Artificial Sequence

<220>
<223> Klammerstrang_0418 zugehoerig zu 3D-DNA-Nanostruktur.

<400> 419
tctacgtagg catcagcgtc gcggcactac acgtgggtct taccatctct cctaaactcg 60

<210> 420
<211> 60
<212> DNA
<213> Artificial Sequence

<220>
<223> Klammerstrang_0419 zugehoerig zu 3D-DNA-Nanostruktur.

<400> 420
cgaagggacg cgggaggcta gggaatgctg catcagtggt taccatctct cctaaactcg 60

<210> 421
<211> 39
<212> DNA
<213> Artificial Sequence

<220>

<223> Klammerstrang_0420 zugehoerig zu 3D-DNA-Nanostruktur.

<400> 421
actccctgat aggcgtcttt caggcgggcg ggactcagg 39

<210> 422
<211> 39
<212> DNA
<213> Artificial Sequence

<220>
<223> Klammerstrang_0421 zugehoerig zu 3D-DNA-Nanostruktur.

<400> 422
gggacgccag actgggctca cgagtgatag gctttccgc 39

<210> 423
<211> 39
<212> DNA
<213> Artificial Sequence

<220>
<223> Klammerstrang_0422 zugehoerig zu 3D-DNA-Nanostruktur.

<400> 423
ttcggcttag gtccttctcg tgtggtgccc gctgccctt 39

<210> 424
<211> 39
<212> DNA
<213> Artificial Sequence

<220>
<223> Klammerstrang_0423 zugehoerig zu 3D-DNA-Nanostruktur.

<400> 424
gcgagccacg cggcgtgtaa ccaacagcat tttctcgct 39

<210> 425
<211> 39
<212> DNA
<213> Artificial Sequence

<220>
<223> Klammerstrang_0424 zugehoerig zu 3D-DNA-Nanostruktur.

<400> 425
tgcggcagca tcaaggacct accatcttga ccggtcgcc 39

<210> 426
<211> 39
<212> DNA
<213> Artificial Sequence

<220>
<223> Klammerstrang_0425 zugehoerig zu 3D-DNA-Nanostruktur.

<400> 426
actccagttg ctagaacggt ctcgcgcaca cacggtggg 39

<210> 427
<211> 39
<212> DNA
<213> Artificial Sequence

<220>
<223> Klammerstrang_0426 zugehoerig zu 3D-DNA-Nanostruktur.

<400> 427
cgtgctttga tccgactgac actgcgtggc cgatgggct 39

<210> 428
<211> 39
<212> DNA
<213> Artificial Sequence

<220>
<223> Klammerstrang_0427 zugehoerig zu 3D-DNA-Nanostruktur.

<400> 428
gtgcccgttg tgagtcggag aaccttgcgc ggcctattg 39

<210> 429
<211> 39
<212> DNA
<213> Artificial Sequence

<220>
<223> Klammerstrang_0428 zugehoerig zu 3D-DNA-Nanostruktur.

<400> 429
cgcagggacg ccgtcgaaac acagcactcc acatcgtac 39

<210> 430
<211> 39
<212> DNA
<213> Artificial Sequence

<220>
<223> Klammerstrang_0429 zugehoerig zu 3D-DNA-Nanostruktur.

<400> 430
cacgactctc acacggagcc agcctatctc ccgttgcgg 39

<210> 431
<211> 39
<212> DNA

<213> Artificial Sequence

<220>
<223> Klammerstrang_0430 zugehoerig zu 3D-DNA-Nanostruktur.

<400> 431
aacagccttg gaccggtcgc tcacagtcct ttcgtgccc 39

<210> 432
<211> 39
<212> DNA
<213> Artificial Sequence

<220>
<223> Klammerstrang_0431 zugehoerig zu 3D-DNA-Nanostruktur.

<400> 432
ctcgtcgttt ccgttcttgc agtgctgcca gaggcggtg 39

<210> 433
<211> 39
<212> DNA
<213> Artificial Sequence

<220>
<223> Klammerstrang_0432 zugehoerig zu 3D-DNA-Nanostruktur.

<400> 433
agaaggaaag tctcaccggg cgccctaggc cttccgagg 39

<210> 434
<211> 39
<212> DNA
<213> Artificial Sequence

<220>
<223> Klammerstrang_0433 zugehoerig zu 3D-DNA-Nanostruktur.

<400> 434
gcctcaatgc tcttcacctg ggtccatgcc cggacggag 39

<210> 435
<211> 39
<212> DNA
<213> Artificial Sequence

<220>
<223> Klammerstrang_0434 zugehoerig zu 3D-DNA-Nanostruktur.

<400> 435
agcagctgac aacctagacg tgtgcctcgg gtagtgcgc 39

<210> 436

<211> 39
<212> DNA
<213> Artificial Sequence

<220>
<223> Klammerstrang_0435 zugehoerig zu 3D-DNA-Nanostruktur.

<400> 436
gaacagagcg cccgtttcgc cgaacgcggt ggataaaca 39

<210> 437
<211> 39
<212> DNA
<213> Artificial Sequence

<220>
<223> Klammerstrang_0436 zugehoerig zu 3D-DNA-Nanostruktur.

<400> 437
acttccggcc cgccgcactc atgcctgtac gggtttagt 39

<210> 438
<211> 39
<212> DNA
<213> Artificial Sequence

<220>
<223> Klammerstrang_0437 zugehoerig zu 3D-DNA-Nanostruktur.

<400> 438
catttacgtc gccagccgcc ggtatccatc tgcgagcac 39

<210> 439
<211> 39
<212> DNA
<213> Artificial Sequence

<220>
<223> Klammerstrang_0438 zugehoerig zu 3D-DNA-Nanostruktur.

<400> 439
agcagcggca tctccagttt tgggccaggg ttgggatcc 39

<210> 440
<211> 39
<212> DNA
<213> Artificial Sequence

<220>
<223> Klammerstrang_0439 zugehoerig zu 3D-DNA-Nanostruktur.

<400> 440
gcatatgcga atcgactcca acgcgcgaaa ggacgggct 39

<210> 441
<211> 39
<212> DNA
<213> Artificial Sequence

<220>
<223> Klammerstrang_0440 zugehoerig zu 3D-DNA-Nanostruktur.

<400> 441
ggcactagcc tcggccacaa actgtaggcg cgctatcga 39

<210> 442
<211> 39
<212> DNA
<213> Artificial Sequence

<220>
<223> Klammerstrang_0441 zugehoerig zu 3D-DNA-Nanostruktur.

<400> 442
ctgctcgcgc gttgaaccta gcctcgagtg gcattgacg 39

<210> 443
<211> 39
<212> DNA
<213> Artificial Sequence

<220>
<223> Klammerstrang_0442 zugehoerig zu 3D-DNA-Nanostruktur.

<400> 443
tcaattccgc gcggagagag aacaacaacc acgcaccgg 39

<210> 444
<211> 39
<212> DNA
<213> Artificial Sequence

<220>
<223> Klammerstrang_0443 zugehoerig zu 3D-DNA-Nanostruktur.

<400> 444
tgaccaccgt cgaaccatcc ggcagtcggg ccataagct 39

<210> 445
<211> 39
<212> DNA
<213> Artificial Sequence

<220>
<223> Klammerstrang_0444 zugehoerig zu 3D-DNA-Nanostruktur.

<400> 445

cctcaacctc gctcgaacgt acgcagccgc aacactgga 39

<210> 446
<211> 39
<212> DNA
<213> Artificial Sequence

<220>
<223> Klammerstrang_0445 zugehoerig zu 3D-DNA-Nanostruktur.

<400> 446
gaagcggcat agctccgtcc tcgggtagta aagcgcgca 39

<210> 447
<211> 39
<212> DNA
<213> Artificial Sequence

<220>
<223> Klammerstrang_0446 zugehoerig zu 3D-DNA-Nanostruktur.

<400> 447
gggaacagcg cataattcgc cgtgtgtgtg gccatcgca 39

<210> 448
<211> 39
<212> DNA
<213> Artificial Sequence

<220>
<223> Klammerstrang_0447 zugehoerig zu 3D-DNA-Nanostruktur.

<400> 448
cgacgccaca acgaacggcc tcctcgaaac cttgtgagg 39

<210> 449
<211> 39
<212> DNA
<213> Artificial Sequence

<220>
<223> Klammerstrang_0448 zugehoerig zu 3D-DNA-Nanostruktur.

<400> 449
gggatgtaac accacgtggc gcacgcactg tgtaggagc 39

<210> 450
<211> 39
<212> DNA
<213> Artificial Sequence

<220>
<223> Klammerstrang_0449 zugehoerig zu 3D-DNA-Nanostruktur.

<400> 450
ctgggaggta cagctagggt gaccggcctc ctgccctca 39

<210> 451
<211> 39
<212> DNA
<213> Artificial Sequence

<220>
<223> Klammerstrang_0450 zugehoerig zu 3D-DNA-Nanostruktur.

<400> 451
gcccttgcca cagacccgca cagcgagttc tggcttcat 39

<210> 452
<211> 39
<212> DNA
<213> Artificial Sequence

<220>
<223> Klammerstrang_0451 zugehoerig zu 3D-DNA-Nanostruktur.

<400> 452
gcgcaacgtg acggtacgcc aacggtcacc agaagtcag 39

<210> 453
<211> 39
<212> DNA
<213> Artificial Sequence

<220>
<223> Klammerstrang_0452 zugehoerig zu 3D-DNA-Nanostruktur.

<400> 453
cggccaacct caaaggcgtc cacggcgaat ctgcgtcta 39

<210> 454
<211> 39
<212> DNA
<213> Artificial Sequence

<220>
<223> Klammerstrang_0453 zugehoerig zu 3D-DNA-Nanostruktur.

<400> 454
cgcgcatagg gagtgtgctg gcgggctgcg agtaagaat 39

<210> 455
<211> 39
<212> DNA
<213> Artificial Sequence

<220>
<223> Klammerstrang_0454 zugehoerig zu 3D-DNA-Nanostruktur.

<400> 455
ctcaggaagc gagcagtaaa tccttggccg accgcagcg 39

<210> 456
<211> 39
<212> DNA
<213> Artificial Sequence

<220>
<223> Klammerstrang_0455 zugehoerig zu 3D-DNA-Nanostruktur.

<400> 456
gagcagagca gcttatccct gggtctccgg catctgcgc 39

<210> 457
<211> 39
<212> DNA
<213> Artificial Sequence

<220>
<223> Klammerstrang_0456 zugehoerig zu 3D-DNA-Nanostruktur.

<400> 457
tggtgttccc tttcgccgcc ttgcaagtcc catcatgcc 39

<210> 458
<211> 39
<212> DNA
<213> Artificial Sequence

<220>
<223> Klammerstrang_0457 zugehoerig zu 3D-DNA-Nanostruktur.

<400> 458
gcaagaagcc tacgcgaagc tgtcgggacc acggcaatg 39

<210> 459
<211> 39
<212> DNA
<213> Artificial Sequence

<220>
<223> Klammerstrang_0458 zugehoerig zu 3D-DNA-Nanostruktur.

<400> 459
ctccgtgctt gttaccttcg agtcgcggca caccacacg 39

<210> 460
<211> 39
<212> DNA
<213> Artificial Sequence

<220>
<223> Klammerstrang_0459 zugehoerig zu 3D-DNA-Nanostruktur.

<400> 460
tctgctcccg tgaatcccgc tcgttcgttc tcccggcta 39

<210> 461
<211> 39
<212> DNA
<213> Artificial Sequence

<220>
<223> Klammerstrang_0460 zugehoerig zu 3D-DNA-Nanostruktur.

<400> 461
atgggaatgt agaagcgctg caaatcgtcg cccgtgccg 39

<210> 462
<211> 39
<212> DNA
<213> Artificial Sequence

<220>
<223> Klammerstrang_0461 zugehoerig zu 3D-DNA-Nanostruktur.

<400> 462
gtctgctcgt agttagtcgc ctcgacgcac gacgggacc 39

<210> 463
<211> 39
<212> DNA
<213> Artificial Sequence

<220>
<223> Klammerstrang_0462 zugehoerig zu 3D-DNA-Nanostruktur.

<400> 463
ggcccaacta gagaagcaga aggcatcccg gtcggccac 39

<210> 464
<211> 39
<212> DNA
<213> Artificial Sequence

<220>
<223> Klammerstrang_0463 zugehoerig zu 3D-DNA-Nanostruktur.

<400> 464
agttcgcacc gccattctcg gcagactccc accgacatg 39

<210> 465
<211> 39

<212> DNA
<213> Artificial Sequence

<220>
<223> Klammerstrang_0464 zugehoerig zu 3D-DNA-Nanostruktur.

<400> 465
gaaaagcacg gcgtgcagtg gcccaggtag gtcgttcca 39

<210> 466
<211> 39
<212> DNA
<213> Artificial Sequence

<220>
<223> Klammerstrang_0465 zugehoerig zu 3D-DNA-Nanostruktur.

<400> 466
ggtaatgcgt tcgggctact gcggtgtgag atcgtgcgg 39

<210> 467
<211> 39
<212> DNA
<213> Artificial Sequence

<220>
<223> Klammerstrang_0466 zugehoerig zu 3D-DNA-Nanostruktur.

<400> 467
tctacgtagg catcagcgtc gcggcactac acgtgggtc 39

<210> 468
<211> 39
<212> DNA
<213> Artificial Sequence

<220>
<223> Klammerstrang_0467 zugehoerig zu 3D-DNA-Nanostruktur.

<400> 468
cgaagggacg cgggaggcta gggaatgctg catcagtgg 39

<210> 469
<211> 60
<212> DNA
<213> Artificial Sequence

<220>
<223> Klammerstrang_0468 zugehoerig zu 3D-DNA-Nanostruktur.

<400> 469
cctacagggt tagctccaca gccgggtccg tgcagatgct tcctctacca cctacatcac 60

<210> 470
<211> 60
<212> DNA
<213> Artificial Sequence

<220>
<223> Klammerstrang_0469 zugehoerig zu 3D-DNA-Nanostruktur.

<400> 470
agaggcctcc tcagcggtcc tacgcggtct tggacgatgt tcctctacca cctacatcac 60

<210> 471
<211> 60
<212> DNA
<213> Artificial Sequence

<220>
<223> Klammerstrang_0470 zugehoerig zu 3D-DNA-Nanostruktur.

<400> 471
cgtactcatc ccgtgccagt gccggcctac tcacctgtgt tcctctacca cctacatcac 60

<210> 472
<211> 60
<212> DNA
<213> Artificial Sequence

<220>
<223> Klammerstrang_0471 zugehoerig zu 3D-DNA-Nanostruktur.

<400> 472
gcctgcgaca atgtgggctg gaacggtttg ctagggcact tcctctacca cctacatcac 60

<210> 473
<211> 60
<212> DNA
<213> Artificial Sequence

<220>
<223> Klammerstrang_0472 zugehoerig zu 3D-DNA-Nanostruktur.

<400> 473
cacccgatgt gttatgacgt gcgtcgccct cgagcacctt tcctctacca cctacatcac 60

<210> 474
<211> 60
<212> DNA
<213> Artificial Sequence

<220>
<223> Klammerstrang_0473 zugehoerig zu 3D-DNA-Nanostruktur.

<400> 474
agcatacaga gggcagcttg gcagggccgc gaagttaggt tcctctacca cctacatcac 60

<210> 475
<211> 60
<212> DNA
<213> Artificial Sequence

<220>
<223> Klammerstrang_0474 zugehoerig zu 3D-DNA-Nanostruktur.

<400> 475
gacgcccgat tgtccttgtt cgccatgccg caaggtgagt tcctctacca cctacatcac 60

<210> 476
<211> 60
<212> DNA
<213> Artificial Sequence

<220>
<223> Klammerstrang_0475 zugehoerig zu 3D-DNA-Nanostruktur.

<400> 476
ggtggcgaga tgacgacagt gcagaaggtg gcggtagggt tcctctacca cctacatcac 60

<210> 477
<211> 60
<212> DNA
<213> Artificial Sequence

<220>
<223> Klammerstrang_0476 zugehoerig zu 3D-DNA-Nanostruktur.

<400> 477
tctgtgaagc gggtggagta cggaggtgtt cttcgcgcct tcctctacca cctacatcac 60

<210> 478
<211> 60
<212> DNA
<213> Artificial Sequence

<220>
<223> Klammerstrang_0477 zugehoerig zu 3D-DNA-Nanostruktur.

<400> 478
ttttgcgcaa tcatggcagc ggactcaaca ccctccggct tcctctacca cctacatcac 60

<210> 479
<211> 60
<212> DNA
<213> Artificial Sequence

<220>
<223> Klammerstrang_0478 zugehoerig zu 3D-DNA-Nanostruktur.

<400> 479
gacctcggag ctagacggga gtgttgcgtg tgcagggtgt tcctctacca cctacatcac 60

<210> 480
<211> 60
<212> DNA
<213> Artificial Sequence

<220>
<223> Klammerstrang_0479 zugehoerig zu 3D-DNA-Nanostruktur.

<400> 480
cggttcgagt gcgcagtgct taacgtgaag cgagggaggt tcctctacca cctacatcac 60

<210> 481
<211> 60
<212> DNA
<213> Artificial Sequence

<220>
<223> Klammerstrang_0480 zugehoerig zu 3D-DNA-Nanostruktur.

<400> 481
cctacagggt tagctccaca gccgggtccg tgcagatgct aacattccta acttctcata 60

<210> 482
<211> 60
<212> DNA
<213> Artificial Sequence

<220>
<223> Klammerstrang_0481 zugehoerig zu 3D-DNA-Nanostruktur.

<400> 482
agaggcctcc tcagcggtcc tacgcggtct tggacgatgt aacattccta acttctcata 60

<210> 483
<211> 60
<212> DNA
<213> Artificial Sequence

<220>
<223> Klammerstrang_0482 zugehoerig zu 3D-DNA-Nanostruktur.

<400> 483
cgtactcatc ccgtgccagt gccggcctac tcacctgtgt aacattccta acttctcata 60

<210> 484
<211> 60
<212> DNA
<213> Artificial Sequence

<220>

<223> Klammerstrang_0483 zugehoerig zu 3D-DNA-Nanostruktur.

<400> 484
gcctgcgaca atgtgggctg gaacggtttg ctagggcact aacattccta acttctcata 60

<210> 485
<211> 60
<212> DNA
<213> Artificial Sequence

<220>
<223> Klammerstrang_0484 zugehoerig zu 3D-DNA-Nanostruktur.

<400> 485
cacccgatgt gttatgacgt gcgtcgccct cgagcacctt aacattccta acttctcata 60

<210> 486
<211> 60
<212> DNA
<213> Artificial Sequence

<220>
<223> Klammerstrang_0485 zugehoerig zu 3D-DNA-Nanostruktur.

<400> 486
agcatacaga gggcagcttg gcagggccgc gaagttaggt aacattccta acttctcata 60

<210> 487
<211> 60
<212> DNA
<213> Artificial Sequence

<220>
<223> Klammerstrang_0486 zugehoerig zu 3D-DNA-Nanostruktur.

<400> 487
gacgcccgat tgtccttgtt cgccatgccg caaggtgagt aacattccta acttctcata 60

<210> 488
<211> 60
<212> DNA
<213> Artificial Sequence

<220>
<223> Klammerstrang_0487 zugehoerig zu 3D-DNA-Nanostruktur.

<400> 488
ggtggcgaga tgacgacagt gcagaaggtg gcggtagggt aacattccta acttctcata 60

<210> 489
<211> 60
<212> DNA
<213> Artificial Sequence

<220>
<223> Klammerstrang_0488 zugehoerig zu 3D-DNA-Nanostruktur.

<400> 489
tctgtgaagc gggtggagta cggaggtgtt cttcgcgcct aacattccta acttctcata 60

<210> 490
<211> 60
<212> DNA
<213> Artificial Sequence

<220>
<223> Klammerstrang_0489 zugehoerig zu 3D-DNA-Nanostruktur.

<400> 490
ttttgcgcaa tcatggcagc ggactcaaca ccctccggct aacattccta acttctcata 60

<210> 491
<211> 60
<212> DNA
<213> Artificial Sequence

<220>
<223> Klammerstrang_0490 zugehoerig zu 3D-DNA-Nanostruktur.

<400> 491
gacctcggag ctagacggga gtgttgcgtg tgcagggtgt aacattccta acttctcata 60

<210> 492
<211> 60
<212> DNA
<213> Artificial Sequence

<220>
<223> Klammerstrang_0491 zugehoerig zu 3D-DNA-Nanostruktur.

<400> 492
cggttcgagt gcgcagtgct taacgtgaag cgagggaggt aacattccta acttctcata 60

<210> 493
<211> 60
<212> DNA
<213> Artificial Sequence

<220>
<223> Klammerstrang_0492 zugehoerig zu 3D-DNA-Nanostruktur.

<400> 493
cctacagggt tagctccaca gccgggtccg tgcagatgct taccatctct cctaaactcg 60

<210> 494
<211> 60
<212> DNA

<213> Artificial Sequence

<220>
<223> Klammerstrang_0493 zugehoerig zu 3D-DNA-Nanostruktur.

<400> 494
agaggcctcc tcagcggtcc tacgcggtct tggacgatgt taccatctct cctaaactcg 60

<210> 495
<211> 60
<212> DNA
<213> Artificial Sequence

<220>
<223> Klammerstrang_0494 zugehoerig zu 3D-DNA-Nanostruktur.

<400> 495
cgtactcatc ccgtgccagt gccggcctac tcacctgtgt taccatctct cctaaactcg 60

<210> 496
<211> 60
<212> DNA
<213> Artificial Sequence

<220>
<223> Klammerstrang_0495 zugehoerig zu 3D-DNA-Nanostruktur.

<400> 496
gcctgcgaca atgtgggctg gaacggtttg ctagggcact taccatctct cctaaactcg 60

<210> 497
<211> 60
<212> DNA
<213> Artificial Sequence

<220>
<223> Klammerstrang_0496 zugehoerig zu 3D-DNA-Nanostruktur.

<400> 497
cacccgatgt gttatgacgt gcgtcgccct cgagcacctt taccatctct cctaaactcg 60

<210> 498
<211> 60
<212> DNA
<213> Artificial Sequence

<220>
<223> Klammerstrang_0497 zugehoerig zu 3D-DNA-Nanostruktur.

<400> 498
agcatacaga gggcagcttg gcagggccgc gaagttaggt taccatctct cctaaactcg 60

<210> 499

<211> 60
<212> DNA
<213> Artificial Sequence

<220>
<223> Klammerstrang_0498 zugehoerig zu 3D-DNA-Nanostruktur.

<400> 499
gacgcccgat tgtccttgtt cgccatgccg caaggtgagt taccatctct cctaaactcg 60

<210> 500
<211> 60
<212> DNA
<213> Artificial Sequence

<220>
<223> Klammerstrang_0499 zugehoerig zu 3D-DNA-Nanostruktur.

<400> 500
ggtggcgaga tgacgacagt gcagaaggtg gcggtagggt taccatctct cctaaactcg 60

<210> 501
<211> 60
<212> DNA
<213> Artificial Sequence

<220>
<223> Klammerstrang_0500 zugehoerig zu 3D-DNA-Nanostruktur.

<400> 501
tctgtgaagc gggtggagta cggaggtgtt cttcgcgcct taccatctct cctaaactcg 60

<210> 502
<211> 60
<212> DNA
<213> Artificial Sequence

<220>
<223> Klammerstrang_0501 zugehoerig zu 3D-DNA-Nanostruktur.

<400> 502
ttttgcgcaa tcatggcagc ggactcaaca ccctccggct taccatctct cctaaactcg 60

<210> 503
<211> 60
<212> DNA
<213> Artificial Sequence

<220>
<223> Klammerstrang_0502 zugehoerig zu 3D-DNA-Nanostruktur.

<400> 503
gacctcggag ctagacggga gtgttgcgtg tgcagggtgt taccatctct cctaaactcg 60

<210> 504
<211> 60
<212> DNA
<213> Artificial Sequence

<220>
<223> Klammerstrang_0503 zugehoerig zu 3D-DNA-Nanostruktur.

<400> 504
cggttcgagt gcgcagtgct taacgtgaag cgagggaggt taccatctct cctaaactcg 60

<210> 505
<211> 31
<212> DNA
<213> Artificial Sequence

<220>
<223> Klammerstrang_0504 zugehoerig zu 2D-DNA-Nanostruktur.

<400> 505
agaaaggaac aactaaagga attcaaaaaa a 31

<210> 506
<211> 30
<212> DNA
<213> Artificial Sequence

<220>
<223> Klammerstrang_0505 zugehoerig zu 2D-DNA-Nanostruktur.

<400> 506
aggctccaga ggctttgagg acacgggtaa 30

<210> 507
<211> 32
<212> DNA
<213> Artificial Sequence

<220>
<223> Klammerstrang_0506 zugehoerig zu 2D-DNA-Nanostruktur.

<400> 507
acggctacaa aaggagcctt taatgtgaga at 32

<210> 508
<211> 30
<212> DNA
<213> Artificial Sequence

<220>
<223> Klammerstrang_0507 zugehoerig zu 2D-DNA-Nanostruktur.

<400> 508

aatacgtttg aaagaggaca gactgacctt 30

<210> 509
<211> 32
<212> DNA
<213> Artificial Sequence

<220>
<223> Klammerstrang_0508 zugehoerig zu 2D-DNA-Nanostruktur.

<400> 509
gaccaactaa tgccactacg aagggggtag ca 32

<210> 510
<211> 30
<212> DNA
<213> Artificial Sequence

<220>
<223> Klammerstrang_0509 zugehoerig zu 2D-DNA-Nanostruktur.

<400> 510
catcaagtaa aacgaactaa cgagttgaga 30

<210> 511
<211> 32
<212> DNA
<213> Artificial Sequence

<220>
<223> Klammerstrang_0510 zugehoerig zu 2D-DNA-Nanostruktur.

<400> 511
tacgttaaag taatcttgac aagaaccgaa ct 32

<210> 512
<211> 30
<212> DNA
<213> Artificial Sequence

<220>
<223> Klammerstrang_0511 zugehoerig zu 2D-DNA-Nanostruktur.

<400> 512
tttaggacaa atgctttaaa caatcaggtc 30

<210> 513
<211> 32
<212> DNA
<213> Artificial Sequence

<220>
<223> Klammerstrang_0512 zugehoerig zu 2D-DNA-Nanostruktur.

<400> 513
atccccctat accacattca actagaaaaa tc 32

<210> 514
<211> 30
<212> DNA
<213> Artificial Sequence

<220>
<223> Klammerstrang_0513 zugehoerig zu 2D-DNA-Nanostruktur.

<400> 514
tttaccccaa catgttttaa atttccatat 30

<210> 515
<211> 32
<212> DNA
<213> Artificial Sequence

<220>
<223> Klammerstrang_0514 zugehoerig zu 2D-DNA-Nanostruktur.

<400> 515
ctgtagcttg actattatag tcagttcatt ga 32

<210> 516
<211> 30
<212> DNA
<213> Artificial Sequence

<220>
<223> Klammerstrang_0515 zugehoerig zu 2D-DNA-Nanostruktur.

<400> 516
aacagttttg taccaaaaac attttatttc 30

<210> 517
<211> 32
<212> DNA
<213> Artificial Sequence

<220>
<223> Klammerstrang_0516 zugehoerig zu 2D-DNA-Nanostruktur.

<400> 517
acaactttca acagtttcag cggatgtatc gg 32

<210> 518
<211> 32
<212> DNA
<213> Artificial Sequence

<220>
<223> Klammerstrang_0517 zugehoerig zu 2D-DNA-Nanostruktur.

<400> 518
tttatcagga cagcatcgga acgacaccaa cc 32

<210> 519
<211> 30
<212> DNA
<213> Artificial Sequence

<220>
<223> Klammerstrang_0518 zugehoerig zu 2D-DNA-Nanostruktur.

<400> 519
cagcgaaact tgctttcgag gtgttgctaa 30

<210> 520
<211> 32
<212> DNA
<213> Artificial Sequence

<220>
<223> Klammerstrang_0519 zugehoerig zu 2D-DNA-Nanostruktur.

<400> 520
taaaacgagg tcaatcataa gggaaccgga ta 32

<210> 521
<211> 30
<212> DNA
<213> Artificial Sequence

<220>
<223> Klammerstrang_0520 zugehoerig zu 2D-DNA-Nanostruktur.

<400> 521
gcgcagacaa gaggcaaaag aatccctcag 30

<210> 522
<211> 32
<212> DNA
<213> Artificial Sequence

<220>
<223> Klammerstrang_0521 zugehoerig zu 2D-DNA-Nanostruktur.

<400> 522
ttcattacgt caggacgttg ggaaatgcag at 32

<210> 523
<211> 30
<212> DNA
<213> Artificial Sequence

<220>
<223> Klammerstrang_0522 zugehoerig zu 2D-DNA-Nanostruktur.

<400> 523
ttataccacc aaatcaacgt aacgaacgag 30

<210> 524
<211> 32
<212> DNA
<213> Artificial Sequence

<220>
<223> Klammerstrang_0523 zugehoerig zu 2D-DNA-Nanostruktur.

<400> 524
acataacggg aatcgtcata aataaagcaa ag 32

<210> 525
<211> 30
<212> DNA
<213> Artificial Sequence

<220>
<223> Klammerstrang_0524 zugehoerig zu 2D-DNA-Nanostruktur.

<400> 525
aatactgccc aaaaggaatt acgtggctca 30

<210> 526
<211> 32
<212> DNA
<213> Artificial Sequence

<220>
<223> Klammerstrang_0525 zugehoerig zu 2D-DNA-Nanostruktur.

<400> 526
cggattgcag agcttaattg ctgaaacgag ta 32

<210> 527
<211> 30
<212> DNA
<213> Artificial Sequence

<220>
<223> Klammerstrang_0526 zugehoerig zu 2D-DNA-Nanostruktur.

<400> 527
gatggcttat caaaaagatt aagagcgtcc 30

<210> 528
<211> 32

<212> DNA
<213> Artificial Sequence

<220>
<223> Klammerstrang_0527 zugehoerig zu 2D-DNA-Nanostruktur.

<400> 528
gatttagtca ataaagcctc agagaaccct ca 32

<210> 529
<211> 32
<212> DNA
<213> Artificial Sequence

<220>
<223> Klammerstrang_0528 zugehoerig zu 2D-DNA-Nanostruktur.

<400> 529
taaatgaatt ttctgtatgg gattaatttc tt 32

<210> 530
<211> 32
<212> DNA
<213> Artificial Sequence

<220>
<223> Klammerstrang_0529 zugehoerig zu 2D-DNA-Nanostruktur.

<400> 530
aaacagcttt ttgcgggatc gtcaacacta aa 32

<210> 531
<211> 32
<212> DNA
<213> Artificial Sequence

<220>
<223> Klammerstrang_0530 zugehoerig zu 2D-DNA-Nanostruktur.

<400> 531
aaggccgctg ataccgatag ttgcgacgtt ag 32

<210> 532
<211> 32
<212> DNA
<213> Artificial Sequence

<220>
<223> Klammerstrang_0531 zugehoerig zu 2D-DNA-Nanostruktur.

<400> 532
acactcatcc atgttactta gccgaaagct gc 32

<210> 533
<211> 32
<212> DNA
<213> Artificial Sequence

<220>
<223> Klammerstrang_0532 zugehoerig zu 2D-DNA-Nanostruktur.

<400> 533
gacctgctct ttgaccccca gcgagggagt ta 32

<210> 534
<211> 32
<212> DNA
<213> Artificial Sequence

<220>
<223> Klammerstrang_0533 zugehoerig zu 2D-DNA-Nanostruktur.

<400> 534
tcattcagat gcgattttaa gaacaggcat ag 32

<210> 535
<211> 32
<212> DNA
<213> Artificial Sequence

<220>
<223> Klammerstrang_0534 zugehoerig zu 2D-DNA-Nanostruktur.

<400> 535
attacctttg aataaggctt gcccaaatcc gc 32

<210> 536
<211> 32
<212> DNA
<213> Artificial Sequence

<220>
<223> Klammerstrang_0535 zugehoerig zu 2D-DNA-Nanostruktur.

<400> 536
taagagcaaa tgtttagact ggataggaag cc 32

<210> 537
<211> 32
<212> DNA
<213> Artificial Sequence

<220>
<223> Klammerstrang_0536 zugehoerig zu 2D-DNA-Nanostruktur.

<400> 537
aatagtaaac actatcataa ccctcattgt ga 32

<210> 538
<211> 32
<212> DNA
<213> Artificial Sequence

<220>
<223> Klammerstrang_0537 zugehoerig zu 2D-DNA-Nanostruktur.

<400> 538
cgaaagactt tgataagagg tcatatttcg ca 32

<210> 539
<211> 32
<212> DNA
<213> Artificial Sequence

<220>
<223> Klammerstrang_0538 zugehoerig zu 2D-DNA-Nanostruktur.

<400> 539
ttgctccttt caaatatcgc gtttgagggg gt 32

<210> 540
<211> 32
<212> DNA
<213> Artificial Sequence

<220>
<223> Klammerstrang_0539 zugehoerig zu 2D-DNA-Nanostruktur.

<400> 540
aatggtcaac aggcaaggca aagagtaatg tg 32

<210> 541
<211> 31
<212> DNA
<213> Artificial Sequence

<220>
<223> Klammerstrang_0540 zugehoerig zu 2D-DNA-Nanostruktur.

<400> 541
tctaaagttt tgtcgtcttt ccagccgaca a 31

<210> 542
<211> 44
<212> DNA
<213> Artificial Sequence

<220>
<223> Klammerstrang_0541 zugehoerig zu 2D-DNA-Nanostruktur.

<400> 542
tgacaactcg ctgaggcttg cattatacca agcgcgatga taaa 44

<210> 543
<211> 32
<212> DNA
<213> Artificial Sequence

<220>
<223> Klammerstrang_0542 zugehoerig zu 2D-DNA-Nanostruktur.

<400> 543
atattcggaa ccatcgccca cgcagagaag ga 32

<210> 544
<211> 32
<212> DNA
<213> Artificial Sequence

<220>
<223> Klammerstrang_0543 zugehoerig zu 2D-DNA-Nanostruktur.

<400> 544
tcatcgccaa caaagtacaa cggacgccag ca 32

<210> 545
<211> 32
<212> DNA
<213> Artificial Sequence

<220>
<223> Klammerstrang_0544 zugehoerig zu 2D-DNA-Nanostruktur.

<400> 545
gatggtttga acgagtagta aatttaccat ta 32

<210> 546
<211> 38
<212> DNA
<213> Artificial Sequence

<220>
<223> Klammerstrang_0545 zugehoerig zu 2D-DNA-Nanostruktur.

<400> 546
cgtttaccag acgacaaaga agttttgcca taattcga 38

<210> 547
<211> 32
<212> DNA
<213> Artificial Sequence

<220>

<223> Klammerstrang_0546 zugehoerig zu 2D-DNA-Nanostruktur.

<400> 547
cttttgcaga taaaaaccaa aataaagact cc 32

<210> 548
<211> 30
<212> DNA
<213> Artificial Sequence

<220>
<223> Klammerstrang_0547 zugehoerig zu 2D-DNA-Nanostruktur.

<400> 548
gcttcaatca ggattagaga gttattttca 30

<210> 549
<211> 32
<212> DNA
<213> Artificial Sequence

<220>
<223> Klammerstrang_0548 zugehoerig zu 2D-DNA-Nanostruktur.

<400> 549
ccaacaggag cgaaccagac cggagccttt ac 32

<210> 550
<211> 30
<212> DNA
<213> Artificial Sequence

<220>
<223> Klammerstrang_0549 zugehoerig zu 2D-DNA-Nanostruktur.

<400> 550
tttggggata gtagtagcat taaaaggccg 30

<210> 551
<211> 32
<212> DNA
<213> Artificial Sequence

<220>
<223> Klammerstrang_0550 zugehoerig zu 2D-DNA-Nanostruktur.

<400> 551
tccacagaca gccctcatag ttagcgtaac ga 32

<210> 552
<211> 32
<212> DNA
<213> Artificial Sequence

<220>
<223> Klammerstrang_0551 zugehoerig zu 2D-DNA-Nanostruktur.

<400> 552
ttaggattgg ctgagactcc tcaataaccg at 32

<210> 553
<211> 30
<212> DNA
<213> Artificial Sequence

<220>
<223> Klammerstrang_0552 zugehoerig zu 2D-DNA-Nanostruktur.

<400> 553
tattaagaag cggggttttg ctcgtagcat 30

<210> 554
<211> 32
<212> DNA
<213> Artificial Sequence

<220>
<223> Klammerstrang_0553 zugehoerig zu 2D-DNA-Nanostruktur.

<400> 554
ttgacaggcc accaccagag ccgcgatttg ta 32

<210> 555
<211> 30
<212> DNA
<213> Artificial Sequence

<220>
<223> Klammerstrang_0554 zugehoerig zu 2D-DNA-Nanostruktur.

<400> 555
caccagaaag gttgaggcag gtcatgaaag 30

<210> 556
<211> 32
<212> DNA
<213> Artificial Sequence

<220>
<223> Klammerstrang_0555 zugehoerig zu 2D-DNA-Nanostruktur.

<400> 556
gcaaggcctc accagtagca ccatgggctt ga 32

<210> 557
<211> 30
<212> DNA

<213> Artificial Sequence

<220>
<223> Klammerstrang_0556 zugehoerig zu 2D-DNA-Nanostruktur.

<400> 557
cagcaaaagg aaacgtcacc aatgagccgc 30

<210> 558
<211> 32
<212> DNA
<213> Artificial Sequence

<220>
<223> Klammerstrang_0557 zugehoerig zu 2D-DNA-Nanostruktur.

<400> 558
ttattacgaa gaactggcat gattgcgaga gg 32

<210> 559
<211> 30
<212> DNA
<213> Artificial Sequence

<220>
<223> Klammerstrang_0558 zugehoerig zu 2D-DNA-Nanostruktur.

<400> 559
atacccaaca gtatgttagc aaattagagc 30

<210> 560
<211> 32
<212> DNA
<213> Artificial Sequence

<220>
<223> Klammerstrang_0559 zugehoerig zu 2D-DNA-Nanostruktur.

<400> 560
agagagaaaa aaatgaaaat agcaagcaaa ct 32

<210> 561
<211> 30
<212> DNA
<213> Artificial Sequence

<220>
<223> Klammerstrang_0560 zugehoerig zu 2D-DNA-Nanostruktur.

<400> 561
ttaacgtcta acataaaaac aggtaacgga 30

<210> 562

<211> 32
<212> DNA
<213> Artificial Sequence

<220>
<223> Klammerstrang_0561 zugehoerig zu 2D-DNA-Nanostruktur.

<400> 562
ccaatagctc atcgtaggaa tcatggcatc aa 32

<210> 563
<211> 32
<212> DNA
<213> Artificial Sequence

<220>
<223> Klammerstrang_0562 zugehoerig zu 2D-DNA-Nanostruktur.

<400> 563
tcaccagtac aaactacaac gcctagtacc ag 32

<210> 564
<211> 32
<212> DNA
<213> Artificial Sequence

<220>
<223> Klammerstrang_0563 zugehoerig zu 2D-DNA-Nanostruktur.

<400> 564
gcggataacc tattattctg aaacagacga tt 32

<210> 565
<211> 32
<212> DNA
<213> Artificial Sequence

<220>
<223> Klammerstrang_0564 zugehoerig zu 2D-DNA-Nanostruktur.

<400> 565
tttcggaagt gccgtcgaga gggtgagttt cg 32

<210> 566
<211> 32
<212> DNA
<213> Artificial Sequence

<220>
<223> Klammerstrang_0565 zugehoerig zu 2D-DNA-Nanostruktur.

<400> 566
ggccttgaag agccaccacc ctcagaaacc at 32

<210> 567
<211> 32
<212> DNA
<213> Artificial Sequence

<220>
<223> Klammerstrang_0566 zugehoerig zu 2D-DNA-Nanostruktur.

<400> 567
ccaccctcta ttcacaaaca aatacctgcc ta 32

<210> 568
<211> 32
<212> DNA
<213> Artificial Sequence

<220>
<223> Klammerstrang_0567 zugehoerig zu 2D-DNA-Nanostruktur.

<400> 568
cgatagcatt gagccatttg ggaacgtaga aa 32

<210> 569
<211> 32
<212> DNA
<213> Artificial Sequence

<220>
<223> Klammerstrang_0568 zugehoerig zu 2D-DNA-Nanostruktur.

<400> 569
tcaccgacgc accgtaatca gtagcagaac cg 32

<210> 570
<211> 32
<212> DNA
<213> Artificial Sequence

<220>
<223> Klammerstrang_0569 zugehoerig zu 2D-DNA-Nanostruktur.

<400> 570
atacataccg aggaaacgca ataagaagcg ca 32

<210> 571
<211> 32
<212> DNA
<213> Artificial Sequence

<220>
<223> Klammerstrang_0570 zugehoerig zu 2D-DNA-Nanostruktur.

<400> 571

aaggaaacat aaaggtggca acattatcac cg 32

<210> 572
<211> 32
<212> DNA
<213> Artificial Sequence

<220>
<223> Klammerstrang_0571 zugehoerig zu 2D-DNA-Nanostruktur.

<400> 572
ttagacggcc aaataagaaa cgatagaagg ct 32

<210> 573
<211> 32
<212> DNA
<213> Artificial Sequence

<220>
<223> Klammerstrang_0572 zugehoerig zu 2D-DNA-Nanostruktur.

<400> 573
atcccaatga gaattaactg aacagttacc ag 32

<210> 574
<211> 32
<212> DNA
<213> Artificial Sequence

<220>
<223> Klammerstrang_0573 zugehoerig zu 2D-DNA-Nanostruktur.

<400> 574
tatccggtct catcgagaac aagcgacaaa ag 32

<210> 575
<211> 31
<212> DNA
<213> Artificial Sequence

<220>
<223> Klammerstrang_0574 zugehoerig zu 2D-DNA-Nanostruktur.

<400> 575
aggaacccat gtaccgtaac acttgatata a 31

<210> 576
<211> 30
<212> DNA
<213> Artificial Sequence

<220>
<223> Klammerstrang_0575 zugehoerig zu 2D-DNA-Nanostruktur.

<400> 576
gtatagcaaa cagttaatgc ccaatcctca 30

<210> 577
<211> 32
<212> DNA
<213> Artificial Sequence

<220>
<223> Klammerstrang_0576 zugehoerig zu 2D-DNA-Nanostruktur.

<400> 577
gcccgtatcc ggaataggtg tatcagccca at 32

<210> 578
<211> 30
<212> DNA
<213> Artificial Sequence

<220>
<223> Klammerstrang_0577 zugehoerig zu 2D-DNA-Nanostruktur.

<400> 578
ttaaagccag agccgccacc ctcgacagaa 30

<210> 579
<211> 32
<212> DNA
<213> Artificial Sequence

<220>
<223> Klammerstrang_0578 zugehoerig zu 2D-DNA-Nanostruktur.

<400> 579
gcctccctca gaatggaaag cgcagtaaca gt 32

<210> 580
<211> 30
<212> DNA
<213> Artificial Sequence

<220>
<223> Klammerstrang_0579 zugehoerig zu 2D-DNA-Nanostruktur.

<400> 580
tcaagtttca ttaaaggtga atataaaaga 30

<210> 581
<211> 32
<212> DNA
<213> Artificial Sequence

<220>
<223> Klammerstrang_0580 zugehoerig zu 2D-DNA-Nanostruktur.

<400> 581
gaaattattg cctttagcgt cagaccggaa cc 32

<210> 582
<211> 30
<212> DNA
<213> Artificial Sequence

<220>
<223> Klammerstrang_0581 zugehoerig zu 2D-DNA-Nanostruktur.

<400> 582
aacgcaaaga tagccgaaca aaccctgaac 30

<210> 583
<211> 32
<212> DNA
<213> Artificial Sequence

<220>
<223> Klammerstrang_0582 zugehoerig zu 2D-DNA-Nanostruktur.

<400> 583
aagtaagcag acaccacgga ataatattga cg 32

<210> 584
<211> 30
<212> DNA
<213> Artificial Sequence

<220>
<223> Klammerstrang_0583 zugehoerig zu 2D-DNA-Nanostruktur.

<400> 584
aaagtcacaa aataaacagc cagcgtttta 30

<210> 585
<211> 32
<212> DNA
<213> Artificial Sequence

<220>
<223> Klammerstrang_0584 zugehoerig zu 2D-DNA-Nanostruktur.

<400> 585
gccagttaga gggtaattga gcgctttaag aa 32

<210> 586
<211> 30
<212> DNA
<213> Artificial Sequence

<220>
<223> Klammerstrang_0585 zugehoerig zu 2D-DNA-Nanostruktur.

<400> 586
gcgaacctcc aagaacgggt atgacaataa 30

<210> 587
<211> 32
<212> DNA
<213> Artificial Sequence

<220>
<223> Klammerstrang_0586 zugehoerig zu 2D-DNA-Nanostruktur.

<400> 587
ccaccctcat tttcagggat agcaaccgta ct 32

<210> 588
<211> 32
<212> DNA
<213> Artificial Sequence

<220>
<223> Klammerstrang_0587 zugehoerig zu 2D-DNA-Nanostruktur.

<400> 588
caggaggtgg ggtcagtgcc ttgagtctct ga 32

<210> 589
<211> 30
<212> DNA
<213> Artificial Sequence

<220>
<223> Klammerstrang_0588 zugehoerig zu 2D-DNA-Nanostruktur.

<400> 589
gttttaactt agtaccgcca cccagagcca 30

<210> 590
<211> 32
<212> DNA
<213> Artificial Sequence

<220>
<223> Klammerstrang_0589 zugehoerig zu 2D-DNA-Nanostruktur.

<400> 590
atttaccggg aaccagagcc accactgtag cg 32

<210> 591
<211> 30

<212> DNA
<213> Artificial Sequence

<220>
<223> Klammerstrang_0590 zugehoerig zu 2D-DNA-Nanostruktur.

<400> 591
aaatcacctt ccagtaagcg tcagtaataa 30

<210> 592
<211> 32
<212> DNA
<213> Artificial Sequence

<220>
<223> Klammerstrang_0591 zugehoerig zu 2D-DNA-Nanostruktur.

<400> 592
cgttttcaag ggagggaagg taaagtttat tt 32

<210> 593
<211> 30
<212> DNA
<213> Artificial Sequence

<220>
<223> Klammerstrang_0592 zugehoerig zu 2D-DNA-Nanostruktur.

<400> 593
accgattgtc ggcattttcg gtcataatca 30

<210> 594
<211> 32
<212> DNA
<213> Artificial Sequence

<220>
<223> Klammerstrang_0593 zugehoerig zu 2D-DNA-Nanostruktur.

<400> 594
tgtcacaatc ttaccgaagc cctttaatat ca 32

<210> 595
<211> 30
<212> DNA
<213> Artificial Sequence

<220>
<223> Klammerstrang_0594 zugehoerig zu 2D-DNA-Nanostruktur.

<400> 595
aatagctatc aatagaaaat tcaacattca 30

<210> 596
<211> 32
<212> DNA
<213> Artificial Sequence

<220>
<223> Klammerstrang_0595 zugehoerig zu 2D-DNA-Nanostruktur.

<400> 596
gagagataga gcgtctttcc agaggttttg aa 32

<210> 597
<211> 30
<212> DNA
<213> Artificial Sequence

<220>
<223> Klammerstrang_0596 zugehoerig zu 2D-DNA-Nanostruktur.

<400> 597
acgctaacac ccacaagaat tgaaaatagc 30

<210> 598
<211> 32
<212> DNA
<213> Artificial Sequence

<220>
<223> Klammerstrang_0597 zugehoerig zu 2D-DNA-Nanostruktur.

<400> 598
gccttaaacc aatcaataat cggcacgcgc ct 32

<210> 599
<211> 32
<212> DNA
<213> Artificial Sequence

<220>
<223> Klammerstrang_0598 zugehoerig zu 2D-DNA-Nanostruktur.

<400> 599
taaatcggga ttcccaattc tgcgatataa tg 32

<210> 600
<211> 30
<212> DNA
<213> Artificial Sequence

<220>
<223> Klammerstrang_0599 zugehoerig zu 2D-DNA-Nanostruktur.

<400> 600
aacgcaaaat cgatgaacgg taccggttga 30

<210> 601
<211> 32
<212> DNA
<213> Artificial Sequence

<220>
<223> Klammerstrang_0600 zugehoerig zu 2D-DNA-Nanostruktur.

<400> 601
aacaagaggg ataaaaattt ttagcataaa gc 32

<210> 602
<211> 30
<212> DNA
<213> Artificial Sequence

<220>
<223> Klammerstrang_0601 zugehoerig zu 2D-DNA-Nanostruktur.

<400> 602
taatcagcgg attgaccgta atcgtaaccg 30

<210> 603
<211> 32
<212> DNA
<213> Artificial Sequence

<220>
<223> Klammerstrang_0602 zugehoerig zu 2D-DNA-Nanostruktur.

<400> 603
acaaacggaa aagccccaaa aacactggag ca 32

<210> 604
<211> 30
<212> DNA
<213> Artificial Sequence

<220>
<223> Klammerstrang_0603 zugehoerig zu 2D-DNA-Nanostruktur.

<400> 604
tgcatctttc ccagtcacga cggcctgcag 30

<210> 605
<211> 32
<212> DNA
<213> Artificial Sequence

<220>
<223> Klammerstrang_0604 zugehoerig zu 2D-DNA-Nanostruktur.

<400> 605
ccagggttgc cagtttgagg ggacccgtgg ga 32

<210> 606
<211> 30
<212> DNA
<213> Artificial Sequence

<220>
<223> Klammerstrang_0605 zugehoerig zu 2D-DNA-Nanostruktur.

<400> 606
gtcgacttcg gccaacgcgc ggggtttttc 30

<210> 607
<211> 32
<212> DNA
<213> Artificial Sequence

<220>
<223> Klammerstrang_0606 zugehoerig zu 2D-DNA-Nanostruktur.

<400> 607
ttaatgaact agaggatccc cgggggtaa cg 32

<210> 608
<211> 30
<212> DNA
<213> Artificial Sequence

<220>
<223> Klammerstrang_0607 zugehoerig zu 2D-DNA-Nanostruktur.

<400> 608
ttttcactca aagggcgaaa aaccatcacc 30

<210> 609
<211> 32
<212> DNA
<213> Artificial Sequence

<220>
<223> Klammerstrang_0608 zugehoerig zu 2D-DNA-Nanostruktur.

<400> 609
ctccaacgca gtgagacggg caaccagctg ca 32

<210> 610
<211> 31
<212> DNA
<213> Artificial Sequence

<220>

<223> Klammerstrang_0609 zugehoerig zu 2D-DNA-Nanostruktur.

<400> 610
caaatcaagt tttttggggt cgaaacgtgg a 31

<210> 611
<211> 30
<212> DNA
<213> Artificial Sequence

<220>
<223> Klammerstrang_0610 zugehoerig zu 2D-DNA-Nanostruktur.

<400> 611
aaattaagtt gaccattaga tacttttgcg 30

<210> 612
<211> 32
<212> DNA
<213> Artificial Sequence

<220>
<223> Klammerstrang_0611 zugehoerig zu 2D-DNA-Nanostruktur.

<400> 612
tatattttgt cattgcctga gagtggaaga tt 32

<210> 613
<211> 30
<212> DNA
<213> Artificial Sequence

<220>
<223> Klammerstrang_0612 zugehoerig zu 2D-DNA-Nanostruktur.

<400> 613
gctatcagaa atgcaatgcc tgaattagca 30

<210> 614
<211> 32
<212> DNA
<213> Artificial Sequence

<220>
<223> Klammerstrang_0613 zugehoerig zu 2D-DNA-Nanostruktur.

<400> 614
gtataagcca acccgtcgga ttctgacgac ag 32

<210> 615
<211> 30
<212> DNA
<213> Artificial Sequence

<220>
<223> Klammerstrang_0614 zugehoerig zu 2D-DNA-Nanostruktur.

<400> 615
gcgagtaaaa atatttaaat tgttacaaag 30

<210> 616
<211> 32
<212> DNA
<213> Artificial Sequence

<220>
<223> Klammerstrang_0615 zugehoerig zu 2D-DNA-Nanostruktur.

<400> 616
tatcggccgc aaggcgatta agtttaccga gc 32

<210> 617
<211> 30
<212> DNA
<213> Artificial Sequence

<220>
<223> Klammerstrang_0616 zugehoerig zu 2D-DNA-Nanostruktur.

<400> 617
gatgtgcttc aggaagatcg cacaatgtga 30

<210> 618
<211> 32
<212> DNA
<213> Artificial Sequence

<220>
<223> Klammerstrang_0617 zugehoerig zu 2D-DNA-Nanostruktur.

<400> 618
tcgaattcgg gaaacctgtc gtgcagctga tt 32

<210> 619
<211> 30
<212> DNA
<213> Artificial Sequence

<220>
<223> Klammerstrang_0618 zugehoerig zu 2D-DNA-Nanostruktur.

<400> 619
ttccagtcgt aatcatggtc ataaaagggg 30

<210> 620
<211> 32
<212> DNA

<213> Artificial Sequence

<220>
<223> Klammerstrang_0619 zugehoerig zu 2D-DNA-Nanostruktur.

<400> 620
gcccttcaga gtccactatt aaagggtgcc gt 32

<210> 621
<211> 30
<212> DNA
<213> Artificial Sequence

<220>
<223> Klammerstrang_0620 zugehoerig zu 2D-DNA-Nanostruktur.

<400> 621
tggaacaacc gcctggccct gaggcccgct 30

<210> 622
<211> 30
<212> DNA
<213> Artificial Sequence

<220>
<223> Klammerstrang_0621 zugehoerig zu 2D-DNA-Nanostruktur.

<400> 622
aaagcactaa atcggaaccc taatccagtt 30

<210> 623
<211> 32
<212> DNA
<213> Artificial Sequence

<220>
<223> Klammerstrang_0622 zugehoerig zu 2D-DNA-Nanostruktur.

<400> 623
taaatcatat aacctgttta gctaaccttt aa 32

<210> 624
<211> 32
<212> DNA
<213> Artificial Sequence

<220>
<223> Klammerstrang_0623 zugehoerig zu 2D-DNA-Nanostruktur.

<400> 624
taggtaaact atttttgaga gatcaaacgt ta 32

<210> 625

<211> 32
<212> DNA
<213> Artificial Sequence

<220>
<223> Klammerstrang_0624 zugehoerig zu 2D-DNA-Nanostruktur.

<400> 625
gagggtagga ttcaaaaggg tgagacatcc aa 32

<210> 626
<211> 32
<212> DNA
<213> Artificial Sequence

<220>
<223> Klammerstrang_0625 zugehoerig zu 2D-DNA-Nanostruktur.

<400> 626
atattttggc tttcatcaac attatccagc ca 32

<210> 627
<211> 32
<212> DNA
<213> Artificial Sequence

<220>
<223> Klammerstrang_0626 zugehoerig zu 2D-DNA-Nanostruktur.

<400> 627
tgtagccatt aaaattcgca ttaaatgccg ga 32

<210> 628
<211> 32
<212> DNA
<213> Artificial Sequence

<220>
<223> Klammerstrang_0627 zugehoerig zu 2D-DNA-Nanostruktur.

<400> 628
gctttccgat tacgccagct ggcggctgtt tc 32

<210> 629
<211> 32
<212> DNA
<213> Artificial Sequence

<220>
<223> Klammerstrang_0628 zugehoerig zu 2D-DNA-Nanostruktur.

<400> 629
tcttcgctgc accgcttctg gtgcggcctt cc 32

<210> 630
<211> 32
<212> DNA
<213> Artificial Sequence

<220>
<223> Klammerstrang_0629 zugehoerig zu 2D-DNA-Nanostruktur.

<400> 630
ctgtgtgatt gcgttgcgct cactagagtt gc 32

<210> 631
<211> 32
<212> DNA
<213> Artificial Sequence

<220>
<223> Klammerstrang_0630 zugehoerig zu 2D-DNA-Nanostruktur.

<400> 631
cacattaaaa ttgttatccg ctcatgcggg cc 32

<210> 632
<211> 32
<212> DNA
<213> Artificial Sequence

<220>
<223> Klammerstrang_0631 zugehoerig zu 2D-DNA-Nanostruktur.

<400> 632
agcaagcgta gggttgagtg ttgtagggag cc 32

<210> 633
<211> 32
<212> DNA
<213> Artificial Sequence

<220>
<223> Klammerstrang_0632 zugehoerig zu 2D-DNA-Nanostruktur.

<400> 633
gcccgagagt ccacgctggt ttgcagctaa ct 32

<210> 634
<211> 32
<212> DNA
<213> Artificial Sequence

<220>
<223> Klammerstrang_0633 zugehoerig zu 2D-DNA-Nanostruktur.

<400> 634

cccgatttag agcttgacgg ggaaaaagaa ta 32

<210> 635
<211> 32
<212> DNA
<213> Artificial Sequence

<220>
<223> Klammerstrang_0634 zugehoerig zu 2D-DNA-Nanostruktur.

<400> 635
ttctactacg cgagctgaaa aggttaccgc gc 32

<210> 636
<211> 30
<212> DNA
<213> Artificial Sequence

<220>
<223> Klammerstrang_0635 zugehoerig zu 2D-DNA-Nanostruktur.

<400> 636
gagacagcta gctgataaat taatttttgt 30

<210> 637
<211> 32
<212> DNA
<213> Artificial Sequence

<220>
<223> Klammerstrang_0636 zugehoerig zu 2D-DNA-Nanostruktur.

<400> 637
caaccgtttc aaatcaccat caattcgagc ca 32

<210> 638
<211> 44
<212> DNA
<213> Artificial Sequence

<220>
<223> Klammerstrang_0637 zugehoerig zu 2D-DNA-Nanostruktur.

<400> 638
taaatcaaaa taattcgcgt ctcggaaacc aggcaaaggg aagg 44

<210> 639
<211> 32
<212> DNA
<213> Artificial Sequence

<220>
<223> Klammerstrang_0638 zugehoerig zu 2D-DNA-Nanostruktur.

<400> 639
gccatcaagc tcatttttta accacaaatc ca 32

<210> 640
<211> 32
<212> DNA
<213> Artificial Sequence

<220>
<223> Klammerstrang_0639 zugehoerig zu 2D-DNA-Nanostruktur.

<400> 640
caactgttgc gccattcgcc attcaaacat ca 32

<210> 641
<211> 32
<212> DNA
<213> Artificial Sequence

<220>
<223> Klammerstrang_0640 zugehoerig zu 2D-DNA-Nanostruktur.

<400> 641
aagcctggta cgagccggaa gcatagatga tg 32

<210> 642
<211> 38
<212> DNA
<213> Artificial Sequence

<220>
<223> Klammerstrang_0641 zugehoerig zu 2D-DNA-Nanostruktur.

<400> 642
cccagcaggc gaaaaatccc ttataaatca agccggcg 38

<210> 643
<211> 32
<212> DNA
<213> Artificial Sequence

<220>
<223> Klammerstrang_0642 zugehoerig zu 2D-DNA-Nanostruktur.

<400> 643
tcggcaaatc ctgtttgatg gtggaccctc aa 32

<210> 644
<211> 31
<212> DNA
<213> Artificial Sequence

<220>
<223> Klammerstrang_0643 zugehoerig zu 2D-DNA-Nanostruktur.

<400> 644
aacgtggcga gaaaggaagg gaaaccagta a 31

<210> 645
<211> 30
<212> DNA
<213> Artificial Sequence

<220>
<223> Klammerstrang_0644 zugehoerig zu 2D-DNA-Nanostruktur.

<400> 645
ttttatttaa gcaaatcaga tattttttgt 30

<210> 646
<211> 32
<212> DNA
<213> Artificial Sequence

<220>
<223> Klammerstrang_0645 zugehoerig zu 2D-DNA-Nanostruktur.

<400> 646
gtaataagtt aggcagaggc atttatgata tt 32

<210> 647
<211> 30
<212> DNA
<213> Artificial Sequence

<220>
<223> Klammerstrang_0646 zugehoerig zu 2D-DNA-Nanostruktur.

<400> 647
catgtaatag aatataaagt accaagccgt 30

<210> 648
<211> 32
<212> DNA
<213> Artificial Sequence

<220>
<223> Klammerstrang_0647 zugehoerig zu 2D-DNA-Nanostruktur.

<400> 648
atcgcaagta tgtaaatgct gatgatagga ac 32

<210> 649
<211> 30
<212> DNA
<213> Artificial Sequence

<220>
<223> Klammerstrang_0648 zugehoerig zu 2D-DNA-Nanostruktur.

<400> 649
tataactaac aaagaacgcg agaacgccaa 30

<210> 650
<211> 32
<212> DNA
<213> Artificial Sequence

<220>
<223> Klammerstrang_0649 zugehoerig zu 2D-DNA-Nanostruktur.

<400> 650
agaaaacaaa gaagatgatg aaacaggctg cg 32

<210> 651
<211> 30
<212> DNA
<213> Artificial Sequence

<220>
<223> Klammerstrang_0650 zugehoerig zu 2D-DNA-Nanostruktur.

<400> 651
ctgagcaaaa attaattaca ttttgggtta 30

<210> 652
<211> 32
<212> DNA
<213> Artificial Sequence

<220>
<223> Klammerstrang_0651 zugehoerig zu 2D-DNA-Nanostruktur.

<400> 652
gcaattcaca tattcctgat tatcaaagtg ta 32

<210> 653
<211> 30
<212> DNA
<213> Artificial Sequence

<220>
<223> Klammerstrang_0652 zugehoerig zu 2D-DNA-Nanostruktur.

<400> 653
attatcattc aatataatcc tgacaattac 30

<210> 654
<211> 32

<212> DNA
<213> Artificial Sequence

<220>
<223> Klammerstrang_0653 zugehoerig zu 2D-DNA-Nanostruktur.

<400> 654
tcaatatcga acctcaaata tcaattccga aa 32

<210> 655
<211> 30
<212> DNA
<213> Artificial Sequence

<220>
<223> Klammerstrang_0654 zugehoerig zu 2D-DNA-Nanostruktur.

<400> 655
accttgcttg gtcagttggc aaagagcgga 30

<210> 656
<211> 30
<212> DNA
<213> Artificial Sequence

<220>
<223> Klammerstrang_0655 zugehoerig zu 2D-DNA-Nanostruktur.

<400> 656
taaaagggac attctggcca acaaagcatc 30

<210> 657
<211> 32
<212> DNA
<213> Artificial Sequence

<220>
<223> Klammerstrang_0656 zugehoerig zu 2D-DNA-Nanostruktur.

<400> 657
gtaccgcaat tctaagaacg cgagtattat tt 32

<210> 658
<211> 32
<212> DNA
<213> Artificial Sequence

<220>
<223> Klammerstrang_0657 zugehoerig zu 2D-DNA-Nanostruktur.

<400> 658
gtaaagtaat cgccatattt aacaaaactt tt 32

<210> 659
<211> 32
<212> DNA
<213> Artificial Sequence

<220>
<223> Klammerstrang_0658 zugehoerig zu 2D-DNA-Nanostruktur.

<400> 659
aattgagaat tctgtccaga cgactaaacc aa 32

<210> 660
<211> 32
<212> DNA
<213> Artificial Sequence

<220>
<223> Klammerstrang_0659 zugehoerig zu 2D-DNA-Nanostruktur.

<400> 660
tcaaatataa cctccggctt aggtaacaat tt 32

<210> 661
<211> 32
<212> DNA
<213> Artificial Sequence

<220>
<223> Klammerstrang_0660 zugehoerig zu 2D-DNA-Nanostruktur.

<400> 661
acctttttat tttagttaat ttcatagggc tt 32

<210> 662
<211> 32
<212> DNA
<213> Artificial Sequence

<220>
<223> Klammerstrang_0661 zugehoerig zu 2D-DNA-Nanostruktur.

<400> 662
catttgaagg cgaattattc atttttgttt gg 32

<210> 663
<211> 32
<212> DNA
<213> Artificial Sequence

<220>
<223> Klammerstrang_0662 zugehoerig zu 2D-DNA-Nanostruktur.

<400> 663
cgcgcagatt accttttta atgggagaga ct 32

<210> 664
<211> 32
<212> DNA
<213> Artificial Sequence

<220>
<223> Klammerstrang_0663 zugehoerig zu 2D-DNA-Nanostruktur.

<400> 664
attatactaa gaaaccacca gaagtcaaca gt 32

<210> 665
<211> 32
<212> DNA
<213> Artificial Sequence

<220>
<223> Klammerstrang_0664 zugehoerig zu 2D-DNA-Nanostruktur.

<400> 665
gcggaacatc tgaataatgg aaggtacaaa at 32

<210> 666
<211> 32
<212> DNA
<213> Artificial Sequence

<220>
<223> Klammerstrang_0665 zugehoerig zu 2D-DNA-Nanostruktur.

<400> 666
tgaaaggagc aaatgaaaaa tctagagata ga 32

<210> 667
<211> 32
<212> DNA
<213> Artificial Sequence

<220>
<223> Klammerstrang_0666 zugehoerig zu 2D-DNA-Nanostruktur.

<400> 667
agccagcaat tgaggaaggt tatcatcatt tt 32

<210> 668
<211> 32
<212> DNA
<213> Artificial Sequence

<220>
<223> Klammerstrang_0667 zugehoerig zu 2D-DNA-Nanostruktur.

<400> 668
accccttctga cctgaaagcg taagacgctg ag 32

<210> 669
<211> 32
<212> DNA
<213> Artificial Sequence

<220>
<223> Klammerstrang_0668 zugehoerig zu 2D-DNA-Nanostruktur.

<400> 669
cttatcattc ccgacttgcg ggagcctaat tt 32

<210> 670
<211> 30
<212> DNA
<213> Artificial Sequence

<220>
<223> Klammerstrang_0669 zugehoerig zu 2D-DNA-Nanostruktur.

<400> 670
acaacatgcc aacgctcaac agtcttctga 30

<210> 671
<211> 32
<212> DNA
<213> Artificial Sequence

<220>
<223> Klammerstrang_0670 zugehoerig zu 2D-DNA-Nanostruktur.

<400> 671
agtataaagt tcagctaatg cagatgtctt tc 32

<210> 672
<211> 30
<212> DNA
<213> Artificial Sequence

<220>
<223> Klammerstrang_0671 zugehoerig zu 2D-DNA-Nanostruktur.

<400> 672
cctaaatcaa aatcataggt ctaaacagta 30

<210> 673
<211> 32
<212> DNA
<213> Artificial Sequence

<220>

<223> Klammerstrang_0672 zugehoerig zu 2D-DNA-Nanostruktur.

<400> 673
gaatttattt aatggtttga aatattctta cc 32

<210> 674
<211> 30
<212> DNA
<213> Artificial Sequence

<220>
<223> Klammerstrang_0673 zugehoerig zu 2D-DNA-Nanostruktur.

<400> 674
cataaatctt tgaataccaa gtgttagaac 30

<210> 675
<211> 32
<212> DNA
<213> Artificial Sequence

<220>
<223> Klammerstrang_0674 zugehoerig zu 2D-DNA-Nanostruktur.

<400> 675
cctgattgca atatatgtga gtgatcaata gt 32

<210> 676
<211> 30
<212> DNA
<213> Artificial Sequence

<220>
<223> Klammerstrang_0675 zugehoerig zu 2D-DNA-Nanostruktur.

<400> 676
ctaccatagt ttgagtaaca tttaaaatat 30

<210> 677
<211> 32
<212> DNA
<213> Artificial Sequence

<220>
<223> Klammerstrang_0676 zugehoerig zu 2D-DNA-Nanostruktur.

<400> 677
attttaaaat caaaattatt tgcacggatt cg 32

<210> 678
<211> 30
<212> DNA
<213> Artificial Sequence

<220>
<223> Klammerstrang_0677 zugehoerig zu 2D-DNA-Nanostruktur.

<400> 678
ctttagggcc tgcaacagtg ccaatacgtg 30

<210> 679
<211> 32
<212> DNA
<213> Artificial Sequence

<220>
<223> Klammerstrang_0678 zugehoerig zu 2D-DNA-Nanostruktur.

<400> 679
ttaacaccag cactaacaac taatcgttat ta 32

<210> 680
<211> 31
<212> DNA
<213> Artificial Sequence

<220>
<223> Klammerstrang_0679 zugehoerig zu 2D-DNA-Nanostruktur.

<400> 680
gcacagacaa tatttttgaa tggggtcagt a 31

<210> 681
<211> 30
<212> DNA
<213> Artificial Sequence

<220>
<223> Klammerstrang_0680 zugehoerig zu 2D-DNA-Nanostruktur.

<400> 681
tgtagaaatc aagattagtt gctcttacca 30

<210> 682
<211> 32
<212> DNA
<213> Artificial Sequence

<220>
<223> Klammerstrang_0681 zugehoerig zu 2D-DNA-Nanostruktur.

<400> 682
gtttatcaat atgcgttata caaaccgacc gt 32

<210> 683
<211> 30
<212> DNA

<213> Artificial Sequence

<220>
<223> Klammerstrang_0682 zugehoerig zu 2D-DNA-Nanostruktur.

<400> 683
ttagtatcac aatagataag tccacgagca 30

<210> 684
<211> 32
<212> DNA
<213> Artificial Sequence

<220>
<223> Klammerstrang_0683 zugehoerig zu 2D-DNA-Nanostruktur.

<400> 684
gtgataaaaa gacgctgaga agagataacc tt 32

<210> 685
<211> 30
<212> DNA
<213> Artificial Sequence

<220>
<223> Klammerstrang_0684 zugehoerig zu 2D-DNA-Nanostruktur.

<400> 685
cttagattta aggcgttaaa taaagcctgt 30

<210> 686
<211> 32
<212> DNA
<213> Artificial Sequence

<220>
<223> Klammerstrang_0685 zugehoerig zu 2D-DNA-Nanostruktur.

<400> 686
gcttctgttc gggagaaaca ataacgtaaa ac 32

<210> 687
<211> 30
<212> DNA
<213> Artificial Sequence

<220>
<223> Klammerstrang_0686 zugehoerig zu 2D-DNA-Nanostruktur.

<400> 687
cttttacaaa atcgtcgcta ttagcgatag 30

<210> 688

<211> 32
<212> DNA
<213> Artificial Sequence

<220>
<223> Klammerstrang_0687 zugehoerig zu 2D-DNA-Nanostruktur.

<400> 688
agaaataaaa atcctttgcc cgaaagatta ga 32

<210> 689
<211> 30
<212> DNA
<213> Artificial Sequence

<220>
<223> Klammerstrang_0688 zugehoerig zu 2D-DNA-Nanostruktur.

<400> 689
ctcgtattag aaattgcgta gatacagtac 30

<210> 690
<211> 32
<212> DNA
<213> Artificial Sequence

<220>
<223> Klammerstrang_0689 zugehoerig zu 2D-DNA-Nanostruktur.

<400> 690
gccgtcaaaa aacagaggtg aggcctatta gt 32

<210> 691
<211> 30
<212> DNA
<213> Artificial Sequence

<220>
<223> Klammerstrang_0690 zugehoerig zu 2D-DNA-Nanostruktur.

<400> 691
cagaagatta gataatacat ttgtcgacaa 30

<210> 692
<211> 30
<212> DNA
<213> Artificial Sequence

<220>
<223> Klammerstrang_0691 zugehoerig zu 2D-DNA-Nanostruktur.

<400> 692
ctttaatgcg cgaactgata gccccaccag 30

<210> 693
<211> 52
<212> DNA
<213> Artificial Sequence

<220>
<223> Klammerstrang_0692 zugehoerig zu 2D-DNA-Nanostruktur.

<400> 693
agaaaggaac aactaaagga attcaaaaaa attcctctac cacctacatc ac 52

<210> 694
<211> 51
<212> DNA
<213> Artificial Sequence

<220>
<223> Klammerstrang_0693 zugehoerig zu 2D-DNA-Nanostruktur.

<400> 694
aggctccaga ggctttgagg acacgggtaa ttcctctacc acctacatca c 51

<210> 695
<211> 53
<212> DNA
<213> Artificial Sequence

<220>
<223> Klammerstrang_0694 zugehoerig zu 2D-DNA-Nanostruktur.

<400> 695
acggctacaa aaggagcctt taatgtgaga atttcctcta ccacctacat cac 53

<210> 696
<211> 51
<212> DNA
<213> Artificial Sequence

<220>
<223> Klammerstrang_0695 zugehoerig zu 2D-DNA-Nanostruktur.

<400> 696
aatacgtttg aaagaggaca gactgacctt ttcctctacc acctacatca c 51

<210> 697
<211> 53
<212> DNA
<213> Artificial Sequence

<220>
<223> Klammerstrang_0696 zugehoerig zu 2D-DNA-Nanostruktur.

<400> 697

gaccaactaa tgccactacg aagggggtag cattcctcta ccacctacat cac 53

<210> 698
<211> 51
<212> DNA
<213> Artificial Sequence

<220>
<223> Klammerstrang_0697 zugehoerig zu 2D-DNA-Nanostruktur.

<400> 698
catcaagtaa aacgaactaa cgagttgaga ttcctctacc acctacatca c 51

<210> 699
<211> 53
<212> DNA
<213> Artificial Sequence

<220>
<223> Klammerstrang_0698 zugehoerig zu 2D-DNA-Nanostruktur.

<400> 699
tacgttaaag taatcttgac aagaaccgaa ctttcctcta ccacctacat cac 53

<210> 700
<211> 51
<212> DNA
<213> Artificial Sequence

<220>
<223> Klammerstrang_0699 zugehoerig zu 2D-DNA-Nanostruktur.

<400> 700
tttaggacaa atgctttaaa caatcaggtc ttcctctacc acctacatca c 51

<210> 701
<211> 53
<212> DNA
<213> Artificial Sequence

<220>
<223> Klammerstrang_0700 zugehoerig zu 2D-DNA-Nanostruktur.

<400> 701
atccccctat accacattca actagaaaaa tcttcctcta ccacctacat cac 53

<210> 702
<211> 51
<212> DNA
<213> Artificial Sequence

<220>
<223> Klammerstrang_0701 zugehoerig zu 2D-DNA-Nanostruktur.

<400> 702
tttaccccaa catgttttaa atttccatat ttcctctacc acctacatca c 51

<210> 703
<211> 53
<212> DNA
<213> Artificial Sequence

<220>
<223> Klammerstrang_0702 zugehoerig zu 2D-DNA-Nanostruktur.

<400> 703
ctgtagcttg actattatag tcagttcatt gattcctcta ccacctacat cac 53

<210> 704
<211> 51
<212> DNA
<213> Artificial Sequence

<220>
<223> Klammerstrang_0703 zugehoerig zu 2D-DNA-Nanostruktur.

<400> 704
aacagttttg taccaaaaac attttatttc ttcctctacc acctacatca c 51

<210> 705
<211> 53
<212> DNA
<213> Artificial Sequence

<220>
<223> Klammerstrang_0704 zugehoerig zu 2D-DNA-Nanostruktur.

<400> 705
acaactttca acagtttcag cggatgtatc ggttcctcta ccacctacat cac 53

<210> 706
<211> 53
<212> DNA
<213> Artificial Sequence

<220>
<223> Klammerstrang_0705 zugehoerig zu 2D-DNA-Nanostruktur.

<400> 706
tttatcagga cagcatcgga acgacaccaa ccttcctcta ccacctacat cac 53

<210> 707
<211> 51
<212> DNA
<213> Artificial Sequence

<220>
<223> Klammerstrang_0706 zugehoerig zu 2D-DNA-Nanostruktur.

<400> 707
cagcgaaact tgctttcgag gtgttgctaa ttcctctacc acctacatca c 51

<210> 708
<211> 53
<212> DNA
<213> Artificial Sequence

<220>
<223> Klammerstrang_0707 zugehoerig zu 2D-DNA-Nanostruktur.

<400> 708
taaaacgagg tcaatcataa gggaaccgga tattcctcta ccacctacat cac 53

<210> 709
<211> 51
<212> DNA
<213> Artificial Sequence

<220>
<223> Klammerstrang_0708 zugehoerig zu 2D-DNA-Nanostruktur.

<400> 709
gcgcagacaa gaggcaaaag aatccctcag ttcctctacc acctacatca c 51

<210> 710
<211> 53
<212> DNA
<213> Artificial Sequence

<220>
<223> Klammerstrang_0709 zugehoerig zu 2D-DNA-Nanostruktur.

<400> 710
ttcattacgt caggacgttg ggaaatgcag atttcctcta ccacctacat cac 53

<210> 711
<211> 51
<212> DNA
<213> Artificial Sequence

<220>
<223> Klammerstrang_0710 zugehoerig zu 2D-DNA-Nanostruktur.

<400> 711
ttataccacc aaatcaacgt aacgaacgag ttcctctacc acctacatca c 51

<210> 712
<211> 53
<212> DNA
<213> Artificial Sequence

<220>
<223> Klammerstrang_0711 zugehoerig zu 2D-DNA-Nanostruktur.

<400> 712
acataacggg aatcgtcata aataaagcaa agttcctcta ccacctacat cac 53

<210> 713
<211> 51
<212> DNA
<213> Artificial Sequence

<220>
<223> Klammerstrang_0712 zugehoerig zu 2D-DNA-Nanostruktur.

<400> 713
aatactgccc aaaaggaatt acgtggctca ttcctctacc acctacatca c 51

<210> 714
<211> 53
<212> DNA
<213> Artificial Sequence

<220>
<223> Klammerstrang_0713 zugehoerig zu 2D-DNA-Nanostruktur.

<400> 714
cggattgcag agcttaattg ctgaaacgag tattcctcta ccacctacat cac 53

<210> 715
<211> 51
<212> DNA
<213> Artificial Sequence

<220>
<223> Klammerstrang_0714 zugehoerig zu 2D-DNA-Nanostruktur.

<400> 715
gatggcttat caaaaagatt aagagcgtcc ttcctctacc acctacatca c 51

<210> 716
<211> 53
<212> DNA
<213> Artificial Sequence

<220>
<223> Klammerstrang_0715 zugehoerig zu 2D-DNA-Nanostruktur.

<400> 716
gatttagtca ataaagcctc agagaaccct cattcctcta ccacctacat cac 53

<210> 717
<211> 53

<212> DNA
<213> Artificial Sequence

<220>
<223> Klammerstrang_0716 zugehoerig zu 2D-DNA-Nanostruktur.

<400> 717
taaatgaatt ttctgtatgg gattaatttc ttttcctcta ccacctacat cac 53

<210> 718
<211> 53
<212> DNA
<213> Artificial Sequence

<220>
<223> Klammerstrang_0717 zugehoerig zu 2D-DNA-Nanostruktur.

<400> 718
aaacagcttt ttgcgggatc gtcaacacta aattcctcta ccacctacat cac 53

<210> 719
<211> 53
<212> DNA
<213> Artificial Sequence

<220>
<223> Klammerstrang_0718 zugehoerig zu 2D-DNA-Nanostruktur.

<400> 719
aaggccgctg ataccgatag ttgcgacgtt agttcctcta ccacctacat cac 53

<210> 720
<211> 53
<212> DNA
<213> Artificial Sequence

<220>
<223> Klammerstrang_0719 zugehoerig zu 2D-DNA-Nanostruktur.

<400> 720
acactcatcc atgttactta gccgaaagct gcttcctcta ccacctacat cac 53

<210> 721
<211> 53
<212> DNA
<213> Artificial Sequence

<220>
<223> Klammerstrang_0720 zugehoerig zu 2D-DNA-Nanostruktur.

<400> 721
gacctgctct ttgacccccca gcgagggagt tattcctcta ccacctacat cac 53

<210> 722
<211> 53
<212> DNA
<213> Artificial Sequence

<220>
<223> Klammerstrang_0721 zugehoerig zu 2D-DNA-Nanostruktur.

<400> 722
tcattcagat gcgattttaa gaacaggcat agttcctcta ccacctacat cac 53

<210> 723
<211> 53
<212> DNA
<213> Artificial Sequence

<220>
<223> Klammerstrang_0722 zugehoerig zu 2D-DNA-Nanostruktur.

<400> 723
attacctttg aataaggctt gcccaaatcc gcttcctcta ccacctacat cac 53

<210> 724
<211> 53
<212> DNA
<213> Artificial Sequence

<220>
<223> Klammerstrang_0723 zugehoerig zu 2D-DNA-Nanostruktur.

<400> 724
taagagcaaa tgtttagact ggataggaag ccttcctcta ccacctacat cac 53

<210> 725
<211> 53
<212> DNA
<213> Artificial Sequence

<220>
<223> Klammerstrang_0724 zugehoerig zu 2D-DNA-Nanostruktur.

<400> 725
aatagtaaac actatcataa ccctcattgt gattcctcta ccacctacat cac 53

<210> 726
<211> 53
<212> DNA
<213> Artificial Sequence

<220>
<223> Klammerstrang_0725 zugehoerig zu 2D-DNA-Nanostruktur.

<400> 726
cgaaagactt tgataagagg tcatatttcg cattcctcta ccacctacat cac 53

<210> 727
<211> 53
<212> DNA
<213> Artificial Sequence

<220>
<223> Klammerstrang_0726 zugehoerig zu 2D-DNA-Nanostruktur.

<400> 727
ttgctccttt caaatatcgc gtttgagggg gtttcctcta ccacctacat cac 53

<210> 728
<211> 53
<212> DNA
<213> Artificial Sequence

<220>
<223> Klammerstrang_0727 zugehoerig zu 2D-DNA-Nanostruktur.

<400> 728
aatggtcaac aggcaaggca aagagtaatg tgttcctcta ccacctacat cac 53

<210> 729
<211> 52
<212> DNA
<213> Artificial Sequence

<220>
<223> Klammerstrang_0728 zugehoerig zu 2D-DNA-Nanostruktur.

<400> 729
tctaaagttt tgtcgtcttt ccagccgaca attcctctac cacctacatc ac 52

<210> 730
<211> 65
<212> DNA
<213> Artificial Sequence

<220>
<223> Klammerstrang_0729 zugehoerig zu 2D-DNA-Nanostruktur.

<400> 730
tgacaactcg ctgaggcttg cattatacca agcgcgatga taaattcctc taccacctac 60

atcac 65

<210> 731
<211> 53
<212> DNA
<213> Artificial Sequence

<220>

<223> Klammerstrang_0730 zugehoerig zu 2D-DNA-Nanostruktur.

<400> 731
atattcggaa ccatcgccca cgcagagaag gattcctcta ccacctacat cac 53

<210> 732
<211> 53
<212> DNA
<213> Artificial Sequence

<220>
<223> Klammerstrang_0731 zugehoerig zu 2D-DNA-Nanostruktur.

<400> 732
tcatcgccaa caaagtacaa cggacgccag cattcctcta ccacctacat cac 53

<210> 733
<211> 53
<212> DNA
<213> Artificial Sequence

<220>
<223> Klammerstrang_0732 zugehoerig zu 2D-DNA-Nanostruktur.

<400> 733
gatggtttga acgagtagta aatttaccat tattcctcta ccacctacat cac 53

<210> 734
<211> 59
<212> DNA
<213> Artificial Sequence

<220>
<223> Klammerstrang_0733 zugehoerig zu 2D-DNA-Nanostruktur.

<400> 734
cgtttaccag acgacaaaga agttttgcca taattcgatt cctctaccac ctacatcac 59

<210> 735
<211> 53
<212> DNA
<213> Artificial Sequence

<220>
<223> Klammerstrang_0734 zugehoerig zu 2D-DNA-Nanostruktur.

<400> 735
cttttgcaga taaaaaccaa aataaagact ccttcctcta ccacctacat cac 53

<210> 736
<211> 51
<212> DNA
<213> Artificial Sequence

<220>
<223> Klammerstrang_0735 zugehoerig zu 2D-DNA-Nanostruktur.

<400> 736
gcttcaatca ggattagaga gttattttca ttcctctacc acctacatca c 51

<210> 737
<211> 53
<212> DNA
<213> Artificial Sequence

<220>
<223> Klammerstrang_0736 zugehoerig zu 2D-DNA-Nanostruktur.

<400> 737
ccaacaggag cgaaccagac cggagccttt acttcctcta ccacctacat cac 53

<210> 738
<211> 51
<212> DNA
<213> Artificial Sequence

<220>
<223> Klammerstrang_0737 zugehoerig zu 2D-DNA-Nanostruktur.

<400> 738
tttggggata gtagtagcat taaaaggccg ttcctctacc acctacatca c 51

<210> 739
<211> 53
<212> DNA
<213> Artificial Sequence

<220>
<223> Klammerstrang_0738 zugehoerig zu 2D-DNA-Nanostruktur.

<400> 739
tccacagaca gccctcatag ttagcgtaac gattcctcta ccacctacat cac 53

<210> 740
<211> 53
<212> DNA
<213> Artificial Sequence

<220>
<223> Klammerstrang_0739 zugehoerig zu 2D-DNA-Nanostruktur.

<400> 740
ttaggattgg ctgagactcc tcaataaccg atttcctcta ccacctacat cac 53

<210> 741
<211> 51
<212> DNA

<213> Artificial Sequence

<220>
<223> Klammerstrang_0740 zugehoerig zu 2D-DNA-Nanostruktur.

<400> 741
tattaagaag cggggttttg ctcgtagcat ttcctctacc acctacatca c 51

<210> 742
<211> 53
<212> DNA
<213> Artificial Sequence

<220>
<223> Klammerstrang_0741 zugehoerig zu 2D-DNA-Nanostruktur.

<400> 742
ttgacaggcc accaccagag ccgcgatttg tattcctcta ccacctacat cac 53

<210> 743
<211> 51
<212> DNA
<213> Artificial Sequence

<220>
<223> Klammerstrang_0742 zugehoerig zu 2D-DNA-Nanostruktur.

<400> 743
caccagaaag gttgaggcag gtcatgaaag ttcctctacc acctacatca c 51

<210> 744
<211> 53
<212> DNA
<213> Artificial Sequence

<220>
<223> Klammerstrang_0743 zugehoerig zu 2D-DNA-Nanostruktur.

<400> 744
gcaaggcctc accagtagca ccatgggctt gattcctcta ccacctacat cac 53

<210> 745
<211> 51
<212> DNA
<213> Artificial Sequence

<220>
<223> Klammerstrang_0744 zugehoerig zu 2D-DNA-Nanostruktur.

<400> 745
cagcaaaagg aaacgtcacc aatgagccgc ttcctctacc acctacatca c 51

<210> 746

<211> 53
<212> DNA
<213> Artificial Sequence

<220>
<223> Klammerstrang_0745 zugehoerig zu 2D-DNA-Nanostruktur.

<400> 746
ttattacgaa gaactggcat gattgcgaga ggttcctcta ccacctacat cac 53

<210> 747
<211> 51
<212> DNA
<213> Artificial Sequence

<220>
<223> Klammerstrang_0746 zugehoerig zu 2D-DNA-Nanostruktur.

<400> 747
atacccaaca gtatgttagc aaattagagc ttcctctacc acctacatca c 51

<210> 748
<211> 53
<212> DNA
<213> Artificial Sequence

<220>
<223> Klammerstrang_0747 zugehoerig zu 2D-DNA-Nanostruktur.

<400> 748
agagagaaaa aaatgaaaat agcaagcaaa ctttcctcta ccacctacat cac 53

<210> 749
<211> 51
<212> DNA
<213> Artificial Sequence

<220>
<223> Klammerstrang_0748 zugehoerig zu 2D-DNA-Nanostruktur.

<400> 749
ttaacgtcta acataaaaac aggtaacgga ttcctctacc acctacatca c 51

<210> 750
<211> 53
<212> DNA
<213> Artificial Sequence

<220>
<223> Klammerstrang_0749 zugehoerig zu 2D-DNA-Nanostruktur.

<400> 750
ccaatagctc atcgtaggaa tcatggcatc aattcctcta ccacctacat cac 53

<210> 751
<211> 53
<212> DNA
<213> Artificial Sequence

<220>
<223> Klammerstrang_0750 zugehoerig zu 2D-DNA-Nanostruktur.

<400> 751
tcaccagtac aaactacaac gcctagtacc agttcctcta ccacctacat cac 53

<210> 752
<211> 53
<212> DNA
<213> Artificial Sequence

<220>
<223> Klammerstrang_0751 zugehoerig zu 2D-DNA-Nanostruktur.

<400> 752
gcggataacc tattattctg aaacagacga ttttcctcta ccacctacat cac 53

<210> 753
<211> 53
<212> DNA
<213> Artificial Sequence

<220>
<223> Klammerstrang_0752 zugehoerig zu 2D-DNA-Nanostruktur.

<400> 753
tttcggaagt gccgtcgaga gggtgagttt cgttcctcta ccacctacat cac 53

<210> 754
<211> 53
<212> DNA
<213> Artificial Sequence

<220>
<223> Klammerstrang_0753 zugehoerig zu 2D-DNA-Nanostruktur.

<400> 754
ggccttgaag agccaccacc ctcagaaacc atttcctcta ccacctacat cac 53

<210> 755
<211> 53
<212> DNA
<213> Artificial Sequence

<220>
<223> Klammerstrang_0754 zugehoerig zu 2D-DNA-Nanostruktur.

<400> 755

ccaccctcta ttcacaaaca aatacctgcc tattcctcta ccacctacat cac 53

<210> 756
<211> 53
<212> DNA
<213> Artificial Sequence

<220>
<223> Klammerstrang_0755 zugehoerig zu 2D-DNA-Nanostruktur.

<400> 756
cgatagcatt gagccatttg ggaacgtaga aattcctcta ccacctacat cac 53

<210> 757
<211> 53
<212> DNA
<213> Artificial Sequence

<220>
<223> Klammerstrang_0756 zugehoerig zu 2D-DNA-Nanostruktur.

<400> 757
tcaccgacgc accgtaatca gtagcagaac cgttcctcta ccacctacat cac 53

<210> 758
<211> 53
<212> DNA
<213> Artificial Sequence

<220>
<223> Klammerstrang_0757 zugehoerig zu 2D-DNA-Nanostruktur.

<400> 758
atacataccg aggaaacgca ataagaagcg cattcctcta ccacctacat cac 53

<210> 759
<211> 53
<212> DNA
<213> Artificial Sequence

<220>
<223> Klammerstrang_0758 zugehoerig zu 2D-DNA-Nanostruktur.

<400> 759
aaggaaacat aaaggtggca acattatcac cgttcctcta ccacctacat cac 53

<210> 760
<211> 53
<212> DNA
<213> Artificial Sequence

<220>
<223> Klammerstrang_0759 zugehoerig zu 2D-DNA-Nanostruktur.

<400> 760
ttagacggcc aaataagaaa cgatagaagg ctttcctcta ccacctacat cac 53

<210> 761
<211> 53
<212> DNA
<213> Artificial Sequence

<220>
<223> Klammerstrang_0760 zugehoerig zu 2D-DNA-Nanostruktur.

<400> 761
atcccaatga gaattaactg aacagttacc agttcctcta ccacctacat cac 53

<210> 762
<211> 53
<212> DNA
<213> Artificial Sequence

<220>
<223> Klammerstrang_0761 zugehoerig zu 2D-DNA-Nanostruktur.

<400> 762
tatccggtct catcgagaac aagcgacaaa agttcctcta ccacctacat cac 53

<210> 763
<211> 52
<212> DNA
<213> Artificial Sequence

<220>
<223> Klammerstrang_0762 zugehoerig zu 2D-DNA-Nanostruktur.

<400> 763
aggaacccat gtaccgtaac acttgatata attcctctac cacctacatc ac 52

<210> 764
<211> 51
<212> DNA
<213> Artificial Sequence

<220>
<223> Klammerstrang_0763 zugehoerig zu 2D-DNA-Nanostruktur.

<400> 764
gtatagcaaa cagttaatgc ccaatcctca ttcctctacc acctacatca c 51

<210> 765
<211> 53
<212> DNA
<213> Artificial Sequence

<220>
<223> Klammerstrang_0764 zugehoerig zu 2D-DNA-Nanostruktur.

<400> 765
gcccgtatcc ggaataggtg tatcagccca atttcctcta ccacctacat cac 53

<210> 766
<211> 51
<212> DNA
<213> Artificial Sequence

<220>
<223> Klammerstrang_0765 zugehoerig zu 2D-DNA-Nanostruktur.

<400> 766
ttaaagccag agccgccacc ctcgacagaa ttcctctacc acctacatca c 51

<210> 767
<211> 53
<212> DNA
<213> Artificial Sequence

<220>
<223> Klammerstrang_0766 zugehoerig zu 2D-DNA-Nanostruktur.

<400> 767
gcctccctca gaatggaaag cgcagtaaca gtttcctcta ccacctacat cac 53

<210> 768
<211> 51
<212> DNA
<213> Artificial Sequence

<220>
<223> Klammerstrang_0767 zugehoerig zu 2D-DNA-Nanostruktur.

<400> 768
tcaagtttca ttaaaggtga atataaaaga ttcctctacc acctacatca c 51

<210> 769
<211> 53
<212> DNA
<213> Artificial Sequence

<220>
<223> Klammerstrang_0768 zugehoerig zu 2D-DNA-Nanostruktur.

<400> 769
gaaattattg cctttagcgt cagaccggaa ccttcctcta ccacctacat cac 53

<210> 770
<211> 51
<212> DNA
<213> Artificial Sequence

<220>
<223> Klammerstrang_0769 zugehoerig zu 2D-DNA-Nanostruktur.

<400> 770
aacgcaaaga tagccgaaca aaccctgaac ttcctctacc acctacatca c 51

<210> 771
<211> 53
<212> DNA
<213> Artificial Sequence

<220>
<223> Klammerstrang_0770 zugehoerig zu 2D-DNA-Nanostruktur.

<400> 771
aagtaagcag acaccacgga ataatattga cgttcctcta ccacctacat cac 53

<210> 772
<211> 51
<212> DNA
<213> Artificial Sequence

<220>
<223> Klammerstrang_0771 zugehoerig zu 2D-DNA-Nanostruktur.

<400> 772
aaagtcacaa aataaacagc cagcgtttta ttcctctacc acctacatca c 51

<210> 773
<211> 53
<212> DNA
<213> Artificial Sequence

<220>
<223> Klammerstrang_0772 zugehoerig zu 2D-DNA-Nanostruktur.

<400> 773
gccagttaga gggtaattga gcgctttaag aattcctcta ccacctacat cac 53

<210> 774
<211> 51
<212> DNA
<213> Artificial Sequence

<220>
<223> Klammerstrang_0773 zugehoerig zu 2D-DNA-Nanostruktur.

<400> 774
gcgaacctcc aagaacgggt atgacaataa ttcctctacc acctacatca c 51

<210> 775
<211> 53

<212> DNA
<213> Artificial Sequence

<220>
<223> Klammerstrang_0774 zugehoerig zu 2D-DNA-Nanostruktur.

<400> 775
ccaccctcat tttcagggat agcaaccgta ctttcctcta ccacctacat cac 53

<210> 776
<211> 53
<212> DNA
<213> Artificial Sequence

<220>
<223> Klammerstrang_0775 zugehoerig zu 2D-DNA-Nanostruktur.

<400> 776
caggaggtgg ggtcagtgcc ttgagtctct gattcctcta ccacctacat cac 53

<210> 777
<211> 51
<212> DNA
<213> Artificial Sequence

<220>
<223> Klammerstrang_0776 zugehoerig zu 2D-DNA-Nanostruktur.

<400> 777
gttttaactt agtaccgcca cccagagcca ttcctctacc acctacatca c 51

<210> 778
<211> 53
<212> DNA
<213> Artificial Sequence

<220>
<223> Klammerstrang_0777 zugehoerig zu 2D-DNA-Nanostruktur.

<400> 778
atttaccggg aaccagagcc accactgtag cgttcctcta ccacctacat cac 53

<210> 779
<211> 51
<212> DNA
<213> Artificial Sequence

<220>
<223> Klammerstrang_0778 zugehoerig zu 2D-DNA-Nanostruktur.

<400> 779
aaatcacctt ccagtaagcg tcagtaataa ttcctctacc acctacatca c 51

<210> 780
<211> 53
<212> DNA
<213> Artificial Sequence

<220>
<223> Klammerstrang_0779 zugehoerig zu 2D-DNA-Nanostruktur.

<400> 780
cgttttcaag ggagggaagg taaagtttat ttttcctcta ccacctacat cac 53

<210> 781
<211> 51
<212> DNA
<213> Artificial Sequence

<220>
<223> Klammerstrang_0780 zugehoerig zu 2D-DNA-Nanostruktur.

<400> 781
accgattgtc ggcattttcg gtcataatca ttcctctacc acctacatca c 51

<210> 782
<211> 53
<212> DNA
<213> Artificial Sequence

<220>
<223> Klammerstrang_0781 zugehoerig zu 2D-DNA-Nanostruktur.

<400> 782
tgtcacaatc ttaccgaagc cctttaatat cattcctcta ccacctacat cac 53

<210> 783
<211> 51
<212> DNA
<213> Artificial Sequence

<220>
<223> Klammerstrang_0782 zugehoerig zu 2D-DNA-Nanostruktur.

<400> 783
aatagctatc aatagaaaat tcaacattca ttcctctacc acctacatca c 51

<210> 784
<211> 53
<212> DNA
<213> Artificial Sequence

<220>
<223> Klammerstrang_0783 zugehoerig zu 2D-DNA-Nanostruktur.

<400> 784
gagagataga gcgtctttcc agaggttttg aattcctcta ccacctacat cac 53

<210> 785
<211> 51
<212> DNA
<213> Artificial Sequence

<220>
<223> Klammerstrang_0784 zugehoerig zu 2D-DNA-Nanostruktur.

<400> 785
acgctaacac ccacaagaat tgaaaatagc ttcctctacc acctacatca c 51

<210> 786
<211> 53
<212> DNA
<213> Artificial Sequence

<220>
<223> Klammerstrang_0785 zugehoerig zu 2D-DNA-Nanostruktur.

<400> 786
gccttaaacc aatcaataat cggcacgcgc ctttcctcta ccacctacat cac 53

<210> 787
<211> 53
<212> DNA
<213> Artificial Sequence

<220>
<223> Klammerstrang_0786 zugehoerig zu 2D-DNA-Nanostruktur.

<400> 787
taaatcggga ttcccaattc tgcgatataa tgttcctcta ccacctacat cac 53

<210> 788
<211> 51
<212> DNA
<213> Artificial Sequence

<220>
<223> Klammerstrang_0787 zugehoerig zu 2D-DNA-Nanostruktur.

<400> 788
aacgcaaaat cgatgaacgg taccggttga ttcctctacc acctacatca c 51

<210> 789
<211> 53
<212> DNA
<213> Artificial Sequence

<220>
<223> Klammerstrang_0788 zugehoerig zu 2D-DNA-Nanostruktur.

<400> 789
aacaagaggg ataaaaattt ttagcataaa gcttcctcta ccacctacat cac 53

<210> 790
<211> 51
<212> DNA
<213> Artificial Sequence

<220>
<223> Klammerstrang_0789 zugehoerig zu 2D-DNA-Nanostruktur.

<400> 790
taatcagcgg attgaccgta atcgtaaccg ttcctctacc acctacatca c 51

<210> 791
<211> 53
<212> DNA
<213> Artificial Sequence

<220>
<223> Klammerstrang_0790 zugehoerig zu 2D-DNA-Nanostruktur.

<400> 791
acaaacggaa aagccccaaa aacactggag cattcctcta ccacctacat cac 53

<210> 792
<211> 51
<212> DNA
<213> Artificial Sequence

<220>
<223> Klammerstrang_0791 zugehoerig zu 2D-DNA-Nanostruktur.

<400> 792
tgcatctttc ccagtcacga cggcctgcag ttcctctacc acctacatca c 51

<210> 793
<211> 53
<212> DNA
<213> Artificial Sequence

<220>
<223> Klammerstrang_0792 zugehoerig zu 2D-DNA-Nanostruktur.

<400> 793
ccagggttgc cagtttgagg ggacccgtgg gattcctcta ccacctacat cac 53

<210> 794
<211> 51
<212> DNA
<213> Artificial Sequence

<220>

<223> Klammerstrang_0793 zugehoerig zu 2D-DNA-Nanostruktur.

<400> 794
gtcgacttcg gccaacgcgc ggggtttttc ttcctctacc acctacatca c 51

<210> 795
<211> 53
<212> DNA
<213> Artificial Sequence

<220>
<223> Klammerstrang_0794 zugehoerig zu 2D-DNA-Nanostruktur.

<400> 795
ttaatgaact agaggatccc cgggggtaa cgttcctcta ccacctacat cac 53

<210> 796
<211> 51
<212> DNA
<213> Artificial Sequence

<220>
<223> Klammerstrang_0795 zugehoerig zu 2D-DNA-Nanostruktur.

<400> 796
ttttcactca aagggcgaaa aaccatcacc ttcctctacc acctacatca c 51

<210> 797
<211> 53
<212> DNA
<213> Artificial Sequence

<220>
<223> Klammerstrang_0796 zugehoerig zu 2D-DNA-Nanostruktur.

<400> 797
ctccaacgca gtgagacggg caaccagctg cattcctcta ccacctacat cac 53

<210> 798
<211> 52
<212> DNA
<213> Artificial Sequence

<220>
<223> Klammerstrang_0797 zugehoerig zu 2D-DNA-Nanostruktur.

<400> 798
caaatcaagt tttttggggt cgaaacgtgg attcctctac cacctacatc ac 52

<210> 799
<211> 51
<212> DNA
<213> Artificial Sequence

<220>
<223> Klammerstrang_0798 zugehoerig zu 2D-DNA-Nanostruktur.

<400> 799
aaattaagtt gaccattaga tacttttgcg ttcctctacc acctacatca c 51

<210> 800
<211> 53
<212> DNA
<213> Artificial Sequence

<220>
<223> Klammerstrang_0799 zugehoerig zu 2D-DNA-Nanostruktur.

<400> 800
tatattttgt cattgcctga gagtggaaga ttttcctcta ccacctacat cac 53

<210> 801
<211> 51
<212> DNA
<213> Artificial Sequence

<220>
<223> Klammerstrang_0800 zugehoerig zu 2D-DNA-Nanostruktur.

<400> 801
gctatcagaa atgcaatgcc tgaattagca ttcctctacc acctacatca c 51

<210> 802
<211> 53
<212> DNA
<213> Artificial Sequence

<220>
<223> Klammerstrang_0801 zugehoerig zu 2D-DNA-Nanostruktur.

<400> 802
gtataagcca acccgtcgga ttctgacgac agttcctcta ccacctacat cac 53

<210> 803
<211> 51
<212> DNA
<213> Artificial Sequence

<220>
<223> Klammerstrang_0802 zugehoerig zu 2D-DNA-Nanostruktur.

<400> 803
gcgagtaaaa atatttaaat tgttacaaag ttcctctacc acctacatca c 51

<210> 804
<211> 53
<212> DNA

<213> Artificial Sequence

<220>
<223> Klammerstrang_0803 zugehoerig zu 2D-DNA-Nanostruktur.

<400> 804
tatcggccgc aaggcgatta agtttaccga gcttcctcta ccacctacat cac 53

<210> 805
<211> 51
<212> DNA
<213> Artificial Sequence

<220>
<223> Klammerstrang_0804 zugehoerig zu 2D-DNA-Nanostruktur.

<400> 805
gatgtgcttc aggaagatcg cacaatgtga ttcctctacc acctacatca c 51

<210> 806
<211> 53
<212> DNA
<213> Artificial Sequence

<220>
<223> Klammerstrang_0805 zugehoerig zu 2D-DNA-Nanostruktur.

<400> 806
tcgaattcgg gaaacctgtc gtgcagctga ttttcctcta ccacctacat cac 53

<210> 807
<211> 51
<212> DNA
<213> Artificial Sequence

<220>
<223> Klammerstrang_0806 zugehoerig zu 2D-DNA-Nanostruktur.

<400> 807
ttccagtcgt aatcatggtc ataaaagggg ttcctctacc acctacatca c 51

<210> 808
<211> 53
<212> DNA
<213> Artificial Sequence

<220>
<223> Klammerstrang_0807 zugehoerig zu 2D-DNA-Nanostruktur.

<400> 808
gcccttcaga gtccactatt aaagggtgcc gtttcctcta ccacctacat cac 53

<210> 809

<211> 51
<212> DNA
<213> Artificial Sequence

<220>
<223> Klammerstrang_0808 zugehoerig zu 2D-DNA-Nanostruktur.

<400> 809
tggaacaacc gcctggccct gaggcccgct ttcctctacc acctacatca c 51

<210> 810
<211> 51
<212> DNA
<213> Artificial Sequence

<220>
<223> Klammerstrang_0809 zugehoerig zu 2D-DNA-Nanostruktur.

<400> 810
aaagcactaa atcggaaccc taatccagtt ttcctctacc acctacatca c 51

<210> 811
<211> 53
<212> DNA
<213> Artificial Sequence

<220>
<223> Klammerstrang_0810 zugehoerig zu 2D-DNA-Nanostruktur.

<400> 811
taaatcatat aacctgttta gctaaccttt aattcctcta ccacctacat cac 53

<210> 812
<211> 53
<212> DNA
<213> Artificial Sequence

<220>
<223> Klammerstrang_0811 zugehoerig zu 2D-DNA-Nanostruktur.

<400> 812
taggtaaact atttttgaga gatcaaacgt tattcctcta ccacctacat cac 53

<210> 813
<211> 53
<212> DNA
<213> Artificial Sequence

<220>
<223> Klammerstrang_0812 zugehoerig zu 2D-DNA-Nanostruktur.

<400> 813
gagggtagga ttcaaaaggg tgagacatcc aattcctcta ccacctacat cac 53

<210> 814
<211> 53
<212> DNA
<213> Artificial Sequence

<220>
<223> Klammerstrang_0813 zugehoerig zu 2D-DNA-Nanostruktur.

<400> 814
atattttggc tttcatcaac attatccagc cattcctcta ccacctacat cac 53

<210> 815
<211> 53
<212> DNA
<213> Artificial Sequence

<220>
<223> Klammerstrang_0814 zugehoerig zu 2D-DNA-Nanostruktur.

<400> 815
tgtagccatt aaaattcgca ttaaatgccg gattcctcta ccacctacat cac 53

<210> 816
<211> 53
<212> DNA
<213> Artificial Sequence

<220>
<223> Klammerstrang_0815 zugehoerig zu 2D-DNA-Nanostruktur.

<400> 816
gctttccgat tacgccagct ggcggctgtt tcttcctcta ccacctacat cac 53

<210> 817
<211> 53
<212> DNA
<213> Artificial Sequence

<220>
<223> Klammerstrang_0816 zugehoerig zu 2D-DNA-Nanostruktur.

<400> 817
tcttcgctgc accgcttctg gtgcggcctt ccttcctcta ccacctacat cac 53

<210> 818
<211> 53
<212> DNA
<213> Artificial Sequence

<220>
<223> Klammerstrang_0817 zugehoerig zu 2D-DNA-Nanostruktur.

<400> 818

ctgtgtgatt gcgttgcgct cactagagtt gcttcctcta ccacctacat cac 53

<210> 819
<211> 53
<212> DNA
<213> Artificial Sequence

<220>
<223> Klammerstrang_0818 zugehoerig zu 2D-DNA-Nanostruktur.

<400> 819
cacattaaaa ttgttatccg ctcatgcggg ccttcctcta ccacctacat cac 53

<210> 820
<211> 53
<212> DNA
<213> Artificial Sequence

<220>
<223> Klammerstrang_0819 zugehoerig zu 2D-DNA-Nanostruktur.

<400> 820
agcaagcgta gggttgagtg ttgtagggag ccttcctcta ccacctacat cac 53

<210> 821
<211> 53
<212> DNA
<213> Artificial Sequence

<220>
<223> Klammerstrang_0820 zugehoerig zu 2D-DNA-Nanostruktur.

<400> 821
gcccgagagt ccacgctggt ttgcagctaa ctttcctcta ccacctacat cac 53

<210> 822
<211> 53
<212> DNA
<213> Artificial Sequence

<220>
<223> Klammerstrang_0821 zugehoerig zu 2D-DNA-Nanostruktur.

<400> 822
cccgatttag agcttgacgg ggaaaaagaa tattcctcta ccacctacat cac 53

<210> 823
<211> 53
<212> DNA
<213> Artificial Sequence

<220>
<223> Klammerstrang_0822 zugehoerig zu 2D-DNA-Nanostruktur.

<400> 823
ttctactacg cgagctgaaa aggttaccgc gcttcctcta ccacctacat cac 53

<210> 824
<211> 51
<212> DNA
<213> Artificial Sequence

<220>
<223> Klammerstrang_0823 zugehoerig zu 2D-DNA-Nanostruktur.

<400> 824
gagacagcta gctgataaat taatttttgt ttcctctacc acctacatca c 51

<210> 825
<211> 53
<212> DNA
<213> Artificial Sequence

<220>
<223> Klammerstrang_0824 zugehoerig zu 2D-DNA-Nanostruktur.

<400> 825
caaccgtttc aaatcaccat caattcgagc cattcctcta ccacctacat cac 53

<210> 826
<211> 65
<212> DNA
<213> Artificial Sequence

<220>
<223> Klammerstrang_0825 zugehoerig zu 2D-DNA-Nanostruktur.

<400> 826
taaatcaaaa taattcgcgt ctcggaaacc aggcaaaggg aaggttcctc taccacctac 60

atcac 65

<210> 827
<211> 53
<212> DNA
<213> Artificial Sequence

<220>
<223> Klammerstrang_0826 zugehoerig zu 2D-DNA-Nanostruktur.

<400> 827
gccatcaagc tcatttttta accacaaatc cattcctcta ccacctacat cac 53

<210> 828
<211> 53
<212> DNA
<213> Artificial Sequence

<220>
<223> Klammerstrang_0827 zugehoerig zu 2D-DNA-Nanostruktur.

<400> 828
caactgttgc gccattcgcc attcaaacat cattcctcta ccacctacat cac 53

<210> 829
<211> 53
<212> DNA
<213> Artificial Sequence

<220>
<223> Klammerstrang_0828 zugehoerig zu 2D-DNA-Nanostruktur.

<400> 829
aagcctggta cgagccggaa gcatagatga tgttcctcta ccacctacat cac 53

<210> 830
<211> 59
<212> DNA
<213> Artificial Sequence

<220>
<223> Klammerstrang_0829 zugehoerig zu 2D-DNA-Nanostruktur.

<400> 830
cccagcaggc gaaaaatccc ttataaatca agccggcgtt cctctaccac ctacatcac 59

<210> 831
<211> 53
<212> DNA
<213> Artificial Sequence

<220>
<223> Klammerstrang_0830 zugehoerig zu 2D-DNA-Nanostruktur.

<400> 831
tcggcaaatc ctgtttgatg gtggaccctc aattcctcta ccacctacat cac 53

<210> 832
<211> 52
<212> DNA
<213> Artificial Sequence

<220>
<223> Klammerstrang_0831 zugehoerig zu 2D-DNA-Nanostruktur.

<400> 832
aacgtggcga gaaaggaagg gaaaccagta attcctctac cacctacatc ac 52

<210> 833
<211> 51

<212> DNA
<213> Artificial Sequence

<220>
<223> Klammerstrang_0832 zugehoerig zu 2D-DNA-Nanostruktur.

<400> 833
ttttatttaa gcaaatcaga tattttttgt ttcctctacc acctacatca c 51

<210> 834
<211> 53
<212> DNA
<213> Artificial Sequence

<220>
<223> Klammerstrang_0833 zugehoerig zu 2D-DNA-Nanostruktur.

<400> 834
gtaataagtt aggcagaggc atttatgata ttttcctcta ccacctacat cac 53

<210> 835
<211> 51
<212> DNA
<213> Artificial Sequence

<220>
<223> Klammerstrang_0834 zugehoerig zu 2D-DNA-Nanostruktur.

<400> 835
catgtaatag aatataaagt accaagccgt ttcctctacc acctacatca c 51

<210> 836
<211> 53
<212> DNA
<213> Artificial Sequence

<220>
<223> Klammerstrang_0835 zugehoerig zu 2D-DNA-Nanostruktur.

<400> 836
atcgcaagta tgtaaatgct gatgatagga acttcctcta ccacctacat cac 53

<210> 837
<211> 51
<212> DNA
<213> Artificial Sequence

<220>
<223> Klammerstrang_0836 zugehoerig zu 2D-DNA-Nanostruktur.

<400> 837
tataactaac aaagaacgcg agaacgccaa ttcctctacc acctacatca c 51

<210> 838
<211> 53
<212> DNA
<213> Artificial Sequence

<220>
<223> Klammerstrang_0837 zugehoerig zu 2D-DNA-Nanostruktur.

<400> 838
agaaaacaaa gaagatgatg aaacaggctg cgttcctcta ccacctacat cac 53

<210> 839
<211> 51
<212> DNA
<213> Artificial Sequence

<220>
<223> Klammerstrang_0838 zugehoerig zu 2D-DNA-Nanostruktur.

<400> 839
ctgagcaaaa attaattaca ttttgggtta ttcctctacc acctacatca c 51

<210> 840
<211> 53
<212> DNA
<213> Artificial Sequence

<220>
<223> Klammerstrang_0839 zugehoerig zu 2D-DNA-Nanostruktur.

<400> 840
gcaattcaca tattcctgat tatcaaagtg tattcctcta ccacctacat cac 53

<210> 841
<211> 51
<212> DNA
<213> Artificial Sequence

<220>
<223> Klammerstrang_0840 zugehoerig zu 2D-DNA-Nanostruktur.

<400> 841
attatcattc aatataatcc tgacaattac ttcctctacc acctacatca c 51

<210> 842
<211> 53
<212> DNA
<213> Artificial Sequence

<220>
<223> Klammerstrang_0841 zugehoerig zu 2D-DNA-Nanostruktur.

<400> 842
tcaatatcga acctcaaata tcaattccga aattcctcta ccacctacat cac 53

<210> 843
<211> 51
<212> DNA
<213> Artificial Sequence

<220>
<223> Klammerstrang_0842 zugehoerig zu 2D-DNA-Nanostruktur.

<400> 843
acccttgcttg gtcagttggc aaagagcgga ttcctctacc acctacatca c 51

<210> 844
<211> 51
<212> DNA
<213> Artificial Sequence

<220>
<223> Klammerstrang_0843 zugehoerig zu 2D-DNA-Nanostruktur.

<400> 844
taaaagggac attctggcca acaaagcatc ttcctctacc acctacatca c 51

<210> 845
<211> 53
<212> DNA
<213> Artificial Sequence

<220>
<223> Klammerstrang_0844 zugehoerig zu 2D-DNA-Nanostruktur.

<400> 845
gtaccgcaat tctaagaacg cgagtattat ttttcctcta ccacctacat cac 53

<210> 846
<211> 53
<212> DNA
<213> Artificial Sequence

<220>
<223> Klammerstrang_0845 zugehoerig zu 2D-DNA-Nanostruktur.

<400> 846
gtaaagtaat cgccatattt aacaaaactt ttttcctcta ccacctacat cac 53

<210> 847
<211> 53
<212> DNA
<213> Artificial Sequence

<220>
<223> Klammerstrang_0846 zugehoerig zu 2D-DNA-Nanostruktur.

<400> 847
aattgagaat tctgtccaga cgactaaacc aattcctcta ccacctacat cac 53

<210> 848
<211> 53
<212> DNA
<213> Artificial Sequence

<220>
<223> Klammerstrang_0847 zugehoerig zu 2D-DNA-Nanostruktur.

<400> 848
tcaaatataa cctccggctt aggtaacaat ttttcctcta ccacctacat cac 53

<210> 849
<211> 53
<212> DNA
<213> Artificial Sequence

<220>
<223> Klammerstrang_0848 zugehoerig zu 2D-DNA-Nanostruktur.

<400> 849
accttttat tttagttaat ttcatagggc ttttcctcta ccacctacat cac 53

<210> 850
<211> 53
<212> DNA
<213> Artificial Sequence

<220>
<223> Klammerstrang_0849 zugehoerig zu 2D-DNA-Nanostruktur.

<400> 850
catttgaagg cgaattattc atttttgttt ggttcctcta ccacctacat cac 53

<210> 851
<211> 53
<212> DNA
<213> Artificial Sequence

<220>
<223> Klammerstrang_0850 zugehoerig zu 2D-DNA-Nanostruktur.

<400> 851
cgcgcagatt acctttttta atgggagaga ctttcctcta ccacctacat cac 53

<210> 852
<211> 53
<212> DNA
<213> Artificial Sequence

<220>

<223> Klammerstrang_0851 zugehoerig zu 2D-DNA-Nanostruktur.

<400> 852
attatactaa gaaaccacca gaagtcaaca gtttcctcta ccacctacat cac 53

<210> 853
<211> 53
<212> DNA
<213> Artificial Sequence

<220>
<223> Klammerstrang_0852 zugehoerig zu 2D-DNA-Nanostruktur.

<400> 853
gcggaacatc tgaataatgg aaggtacaaa atttcctcta ccacctacat cac 53

<210> 854
<211> 53
<212> DNA
<213> Artificial Sequence

<220>
<223> Klammerstrang_0853 zugehoerig zu 2D-DNA-Nanostruktur.

<400> 854
tgaaaggagc aaatgaaaaa tctagagata gattcctcta ccacctacat cac 53

<210> 855
<211> 53
<212> DNA
<213> Artificial Sequence

<220>
<223> Klammerstrang_0854 zugehoerig zu 2D-DNA-Nanostruktur.

<400> 855
agccagcaat tgaggaaggt tatcatcatt ttttcctcta ccacctacat cac 53

<210> 856
<211> 53
<212> DNA
<213> Artificial Sequence

<220>
<223> Klammerstrang_0855 zugehoerig zu 2D-DNA-Nanostruktur.

<400> 856
acccttctga cctgaaagcg taagacgctg agttcctcta ccacctacat cac 53

<210> 857
<211> 53
<212> DNA
<213> Artificial Sequence

<220>
<223> Klammerstrang_0856 zugehoerig zu 2D-DNA-Nanostruktur.

<400> 857
cttatcattc ccgacttgcg ggagcctaat ttttcctcta ccacctacat cac 53

<210> 858
<211> 51
<212> DNA
<213> Artificial Sequence

<220>
<223> Klammerstrang_0857 zugehoerig zu 2D-DNA-Nanostruktur.

<400> 858
acaacatgcc aacgctcaac agtcttctga ttcctctacc acctacatca c 51

<210> 859
<211> 53
<212> DNA
<213> Artificial Sequence

<220>
<223> Klammerstrang_0858 zugehoerig zu 2D-DNA-Nanostruktur.

<400> 859
agtataaagt tcagctaatg cagatgtctt tcttcctcta ccacctacat cac 53

<210> 860
<211> 51
<212> DNA
<213> Artificial Sequence

<220>
<223> Klammerstrang_0859 zugehoerig zu 2D-DNA-Nanostruktur.

<400> 860
cctaaatcaa aatcataggt ctaaacagta ttcctctacc acctacatca c 51

<210> 861
<211> 53
<212> DNA
<213> Artificial Sequence

<220>
<223> Klammerstrang_0860 zugehoerig zu 2D-DNA-Nanostruktur.

<400> 861
gaatttattt aatggtttga aatattctta ccttcctcta ccacctacat cac 53

<210> 862
<211> 51
<212> DNA

<213> Artificial Sequence

<220>
<223> Klammerstrang_0861 zugehoerig zu 2D-DNA-Nanostruktur.

<400> 862
cataaatctt tgaataccaa gtgttagaac ttcctctacc acctacatca c 51

<210> 863
<211> 53
<212> DNA
<213> Artificial Sequence

<220>
<223> Klammerstrang_0862 zugehoerig zu 2D-DNA-Nanostruktur.

<400> 863
cctgattgca atatatgtga gtgatcaata gtttcctcta ccacctacat cac 53

<210> 864
<211> 51
<212> DNA
<213> Artificial Sequence

<220>
<223> Klammerstrang_0863 zugehoerig zu 2D-DNA-Nanostruktur.

<400> 864
ctaccatagt ttgagtaaca tttaaaatat ttcctctacc acctacatca c 51

<210> 865
<211> 53
<212> DNA
<213> Artificial Sequence

<220>
<223> Klammerstrang_0864 zugehoerig zu 2D-DNA-Nanostruktur.

<400> 865
attttaaaat caaaattatt tgcacggatt cgttcctcta ccacctacat cac 53

<210> 866
<211> 51
<212> DNA
<213> Artificial Sequence

<220>
<223> Klammerstrang_0865 zugehoerig zu 2D-DNA-Nanostruktur.

<400> 866
ctttagggcc tgcaacagtg ccaatacgtg ttcctctacc acctacatca c 51

<210> 867

<211> 53
<212> DNA
<213> Artificial Sequence

<220>
<223> Klammerstrang_0866 zugehoerig zu 2D-DNA-Nanostruktur.

<400> 867
ttaacaccag cactaacaac taatcgttat tattcctcta ccacctacat cac 53

<210> 868
<211> 52
<212> DNA
<213> Artificial Sequence

<220>
<223> Klammerstrang_0867 zugehoerig zu 2D-DNA-Nanostruktur.

<400> 868
gcacagacaa tatttttgaa tggggtcagt attcctctac cacctacatc ac 52

<210> 869
<211> 51
<212> DNA
<213> Artificial Sequence

<220>
<223> Klammerstrang_0868 zugehoerig zu 2D-DNA-Nanostruktur.

<400> 869
tgtagaaatc aagattagtt gctcttacca ttcctctacc acctacatca c 51

<210> 870
<211> 53
<212> DNA
<213> Artificial Sequence

<220>
<223> Klammerstrang_0869 zugehoerig zu 2D-DNA-Nanostruktur.

<400> 870
gtttatcaat atgcgttata caaaccgacc gtttcctcta ccacctacat cac 53

<210> 871
<211> 51
<212> DNA
<213> Artificial Sequence

<220>
<223> Klammerstrang_0870 zugehoerig zu 2D-DNA-Nanostruktur.

<400> 871
ttagtatcac aatagataag tccacgagca ttcctctacc acctacatca c 51

<210> 872
<211> 53
<212> DNA
<213> Artificial Sequence

<220>
<223> Klammerstrang_0871 zugehoerig zu 2D-DNA-Nanostruktur.

<400> 872
gtgataaaaa gacgctgaga agagataacc ttttcctcta ccacctacat cac 53

<210> 873
<211> 51
<212> DNA
<213> Artificial Sequence

<220>
<223> Klammerstrang_0872 zugehoerig zu 2D-DNA-Nanostruktur.

<400> 873
cttagattta aggcgttaaa taaagcctgt ttcctctacc acctacatca c 51

<210> 874
<211> 53
<212> DNA
<213> Artificial Sequence

<220>
<223> Klammerstrang_0873 zugehoerig zu 2D-DNA-Nanostruktur.

<400> 874
gcttctgttc gggagaaaca ataacgtaaa acttcctcta ccacctacat cac 53

<210> 875
<211> 51
<212> DNA
<213> Artificial Sequence

<220>
<223> Klammerstrang_0874 zugehoerig zu 2D-DNA-Nanostruktur.

<400> 875
cttttacaaa atcgtcgcta ttagcgatag ttcctctacc acctacatca c 51

<210> 876
<211> 53
<212> DNA
<213> Artificial Sequence

<220>
<223> Klammerstrang_0875 zugehoerig zu 2D-DNA-Nanostruktur.

<400> 876

agaaataaaa atcctttgcc cgaaagatta gattcctcta ccacctacat cac 53

<210> 877
<211> 51
<212> DNA
<213> Artificial Sequence

<220>
<223> Klammerstrang_0876 zugehoerig zu 2D-DNA-Nanostruktur.

<400> 877
ctcgtattag aaattgcgta gatacagtac ttcctctacc acctacatca c 51

<210> 878
<211> 53
<212> DNA
<213> Artificial Sequence

<220>
<223> Klammerstrang_0877 zugehoerig zu 2D-DNA-Nanostruktur.

<400> 878
gccgtcaaaa aacagaggtg aggcctatta gtttcctcta ccacctacat cac 53

<210> 879
<211> 51
<212> DNA
<213> Artificial Sequence

<220>
<223> Klammerstrang_0878 zugehoerig zu 2D-DNA-Nanostruktur.

<400> 879
cagaagatta gataatacat ttgtcgacaa ttcctctacc acctacatca c 51

<210> 880
<211> 51
<212> DNA
<213> Artificial Sequence

<220>
<223> Klammerstrang_0879 zugehoerig zu 2D-DNA-Nanostruktur.

<400> 880
ctttaatgcg cgaactgata gccccaccag ttcctctacc acctacatca c 51

<210> 881
<211> 52
<212> DNA
<213> Artificial Sequence

<220>
<223> Klammerstrang_0880 zugehoerig zu 2D-DNA-Nanostruktur.

<400> 881
agaaaggaac aactaaagga attcaaaaaa ataacattcc taacttctca ta 52

<210> 882
<211> 51
<212> DNA
<213> Artificial Sequence

<220>
<223> Klammerstrang_0881 zugehoerig zu 2D-DNA-Nanostruktur.

<400> 882
aggctccaga ggctttgagg acacgggtaa taacattcct aacttctcat a 51

<210> 883
<211> 53
<212> DNA
<213> Artificial Sequence

<220>
<223> Klammerstrang_0882 zugehoerig zu 2D-DNA-Nanostruktur.

<400> 883
acggctacaa aaggagcctt taatgtgaga attaacattc ctaacttctc ata 53

<210> 884
<211> 51
<212> DNA
<213> Artificial Sequence

<220>
<223> Klammerstrang_0883 zugehoerig zu 2D-DNA-Nanostruktur.

<400> 884
aatacgtttg aaagaggaca gactgacctt taacattcct aacttctcat a 51

<210> 885
<211> 53
<212> DNA
<213> Artificial Sequence

<220>
<223> Klammerstrang_0884 zugehoerig zu 2D-DNA-Nanostruktur.

<400> 885
gaccaactaa tgccactacg aaggggtag cataacattc ctaacttctc ata 53

<210> 886
<211> 51
<212> DNA
<213> Artificial Sequence

<220>
<223> Klammerstrang_0885 zugehoerig zu 2D-DNA-Nanostruktur.

<400> 886
catcaagtaa aacgaactaa cgagttgaga taacattcct aacttctcat a 51

<210> 887
<211> 53
<212> DNA
<213> Artificial Sequence

<220>
<223> Klammerstrang_0886 zugehoerig zu 2D-DNA-Nanostruktur.

<400> 887
tacgttaaag taatcttgac aagaaccgaa cttaacattc ctaacttctc ata 53

<210> 888
<211> 51
<212> DNA
<213> Artificial Sequence

<220>
<223> Klammerstrang_0887 zugehoerig zu 2D-DNA-Nanostruktur.

<400> 888
tttaggacaa atgctttaaa caatcaggtc taacattcct aacttctcat a 51

<210> 889
<211> 53
<212> DNA
<213> Artificial Sequence

<220>
<223> Klammerstrang_0888 zugehoerig zu 2D-DNA-Nanostruktur.

<400> 889
atccccctat accacattca actagaaaaa tctaacattc ctaacttctc ata 53

<210> 890
<211> 51
<212> DNA
<213> Artificial Sequence

<220>
<223> Klammerstrang_0889 zugehoerig zu 2D-DNA-Nanostruktur.

<400> 890
tttaccccaa catgttttaa atttccatat taacattcct aacttctcat a 51

<210> 891
<211> 53
<212> DNA
<213> Artificial Sequence

<220>
<223> Klammerstrang_0890 zugehoerig zu 2D-DNA-Nanostruktur.

<400> 891
ctgtagcttg actattatag tcagttcatt gataacattc ctaacttctc ata 53

<210> 892
<211> 51
<212> DNA
<213> Artificial Sequence

<220>
<223> Klammerstrang_0891 zugehoerig zu 2D-DNA-Nanostruktur.

<400> 892
aacagttttg taccaaaaac attttatttc taacattcct aacttctcat a 51

<210> 893
<211> 53
<212> DNA
<213> Artificial Sequence

<220>
<223> Klammerstrang_0892 zugehoerig zu 2D-DNA-Nanostruktur.

<400> 893
acaactttca acagtttcag cggatgtatc ggtaacattc ctaacttctc ata 53

<210> 894
<211> 53
<212> DNA
<213> Artificial Sequence

<220>
<223> Klammerstrang_0893 zugehoerig zu 2D-DNA-Nanostruktur.

<400> 894
tttatcagga cagcatcgga acgacaccaa cctaacattc ctaacttctc ata 53

<210> 895
<211> 51
<212> DNA
<213> Artificial Sequence

<220>
<223> Klammerstrang_0894 zugehoerig zu 2D-DNA-Nanostruktur.

<400> 895
cagcgaaact tgctttcgag gtgttgctaa taacattcct aacttctcat a 51

<210> 896
<211> 53

<212> DNA
<213> Artificial Sequence

<220>
<223> Klammerstrang_0895 zugehoerig zu 2D-DNA-Nanostruktur.

<400> 896
taaaacgagg tcaatcataa gggaaccgga tataacattc ctaacttctc ata 53

<210> 897
<211> 51
<212> DNA
<213> Artificial Sequence

<220>
<223> Klammerstrang_0896 zugehoerig zu 2D-DNA-Nanostruktur.

<400> 897
gcgcagacaa gaggcaaaag aatccctcag taacattcct aacttctcat a 51

<210> 898
<211> 53
<212> DNA
<213> Artificial Sequence

<220>
<223> Klammerstrang_0897 zugehoerig zu 2D-DNA-Nanostruktur.

<400> 898
ttcattacgt caggacgttg ggaaatgcag attaacattc ctaacttctc ata 53

<210> 899
<211> 51
<212> DNA
<213> Artificial Sequence

<220>
<223> Klammerstrang_0898 zugehoerig zu 2D-DNA-Nanostruktur.

<400> 899
ttataccacc aaatcaacgt aacgaacgag taacattcct aacttctcat a 51

<210> 900
<211> 53
<212> DNA
<213> Artificial Sequence

<220>
<223> Klammerstrang_0899 zugehoerig zu 2D-DNA-Nanostruktur.

<400> 900
acataacggg aatcgtcata aataaagcaa agtaacattc ctaacttctc ata 53

<210> 901
<211> 51
<212> DNA
<213> Artificial Sequence

<220>
<223> Klammerstrang_0900 zugehoerig zu 2D-DNA-Nanostruktur.

<400> 901
aatactgccc aaaaggaatt acgtggctca taacattcct aacttctcat a 51

<210> 902
<211> 53
<212> DNA
<213> Artificial Sequence

<220>
<223> Klammerstrang_0901 zugehoerig zu 2D-DNA-Nanostruktur.

<400> 902
cggattgcag agcttaattg ctgaaacgag tataacattc ctaacttctc ata 53

<210> 903
<211> 51
<212> DNA
<213> Artificial Sequence

<220>
<223> Klammerstrang_0902 zugehoerig zu 2D-DNA-Nanostruktur.

<400> 903
gatggcttat caaaaagatt aagagcgtcc taacattcct aacttctcat a 51

<210> 904
<211> 53
<212> DNA
<213> Artificial Sequence

<220>
<223> Klammerstrang_0903 zugehoerig zu 2D-DNA-Nanostruktur.

<400> 904
gatttagtca ataaagcctc agagaaccct cataacattc ctaacttctc ata 53

<210> 905
<211> 53
<212> DNA
<213> Artificial Sequence

<220>
<223> Klammerstrang_0904 zugehoerig zu 2D-DNA-Nanostruktur.

<400> 905
taaatgaatt ttctgtatgg gattaatttc tttaacattc ctaacttctc ata 53

<210> 906
<211> 53
<212> DNA
<213> Artificial Sequence

<220>
<223> Klammerstrang_0905 zugehoerig zu 2D-DNA-Nanostruktur.

<400> 906
aaacagcttt ttgcgggatc gtcaacacta aataacattc ctaacttctc ata 53

<210> 907
<211> 53
<212> DNA
<213> Artificial Sequence

<220>
<223> Klammerstrang_0906 zugehoerig zu 2D-DNA-Nanostruktur.

<400> 907
aaggccgctg ataccgatag ttgcgacgtt agtaacattc ctaacttctc ata 53

<210> 908
<211> 53
<212> DNA
<213> Artificial Sequence

<220>
<223> Klammerstrang_0907 zugehoerig zu 2D-DNA-Nanostruktur.

<400> 908
acactcatcc atgttactta gccgaaagct gctaacattc ctaacttctc ata 53

<210> 909
<211> 53
<212> DNA
<213> Artificial Sequence

<220>
<223> Klammerstrang_0908 zugehoerig zu 2D-DNA-Nanostruktur.

<400> 909
gacctgctct ttgacccccca gcgagggagt tataacattc ctaacttctc ata 53

<210> 910
<211> 53
<212> DNA
<213> Artificial Sequence

<220>
<223> Klammerstrang_0909 zugehoerig zu 2D-DNA-Nanostruktur.

<400> 910
tcattcagat gcgattttaa gaacaggcat agtaacattc ctaacttctc ata 53

<210> 911
<211> 53
<212> DNA
<213> Artificial Sequence

<220>
<223> Klammerstrang_0910 zugehoerig zu 2D-DNA-Nanostruktur.

<400> 911
attacctttg aataaggctt gcccaaatcc gctaacattc ctaacttctc ata 53

<210> 912
<211> 53
<212> DNA
<213> Artificial Sequence

<220>
<223> Klammerstrang_0911 zugehoerig zu 2D-DNA-Nanostruktur.

<400> 912
taagagcaaa tgtttagact ggataggaag cctaacattc ctaacttctc ata 53

<210> 913
<211> 53
<212> DNA
<213> Artificial Sequence

<220>
<223> Klammerstrang_0912 zugehoerig zu 2D-DNA-Nanostruktur.

<400> 913
aatagtaaac actatcataa ccctcattgt gataacattc ctaacttctc ata 53

<210> 914
<211> 53
<212> DNA
<213> Artificial Sequence

<220>
<223> Klammerstrang_0913 zugehoerig zu 2D-DNA-Nanostruktur.

<400> 914
cgaaagactt tgataagagg tcatatttcg cataacattc ctaacttctc ata 53

<210> 915
<211> 53
<212> DNA
<213> Artificial Sequence

<220>

<223> Klammerstrang_0914 zugehoerig zu 2D-DNA-Nanostruktur.

<400> 915
ttgctccttt caaatatcgc gtttgagggg gttaacattc ctaacttctc ata 53

<210> 916
<211> 53
<212> DNA
<213> Artificial Sequence

<220>
<223> Klammerstrang_0915 zugehoerig zu 2D-DNA-Nanostruktur.

<400> 916
aatggtcaac aggcaaggca aagagtaatg tgtaacattc ctaacttctc ata 53

<210> 917
<211> 52
<212> DNA
<213> Artificial Sequence

<220>
<223> Klammerstrang_0916 zugehoerig zu 2D-DNA-Nanostruktur.

<400> 917
tctaaagttt tgtcgtcttt ccagccgaca ataacattcc taacttctca ta 52

<210> 918
<211> 65
<212> DNA
<213> Artificial Sequence

<220>
<223> Klammerstrang_0917 zugehoerig zu 2D-DNA-Nanostruktur.

<400> 918
tgacaactcg ctgaggcttg cattatacca agcgcgatga taaataacat tcctaacttc 60

tcata 65

<210> 919
<211> 53
<212> DNA
<213> Artificial Sequence

<220>
<223> Klammerstrang_0918 zugehoerig zu 2D-DNA-Nanostruktur.

<400> 919
atattcggaa ccatcgccca cgcagagaag gataacattc ctaacttctc ata 53

<210> 920
<211> 53
<212> DNA

<213> Artificial Sequence

<220>
<223> Klammerstrang_0919 zugehoerig zu 2D-DNA-Nanostruktur.

<400> 920
tcatcgccaa caaagtacaa cggacgccag cataacattc ctaacttctc ata 53

<210> 921
<211> 53
<212> DNA
<213> Artificial Sequence

<220>
<223> Klammerstrang_0920 zugehoerig zu 2D-DNA-Nanostruktur.

<400> 921
gatggtttga acgagtagta aatttaccat tataacattc ctaacttctc ata 53

<210> 922
<211> 59
<212> DNA
<213> Artificial Sequence

<220>
<223> Klammerstrang_0921 zugehoerig zu 2D-DNA-Nanostruktur.

<400> 922
cgtttaccag acgacaaaga agttttgcca taattcgata acattcctaa cttctcata 59

<210> 923
<211> 53
<212> DNA
<213> Artificial Sequence

<220>
<223> Klammerstrang_0922 zugehoerig zu 2D-DNA-Nanostruktur.

<400> 923
cttttgcaga taaaaaccaa aataaagact cctaacattc ctaacttctc ata 53

<210> 924
<211> 51
<212> DNA
<213> Artificial Sequence

<220>
<223> Klammerstrang_0923 zugehoerig zu 2D-DNA-Nanostruktur.

<400> 924
gcttcaatca ggattagaga gttattttca taacattcct aacttctcat a 51

<210> 925

<211> 53
<212> DNA
<213> Artificial Sequence

<220>
<223> Klammerstrang_0924 zugehoerig zu 2D-DNA-Nanostruktur.

<400> 925
ccaacaggag cgaaccagac cggagccttt actaacattc ctaacttctc ata 53

<210> 926
<211> 51
<212> DNA
<213> Artificial Sequence

<220>
<223> Klammerstrang_0925 zugehoerig zu 2D-DNA-Nanostruktur.

<400> 926
tttggggata gtagtagcat taaaaggccg taacattcct aacttctcat a 51

<210> 927
<211> 53
<212> DNA
<213> Artificial Sequence

<220>
<223> Klammerstrang_0926 zugehoerig zu 2D-DNA-Nanostruktur.

<400> 927
tccacagaca gccctcatag ttagcgtaac gataacattc ctaacttctc ata 53

<210> 928
<211> 53
<212> DNA
<213> Artificial Sequence

<220>
<223> Klammerstrang_0927 zugehoerig zu 2D-DNA-Nanostruktur.

<400> 928
ttaggattgg ctgagactcc tcaataaccg attaacattc ctaacttctc ata 53

<210> 929
<211> 51
<212> DNA
<213> Artificial Sequence

<220>
<223> Klammerstrang_0928 zugehoerig zu 2D-DNA-Nanostruktur.

<400> 929
tattaagaag cggggttttg ctcgtagcat taacattcct aacttctcat a 51

<210> 930
<211> 53
<212> DNA
<213> Artificial Sequence

<220>
<223> Klammerstrang_0929 zugehoerig zu 2D-DNA-Nanostruktur.

<400> 930
ttgacaggcc accaccagag ccgcgatttg tataacattc ctaacttctc ata 53

<210> 931
<211> 51
<212> DNA
<213> Artificial Sequence

<220>
<223> Klammerstrang_0930 zugehoerig zu 2D-DNA-Nanostruktur.

<400> 931
caccagaaag gttgaggcag gtcatgaaag taacattcct aacttctcat a 51

<210> 932
<211> 53
<212> DNA
<213> Artificial Sequence

<220>
<223> Klammerstrang_0931 zugehoerig zu 2D-DNA-Nanostruktur.

<400> 932
gcaaggcctc accagtagca ccatgggctt gataacattc ctaacttctc ata 53

<210> 933
<211> 51
<212> DNA
<213> Artificial Sequence

<220>
<223> Klammerstrang_0932 zugehoerig zu 2D-DNA-Nanostruktur.

<400> 933
cagcaaaagg aaacgtcacc aatgagccgc taacattcct aacttctcat a 51

<210> 934
<211> 53
<212> DNA
<213> Artificial Sequence

<220>
<223> Klammerstrang_0933 zugehoerig zu 2D-DNA-Nanostruktur.

<400> 934

ttattacgaa gaactggcat gattgcgaga ggtaacattc ctaacttctc ata 53

<210> 935
<211> 51
<212> DNA
<213> Artificial Sequence

<220>
<223> Klammerstrang_0934 zugehoerig zu 2D-DNA-Nanostruktur.

<400> 935
atacccaaca gtatgttagc aaattagagc taacattcct aacttctcat a 51

<210> 936
<211> 53
<212> DNA
<213> Artificial Sequence

<220>
<223> Klammerstrang_0935 zugehoerig zu 2D-DNA-Nanostruktur.

<400> 936
agagagaaaa aaatgaaaat agcaagcaaa cttaacattc ctaacttctc ata 53

<210> 937
<211> 51
<212> DNA
<213> Artificial Sequence

<220>
<223> Klammerstrang_0936 zugehoerig zu 2D-DNA-Nanostruktur.

<400> 937
ttaacgtcta acataaaaac aggtaacgga taacattcct aacttctcat a 51

<210> 938
<211> 53
<212> DNA
<213> Artificial Sequence

<220>
<223> Klammerstrang_0937 zugehoerig zu 2D-DNA-Nanostruktur.

<400> 938
ccaatagctc atcgtaggaa tcatggcatc aataacattc ctaacttctc ata 53

<210> 939
<211> 53
<212> DNA
<213> Artificial Sequence

<220>
<223> Klammerstrang_0938 zugehoerig zu 2D-DNA-Nanostruktur.

<400> 939
tcaccagtac aaactacaac gcctagtacc agtaacattc ctaacttctc ata 53

<210> 940
<211> 53
<212> DNA
<213> Artificial Sequence

<220>
<223> Klammerstrang_0939 zugehoerig zu 2D-DNA-Nanostruktur.

<400> 940
gcggataacc tattattctg aaacagacga tttaacattc ctaacttctc ata 53

<210> 941
<211> 53
<212> DNA
<213> Artificial Sequence

<220>
<223> Klammerstrang_0940 zugehoerig zu 2D-DNA-Nanostruktur.

<400> 941
tttcggaagt gccgtcgaga gggtgagttt cgtaacattc ctaacttctc ata 53

<210> 942
<211> 53
<212> DNA
<213> Artificial Sequence

<220>
<223> Klammerstrang_0941 zugehoerig zu 2D-DNA-Nanostruktur.

<400> 942
ggccttgaag agccaccacc ctcagaaacc attaacattc ctaacttctc ata 53

<210> 943
<211> 53
<212> DNA
<213> Artificial Sequence

<220>
<223> Klammerstrang_0942 zugehoerig zu 2D-DNA-Nanostruktur.

<400> 943
ccaccctcta ttcacaaaca aatacctgcc tataacattc ctaacttctc ata 53

<210> 944
<211> 53
<212> DNA
<213> Artificial Sequence

<220>
<223> Klammerstrang_0943 zugehoerig zu 2D-DNA-Nanostruktur.

<400> 944
cgatagcatt gagccatttg ggaacgtaga aataacattc ctaacttctc ata 53

<210> 945
<211> 53
<212> DNA
<213> Artificial Sequence

<220>
<223> Klammerstrang_0944 zugehoerig zu 2D-DNA-Nanostruktur.

<400> 945
tcaccgacgc accgtaatca gtagcagaac cgtaacattc ctaacttctc ata 53

<210> 946
<211> 53
<212> DNA
<213> Artificial Sequence

<220>
<223> Klammerstrang_0945 zugehoerig zu 2D-DNA-Nanostruktur.

<400> 946
atacataccg aggaaacgca ataagaagcg cataacattc ctaacttctc ata 53

<210> 947
<211> 53
<212> DNA
<213> Artificial Sequence

<220>
<223> Klammerstrang_0946 zugehoerig zu 2D-DNA-Nanostruktur.

<400> 947
aaggaaacat aaaggtggca acattatcac cgtaacattc ctaacttctc ata 53

<210> 948
<211> 53
<212> DNA
<213> Artificial Sequence

<220>
<223> Klammerstrang_0947 zugehoerig zu 2D-DNA-Nanostruktur.

<400> 948
ttagacggcc aaataagaaa cgatagaagg cttaacattc ctaacttctc ata 53

<210> 949
<211> 53
<212> DNA
<213> Artificial Sequence

<220>
<223> Klammerstrang_0948 zugehoerig zu 2D-DNA-Nanostruktur.

<400> 949
atcccaatga gaattaactg aacagttacc agtaacattc ctaacttctc ata 53

<210> 950
<211> 53
<212> DNA
<213> Artificial Sequence

<220>
<223> Klammerstrang_0949 zugehoerig zu 2D-DNA-Nanostruktur.

<400> 950
tatccggtct catcgagaac aagcgacaaa agtaacattc ctaacttctc ata 53

<210> 951
<211> 52
<212> DNA
<213> Artificial Sequence

<220>
<223> Klammerstrang_0950 zugehoerig zu 2D-DNA-Nanostruktur.

<400> 951
aggaacccat gtaccgtaac acttgatata ataacattcc taacttctca ta 52

<210> 952
<211> 51
<212> DNA
<213> Artificial Sequence

<220>
<223> Klammerstrang_0951 zugehoerig zu 2D-DNA-Nanostruktur.

<400> 952
gtatagcaaa cagttaatgc ccaatcctca taacattcct aacttctcat a 51

<210> 953
<211> 53
<212> DNA
<213> Artificial Sequence

<220>
<223> Klammerstrang_0952 zugehoerig zu 2D-DNA-Nanostruktur.

<400> 953
gcccgtatcc ggaataggtg tatcagccca attaacattc ctaacttctc ata 53

<210> 954
<211> 51

<212> DNA
<213> Artificial Sequence

<220>
<223> Klammerstrang_0953 zugehoerig zu 2D-DNA-Nanostruktur.

<400> 954
ttaaagccag agccgccacc ctcgacagaa taacattcct aacttctcat a 51

<210> 955
<211> 53
<212> DNA
<213> Artificial Sequence

<220>
<223> Klammerstrang_0954 zugehoerig zu 2D-DNA-Nanostruktur.

<400> 955
gcctccctca gaatggaaag cgcagtaaca gttaacattc ctaacttctc ata 53

<210> 956
<211> 51
<212> DNA
<213> Artificial Sequence

<220>
<223> Klammerstrang_0955 zugehoerig zu 2D-DNA-Nanostruktur.

<400> 956
tcaagtttca ttaaaggtga atataaaaga taacattcct aacttctcat a 51

<210> 957
<211> 53
<212> DNA
<213> Artificial Sequence

<220>
<223> Klammerstrang_0956 zugehoerig zu 2D-DNA-Nanostruktur.

<400> 957
gaaattattg cctttagcgt cagaccggaa cctaacattc ctaacttctc ata 53

<210> 958
<211> 51
<212> DNA
<213> Artificial Sequence

<220>
<223> Klammerstrang_0957 zugehoerig zu 2D-DNA-Nanostruktur.

<400> 958
aacgcaaaga tagccgaaca aaccctgaac taacattcct aacttctcat a 51

<210> 959
<211> 53
<212> DNA
<213> Artificial Sequence

<220>
<223> Klammerstrang_0958 zugehoerig zu 2D-DNA-Nanostruktur.

<400> 959
aagtaagcag acaccacgga ataatattga cgtaacattc ctaacttctc ata 53

<210> 960
<211> 51
<212> DNA
<213> Artificial Sequence

<220>
<223> Klammerstrang_0959 zugehoerig zu 2D-DNA-Nanostruktur.

<400> 960
aaagtcacaa aataaacagc cagcgtttta taacattcct aacttctcat a 51

<210> 961
<211> 53
<212> DNA
<213> Artificial Sequence

<220>
<223> Klammerstrang_0960 zugehoerig zu 2D-DNA-Nanostruktur.

<400> 961
gccagttaga gggtaattga gcgctttaag aataacattc ctaacttctc ata 53

<210> 962
<211> 51
<212> DNA
<213> Artificial Sequence

<220>
<223> Klammerstrang_0961 zugehoerig zu 2D-DNA-Nanostruktur.

<400> 962
gcgaacctcc aagaacgggt atgacaataa taacattcct aacttctcat a 51

<210> 963
<211> 53
<212> DNA
<213> Artificial Sequence

<220>
<223> Klammerstrang_0962 zugehoerig zu 2D-DNA-Nanostruktur.

<400> 963
ccaccctcat tttcagggat agcaaccgta cttaacattc ctaacttctc ata 53

<210> 964
<211> 53
<212> DNA
<213> Artificial Sequence

<220>
<223> Klammerstrang_0963 zugehoerig zu 2D-DNA-Nanostruktur.

<400> 964
caggaggtgg ggtcagtgcc ttgagtctct gataacattc ctaacttctc ata 53

<210> 965
<211> 51
<212> DNA
<213> Artificial Sequence

<220>
<223> Klammerstrang_0964 zugehoerig zu 2D-DNA-Nanostruktur.

<400> 965
gttttaactt agtaccgcca cccagagcca taacattcct aacttctcat a 51

<210> 966
<211> 53
<212> DNA
<213> Artificial Sequence

<220>
<223> Klammerstrang_0965 zugehoerig zu 2D-DNA-Nanostruktur.

<400> 966
atttaccggg aaccagagcc accactgtag cgtaacattc ctaacttctc ata 53

<210> 967
<211> 51
<212> DNA
<213> Artificial Sequence

<220>
<223> Klammerstrang_0966 zugehoerig zu 2D-DNA-Nanostruktur.

<400> 967
aaatcacctt ccagtaagcg tcagtaataa taacattcct aacttctcat a 51

<210> 968
<211> 53
<212> DNA
<213> Artificial Sequence

<220>
<223> Klammerstrang_0967 zugehoerig zu 2D-DNA-Nanostruktur.

<400> 968
cgttttcaag ggagggaagg taaagtttat tttaacattc ctaacttctc ata 53

<210> 969
<211> 51
<212> DNA
<213> Artificial Sequence

<220>
<223> Klammerstrang_0968 zugehoerig zu 2D-DNA-Nanostruktur.

<400> 969
accgattgtc ggcattttcg gtcataatca taacattcct aacttctcat a 51

<210> 970
<211> 53
<212> DNA
<213> Artificial Sequence

<220>
<223> Klammerstrang_0969 zugehoerig zu 2D-DNA-Nanostruktur.

<400> 970
tgtcacaatc ttaccgaagc cctttaatat cataacattc ctaacttctc ata 53

<210> 971
<211> 51
<212> DNA
<213> Artificial Sequence

<220>
<223> Klammerstrang_0970 zugehoerig zu 2D-DNA-Nanostruktur.

<400> 971
aatagctatc aatagaaaat tcaacattca taacattcct aacttctcat a 51

<210> 972
<211> 53
<212> DNA
<213> Artificial Sequence

<220>
<223> Klammerstrang_0971 zugehoerig zu 2D-DNA-Nanostruktur.

<400> 972
gagagataga gcgtctttcc agaggttttg aataacattc ctaacttctc ata 53

<210> 973
<211> 51
<212> DNA
<213> Artificial Sequence

<220>

<223> Klammerstrang_0972 zugehoerig zu 2D-DNA-Nanostruktur.

<400> 973
acgctaacac ccacaagaat tgaaaatagc taacattcct aacttctcat a 51

<210> 974
<211> 53
<212> DNA
<213> Artificial Sequence

<220>
<223> Klammerstrang_0973 zugehoerig zu 2D-DNA-Nanostruktur.

<400> 974
gccttaaacc aatcaataat cggcacgcgc cttaacattc ctaacttctc ata 53

<210> 975
<211> 53
<212> DNA
<213> Artificial Sequence

<220>
<223> Klammerstrang_0974 zugehoerig zu 2D-DNA-Nanostruktur.

<400> 975
taaatcggga ttcccaattc tgcgatataa tgtaacattc ctaacttctc ata 53

<210> 976
<211> 51
<212> DNA
<213> Artificial Sequence

<220>
<223> Klammerstrang_0975 zugehoerig zu 2D-DNA-Nanostruktur.

<400> 976
aacgcaaaat cgatgaacgg taccggttga taacattcct aacttctcat a 51

<210> 977
<211> 53
<212> DNA
<213> Artificial Sequence

<220>
<223> Klammerstrang_0976 zugehoerig zu 2D-DNA-Nanostruktur.

<400> 977
aacaagaggg ataaaaattt ttagcataaa gctaacattc ctaacttctc ata 53

<210> 978
<211> 51
<212> DNA
<213> Artificial Sequence

<220>
<223> Klammerstrang_0977 zugehoerig zu 2D-DNA-Nanostruktur.

<400> 978
taatcagcgg attgaccgta atcgtaaccg taacattcct aacttctcat a 51

<210> 979
<211> 53
<212> DNA
<213> Artificial Sequence

<220>
<223> Klammerstrang_0978 zugehoerig zu 2D-DNA-Nanostruktur.

<400> 979
acaaacggaa aagccccaaa aacactggag cataacattc ctaacttctc ata 53

<210> 980
<211> 51
<212> DNA
<213> Artificial Sequence

<220>
<223> Klammerstrang_0979 zugehoerig zu 2D-DNA-Nanostruktur.

<400> 980
tgcatctttc ccagtcacga cggcctgcag taacattcct aacttctcat a 51

<210> 981
<211> 53
<212> DNA
<213> Artificial Sequence

<220>
<223> Klammerstrang_0980 zugehoerig zu 2D-DNA-Nanostruktur.

<400> 981
ccagggttgc cagtttgagg ggacccgtgg gataacattc ctaacttctc ata 53

<210> 982
<211> 51
<212> DNA
<213> Artificial Sequence

<220>
<223> Klammerstrang_0981 zugehoerig zu 2D-DNA-Nanostruktur.

<400> 982
gtcgacttcg gccaacgcgc ggggtttttc taacattcct aacttctcat a 51

<210> 983
<211> 53
<212> DNA

<213> Artificial Sequence

<220>
<223> Klammerstrang_0982 zugehoerig zu 2D-DNA-Nanostruktur.

<400> 983
ttaatgaact agaggatccc cggggggtaa cgtaacattc ctaacttctc ata 53

<210> 984
<211> 51
<212> DNA
<213> Artificial Sequence

<220>
<223> Klammerstrang_0983 zugehoerig zu 2D-DNA-Nanostruktur.

<400> 984
ttttcactca aagggcgaaa aaccatcacc taacattcct aacttctcat a 51

<210> 985
<211> 53
<212> DNA
<213> Artificial Sequence

<220>
<223> Klammerstrang_0984 zugehoerig zu 2D-DNA-Nanostruktur.

<400> 985
ctccaacgca gtgagacggg caaccagctg cataacattc ctaacttctc ata 53

<210> 986
<211> 52
<212> DNA
<213> Artificial Sequence

<220>
<223> Klammerstrang_0985 zugehoerig zu 2D-DNA-Nanostruktur.

<400> 986
caaatcaagt tttttggggt cgaaacgtgg ataacattcc taacttctca ta 52

<210> 987
<211> 51
<212> DNA
<213> Artificial Sequence

<220>
<223> Klammerstrang_0986 zugehoerig zu 2D-DNA-Nanostruktur.

<400> 987
aaattaagtt gaccattaga tacttttgcg taacattcct aacttctcat a 51

<210> 988

<211> 53
<212> DNA
<213> Artificial Sequence

<220>
<223> Klammerstrang_0987 zugehoerig zu 2D-DNA-Nanostruktur.

<400> 988
tatattttgt cattgcctga gagtggaaga tttaacattc ctaacttctc ata 53

<210> 989
<211> 51
<212> DNA
<213> Artificial Sequence

<220>
<223> Klammerstrang_0988 zugehoerig zu 2D-DNA-Nanostruktur.

<400> 989
gctatcagaa atgcaatgcc tgaattagca taacattcct aacttctcat a 51

<210> 990
<211> 53
<212> DNA
<213> Artificial Sequence

<220>
<223> Klammerstrang_0989 zugehoerig zu 2D-DNA-Nanostruktur.

<400> 990
gtataagcca acccgtcgga ttctgacgac agtaacattc ctaacttctc ata 53

<210> 991
<211> 51
<212> DNA
<213> Artificial Sequence

<220>
<223> Klammerstrang_0990 zugehoerig zu 2D-DNA-Nanostruktur.

<400> 991
gcgagtaaaa atatttaaat tgttacaaag taacattcct aacttctcat a 51

<210> 992
<211> 53
<212> DNA
<213> Artificial Sequence

<220>
<223> Klammerstrang_0991 zugehoerig zu 2D-DNA-Nanostruktur.

<400> 992
tatcggccgc aaggcgatta agtttaccga gctaacattc ctaacttctc ata 53

<210> 993
<211> 51
<212> DNA
<213> Artificial Sequence

<220>
<223> Klammerstrang_0992 zugehoerig zu 2D-DNA-Nanostruktur.

<400> 993
gatgtgcttc aggaagatcg cacaatgtga taacattcct aacttctcat a 51

<210> 994
<211> 53
<212> DNA
<213> Artificial Sequence

<220>
<223> Klammerstrang_0993 zugehoerig zu 2D-DNA-Nanostruktur.

<400> 994
tcgaattcgg gaaacctgtc gtgcagctga tttaacattc ctaacttctc ata 53

<210> 995
<211> 51
<212> DNA
<213> Artificial Sequence

<220>
<223> Klammerstrang_0994 zugehoerig zu 2D-DNA-Nanostruktur.

<400> 995
ttccagtcgt aatcatggtc ataaaagggg taacattcct aacttctcat a 51

<210> 996
<211> 53
<212> DNA
<213> Artificial Sequence

<220>
<223> Klammerstrang_0995 zugehoerig zu 2D-DNA-Nanostruktur.

<400> 996
gcccttcaga gtccactatt aaagggtgcc gttaacattc ctaacttctc ata 53

<210> 997
<211> 51
<212> DNA
<213> Artificial Sequence

<220>
<223> Klammerstrang_0996 zugehoerig zu 2D-DNA-Nanostruktur.

<400> 997

tggaacaacc gcctggccct gaggcccgct taacattcct aacttctcat a 51

<210> 998
<211> 51
<212> DNA
<213> Artificial Sequence

<220>
<223> Klammerstrang_0997 zugehoerig zu 2D-DNA-Nanostruktur.

<400> 998
aaagcactaa atcggaaccc taatccagtt taacattcct aacttctcat a 51

<210> 999
<211> 53
<212> DNA
<213> Artificial Sequence

<220>
<223> Klammerstrang_0998 zugehoerig zu 2D-DNA-Nanostruktur.

<400> 999
taaatcatat aacctgttta gctaaccttt aataacattc ctaacttctc ata 53

<210> 1000
<211> 53
<212> DNA
<213> Artificial Sequence

<220>
<223> Klammerstrang_0999 zugehoerig zu 2D-DNA-Nanostruktur.

<400> 1000
taggtaaact atttttgaga gatcaaacgt tataacattc ctaacttctc ata 53

<210> 1001
<211> 53
<212> DNA
<213> Artificial Sequence

<220>
<223> Klammerstrang_1000 zugehoerig zu 2D-DNA-Nanostruktur.

<400> 1001
gagggtagga ttcaaaaggg tgagacatcc aataacattc ctaacttctc ata 53

<210> 1002
<211> 53
<212> DNA
<213> Artificial Sequence

<220>
<223> Klammerstrang_1001 zugehoerig zu 2D-DNA-Nanostruktur.

<400> 1002
atattttggc tttcatcaac attatccagc cataacattc ctaacttctc ata 53

<210> 1003
<211> 53
<212> DNA
<213> Artificial Sequence

<220>
<223> Klammerstrang_1002 zugehoerig zu 2D-DNA-Nanostruktur.

<400> 1003
tgtagccatt aaaattcgca ttaaatgccg gataacattc ctaacttctc ata 53

<210> 1004
<211> 53
<212> DNA
<213> Artificial Sequence

<220>
<223> Klammerstrang_1003 zugehoerig zu 2D-DNA-Nanostruktur.

<400> 1004
gctttccgat tacgccagct ggcggctgtt tctaacattc ctaacttctc ata 53

<210> 1005
<211> 53
<212> DNA
<213> Artificial Sequence

<220>
<223> Klammerstrang_1004 zugehoerig zu 2D-DNA-Nanostruktur.

<400> 1005
tcttcgctgc accgcttctg gtgcggcctt cctaacattc ctaacttctc ata 53

<210> 1006
<211> 53
<212> DNA
<213> Artificial Sequence

<220>
<223> Klammerstrang_1005 zugehoerig zu 2D-DNA-Nanostruktur.

<400> 1006
ctgtgtgatt gcgttgcgct cactagagtt gctaacattc ctaacttctc ata 53

<210> 1007
<211> 53
<212> DNA
<213> Artificial Sequence

<220>
<223> Klammerstrang_1006 zugehoerig zu 2D-DNA-Nanostruktur.

<400> 1007
cacattaaaa ttgttatccg ctcatgcggg cctaacattc ctaacttctc ata 53

<210> 1008
<211> 53
<212> DNA
<213> Artificial Sequence

<220>
<223> Klammerstrang_1007 zugehoerig zu 2D-DNA-Nanostruktur.

<400> 1008
agcaagcgta gggttgagtg ttgtagggag cctaacattc ctaacttctc ata 53

<210> 1009
<211> 53
<212> DNA
<213> Artificial Sequence

<220>
<223> Klammerstrang_1008 zugehoerig zu 2D-DNA-Nanostruktur.

<400> 1009
gcccgagagt ccacgctggt ttgcagctaa cttaacattc ctaacttctc ata 53

<210> 1010
<211> 53
<212> DNA
<213> Artificial Sequence

<220>
<223> Klammerstrang_1009 zugehoerig zu 2D-DNA-Nanostruktur.

<400> 1010
cccgatttag agcttgacgg ggaaaaagaa tataacattc ctaacttctc ata 53

<210> 1011
<211> 53
<212> DNA
<213> Artificial Sequence

<220>
<223> Klammerstrang_1010 zugehoerig zu 2D-DNA-Nanostruktur.

<400> 1011
ttctactacg cgagctgaaa aggttaccgc gctaacattc ctaacttctc ata 53

<210> 1012
<211> 51
<212> DNA
<213> Artificial Sequence

<220>
<223> Klammerstrang_1011 zugehoerig zu 2D-DNA-Nanostruktur.

<400> 1012
gagacagcta gctgataaat taatttttgt taacattcct aacttctcat a 51

<210> 1013
<211> 53
<212> DNA
<213> Artificial Sequence

<220>
<223> Klammerstrang_1012 zugehoerig zu 2D-DNA-Nanostruktur.

<400> 1013
caaccgtttc aaatcaccat caattcgagc cataacattc ctaacttctc ata 53

<210> 1014
<211> 65
<212> DNA
<213> Artificial Sequence

<220>
<223> Klammerstrang_1013 zugehoerig zu 2D-DNA-Nanostruktur.

<400> 1014
taaatcaaaa taattcgcgt ctcggaaacc aggcaaaggg aaggtaacat tcctaacttc 60

tcata 65

<210> 1015
<211> 53
<212> DNA
<213> Artificial Sequence

<220>
<223> Klammerstrang_1014 zugehoerig zu 2D-DNA-Nanostruktur.

<400> 1015
gccatcaagc tcatttttta accacaaatc cataacattc ctaacttctc ata 53

<210> 1016
<211> 53
<212> DNA
<213> Artificial Sequence

<220>
<223> Klammerstrang_1015 zugehoerig zu 2D-DNA-Nanostruktur.

<400> 1016
caactgttgc gccattcgcc attcaaacat cataacattc ctaacttctc ata 53

<210> 1017
<211> 53
<212> DNA
<213> Artificial Sequence

<220>
<223> Klammerstrang_1016 zugehoerig zu 2D-DNA-Nanostruktur.

<400> 1017
aagcctggta cgagccggaa gcatagatga tgtaacattc ctaacttctc ata 53

<210> 1018
<211> 59
<212> DNA
<213> Artificial Sequence

<220>
<223> Klammerstrang_1017 zugehoerig zu 2D-DNA-Nanostruktur.

<400> 1018
cccagcaggc gaaaaatccc ttataaatca agccggcgta acattcctaa cttctcata 59

<210> 1019
<211> 53
<212> DNA
<213> Artificial Sequence

<220>
<223> Klammerstrang_1018 zugehoerig zu 2D-DNA-Nanostruktur.

<400> 1019
tcggcaaatc ctgtttgatg gtggaccctc aataacattc ctaacttctc ata 53

<210> 1020
<211> 52
<212> DNA
<213> Artificial Sequence

<220>
<223> Klammerstrang_1019 zugehoerig zu 2D-DNA-Nanostruktur.

<400> 1020
aacgtggcga gaaaggaagg gaaaccagta ataacattcc taacttctca ta 52

<210> 1021
<211> 51
<212> DNA
<213> Artificial Sequence

<220>
<223> Klammerstrang_1020 zugehoerig zu 2D-DNA-Nanostruktur.

<400> 1021
ttttatttaa gcaaatcaga tattttttgt taacattcct aacttctcat a 51

<210> 1022
<211> 53
<212> DNA
<213> Artificial Sequence

<220>
<223> Klammerstrang_1021 zugehoerig zu 2D-DNA-Nanostruktur.

<400> 1022
gtaataagtt aggcagaggc atttatgata tttaacattc ctaacttctc ata 53

<210> 1023
<211> 51
<212> DNA
<213> Artificial Sequence

<220>
<223> Klammerstrang_1022 zugehoerig zu 2D-DNA-Nanostruktur.

<400> 1023
catgtaatag aatataaagt accaagccgt taacattcct aacttctcat a 51

<210> 1024
<211> 53
<212> DNA
<213> Artificial Sequence

<220>
<223> Klammerstrang_1023 zugehoerig zu 2D-DNA-Nanostruktur.

<400> 1024
atcgcaagta tgtaaatgct gatgatagga actaacattc ctaacttctc ata 53

<210> 1025
<211> 51
<212> DNA
<213> Artificial Sequence

<220>
<223> Klammerstrang_1024 zugehoerig zu 2D-DNA-Nanostruktur.

<400> 1025
tataactaac aaagaacgcg agaacgccaa taacattcct aacttctcat a 51

<210> 1026
<211> 53
<212> DNA
<213> Artificial Sequence

<220>
<223> Klammerstrang_1025 zugehoerig zu 2D-DNA-Nanostruktur.

<400> 1026
agaaaacaaa gaagatgatg aaacaggctg cgtaacattc ctaacttctc ata 53

<210> 1027
<211> 51
<212> DNA
<213> Artificial Sequence

<220>
<223> Klammerstrang_1026 zugehoerig zu 2D-DNA-Nanostruktur.

<400> 1027
ctgagcaaaa attaattaca ttttgggtta taacattcct aacttctcat a 51

<210> 1028
<211> 53
<212> DNA
<213> Artificial Sequence

<220>
<223> Klammerstrang_1027 zugehoerig zu 2D-DNA-Nanostruktur.

<400> 1028
gcaattcaca tattcctgat tatcaaagtg tataacattc ctaacttctc ata 53

<210> 1029
<211> 51
<212> DNA
<213> Artificial Sequence

<220>
<223> Klammerstrang_1028 zugehoerig zu 2D-DNA-Nanostruktur.

<400> 1029
attatcattc aatataatcc tgacaattac taacattcct aacttctcat a 51

<210> 1030
<211> 53
<212> DNA
<213> Artificial Sequence

<220>
<223> Klammerstrang_1029 zugehoerig zu 2D-DNA-Nanostruktur.

<400> 1030
tcaatatcga acctcaaata tcaattccga aataacattc ctaacttctc ata 53

<210> 1031
<211> 51
<212> DNA
<213> Artificial Sequence

<220>

<223> Klammerstrang_1030 zugehoerig zu 2D-DNA-Nanostruktur.

<400> 1031
acttgcttg gtcagttggc aaagagcgga taacattcct aacttctcat a 51

<210> 1032
<211> 51
<212> DNA
<213> Artificial Sequence

<220>
<223> Klammerstrang_1031 zugehoerig zu 2D-DNA-Nanostruktur.

<400> 1032
taaaagggac attctggcca acaaagcatc taacattcct aacttctcat a 51

<210> 1033
<211> 53
<212> DNA
<213> Artificial Sequence

<220>
<223> Klammerstrang_1032 zugehoerig zu 2D-DNA-Nanostruktur.

<400> 1033
gtaccgcaat tctaagaacg cgagtattat tttaacattc ctaacttctc ata 53

<210> 1034
<211> 53
<212> DNA
<213> Artificial Sequence

<220>
<223> Klammerstrang_1033 zugehoerig zu 2D-DNA-Nanostruktur.

<400> 1034
gtaaagtaat cgccatattt aacaaaactt tttaacattc ctaacttctc ata 53

<210> 1035
<211> 53
<212> DNA
<213> Artificial Sequence

<220>
<223> Klammerstrang_1034 zugehoerig zu 2D-DNA-Nanostruktur.

<400> 1035
aattgagaat tctgtccaga cgactaaacc aataacattc ctaacttctc ata 53

<210> 1036
<211> 53
<212> DNA
<213> Artificial Sequence

<220>
<223> Klammerstrang_1035 zugehoerig zu 2D-DNA-Nanostruktur.

<400> 1036
tcaaatataa cctccggctt aggtaacaat tttaacattc ctaacttctc ata 53

<210> 1037
<211> 53
<212> DNA
<213> Artificial Sequence

<220>
<223> Klammerstrang_1036 zugehoerig zu 2D-DNA-Nanostruktur.

<400> 1037
accttttat tttagttaat ttcatagggc tttaacattc ctaacttctc ata 53

<210> 1038
<211> 53
<212> DNA
<213> Artificial Sequence

<220>
<223> Klammerstrang_1037 zugehoerig zu 2D-DNA-Nanostruktur.

<400> 1038
catttgaagg cgaattattc atttttgttt ggtaacattc ctaacttctc ata 53

<210> 1039
<211> 53
<212> DNA
<213> Artificial Sequence

<220>
<223> Klammerstrang_1038 zugehoerig zu 2D-DNA-Nanostruktur.

<400> 1039
cgcgcagatt accttttta atgggagaga cttaacattc ctaacttctc ata 53

<210> 1040
<211> 53
<212> DNA
<213> Artificial Sequence

<220>
<223> Klammerstrang_1039 zugehoerig zu 2D-DNA-Nanostruktur.

<400> 1040
attatactaa gaaaccacca gaagtcaaca gttaacattc ctaacttctc ata 53

<210> 1041
<211> 53
<212> DNA

<213> Artificial Sequence

<220>
<223> Klammerstrang_1040 zugehoerig zu 2D-DNA-Nanostruktur.

<400> 1041
gcggaacatc tgaataatgg aaggtacaaa attaacattc ctaacttctc ata 53

<210> 1042
<211> 53
<212> DNA
<213> Artificial Sequence

<220>
<223> Klammerstrang_1041 zugehoerig zu 2D-DNA-Nanostruktur.

<400> 1042
tgaaaggagc aaatgaaaaa tctagagata gataacattc ctaacttctc ata 53

<210> 1043
<211> 53
<212> DNA
<213> Artificial Sequence

<220>
<223> Klammerstrang_1042 zugehoerig zu 2D-DNA-Nanostruktur.

<400> 1043
agccagcaat tgaggaaggt tatcatcatt tttaacattc ctaacttctc ata 53

<210> 1044
<211> 53
<212> DNA
<213> Artificial Sequence

<220>
<223> Klammerstrang_1043 zugehoerig zu 2D-DNA-Nanostruktur.

<400> 1044
acccttctga cctgaaagcg taagacgctg agtaacattc ctaacttctc ata 53

<210> 1045
<211> 53
<212> DNA
<213> Artificial Sequence

<220>
<223> Klammerstrang_1044 zugehoerig zu 2D-DNA-Nanostruktur.

<400> 1045
cttatcattc ccgacttgcg ggagcctaat tttaacattc ctaacttctc ata 53

<210> 1046

<211> 51
<212> DNA
<213> Artificial Sequence

<220>
<223> Klammerstrang_1045 zugehoerig zu 2D-DNA-Nanostruktur.

<400> 1046
acaacatgcc aacgctcaac agtcttctga taacattcct aacttctcat a 51

<210> 1047
<211> 53
<212> DNA
<213> Artificial Sequence

<220>
<223> Klammerstrang_1046 zugehoerig zu 2D-DNA-Nanostruktur.

<400> 1047
agtataaagt tcagctaatg cagatgtctt tctaacattc ctaacttctc ata 53

<210> 1048
<211> 51
<212> DNA
<213> Artificial Sequence

<220>
<223> Klammerstrang_1047 zugehoerig zu 2D-DNA-Nanostruktur.

<400> 1048
cctaaatcaa aatcataggt ctaaacagta taacattcct aacttctcat a 51

<210> 1049
<211> 53
<212> DNA
<213> Artificial Sequence

<220>
<223> Klammerstrang_1048 zugehoerig zu 2D-DNA-Nanostruktur.

<400> 1049
gaatttattt aatggtttga aatattctta cctaacattc ctaacttctc ata 53

<210> 1050
<211> 51
<212> DNA
<213> Artificial Sequence

<220>
<223> Klammerstrang_1049 zugehoerig zu 2D-DNA-Nanostruktur.

<400> 1050
cataaatctt tgaataccaa gtgttagaac taacattcct aacttctcat a 51

<210> 1051
<211> 53
<212> DNA
<213> Artificial Sequence

<220>
<223> Klammerstrang_1050 zugehoerig zu 2D-DNA-Nanostruktur.

<400> 1051
cctgattgca atatatgtga gtgatcaata gttaacattc ctaacttctc ata 53

<210> 1052
<211> 51
<212> DNA
<213> Artificial Sequence

<220>
<223> Klammerstrang_1051 zugehoerig zu 2D-DNA-Nanostruktur.

<400> 1052
ctaccatagt ttgagtaaca tttaaaatat taacattcct aacttctcat a 51

<210> 1053
<211> 53
<212> DNA
<213> Artificial Sequence

<220>
<223> Klammerstrang_1052 zugehoerig zu 2D-DNA-Nanostruktur.

<400> 1053
attttaaaat caaaattatt tgcacggatt cgtaacattc ctaacttctc ata 53

<210> 1054
<211> 51
<212> DNA
<213> Artificial Sequence

<220>
<223> Klammerstrang_1053 zugehoerig zu 2D-DNA-Nanostruktur.

<400> 1054
ctttagggcc tgcaacagtg ccaatacgtg taacattcct aacttctcat a 51

<210> 1055
<211> 53
<212> DNA
<213> Artificial Sequence

<220>
<223> Klammerstrang_1054 zugehoerig zu 2D-DNA-Nanostruktur.

<400> 1055

ttaacaccag cactaacaac taatcgttat tataacattc ctaacttctc ata 53

<210> 1056
<211> 52
<212> DNA
<213> Artificial Sequence

<220>
<223> Klammerstrang_1055 zugehoerig zu 2D-DNA-Nanostruktur.

<400> 1056
gcacagacaa tatttttgaa tggggtcagt ataacattcc taacttctca ta 52

<210> 1057
<211> 51
<212> DNA
<213> Artificial Sequence

<220>
<223> Klammerstrang_1056 zugehoerig zu 2D-DNA-Nanostruktur.

<400> 1057
tgtagaaatc aagattagtt gctcttacca taacattcct aacttctcat a 51

<210> 1058
<211> 53
<212> DNA
<213> Artificial Sequence

<220>
<223> Klammerstrang_1057 zugehoerig zu 2D-DNA-Nanostruktur.

<400> 1058
gtttatcaat atgcgttata caaaccgacc gttaacattc ctaacttctc ata 53

<210> 1059
<211> 51
<212> DNA
<213> Artificial Sequence

<220>
<223> Klammerstrang_1058 zugehoerig zu 2D-DNA-Nanostruktur.

<400> 1059
ttagtatcac aatagataag tccacgagca taacattcct aacttctcat a 51

<210> 1060
<211> 53
<212> DNA
<213> Artificial Sequence

<220>
<223> Klammerstrang_1059 zugehoerig zu 2D-DNA-Nanostruktur.

<400> 1060
gtgataaaaa gacgctgaga agagataacc tttaacattc ctaacttctc ata 53

<210> 1061
<211> 51
<212> DNA
<213> Artificial Sequence

<220>
<223> Klammerstrang_1060 zugehoerig zu 2D-DNA-Nanostruktur.

<400> 1061
cttagattta aggcgttaaa taaagcctgt taacattcct aacttctcat a 51

<210> 1062
<211> 53
<212> DNA
<213> Artificial Sequence

<220>
<223> Klammerstrang_1061 zugehoerig zu 2D-DNA-Nanostruktur.

<400> 1062
gcttctgttc gggagaaaca ataacgtaaa actaacattc ctaacttctc ata 53

<210> 1063
<211> 51
<212> DNA
<213> Artificial Sequence

<220>
<223> Klammerstrang_1062 zugehoerig zu 2D-DNA-Nanostruktur.

<400> 1063
cttttacaaa atcgtcgcta ttagcgatag taacattcct aacttctcat a 51

<210> 1064
<211> 53
<212> DNA
<213> Artificial Sequence

<220>
<223> Klammerstrang_1063 zugehoerig zu 2D-DNA-Nanostruktur.

<400> 1064
agaaataaaa atcctttgcc cgaaagatta gataacattc ctaacttctc ata 53

<210> 1065
<211> 51
<212> DNA
<213> Artificial Sequence

<220>
<223> Klammerstrang_1064 zugehoerig zu 2D-DNA-Nanostruktur.

<400> 1065
ctcgtattag aaattgcgta gatacagtac taacattcct aacttctcat a 51

<210> 1066
<211> 53
<212> DNA
<213> Artificial Sequence

<220>
<223> Klammerstrang_1065 zugehoerig zu 2D-DNA-Nanostruktur.

<400> 1066
gccgtcaaaa aacagaggtg aggcctatta gttaacattc ctaacttctc ata 53

<210> 1067
<211> 51
<212> DNA
<213> Artificial Sequence

<220>
<223> Klammerstrang_1066 zugehoerig zu 2D-DNA-Nanostruktur.

<400> 1067
cagaagatta gataatacat ttgtcgacaa taacattcct aacttctcat a 51

<210> 1068
<211> 51
<212> DNA
<213> Artificial Sequence

<220>
<223> Klammerstrang_1067 zugehoerig zu 2D-DNA-Nanostruktur.

<400> 1068
ctttaatgcg cgaactgata gccccaccag taacattcct aacttctcat a 51

<210> 1069
<211> 52
<212> DNA
<213> Artificial Sequence

<220>
<223> Klammerstrang_1068 zugehoerig zu 2D-DNA-Nanostruktur.

<400> 1069
agaaaggaac aactaaagga attcaaaaaa attaccatct ctcctaaact cg 52

<210> 1070
<211> 51
<212> DNA
<213> Artificial Sequence

<220>
<223> Klammerstrang_1069 zugehoerig zu 2D-DNA-Nanostruktur.

<400> 1070
aggctccaga ggctttgagg acacgggtaa ttaccatctc tcctaaactc g 51

<210> 1071
<211> 53
<212> DNA
<213> Artificial Sequence

<220>
<223> Klammerstrang_1070 zugehoerig zu 2D-DNA-Nanostruktur.

<400> 1071
acggctacaa aaggagcctt taatgtgaga atttaccatc tctcctaaac tcg 53

<210> 1072
<211> 51
<212> DNA
<213> Artificial Sequence

<220>
<223> Klammerstrang_1071 zugehoerig zu 2D-DNA-Nanostruktur.

<400> 1072
aatacgtttg aaagaggaca gactgacctt ttaccatctc tcctaaactc g 51

<210> 1073
<211> 53
<212> DNA
<213> Artificial Sequence

<220>
<223> Klammerstrang_1072 zugehoerig zu 2D-DNA-Nanostruktur.

<400> 1073
gaccaactaa tgccactacg aaggggtag cattaccatc tctcctaaac tcg 53

<210> 1074
<211> 51
<212> DNA
<213> Artificial Sequence

<220>
<223> Klammerstrang_1073 zugehoerig zu 2D-DNA-Nanostruktur.

<400> 1074
catcaagtaa aacgaactaa cgagttgaga ttaccatctc tcctaaactc g 51

<210> 1075
<211> 53

<212> DNA
<213> Artificial Sequence

<220>
<223> Klammerstrang_1074 zugehoerig zu 2D-DNA-Nanostruktur.

<400> 1075
tacgttaaag taatcttgac aagaaccgaa ctttaccatc tctcctaaac tcg 53

<210> 1076
<211> 51
<212> DNA
<213> Artificial Sequence

<220>
<223> Klammerstrang_1075 zugehoerig zu 2D-DNA-Nanostruktur.

<400> 1076
tttaggacaa atgctttaaa caatcaggtc ttaccatctc tcctaaactc g 51

<210> 1077
<211> 53
<212> DNA
<213> Artificial Sequence

<220>
<223> Klammerstrang_1076 zugehoerig zu 2D-DNA-Nanostruktur.

<400> 1077
atccccctat accacattca actagaaaaa tcttaccatc tctcctaaac tcg 53

<210> 1078
<211> 51
<212> DNA
<213> Artificial Sequence

<220>
<223> Klammerstrang_1077 zugehoerig zu 2D-DNA-Nanostruktur.

<400> 1078
tttaccccaa catgttttaa atttccatat ttaccatctc tcctaaactc g 51

<210> 1079
<211> 53
<212> DNA
<213> Artificial Sequence

<220>
<223> Klammerstrang_1078 zugehoerig zu 2D-DNA-Nanostruktur.

<400> 1079
ctgtagcttg actattatag tcagttcatt gattaccatc tctcctaaac tcg 53

<210> 1080
<211> 51
<212> DNA
<213> Artificial Sequence

<220>
<223> Klammerstrang_1079 zugehoerig zu 2D-DNA-Nanostruktur.

<400> 1080
aacagttttg taccaaaaac attttatttc ttaccatctc tcctaaactc g 51

<210> 1081
<211> 53
<212> DNA
<213> Artificial Sequence

<220>
<223> Klammerstrang_1080 zugehoerig zu 2D-DNA-Nanostruktur.

<400> 1081
acaactttca acagtttcag cggatgtatc ggttaccatc tctcctaaac tcg 53

<210> 1082
<211> 53
<212> DNA
<213> Artificial Sequence

<220>
<223> Klammerstrang_1081 zugehoerig zu 2D-DNA-Nanostruktur.

<400> 1082
tttatcagga cagcatcgga acgacaccaa ccttaccatc tctcctaaac tcg 53

<210> 1083
<211> 51
<212> DNA
<213> Artificial Sequence

<220>
<223> Klammerstrang_1082 zugehoerig zu 2D-DNA-Nanostruktur.

<400> 1083
cagcgaaact tgctttcgag gtgttgctaa ttaccatctc tcctaaactc g 51

<210> 1084
<211> 53
<212> DNA
<213> Artificial Sequence

<220>
<223> Klammerstrang_1083 zugehoerig zu 2D-DNA-Nanostruktur.

<400> 1084
taaaacgagg tcaatcataa gggaaccgga tattaccatc tctcctaaac tcg 53

<210> 1085
<211> 51
<212> DNA
<213> Artificial Sequence

<220>
<223> Klammerstrang_1084 zugehoerig zu 2D-DNA-Nanostruktur.

<400> 1085
gcgcagacaa gaggcaaaag aatccctcag ttaccatctc tcctaaactc g 51

<210> 1086
<211> 53
<212> DNA
<213> Artificial Sequence

<220>
<223> Klammerstrang_1085 zugehoerig zu 2D-DNA-Nanostruktur.

<400> 1086
ttcattacgt caggacgttg ggaaatgcag atttaccatc tctcctaaac tcg 53

<210> 1087
<211> 51
<212> DNA
<213> Artificial Sequence

<220>
<223> Klammerstrang_1086 zugehoerig zu 2D-DNA-Nanostruktur.

<400> 1087
ttataccacc aaatcaacgt aacgaacgag ttaccatctc tcctaaactc g 51

<210> 1088
<211> 53
<212> DNA
<213> Artificial Sequence

<220>
<223> Klammerstrang_1087 zugehoerig zu 2D-DNA-Nanostruktur.

<400> 1088
acataacggg aatcgtcata aataaagcaa agttaccatc tctcctaaac tcg 53

<210> 1089
<211> 51
<212> DNA
<213> Artificial Sequence

<220>
<223> Klammerstrang_1088 zugehoerig zu 2D-DNA-Nanostruktur.

<400> 1089
aatactgccc aaaaggaatt acgtggctca ttaccatctc tcctaaactc g 51

<210> 1090
<211> 53
<212> DNA
<213> Artificial Sequence

<220>
<223> Klammerstrang_1089 zugehoerig zu 2D-DNA-Nanostruktur.

<400> 1090
cggattgcag agcttaattg ctgaaacgag tattaccatc tctcctaaac tcg 53

<210> 1091
<211> 51
<212> DNA
<213> Artificial Sequence

<220>
<223> Klammerstrang_1090 zugehoerig zu 2D-DNA-Nanostruktur.

<400> 1091
gatggcttat caaaaagatt aagagcgtcc ttaccatctc tcctaaactc g 51

<210> 1092
<211> 53
<212> DNA
<213> Artificial Sequence

<220>
<223> Klammerstrang_1091 zugehoerig zu 2D-DNA-Nanostruktur.

<400> 1092
gatttagtca ataaagcctc agagaaccct cattaccatc tctcctaaac tcg 53

<210> 1093
<211> 53
<212> DNA
<213> Artificial Sequence

<220>
<223> Klammerstrang_1092 zugehoerig zu 2D-DNA-Nanostruktur.

<400> 1093
taaatgaatt ttctgtatgg gattaatttc ttttaccatc tctcctaaac tcg 53

<210> 1094
<211> 53
<212> DNA
<213> Artificial Sequence

<220>

<223> Klammerstrang_1093 zugehoerig zu 2D-DNA-Nanostruktur.

<400> 1094
aaacagcttt ttgcgggatc gtcaacacta aattaccatc tctcctaaac tcg 53

<210> 1095
<211> 53
<212> DNA
<213> Artificial Sequence

<220>
<223> Klammerstrang_1094 zugehoerig zu 2D-DNA-Nanostruktur.

<400> 1095
aaggccgctg ataccgatag ttgcgacgtt agttaccatc tctcctaaac tcg 53

<210> 1096
<211> 53
<212> DNA
<213> Artificial Sequence

<220>
<223> Klammerstrang_1095 zugehoerig zu 2D-DNA-Nanostruktur.

<400> 1096
acactcatcc atgttactta gccgaaagct gcttaccatc tctcctaaac tcg 53

<210> 1097
<211> 53
<212> DNA
<213> Artificial Sequence

<220>
<223> Klammerstrang_1096 zugehoerig zu 2D-DNA-Nanostruktur.

<400> 1097
gacctgctct ttgacccccca gcgagggagt tattaccatc tctcctaaac tcg 53

<210> 1098
<211> 53
<212> DNA
<213> Artificial Sequence

<220>
<223> Klammerstrang_1097 zugehoerig zu 2D-DNA-Nanostruktur.

<400> 1098
tcattcagat gcgattttaa gaacaggcat agttaccatc tctcctaaac tcg 53

<210> 1099
<211> 53
<212> DNA
<213> Artificial Sequence

<220>
<223> Klammerstrang_1098 zugehoerig zu 2D-DNA-Nanostruktur.

<400> 1099
attacctttg aataaggctt gcccaaatcc gcttaccatc tctcctaaac tcg 53

<210> 1100
<211> 53
<212> DNA
<213> Artificial Sequence

<220>
<223> Klammerstrang_1099 zugehoerig zu 2D-DNA-Nanostruktur.

<400> 1100
taagagcaaa tgtttagact ggataggaag ccttaccatc tctcctaaac tcg 53

<210> 1101
<211> 53
<212> DNA
<213> Artificial Sequence

<220>
<223> Klammerstrang_1100 zugehoerig zu 2D-DNA-Nanostruktur.

<400> 1101
aatagtaaac actatcataa ccctcattgt gattaccatc tctcctaaac tcg 53

<210> 1102
<211> 53
<212> DNA
<213> Artificial Sequence

<220>
<223> Klammerstrang_1101 zugehoerig zu 2D-DNA-Nanostruktur.

<400> 1102
cgaaagactt tgataagagg tcatatttcg cattaccatc tctcctaaac tcg 53

<210> 1103
<211> 53
<212> DNA
<213> Artificial Sequence

<220>
<223> Klammerstrang_1102 zugehoerig zu 2D-DNA-Nanostruktur.

<400> 1103
ttgctccttt caaatatcgc gtttgagggg gtttaccatc tctcctaaac tcg 53

<210> 1104
<211> 53
<212> DNA

<213> Artificial Sequence

<220>
<223> Klammerstrang_1103 zugehoerig zu 2D-DNA-Nanostruktur.

<400> 1104
aatggtcaac aggcaaggca aagagtaatg tgttaccatc tctcctaaac tcg 53

<210> 1105
<211> 52
<212> DNA
<213> Artificial Sequence

<220>
<223> Klammerstrang_1104 zugehoerig zu 2D-DNA-Nanostruktur.

<400> 1105
tctaaagttt tgtcgtcttt ccagccgaca attaccatct ctcctaaact cg 52

<210> 1106
<211> 65
<212> DNA
<213> Artificial Sequence

<220>
<223> Klammerstrang_1105 zugehoerig zu 2D-DNA-Nanostruktur.

<400> 1106
tgacaactcg ctgaggcttg cattatacca agcgcgatga taaattacca tctctcctaa 60

actcg 65

<210> 1107
<211> 53
<212> DNA
<213> Artificial Sequence

<220>
<223> Klammerstrang_1106 zugehoerig zu 2D-DNA-Nanostruktur.

<400> 1107
atattcggaa ccatcgccca cgcagagaag gattaccatc tctcctaaac tcg 53

<210> 1108
<211> 53
<212> DNA
<213> Artificial Sequence

<220>
<223> Klammerstrang_1107 zugehoerig zu 2D-DNA-Nanostruktur.

<400> 1108
tcatcgccaa caaagtacaa cggacgccag cattaccatc tctcctaaac tcg 53

<210> 1109
<211> 53
<212> DNA
<213> Artificial Sequence

<220>
<223> Klammerstrang_1108 zugehoerig zu 2D-DNA-Nanostruktur.

<400> 1109
gatggtttga acgagtagta aatttaccat tattaccatc tctcctaaac tcg 53

<210> 1110
<211> 59
<212> DNA
<213> Artificial Sequence

<220>
<223> Klammerstrang_1109 zugehoerig zu 2D-DNA-Nanostruktur.

<400> 1110
cgtttaccag acgacaaaga agttttgcca taattcgatt accatctctc ctaaactcg 59

<210> 1111
<211> 53
<212> DNA
<213> Artificial Sequence

<220>
<223> Klammerstrang_1110 zugehoerig zu 2D-DNA-Nanostruktur.

<400> 1111
cttttgcaga taaaaaccaa aataaagact ccttaccatc tctcctaaac tcg 53

<210> 1112
<211> 51
<212> DNA
<213> Artificial Sequence

<220>
<223> Klammerstrang_1111 zugehoerig zu 2D-DNA-Nanostruktur.

<400> 1112
gcttcaatca ggattagaga gttattttca ttaccatctc tcctaaactc g 51

<210> 1113
<211> 53
<212> DNA
<213> Artificial Sequence

<220>
<223> Klammerstrang_1112 zugehoerig zu 2D-DNA-Nanostruktur.

<400> 1113

ccaacaggag cgaaccagac cggagccttt acttaccatc tctcctaaac tcg 53

<210> 1114
<211> 51
<212> DNA
<213> Artificial Sequence

<220>
<223> Klammerstrang_1113 zugehoerig zu 2D-DNA-Nanostruktur.

<400> 1114
tttggggata gtagtagcat taaaaggccg ttaccatctc tcctaaactc g 51

<210> 1115
<211> 53
<212> DNA
<213> Artificial Sequence

<220>
<223> Klammerstrang_1114 zugehoerig zu 2D-DNA-Nanostruktur.

<400> 1115
tccacagaca gccctcatag ttagcgtaac gattaccatc tctcctaaac tcg 53

<210> 1116
<211> 53
<212> DNA
<213> Artificial Sequence

<220>
<223> Klammerstrang_1115 zugehoerig zu 2D-DNA-Nanostruktur.

<400> 1116
ttaggattgg ctgagactcc tcaataaccg atttaccatc tctcctaaac tcg 53

<210> 1117
<211> 51
<212> DNA
<213> Artificial Sequence

<220>
<223> Klammerstrang_1116 zugehoerig zu 2D-DNA-Nanostruktur.

<400> 1117
tattaagaag cggggttttg ctcgtagcat ttaccatctc tcctaaactc g 51

<210> 1118
<211> 53
<212> DNA
<213> Artificial Sequence

<220>
<223> Klammerstrang_1117 zugehoerig zu 2D-DNA-Nanostruktur.

<400> 1118
ttgacaggcc accaccagag ccgcgatttg tattaccatc tctcctaaac tcg 53

<210> 1119
<211> 51
<212> DNA
<213> Artificial Sequence

<220>
<223> Klammerstrang_1118 zugehoerig zu 2D-DNA-Nanostruktur.

<400> 1119
caccagaaag gttgaggcag gtcatgaaag ttaccatctc tcctaaactc g 51

<210> 1120
<211> 53
<212> DNA
<213> Artificial Sequence

<220>
<223> Klammerstrang_1119 zugehoerig zu 2D-DNA-Nanostruktur.

<400> 1120
gcaaggcctc accagtagca ccatgggctt gattaccatc tctcctaaac tcg 53

<210> 1121
<211> 51
<212> DNA
<213> Artificial Sequence

<220>
<223> Klammerstrang_1120 zugehoerig zu 2D-DNA-Nanostruktur.

<400> 1121
cagcaaaagg aaacgtcacc aatgagccgc ttaccatctc tcctaaactc g 51

<210> 1122
<211> 53
<212> DNA
<213> Artificial Sequence

<220>
<223> Klammerstrang_1121 zugehoerig zu 2D-DNA-Nanostruktur.

<400> 1122
ttattacgaa gaactggcat gattgcgaga ggttaccatc tctcctaaac tcg 53

<210> 1123
<211> 51
<212> DNA
<213> Artificial Sequence

<220>
<223> Klammerstrang_1122 zugehoerig zu 2D-DNA-Nanostruktur.

<400> 1123
atacccaaca gtatgttagc aaattagagc ttaccatctc tcctaaactc g 51

<210> 1124
<211> 53
<212> DNA
<213> Artificial Sequence

<220>
<223> Klammerstrang_1123 zugehoerig zu 2D-DNA-Nanostruktur.

<400> 1124
agagagaaaa aaatgaaaat agcaagcaaa ctttaccatc tctcctaaac tcg 53

<210> 1125
<211> 51
<212> DNA
<213> Artificial Sequence

<220>
<223> Klammerstrang_1124 zugehoerig zu 2D-DNA-Nanostruktur.

<400> 1125
ttaacgtcta acataaaaac aggtaacgga ttaccatctc tcctaaactc g 51

<210> 1126
<211> 53
<212> DNA
<213> Artificial Sequence

<220>
<223> Klammerstrang_1125 zugehoerig zu 2D-DNA-Nanostruktur.

<400> 1126
ccaatagctc atcgtaggaa tcatggcatc aattaccatc tctcctaaac tcg 53

<210> 1127
<211> 53
<212> DNA
<213> Artificial Sequence

<220>
<223> Klammerstrang_1126 zugehoerig zu 2D-DNA-Nanostruktur.

<400> 1127
tcaccagtac aaactacaac gcctagtacc agttaccatc tctcctaaac tcg 53

<210> 1128
<211> 53
<212> DNA
<213> Artificial Sequence

<220>
<223> Klammerstrang_1127 zugehoerig zu 2D-DNA-Nanostruktur.

<400> 1128
gcggataacc tattattctg aaacagacga ttttaccatc tctcctaaac tcg 53

<210> 1129
<211> 53
<212> DNA
<213> Artificial Sequence

<220>
<223> Klammerstrang_1128 zugehoerig zu 2D-DNA-Nanostruktur.

<400> 1129
tttcggaagt gccgtcgaga gggtgagttt cgttaccatc tctcctaaac tcg 53

<210> 1130
<211> 53
<212> DNA
<213> Artificial Sequence

<220>
<223> Klammerstrang_1129 zugehoerig zu 2D-DNA-Nanostruktur.

<400> 1130
ggccttgaag agccaccacc ctcagaaacc atttaccatc tctcctaaac tcg 53

<210> 1131
<211> 53
<212> DNA
<213> Artificial Sequence

<220>
<223> Klammerstrang_1130 zugehoerig zu 2D-DNA-Nanostruktur.

<400> 1131
ccaccctcta ttcacaaaca aatacctgcc tattaccatc tctcctaaac tcg 53

<210> 1132
<211> 53
<212> DNA
<213> Artificial Sequence

<220>
<223> Klammerstrang_1131 zugehoerig zu 2D-DNA-Nanostruktur.

<400> 1132
cgatagcatt gagccatttg ggaacgtaga aattaccatc tctcctaaac tcg 53

<210> 1133
<211> 53

<212> DNA
<213> Artificial Sequence

<220>
<223> Klammerstrang_1132 zugehoerig zu 2D-DNA-Nanostruktur.

<400> 1133
tcaccgacgc accgtaatca gtagcagaac cgttaccatc tctcctaaac tcg 53

<210> 1134
<211> 53
<212> DNA
<213> Artificial Sequence

<220>
<223> Klammerstrang_1133 zugehoerig zu 2D-DNA-Nanostruktur.

<400> 1134
atacataccg aggaaacgca ataagaagcg cattaccatc tctcctaaac tcg 53

<210> 1135
<211> 53
<212> DNA
<213> Artificial Sequence

<220>
<223> Klammerstrang_1134 zugehoerig zu 2D-DNA-Nanostruktur.

<400> 1135
aaggaaacat aaaggtggca acattatcac cgttaccatc tctcctaaac tcg 53

<210> 1136
<211> 53
<212> DNA
<213> Artificial Sequence

<220>
<223> Klammerstrang_1135 zugehoerig zu 2D-DNA-Nanostruktur.

<400> 1136
ttagacggcc aaataagaaa cgatagaagg ctttaccatc tctcctaaac tcg 53

<210> 1137
<211> 53
<212> DNA
<213> Artificial Sequence

<220>
<223> Klammerstrang_1136 zugehoerig zu 2D-DNA-Nanostruktur.

<400> 1137
atcccaatga gaattaactg aacagttacc agttaccatc tctcctaaac tcg 53

<210> 1138
<211> 53
<212> DNA
<213> Artificial Sequence

<220>
<223> Klammerstrang_1137 zugehoerig zu 2D-DNA-Nanostruktur.

<400> 1138
tatccggtct catcgagaac aagcgacaaa agttaccatc tctcctaaac tcg 53

<210> 1139
<211> 52
<212> DNA
<213> Artificial Sequence

<220>
<223> Klammerstrang_1138 zugehoerig zu 2D-DNA-Nanostruktur.

<400> 1139
aggaacccat gtaccgtaac acttgatata attaccatct ctcctaaact cg 52

<210> 1140
<211> 51
<212> DNA
<213> Artificial Sequence

<220>
<223> Klammerstrang_1139 zugehoerig zu 2D-DNA-Nanostruktur.

<400> 1140
gtatagcaaa cagttaatgc ccaatcctca ttaccatctc tcctaaactc g 51

<210> 1141
<211> 53
<212> DNA
<213> Artificial Sequence

<220>
<223> Klammerstrang_1140 zugehoerig zu 2D-DNA-Nanostruktur.

<400> 1141
gcccgtatcc ggaataggtg tatcagccca atttaccatc tctcctaaac tcg 53

<210> 1142
<211> 51
<212> DNA
<213> Artificial Sequence

<220>
<223> Klammerstrang_1141 zugehoerig zu 2D-DNA-Nanostruktur.

<400> 1142
ttaaagccag agccgccacc ctcgacagaa ttaccatctc tcctaaactc g 51

<210> 1143
<211> 53
<212> DNA
<213> Artificial Sequence

<220>
<223> Klammerstrang_1142 zugehoerig zu 2D-DNA-Nanostruktur.

<400> 1143
gcctccctca gaatggaaag cgcagtaaca gtttaccatc tctcctaaac tcg 53

<210> 1144
<211> 51
<212> DNA
<213> Artificial Sequence

<220>
<223> Klammerstrang_1143 zugehoerig zu 2D-DNA-Nanostruktur.

<400> 1144
tcaagtttca ttaaaggtga atataaaaga ttaccatctc tcctaaactc g 51

<210> 1145
<211> 53
<212> DNA
<213> Artificial Sequence

<220>
<223> Klammerstrang_1144 zugehoerig zu 2D-DNA-Nanostruktur.

<400> 1145
gaaattattg cctttagcgt cagaccggaa ccttaccatc tctcctaaac tcg 53

<210> 1146
<211> 51
<212> DNA
<213> Artificial Sequence

<220>
<223> Klammerstrang_1145 zugehoerig zu 2D-DNA-Nanostruktur.

<400> 1146
aacgcaaaga tagccgaaca aaccctgaac ttaccatctc tcctaaactc g 51

<210> 1147
<211> 53
<212> DNA
<213> Artificial Sequence

<220>
<223> Klammerstrang_1146 zugehoerig zu 2D-DNA-Nanostruktur.

<400> 1147
aagtaagcag acaccacgga ataatattga cgttaccatc tctcctaaac tcg 53

<210> 1148
<211> 51
<212> DNA
<213> Artificial Sequence

<220>
<223> Klammerstrang_1147 zugehoerig zu 2D-DNA-Nanostruktur.

<400> 1148
aaagtcacaa aataaacagc cagcgtttta ttaccatctc tcctaaactc g 51

<210> 1149
<211> 53
<212> DNA
<213> Artificial Sequence

<220>
<223> Klammerstrang_1148 zugehoerig zu 2D-DNA-Nanostruktur.

<400> 1149
gccagttaga gggtaattga gcgctttaag aattaccatc tctcctaaac tcg 53

<210> 1150
<211> 51
<212> DNA
<213> Artificial Sequence

<220>
<223> Klammerstrang_1149 zugehoerig zu 2D-DNA-Nanostruktur.

<400> 1150
gcgaacctcc aagaacgggt atgacaataa ttaccatctc tcctaaactc g 51

<210> 1151
<211> 53
<212> DNA
<213> Artificial Sequence

<220>
<223> Klammerstrang_1150 zugehoerig zu 2D-DNA-Nanostruktur.

<400> 1151
ccaccctcat tttcagggat agcaaccgta ctttaccatc tctcctaaac tcg 53

<210> 1152
<211> 53
<212> DNA
<213> Artificial Sequence

<220>

<223> Klammerstrang_1151 zugehoerig zu 2D-DNA-Nanostruktur.

<400> 1152
caggaggtgg ggtcagtgcc ttgagtctct gattaccatc tctcctaaac tcg 53

<210> 1153
<211> 51
<212> DNA
<213> Artificial Sequence

<220>
<223> Klammerstrang_1152 zugehoerig zu 2D-DNA-Nanostruktur.

<400> 1153
gttttaactt agtaccgcca cccagagcca ttaccatctc tcctaaactc g 51

<210> 1154
<211> 53
<212> DNA
<213> Artificial Sequence

<220>
<223> Klammerstrang_1153 zugehoerig zu 2D-DNA-Nanostruktur.

<400> 1154
atttaccggg aaccagagcc accactgtag cgttaccatc tctcctaaac tcg 53

<210> 1155
<211> 51
<212> DNA
<213> Artificial Sequence

<220>
<223> Klammerstrang_1154 zugehoerig zu 2D-DNA-Nanostruktur.

<400> 1155
aaatcacctt ccagtaagcg tcagtaataa ttaccatctc tcctaaactc g 51

<210> 1156
<211> 53
<212> DNA
<213> Artificial Sequence

<220>
<223> Klammerstrang_1155 zugehoerig zu 2D-DNA-Nanostruktur.

<400> 1156
cgttttcaag ggagggaagg taaagtttat ttttaccatc tctcctaaac tcg 53

<210> 1157
<211> 51
<212> DNA
<213> Artificial Sequence

<220>
<223> Klammerstrang_1156 zugehoerig zu 2D-DNA-Nanostruktur.

<400> 1157
accgattgtc ggcattttcg gtcataatca ttaccatctc tcctaaactc g 51

<210> 1158
<211> 53
<212> DNA
<213> Artificial Sequence

<220>
<223> Klammerstrang_1157 zugehoerig zu 2D-DNA-Nanostruktur.

<400> 1158
tgtcacaatc ttaccgaagc cctttaatat cattaccatc tctcctaaac tcg 53

<210> 1159
<211> 51
<212> DNA
<213> Artificial Sequence

<220>
<223> Klammerstrang_1158 zugehoerig zu 2D-DNA-Nanostruktur.

<400> 1159
aatagctatc aatagaaaat tcaacattca ttaccatctc tcctaaactc g 51

<210> 1160
<211> 53
<212> DNA
<213> Artificial Sequence

<220>
<223> Klammerstrang_1159 zugehoerig zu 2D-DNA-Nanostruktur.

<400> 1160
gagagataga gcgtctttcc agaggttttg aattaccatc tctcctaaac tcg 53

<210> 1161
<211> 51
<212> DNA
<213> Artificial Sequence

<220>
<223> Klammerstrang_1160 zugehoerig zu 2D-DNA-Nanostruktur.

<400> 1161
acgctaacac ccacaagaat tgaaaatagc ttaccatctc tcctaaactc g 51

<210> 1162
<211> 53
<212> DNA

<213> Artificial Sequence

<220>
<223> Klammerstrang_1161 zugehoerig zu 2D-DNA-Nanostruktur.

<400> 1162
gccttaaacc aatcaataat cggcacgcgc ctttaccatc tctcctaaac tcg 53

<210> 1163
<211> 53
<212> DNA
<213> Artificial Sequence

<220>
<223> Klammerstrang_1162 zugehoerig zu 2D-DNA-Nanostruktur.

<400> 1163
taaatcggga ttcccaattc tgcgatataa tgttaccatc tctcctaaac tcg 53

<210> 1164
<211> 51
<212> DNA
<213> Artificial Sequence

<220>
<223> Klammerstrang_1163 zugehoerig zu 2D-DNA-Nanostruktur.

<400> 1164
aacgcaaaat cgatgaacgg taccggttga ttaccatctc tcctaaactc g 51

<210> 1165
<211> 53
<212> DNA
<213> Artificial Sequence

<220>
<223> Klammerstrang_1164 zugehoerig zu 2D-DNA-Nanostruktur.

<400> 1165
aacaagaggg ataaaaattt ttagcataaa gcttaccatc tctcctaaac tcg 53

<210> 1166
<211> 51
<212> DNA
<213> Artificial Sequence

<220>
<223> Klammerstrang_1165 zugehoerig zu 2D-DNA-Nanostruktur.

<400> 1166
taatcagcgg attgaccgta atcgtaaccg ttaccatctc tcctaaactc g 51

<210> 1167

<211> 53
<212> DNA
<213> Artificial Sequence

<220>
<223> Klammerstrang_1166 zugehoerig zu 2D-DNA-Nanostruktur.

<400> 1167
acaaacggaa aagccccaaa aacactggag cattaccatc tctcctaaac tcg 53

<210> 1168
<211> 51
<212> DNA
<213> Artificial Sequence

<220>
<223> Klammerstrang_1167 zugehoerig zu 2D-DNA-Nanostruktur.

<400> 1168
tgcatctttc ccagtcacga cggcctgcag ttaccatctc tcctaaactc g 51

<210> 1169
<211> 53
<212> DNA
<213> Artificial Sequence

<220>
<223> Klammerstrang_1168 zugehoerig zu 2D-DNA-Nanostruktur.

<400> 1169
ccagggttgc cagtttgagg ggacccgtgg gattaccatc tctcctaaac tcg 53

<210> 1170
<211> 51
<212> DNA
<213> Artificial Sequence

<220>
<223> Klammerstrang_1169 zugehoerig zu 2D-DNA-Nanostruktur.

<400> 1170
gtcgacttcg gccaacgcgc ggggtttttc ttaccatctc tcctaaactc g 51

<210> 1171
<211> 53
<212> DNA
<213> Artificial Sequence

<220>
<223> Klammerstrang_1170 zugehoerig zu 2D-DNA-Nanostruktur.

<400> 1171
ttaatgaact agaggatccc cgggggtaa cgttaccatc tctcctaaac tcg 53

<210> 1172
<211> 51
<212> DNA
<213> Artificial Sequence

<220>
<223> Klammerstrang_1171 zugehoerig zu 2D-DNA-Nanostruktur.

<400> 1172
ttttcactca aagggcgaaa aaccatcacc ttaccatctc tcctaaactc g 51

<210> 1173
<211> 53
<212> DNA
<213> Artificial Sequence

<220>
<223> Klammerstrang_1172 zugehoerig zu 2D-DNA-Nanostruktur.

<400> 1173
ctccaacgca gtgagacggg caaccagctg cattaccatc tctcctaaac tcg 53

<210> 1174
<211> 52
<212> DNA
<213> Artificial Sequence

<220>
<223> Klammerstrang_1173 zugehoerig zu 2D-DNA-Nanostruktur.

<400> 1174
caaatcaagt tttttggggt cgaaacgtgg attaccatct ctcctaaact cg 52

<210> 1175
<211> 51
<212> DNA
<213> Artificial Sequence

<220>
<223> Klammerstrang_1174 zugehoerig zu 2D-DNA-Nanostruktur.

<400> 1175
aaattaagtt gaccattaga tacttttgcg ttaccatctc tcctaaactc g 51

<210> 1176
<211> 53
<212> DNA
<213> Artificial Sequence

<220>
<223> Klammerstrang_1175 zugehoerig zu 2D-DNA-Nanostruktur.

<400> 1176

tatattttgt cattgcctga gagtggaaga ttttaccatc tctcctaaac tcg 53

<210> 1177
<211> 51
<212> DNA
<213> Artificial Sequence

<220>
<223> Klammerstrang_1176 zugehoerig zu 2D-DNA-Nanostruktur.

<400> 1177
gctatcagaa atgcaatgcc tgaattagca ttaccatctc tcctaaactc g 51

<210> 1178
<211> 53
<212> DNA
<213> Artificial Sequence

<220>
<223> Klammerstrang_1177 zugehoerig zu 2D-DNA-Nanostruktur.

<400> 1178
gtataagcca acccgtcgga ttctgacgac agttaccatc tctcctaaac tcg 53

<210> 1179
<211> 51
<212> DNA
<213> Artificial Sequence

<220>
<223> Klammerstrang_1178 zugehoerig zu 2D-DNA-Nanostruktur.

<400> 1179
gcgagtaaaa atatttaaat tgttacaaag ttaccatctc tcctaaactc g 51

<210> 1180
<211> 53
<212> DNA
<213> Artificial Sequence

<220>
<223> Klammerstrang_1179 zugehoerig zu 2D-DNA-Nanostruktur.

<400> 1180
tatcggccgc aaggcgatta agtttaccga gcttaccatc tctcctaaac tcg 53

<210> 1181
<211> 51
<212> DNA
<213> Artificial Sequence

<220>
<223> Klammerstrang_1180 zugehoerig zu 2D-DNA-Nanostruktur.

<400> 1181
gatgtgcttc aggaagatcg cacaatgtga ttaccatctc tcctaaactc g 51

<210> 1182
<211> 53
<212> DNA
<213> Artificial Sequence

<220>
<223> Klammerstrang_1181 zugehoerig zu 2D-DNA-Nanostruktur.

<400> 1182
tcgaattcgg gaaacctgtc gtgcagctga ttttaccatc tctcctaaac tcg 53

<210> 1183
<211> 51
<212> DNA
<213> Artificial Sequence

<220>
<223> Klammerstrang_1182 zugehoerig zu 2D-DNA-Nanostruktur.

<400> 1183
ttccagtcgt aatcatggtc ataaaagggg ttaccatctc tcctaaactc g 51

<210> 1184
<211> 53
<212> DNA
<213> Artificial Sequence

<220>
<223> Klammerstrang_1183 zugehoerig zu 2D-DNA-Nanostruktur.

<400> 1184
gcccttcaga gtccactatt aaagggtgcc gtttaccatc tctcctaaac tcg 53

<210> 1185
<211> 51
<212> DNA
<213> Artificial Sequence

<220>
<223> Klammerstrang_1184 zugehoerig zu 2D-DNA-Nanostruktur.

<400> 1185
tggaacaacc gcctggccct gaggcccgct ttaccatctc tcctaaactc g 51

<210> 1186
<211> 51
<212> DNA
<213> Artificial Sequence

<220>
<223> Klammerstrang_1185 zugehoerig zu 2D-DNA-Nanostruktur.

<400> 1186
aaagcactaa atcggaaccc taatccagtt ttaccatctc tcctaaactc g 51

<210> 1187
<211> 53
<212> DNA
<213> Artificial Sequence

<220>
<223> Klammerstrang_1186 zugehoerig zu 2D-DNA-Nanostruktur.

<400> 1187
taaatcatat aacctgttta gctaaccttt aattaccatc tctcctaaac tcg 53

<210> 1188
<211> 53
<212> DNA
<213> Artificial Sequence

<220>
<223> Klammerstrang_1187 zugehoerig zu 2D-DNA-Nanostruktur.

<400> 1188
taggtaaact atttttgaga gatcaaacgt tattaccatc tctcctaaac tcg 53

<210> 1189
<211> 53
<212> DNA
<213> Artificial Sequence

<220>
<223> Klammerstrang_1188 zugehoerig zu 2D-DNA-Nanostruktur.

<400> 1189
gagggtagga ttcaaaaggg tgagacatcc aattaccatc tctcctaaac tcg 53

<210> 1190
<211> 53
<212> DNA
<213> Artificial Sequence

<220>
<223> Klammerstrang_1189 zugehoerig zu 2D-DNA-Nanostruktur.

<400> 1190
atattttggc tttcatcaac attatccagc cattaccatc tctcctaaac tcg 53

<210> 1191
<211> 53
<212> DNA
<213> Artificial Sequence

<220>
<223> Klammerstrang_1190 zugehoerig zu 2D-DNA-Nanostruktur.

<400> 1191
tgtagccatt aaaattcgca ttaaatgccg gattaccatc tctcctaaac tcg 53

<210> 1192
<211> 53
<212> DNA
<213> Artificial Sequence

<220>
<223> Klammerstrang_1191 zugehoerig zu 2D-DNA-Nanostruktur.

<400> 1192
gctttccgat tacgccagct ggcggctgtt tcttaccatc tctcctaaac tcg 53

<210> 1193
<211> 53
<212> DNA
<213> Artificial Sequence

<220>
<223> Klammerstrang_1192 zugehoerig zu 2D-DNA-Nanostruktur.

<400> 1193
tcttcgctgc accgcttctg gtgcggcctt ccttaccatc tctcctaaac tcg 53

<210> 1194
<211> 53
<212> DNA
<213> Artificial Sequence

<220>
<223> Klammerstrang_1193 zugehoerig zu 2D-DNA-Nanostruktur.

<400> 1194
ctgtgtgatt gcgttgcgct cactagagtt gcttaccatc tctcctaaac tcg 53

<210> 1195
<211> 53
<212> DNA
<213> Artificial Sequence

<220>
<223> Klammerstrang_1194 zugehoerig zu 2D-DNA-Nanostruktur.

<400> 1195
cacattaaaa ttgttatccg ctcatgcggg ccttaccatc tctcctaaac tcg 53

<210> 1196
<211> 53

<212> DNA
<213> Artificial Sequence

<220>
<223> Klammerstrang_1195 zugehoerig zu 2D-DNA-Nanostruktur.

<400> 1196
agcaagcgta gggttgagtg ttgtagggag ccttaccatc tctcctaaac tcg 53

<210> 1197
<211> 53
<212> DNA
<213> Artificial Sequence

<220>
<223> Klammerstrang_1196 zugehoerig zu 2D-DNA-Nanostruktur.

<400> 1197
gcccgagagt ccacgctggt ttgcagctaa ctttaccatc tctcctaaac tcg 53

<210> 1198
<211> 53
<212> DNA
<213> Artificial Sequence

<220>
<223> Klammerstrang_1197 zugehoerig zu 2D-DNA-Nanostruktur.

<400> 1198
cccgatttag agcttgacgg ggaaaaagaa tattaccatc tctcctaaac tcg 53

<210> 1199
<211> 53
<212> DNA
<213> Artificial Sequence

<220>
<223> Klammerstrang_1198 zugehoerig zu 2D-DNA-Nanostruktur.

<400> 1199
ttctactacg cgagctgaaa aggttaccgc gcttaccatc tctcctaaac tcg 53

<210> 1200
<211> 51
<212> DNA
<213> Artificial Sequence

<220>
<223> Klammerstrang_1199 zugehoerig zu 2D-DNA-Nanostruktur.

<400> 1200
gagacagcta gctgataaat taatttttgt ttaccatctc tcctaaactc g 51

<210> 1201
<211> 53
<212> DNA
<213> Artificial Sequence

<220>
<223> Klammerstrang_1200 zugehoerig zu 2D-DNA-Nanostruktur.

<400> 1201
caaccgtttc aaatcaccat caattcgagc cattaccatc tctcctaaac tcg 53

<210> 1202
<211> 65
<212> DNA
<213> Artificial Sequence

<220>
<223> Klammerstrang_1201 zugehoerig zu 2D-DNA-Nanostruktur.

<400> 1202
taaatcaaaa taattcgcgt ctcggaaacc aggcaaaggg aaggttacca tctctcctaa 60

actcg 65

<210> 1203
<211> 53
<212> DNA
<213> Artificial Sequence

<220>
<223> Klammerstrang_1202 zugehoerig zu 2D-DNA-Nanostruktur.

<400> 1203
gccatcaagc tcatttttta accacaaatc cattaccatc tctcctaaac tcg 53

<210> 1204
<211> 53
<212> DNA
<213> Artificial Sequence

<220>
<223> Klammerstrang_1203 zugehoerig zu 2D-DNA-Nanostruktur.

<400> 1204
caactgttgc gccattcgcc attcaaacat cattaccatc tctcctaaac tcg 53

<210> 1205
<211> 53
<212> DNA
<213> Artificial Sequence

<220>
<223> Klammerstrang_1204 zugehoerig zu 2D-DNA-Nanostruktur.

<400> 1205
aagcctggta cgagccggaa gcatagatga tgttaccatc tctcctaaac tcg 53

<210> 1206
<211> 59
<212> DNA
<213> Artificial Sequence

<220>
<223> Klammerstrang_1205 zugehoerig zu 2D-DNA-Nanostruktur.

<400> 1206
cccagcaggc gaaaaatccc ttataaatca agccggcgtt accatctctc ctaaactcg 59

<210> 1207
<211> 53
<212> DNA
<213> Artificial Sequence

<220>
<223> Klammerstrang_1206 zugehoerig zu 2D-DNA-Nanostruktur.

<400> 1207
tcggcaaatc ctgtttgatg gtggaccctc aattaccatc tctcctaaac tcg 53

<210> 1208
<211> 52
<212> DNA
<213> Artificial Sequence

<220>
<223> Klammerstrang_1207 zugehoerig zu 2D-DNA-Nanostruktur.

<400> 1208
aacgtggcga gaaaggaagg gaaaccagta attaccatct ctcctaaact cg 52

<210> 1209
<211> 51
<212> DNA
<213> Artificial Sequence

<220>
<223> Klammerstrang_1208 zugehoerig zu 2D-DNA-Nanostruktur.

<400> 1209
ttttatttaa gcaaatcaga tattttttgt ttaccatctc tcctaaactc g 51

<210> 1210
<211> 53
<212> DNA
<213> Artificial Sequence

<220>

<223> Klammerstrang_1209 zugehoerig zu 2D-DNA-Nanostruktur.

<400> 1210
gtaataagtt aggcagaggc atttatgata ttttaccatc tctcctaaac tcg 53

<210> 1211
<211> 51
<212> DNA
<213> Artificial Sequence

<220>
<223> Klammerstrang_1210 zugehoerig zu 2D-DNA-Nanostruktur.

<400> 1211
catgtaatag aatataaagt accaagccgt ttaccatctc tcctaaactc g 51

<210> 1212
<211> 53
<212> DNA
<213> Artificial Sequence

<220>
<223> Klammerstrang_1211 zugehoerig zu 2D-DNA-Nanostruktur.

<400> 1212
atcgcaagta tgtaaatgct gatgatagga acttaccatc tctcctaaac tcg 53

<210> 1213
<211> 51
<212> DNA
<213> Artificial Sequence

<220>
<223> Klammerstrang_1212 zugehoerig zu 2D-DNA-Nanostruktur.

<400> 1213
tataactaac aaagaacgcg agaacgccaa ttaccatctc tcctaaactc g 51

<210> 1214
<211> 53
<212> DNA
<213> Artificial Sequence

<220>
<223> Klammerstrang_1213 zugehoerig zu 2D-DNA-Nanostruktur.

<400> 1214
agaaaacaaa gaagatgatg aaacaggctg cgttaccatc tctcctaaac tcg 53

<210> 1215
<211> 51
<212> DNA
<213> Artificial Sequence

<220>
<223> Klammerstrang_1214 zugehoerig zu 2D-DNA-Nanostruktur.

<400> 1215
ctgagcaaaa attaattaca ttttgggtta ttaccatctc tcctaaactc g 51

<210> 1216
<211> 53
<212> DNA
<213> Artificial Sequence

<220>
<223> Klammerstrang_1215 zugehoerig zu 2D-DNA-Nanostruktur.

<400> 1216
gcaattcaca tattcctgat tatcaaagtg tattaccatc tctcctaaac tcg 53

<210> 1217
<211> 51
<212> DNA
<213> Artificial Sequence

<220>
<223> Klammerstrang_1216 zugehoerig zu 2D-DNA-Nanostruktur.

<400> 1217
attatcattc aatataatcc tgacaattac ttaccatctc tcctaaactc g 51

<210> 1218
<211> 53
<212> DNA
<213> Artificial Sequence

<220>
<223> Klammerstrang_1217 zugehoerig zu 2D-DNA-Nanostruktur.

<400> 1218
tcaatatcga acctcaaata tcaattccga aattaccatc tctcctaaac tcg 53

<210> 1219
<211> 51
<212> DNA
<213> Artificial Sequence

<220>
<223> Klammerstrang_1218 zugehoerig zu 2D-DNA-Nanostruktur.

<400> 1219
accttgcttg gtcagttggc aaagagcgga ttaccatctc tcctaaactc g 51

<210> 1220
<211> 51
<212> DNA

<213> Artificial Sequence

<220>
<223> Klammerstrang_1219 zugehoerig zu 2D-DNA-Nanostruktur.

<400> 1220
taaaagggac attctggcca acaaagcatc ttaccatctc tcctaaactc g 51

<210> 1221
<211> 53
<212> DNA
<213> Artificial Sequence

<220>
<223> Klammerstrang_1220 zugehoerig zu 2D-DNA-Nanostruktur.

<400> 1221
gtaccgcaat tctaagaacg cgagtattat ttttaccatc tctcctaaac tcg 53

<210> 1222
<211> 53
<212> DNA
<213> Artificial Sequence

<220>
<223> Klammerstrang_1221 zugehoerig zu 2D-DNA-Nanostruktur.

<400> 1222
gtaaagtaat cgccatattt aacaaaactt ttttaccatc tctcctaaac tcg 53

<210> 1223
<211> 53
<212> DNA
<213> Artificial Sequence

<220>
<223> Klammerstrang_1222 zugehoerig zu 2D-DNA-Nanostruktur.

<400> 1223
aattgagaat tctgtccaga cgactaaacc aattaccatc tctcctaaac tcg 53

<210> 1224
<211> 53
<212> DNA
<213> Artificial Sequence

<220>
<223> Klammerstrang_1223 zugehoerig zu 2D-DNA-Nanostruktur.

<400> 1224
tcaaatataa cctccggctt aggtaacaat ttttaccatc tctcctaaac tcg 53

<210> 1225

<211> 53
<212> DNA
<213> Artificial Sequence

<220>
<223> Klammerstrang_1224 zugehoerig zu 2D-DNA-Nanostruktur.

<400> 1225
accttttat tttagttaat ttcatagggc ttttaccatc tctcctaaac tcg 53

<210> 1226
<211> 53
<212> DNA
<213> Artificial Sequence

<220>
<223> Klammerstrang_1225 zugehoerig zu 2D-DNA-Nanostruktur.

<400> 1226
catttgaagg cgaattattc atttttgttt ggttaccatc tctcctaaac tcg 53

<210> 1227
<211> 53
<212> DNA
<213> Artificial Sequence

<220>
<223> Klammerstrang_1226 zugehoerig zu 2D-DNA-Nanostruktur.

<400> 1227
cgcgcagatt acctttttta atgggagaga ctttaccatc tctcctaaac tcg 53

<210> 1228
<211> 53
<212> DNA
<213> Artificial Sequence

<220>
<223> Klammerstrang_1227 zugehoerig zu 2D-DNA-Nanostruktur.

<400> 1228
attatactaa gaaaccacca gaagtcaaca gtttaccatc tctcctaaac tcg 53

<210> 1229
<211> 53
<212> DNA
<213> Artificial Sequence

<220>
<223> Klammerstrang_1228 zugehoerig zu 2D-DNA-Nanostruktur.

<400> 1229
gcggaacatc tgaataatgg aaggtacaaa atttaccatc tctcctaaac tcg 53

<210> 1230
<211> 53
<212> DNA
<213> Artificial Sequence

<220>
<223> Klammerstrang_1229 zugehoerig zu 2D-DNA-Nanostruktur.

<400> 1230
tgaaaggagc aaatgaaaaa tctagagata gattaccatc tctcctaaac tcg 53

<210> 1231
<211> 53
<212> DNA
<213> Artificial Sequence

<220>
<223> Klammerstrang_1230 zugehoerig zu 2D-DNA-Nanostruktur.

<400> 1231
agccagcaat tgaggaaggt tatcatcatt ttttaccatc tctcctaaac tcg 53

<210> 1232
<211> 53
<212> DNA
<213> Artificial Sequence

<220>
<223> Klammerstrang_1231 zugehoerig zu 2D-DNA-Nanostruktur.

<400> 1232
acccttctga cctgaaagcg taagacgctg agttaccatc tctcctaaac tcg 53

<210> 1233
<211> 53
<212> DNA
<213> Artificial Sequence

<220>
<223> Klammerstrang_1232 zugehoerig zu 2D-DNA-Nanostruktur.

<400> 1233
cttatcattc ccgacttgcg ggagcctaat ttttaccatc tctcctaaac tcg 53

<210> 1234
<211> 51
<212> DNA
<213> Artificial Sequence

<220>
<223> Klammerstrang_1233 zugehoerig zu 2D-DNA-Nanostruktur.

<400> 1234

acaacatgcc aacgctcaac agtcttctga ttaccatctc tcctaaactc g 51

<210> 1235
<211> 53
<212> DNA
<213> Artificial Sequence

<220>
<223> Klammerstrang_1234 zugehoerig zu 2D-DNA-Nanostruktur.

<400> 1235
agtataaagt tcagctaatg cagatgtctt tcttaccatc tctcctaaac tcg 53

<210> 1236
<211> 51
<212> DNA
<213> Artificial Sequence

<220>
<223> Klammerstrang_1235 zugehoerig zu 2D-DNA-Nanostruktur.

<400> 1236
cctaaatcaa aatcataggt ctaaacagta ttaccatctc tcctaaactc g 51

<210> 1237
<211> 53
<212> DNA
<213> Artificial Sequence

<220>
<223> Klammerstrang_1236 zugehoerig zu 2D-DNA-Nanostruktur.

<400> 1237
gaatttattt aatggtttga aatattctta ccttaccatc tctcctaaac tcg 53

<210> 1238
<211> 51
<212> DNA
<213> Artificial Sequence

<220>
<223> Klammerstrang_1237 zugehoerig zu 2D-DNA-Nanostruktur.

<400> 1238
cataaatctt tgaataccaa gtgttagaac ttaccatctc tcctaaactc g 51

<210> 1239
<211> 53
<212> DNA
<213> Artificial Sequence

<220>
<223> Klammerstrang_1238 zugehoerig zu 2D-DNA-Nanostruktur.

<400> 1239
cctgattgca atatatgtga gtgatcaata gtttaccatc tctcctaaac tcg 53

<210> 1240
<211> 51
<212> DNA
<213> Artificial Sequence

<220>
<223> Klammerstrang_1239 zugehoerig zu 2D-DNA-Nanostruktur.

<400> 1240
ctaccatagt ttgagtaaca tttaaaatat ttaccatctc tcctaaactc g 51

<210> 1241
<211> 53
<212> DNA
<213> Artificial Sequence

<220>
<223> Klammerstrang_1240 zugehoerig zu 2D-DNA-Nanostruktur.

<400> 1241
attttaaaat caaaattatt tgcacggatt cgttaccatc tctcctaaac tcg 53

<210> 1242
<211> 51
<212> DNA
<213> Artificial Sequence

<220>
<223> Klammerstrang_1241 zugehoerig zu 2D-DNA-Nanostruktur.

<400> 1242
ctttagggcc tgcaacagtg ccaatacgtg ttaccatctc tcctaaactc g 51

<210> 1243
<211> 53
<212> DNA
<213> Artificial Sequence

<220>
<223> Klammerstrang_1242 zugehoerig zu 2D-DNA-Nanostruktur.

<400> 1243
ttaacaccag cactaacaac taatcgttat tattaccatc tctcctaaac tcg 53

<210> 1244
<211> 52
<212> DNA
<213> Artificial Sequence

<220>
<223> Klammerstrang_1243 zugehoerig zu 2D-DNA-Nanostruktur.

<400> 1244
gcacagacaa tatttttgaa tggggtcagt attaccatct ctcctaaact cg 52

<210> 1245
<211> 51
<212> DNA
<213> Artificial Sequence

<220>
<223> Klammerstrang_1244 zugehoerig zu 2D-DNA-Nanostruktur.

<400> 1245
tgtagaaatc aagattagtt gctcttacca ttaccatctc tcctaaactc g 51

<210> 1246
<211> 53
<212> DNA
<213> Artificial Sequence

<220>
<223> Klammerstrang_1245 zugehoerig zu 2D-DNA-Nanostruktur.

<400> 1246
gtttatcaat atgcgttata caaaccgacc gtttaccatc tctcctaaac tcg 53

<210> 1247
<211> 51
<212> DNA
<213> Artificial Sequence

<220>
<223> Klammerstrang_1246 zugehoerig zu 2D-DNA-Nanostruktur.

<400> 1247
ttagtatcac aatagataag tccacgagca ttaccatctc tcctaaactc g 51

<210> 1248
<211> 53
<212> DNA
<213> Artificial Sequence

<220>
<223> Klammerstrang_1247 zugehoerig zu 2D-DNA-Nanostruktur.

<400> 1248
gtgataaaaa gacgctgaga agagataacc ttttaccatc tctcctaaac tcg 53

<210> 1249
<211> 51
<212> DNA
<213> Artificial Sequence

<220>
<223> Klammerstrang_1248 zugehoerig zu 2D-DNA-Nanostruktur.

<400> 1249
cttagattta aggcgttaaa taaagcctgt ttaccatctc tcctaaactc g 51

<210> 1250
<211> 53
<212> DNA
<213> Artificial Sequence

<220>
<223> Klammerstrang_1249 zugehoerig zu 2D-DNA-Nanostruktur.

<400> 1250
gcttctgttc gggagaaaca ataacgtaaa acttaccatc tctcctaaac tcg 53

<210> 1251
<211> 51
<212> DNA
<213> Artificial Sequence

<220>
<223> Klammerstrang_1250 zugehoerig zu 2D-DNA-Nanostruktur.

<400> 1251
cttttacaaa atcgtcgcta ttagcgatag ttaccatctc tcctaaactc g 51

<210> 1252
<211> 53
<212> DNA
<213> Artificial Sequence

<220>
<223> Klammerstrang_1251 zugehoerig zu 2D-DNA-Nanostruktur.

<400> 1252
agaaataaaa atcctttgcc cgaaagatta gattaccatc tctcctaaac tcg 53

<210> 1253
<211> 51
<212> DNA
<213> Artificial Sequence

<220>
<223> Klammerstrang_1252 zugehoerig zu 2D-DNA-Nanostruktur.

<400> 1253
ctcgtattag aaattgcgta gatacagtac ttaccatctc tcctaaactc g 51

<210> 1254
<211> 53

<212> DNA
<213> Artificial Sequence

<220>
<223> Klammerstrang_1253 zugehoerig zu 2D-DNA-Nanostruktur.

<400> 1254
gccgtcaaaa aacagaggtg aggcctatta gtttaccatc tctcctaaac tcg 53

<210> 1255
<211> 51
<212> DNA
<213> Artificial Sequence

<220>
<223> Klammerstrang_1254 zugehoerig zu 2D-DNA-Nanostruktur.

<400> 1255
cagaagatta gataatacat ttgtcgacaa ttaccatctc tcctaaactc g 51

<210> 1256
<211> 51
<212> DNA
<213> Artificial Sequence

<220>
<223> Klammerstrang_1255 zugehoerig zu 2D-DNA-Nanostruktur.

<400> 1256
ctttaatgcg cgaactgata gccccaccag ttaccatctc tcctaaactc g 51

<210> 1257
<211> 7249
<212> DNA
<213> Phage M13mp18

<220>
<223> scaffold DNA p7249_tillibit.txt

<400> 1257
aatgctacta ctattagtag aattgatgcc accttttcag ctcgcgcccc aaatgaaaat 60

atagctaaac aggttattga ccatttgcga aatgtatcta atggtcaaac taaatctact 120

cgttcgcaga attgggaatc aactgttata tggaatgaaa cttccagaca ccgtacttta 180

gttgcatatt taaaacatgt tgagctacag cattatattc agcaattaag ctctaagcca 240

tccgcaaaaa tgacctctta tcaaaaggag caattaaagg tactctctaa tcctgacctg 300

ttggagtttg cttccggtct ggttcgcttt gaagctcgaa ttaaaacgcg atatttgaag 360

tctttcgggc ttcctcttaa tctttttgat gcaatccgct ttgcttctga ctataatagt 420

cagggtaaag acctgatttt tgatttatgg tcattctcgt tttctgaact gtttaaagca 480

tttgaggggg attcaatgaa tatttatgac gattccgcag tattggacgc tatccagtct 540
aaacatttta ctattacccc ctctggcaaa acttcttttg caaaagcctc tcgctatttt 600
ggtttttatc gtcgtctggt aaacgagggt tatgatagtg ttgctcttac tatgcctcgt 660
aattcctttt ggcgttatgt atctgcatta gttgaatgtg gtattcctaa atctcaactg 720
atgaatcttt ctacctgtaa taatgttgtt ccgttagttc gttttattaa cgtagatttt 780
tcttcccaac gtcctgactg gtataatgag ccagttctta aaatcgcata aggtaattca 840
caatgattaa agttgaaatt aaaccatctc aagcccaatt tactactcgt tctggtgttt 900
ctcgtcaggg caagccttat tcactgaatg agcagctttg ttacgttgat ttgggtaatg 960
aatatccggt tcttgtcaag attactcttg atgaaggtca gccagcctat gcgcctggtc 1020
tgtacaccgt tcatctgtcc tctttcaaag ttggtcagtt cggttccctt atgattgacc 1080
gtctgcgcct cgttccggct aagtaacatg gagcaggtcg cggatttcga cacaatttat 1140
caggcgatga tacaaatctc cgttgtactt tgtttcgcgc ttggtataat cgctgggggt 1200
caaagatgag tgttttagtg tattcttttg cctctttcgt tttaggttgg tgccttcgta 1260
gtggcattac gtattttacc cgtttaatgg aaacttcctc atgaaaaagt ctttagtcct 1320
caaagcctct gtagccgttg ctaccctcgt tccgatgctg tctttcgctg ctgagggtga 1380
cgatcccgca aaagcggcct ttaactccct gcaagcctca gcgaccgaat atatcggtta 1440
tgcgtgggcg atggttgttg tcattgtcgg cgcaactatc ggtatcaagc tgtttaagaa 1500
attcacctcg aaagcaagct gataaaccga tacaattaaa ggctcctttt ggagcctttt 1560
ttttggagat tttcaacgtg aaaaaattat tattcgcaat tcctttagtt gttcctttct 1620
attctcactc cgctgaaact gttgaaagtt gtttagcaaa atcccataca gaaaattcat 1680
ttactaacgt ctggaaagac gacaaaactt tagatcgtta cgctaactat gagggctgtc 1740
tgtggaatgc tacaggcgtt gtagtttgta ctggtgacga aactcagtgt tacggtacat 1800
gggttcctat tgggcttgct atccctgaaa atgagggtgg tggctctgag ggtggcggtt 1860
ctgagggtgg cggttctgag ggtggcggta ctaaacctcc tgagtacggt gatacaccta 1920
ttccgggcta tacttatatc aaccctctcg acggcactta tccgcctggt actgagcaaa 1980
accccgctaa tcctaatcct tctcttgagg agtctcagcc tcttaatact ttcatgtttc 2040
agaataatag gttccgaaat aggcaggggg cattaactgt ttatacgggc actgttactc 2100
aaggcactga ccccgttaaa acttattacc agtacactcc tgtatcatca aaagccatgt 2160
atgacgctta ctggaacggt aaattcagag actgcgcttt ccattctggc tttaatgagg 2220
atttatttgt ttgtgaatat caaggccaat cgtctgacct gcctcaacct cctgtcaatg 2280
ctggcggcgg ctctggtggt ggttctggtg gcggctctga gggtggtggc tctgagggtg 2340
gcggttctga gggtggcggc tctgagggag gcggttccgg tggtggctct ggttccggtg 2400

attttgatta tgaaaagatg gcaaacgcta ataaggggc tatgaccgaa aatgccgatg 2460
aaaacgcgct acagtctgac gctaaaggca aacttgattc tgtcgctact gattacggtg 2520
ctgctatcga tggtttcatt ggtgacgttt ccggccttgc taatggtaat ggtgctactg 2580
gtgattttgc tggctctaat tcccaaatgg ctcaagtcgg tgacggtgat aattcacctt 2640
taatgaataa tttccgtcaa tatttacctt ccctccctca atcggttgaa tgtcgccctt 2700
ttgtctttgg cgctggtaaa ccatatgaat tttctattga ttgtgacaaa ataaacttat 2760
tccgtggtgt ctttgcgttt cttttatatg ttgccacctt tatgtatgta ttttctacgt 2820
ttgctaacat actgcgtaat aaggagtctt aatcatgcca gttcttttgg gtattccgtt 2880
attattgcgt ttcctcggtt tccttctggt aactttgttc ggctatctgc ttacttttct 2940
taaaaagggc ttcggtaaga tagctattgc tatttcattg tttcttgctc ttattattgg 3000
gcttaactca attcttgtgg gttatctctc tgatattagc gctcaattac cctctgactt 3060
tgttcagggt gttcagttaa ttctcccgtc taatgcgctt ccctgttttt atgttattct 3120
ctctgtaaag gctgctattt tcatttttga cgttaaacaa aaaatcgttt cttatttgga 3180
ttgggataaa taatatggct gtttattttg taactggcaa attaggctct ggaaagacgc 3240
tcgttagcgt tggtaagatt caggataaaa ttgtagctgg gtgcaaaata gcaactaatc 3300
ttgatttaag gcttcaaaac ctcccgcaag tcgggaggtt cgctaaaacg cctcgcgttc 3360
ttagaatacc ggataagcct tctatatctg atttgcttgc tattgggcgc ggtaatgatt 3420
cctacgatga aaataaaaac ggcttgcttg ttctcgatga gtgcggtact tggtttaata 3480
cccgttcttg gaatgataag gaaagacagc cgattattga ttggtttcta catgctcgta 3540
aattaggatg ggatattatt tttcttgttc aggacttatc tattgttgat aaacaggcgc 3600
gttctgcatt agctgaacat gttgtttatt gtcgtcgtct ggacagaatt actttacctt 3660
ttgtcggtac tttatattct cttattactg gctcgaaaat gcctctgcct aaattacatg 3720
ttggcgttgt taaatatggc gattctcaat taagccctac tgttgagcgt tggctttata 3780
ctggtaagaa tttgtataac gcatatgata ctaaacaggc tttttctagt aattatgatt 3840
ccggtgttta ttcttattta acgccttatt tatcacacgg tcggtatttc aaaccattaa 3900
atttaggtca gaagatgaaa ttaactaaaa tatatttgaa aaagttttct cgcgttcttt 3960
gtcttgcgat tggatttgca tcagcattta catatagtta tataacccaa cctaagccgg 4020
aggttaaaaa ggtagtctct cagacctatg attttgataa attcactatt gactcttctc 4080
agcgtcttaa tctaagctat cgctatgttt tcaaggattc taagggaaaa ttaattaata 4140
gcgacgattt acagaagcaa ggttattcac tcacatatat tgatttatgt actgtttcca 4200
ttaaaaaagg taattcaaat gaaattgtta aatgtaatta attttgtttt cttgatgttt 4260

gtttcatcat cttcttttgc tcaggtaatt gaaatgaata attcgcctct gcgcgatttt 4320
gtaacttggt attcaaagca atcaggcgaa tccgttattg tttctcccga tgtaaaaggt 4380
actgttactg tatattcatc tgacgttaaa cctgaaaatc tacgcaattt ctttatttct 4440
gttttacgtg caaataattt tgatatggta ggttctaacc cttccattat tcagaagtat 4500
aatccaaaca atcaggatta tattgatgaa ttgccatcat ctgataatca ggaatatgat 4560
gataattccg ctccttctgg tggtttcttt gttccgcaaa atgataatgt tactcaaact 4620
tttaaaatta ataacgttcg ggcaaaggat ttaatacgag ttgtcgaatt gtttgtaaag 4680
tctaatactt ctaaatcctc aaatgtatta tctattgacg gctctaatct attagttgtt 4740
agtgctccta aagatatttt agataacctt cctcaattcc tttcaactgt tgatttgcca 4800
actgaccaga tattgattga gggtttgata tttgaggttc agcaaggtga tgctttagat 4860
ttttcatttg ctgctggctc tcagcgtggc actgttgcag gcggtgttaa tactgaccgc 4920
ctcacctctg ttttatcttc tgctggtggt tcgttcggta tttttaatgg cgatgtttta 4980
gggctatcag ttcgcgcatt aaagactaat agccattcaa aaatattgtc tgtgccacgt 5040
attcttacgc tttcaggtca gaagggttct atctctgttg gccagaatgt cccttttatt 5100
actggtcgtg tgactggtga atctgccaat gtaaataatc catttcagac gattgagcgt 5160
caaaatgtag gtatttccat gagcgttttt cctgttgcaa tggctggcgg taatattgtt 5220
ctggatatta ccagcaaggc cgatagtttg agttcttcta ctcaggcaag tgatgttatt 5280
actaatcaaa gaagtattgc tacaacggtt aatttgcgtg atggacagac tcttttactc 5340
ggtggcctca ctgattataa aaacacttct caggattctg gcgtaccgtt cctgtctaaa 5400
atccctttaa tcggcctcct gtttagctcc cgctctgatt ctaacgagga aagcacgtta 5460
tacgtgctcg tcaaagcaac catagtacgc gccctgtagc ggcgcattaa gcgcggcggg 5520
tgtggtggtt acgcgcagcg tgaccgctac acttgccagc gccctagcgc ccgctccttt 5580
cgctttcttc ccttcctttc tcgccacgtt cgccggcttt ccccgtcaag ctctaaatcg 5640
ggggctccct ttagggttcc gatttagtgc tttacggcac ctcgacccca aaaaacttga 5700
tttgggtgat ggttcacgta gtgggccatc gccctgatag acggtttttc gccctttgac 5760
gttggagtcc acgttcttta atagtggact cttgttccaa actggaacaa cactcaaccc 5820
tatctcgggc tattcttttg atttataagg gattttgccg atttcggaac caccatcaaa 5880
caggattttc gcctgctggg gcaaaccagc gtggaccgct tgctgcaact ctctcagggc 5940
caggcggtga agggcaatca gctgttgccc gtctcactgg tgaaaagaaa aaccaccctg 6000
gcgcccaata cgcaaaccgc ctctccccgc gcgttggccg attcattaat gcagctggca 6060
cgacaggttt cccgactgga aagcgggcag tgagcgcaac gcaattaatg tgagttagct 6120
cactcattag gcaccccagg ctttacactt tatgcttccg gctcgtatgt tgtgtggaat 6180

tgtgagcgga taacaatttc acacaggaaa cagctatgac catgattacg aattcgagct 6240

cggtacccgg ggatcctcta gagtcgacct gcaggcatgc aagcttggca ctggccgtcg 6300

ttttacaacg tcgtgactgg gaaaaccctg gcgttaccca acttaatcgc cttgcagcac 6360

atccccctt cgccagctgg cgtaatagcg aagaggcccg caccgatcgc ccttcccaac 6420

agttgcgcag cctgaatggc gaatggcgct ttgcctggtt tccggcacca gaagcggtgc 6480

cggaaagctg gctggagtgc gatcttcctg aggccgatac tgtcgtcgtc ccctcaaact 6540

ggcagatgca cggttacgat gcgcccatct acaccaacgt gacctatccc attacggtca 6600

atccgccgtt tgttcccacg gagaatccga cgggttgtta ctcgctcaca tttaatgttg 6660

atgaaagctg gctacaggaa ggccagacgc gaattatttt tgatggcgtt cctattggtt 6720

aaaaaatgag ctgatttaac aaaaatttaa tgcgaatttt aacaaaatat taacgtttac 6780

aatttaaata tttgcttata caatcttcct gtttttgggg cttttctgat tatcaaccgg 6840

ggtacatatg attgacatgc tagttttacg attaccgttc atcgattctc ttgtttgctc 6900

cagactctca ggcaatgacc tgatagcctt tgtagatctc tcaaaaatag ctaccctctc 6960

cggcattaat ttatcagcta gaacggttga atatcatatt gatggtgatt tgactgtctc 7020

cggcctttct caccctttg aatctttacc tacacattac tcaggcattg catttaaaat 7080

atatgagggt tctaaaaatt tttatccttg cgttgaaata aaggcttctc ccgcaaaagt 7140

attacagggt cataatgttt ttggtacaac cgatttagct ttatgctctg aggctttatt 7200

gcttaatttt gctaattctt tgccttgcct gtatgattta ttggatgtt 7249

<210> 1258
<211> 7308
<212> DNA
<213> Phage M13mp18

<220>
<223> scaffold DNA p7308_NatChem2012.txt

<400> 1258
aatgctacta ctattagtag aattgatgcc accttttcag ctcgcgcccc aaatgaaaat 60

atagctaaac aggttattga ccatttgcga aatgtatcta atggtcaaac taaatctact 120

cgttcgcaga attgggaatc aactgttata tggaatgaaa cttccagaca ccgtacttta 180

gttgcatatt taaaacatgt tgagctacag cattatattc agcaattaag ctctaagcca 240

tccgcaaaaa tgacctctta tcaaaaggag caattaaagg tactctctaa tcctgacctg 300

ttggagtttg cttccggtct ggttcgcttt gaagctcgaa ttaaaacgcg atatttgaag 360

tctttcgggc ttcctcttaa tctttttgat gcaatccgct ttgcttctga ctataatagt 420

cagggtaaag acctgatttt tgatttatgg tcattctcgt ttctgaact gtttaaagca 480

tttgaggggg attcaatgaa tatttatgac gattccgcag tattggacgc tatccagtct 540

aaacatttta ctattacccc ctctggcaaa acttcttttg caaaagcctc tcgctatttt 600

ggtttttatc gtcgtctggt aaacgagggt tatgatagtg ttgctcttac tatgcctcgt 660

aattcctttt ggcgttatgt atctgcatta gttgaatgtg gtattcctaa atctcaactg 720

atgaatcttt ctacctgtaa taatgttgtt ccgttagttc gttttattaa cgtagatttt 780

tcttcccaac gtcctgactg gtataatgag ccagttctta aaatcgcata aggtaattca 840

caatgattaa agttgaaatt aaaccatctc aagcccaatt tactactcgt tctggtgttt 900

ctcgtcaggg caagccttat tcactgaatg agcagctttg ttacgttgat ttgggtaatg 960

aatatccggt tcttgtcaag attactcttg atgaaggtca gccagcctat gcgcctggtc 1020

tgtacaccgt tcatctgtcc tctttcaaag ttggtcagtt cggttccctt atgattgacc 1080

gtctgcgcct cgttccggct aagtaacatg gagcaggtcg cggatttcga cacaatttat 1140

caggcgatga tacaaatctc cgttgtactt tgtttcgcgc ttggtataat cgctgggggt 1200

caaagatgag tgttttagtg tattcttttg cctctttcgt tttaggttgg tgccttcgta 1260

gtggcattac gtattttacc cgtttaatgg aaacttcctc atgaaaaagt ctttagtcct 1320

caaagcctct gtagccgttg ctaccctcgt tccgatgctg tctttcgctg ctgagggtga 1380

cgatcccgca aaagcggcct ttaactccct gcaagcctca gcgaccgaat atatcggtta 1440

tgcgtgggcg atggttgttg tcattgtcgg cgcaactatc ggtatcaagc tgtttaagaa 1500

attcacctcg aaagcaagct gataaaccga tacaattaaa ggctcctttt ggagcctttt 1560

ttttggagat tttcaacgtg aaaaaattat tattcgcaat tcctttagtt gttcctttct 1620

attctcactc cgctgaaact gttgaaagtt gtttagcaaa atcccataca gaaaattcat 1680

ttactaacgt ctggaaagac gacaaaactt tagatcgtta cgctaactat gagggctgtc 1740

tgtggaatgc tacaggcgtt gtagtttgta ctggtgacga aactcagtgt tacggtacat 1800

gggttcctat tgggcttgct atccctgaaa atgagggtgg tggctctgag ggtggcggtt 1860

ctgagggtgg cggttctgag ggtggcggta ctaaacctcc tgagtacggt gatacaccta 1920

ttccgggcta tacttatatc aaccctctcg acggcactta tccgcctggt actgagcaaa 1980

accccgctaa tcctaatcct tctcttgagg agtctcagcc tcttaatact ttcatgtttc 2040

agaataatag gttccgaaat aggcaggggg cattaactgt ttatacgggc actgttactc 2100

aaggcactga ccccgttaaa acttattacc agtacactcc tgtatcatca aaagccatgt 2160

atgacgctta ctggaacggt aaattcagag actgcgcttt ccattctggc tttaatgagg 2220

atttatttgt ttgtgaatat caaggccaat cgtctgacct gcctcaacct cctgtcaatg 2280

ctggcggcgg ctctggtggt ggttctggtg gcggctctga gggtggtggc tctgagggtg 2340

gcggttctga gggtggcggc tctgagggag gcggttccgg tggtggctct ggttccggtg 2400
attttgatta tgaaaagatg gcaaacgcta ataagggggc tatgaccgaa aatgccgatg 2460
aaaacgcgct acagtctgac gctaaaggca aacttgattc tgtcgctact gattacggtg 2520
ctgctatcga tggtttcatt ggtgacgttt ccggccttgc taatggtaat ggtgctactg 2580
gtgattttgc tggctctaat tcccaaatgg ctcaagtcgg tgacggtgat aattcacctt 2640
taatgaataa tttccgtcaa tatttacctt ccctccctca atcggttgaa tgtcgccctt 2700
ttgtctttgg cgctggtaaa ccatatgaat tttctattga ttgtgacaaa ataaacttat 2760
tccgtggtgt ctttgcgttt cttttatatg ttgccacctt tatgtatgta ttttctacgt 2820
ttgctaacat actgcgtaat aaggagtctt aatcatgcca gttcttttgg gtattccgtt 2880
attattgcgt ttcctcggtt tccttctggt aactttgttc ggctatctgc ttacttttct 2940
taaaaagggc ttcggtaaga tagctattgc tatttcattg tttcttgctc ttattattgg 3000
gcttaactca attcttgtgg gttatctctc tgatattagc gctcaattac cctctgactt 3060
tgttcagggt gttcagttaa ttctcccgtc taatgcgctt ccctgttttt atgttattct 3120
ctctgtaaag gctgctattt tcatttttga cgttaaacaa aaaatcgttt cttatttgga 3180
ttgggataaa taatatggct gtttattttg taactggcaa attaggctct ggaaagacgc 3240
tcgttagcgt tggtaagatt caggataaaa ttgtagctgg gtgcaaaata gcaactaatc 3300
ttgatttaag gcttcaaaac ctcccgcaag tcgggaggtt cgctaaaacg cctcgcgttc 3360
ttagaatacc ggataagcct tctatatctg atttgcttgc tattgggcgc ggtaatgatt 3420
cctacgatga aaataaaaac ggcttgcttg ttctcgatga gtgcggtact tggtttaata 3480
cccgttcttg gaatgataag gaaagacagc cgattattga ttggtttcta catgctcgta 3540
aattaggatg ggatattatt tttcttgttc aggacttatc tattgttgat aaacaggcgc 3600
gttctgcatt agctgaacat gttgtttatt gtcgtcgtct ggacagaatt actttacctt 3660
ttgtcggtac tttatattct cttattactg gctcgaaaat gcctctgcct aaattacatg 3720
ttggcgttgt taaatatggc gattctcaat taagccctac tgttgagcgt tggctttata 3780
ctggtaagaa tttgtataac gcatatgata ctaaacaggc tttttctagt aattatgatt 3840
ccggtgttta ttcttattta acgccttatt tatcacacgg tcggtatttc aaaccattaa 3900
atttaggtca gaagatgaaa ttaactaaaa tatatttgaa aaagttttct cgcgttcttt 3960
gtcttgcgat tggatttgca tcagcattta catatagtta tataacccaa cctaagccgg 4020
aggttaaaaa ggtagtctct cagacctatg attttgataa attcactatt gactcttctc 4080
agcgtcttaa tctaagctat cgctatgttt tcaaggattc taagggaaaa ttaattaata 4140
gcgacgattt acagaagcaa ggttattcac tcacatatat tgatttatgt actgtttcca 4200
ttaaaaaagg taattcaaat gaaattgtta aatgtaatta attttgtttt cttgatgttt 4260

gtttcatcat cttcttttgc tcaggtaatt gaaatgaata attcgcctct gcgcgatttt 4320
gtaacttggt attcaaagca atcaggcgaa tccgttattg tttctcccga tgtaaaaggt 4380
actgttactg tatattcatc tgacgttaaa cctgaaaatc tacgcaattt ctttatttct 4440
gttttacgtg caaataattt tgatatggta ggttctaacc cttccattat tcagaagtat 4500
aatccaaaca atcaggatta tattgatgaa ttgccatcat ctgataatca ggaatatgat 4560
gataattccg ctccttctgg tggtttcttt gttccgcaaa atgataatgt tactcaaact 4620
tttaaaatta ataacgttcg ggcaaaggat ttaatacgag ttgtcgaatt gtttgtaaag 4680
tctaatactt ctaaatcctc aaatgtatta tctattgacg gctctaatct attagttgtt 4740
agtgctccta aagatatttt agataacctt cctcaattcc tttcaactgt tgatttgcca 4800
actgaccaga tattgattga gggtttgata tttgaggttc agcaaggtga tgctttagat 4860
ttttcatttg ctgctggctc tcagcgtggc actgttgcag gcggtgttaa tactgaccgc 4920
ctcacctctg ttttatcttc tgctggtggt tcgttcggta tttttaatgg cgatgtttta 4980
gggctatcag ttcgcgcatt aaagactaat agccattcaa aaatattgtc tgtgccacgt 5040
attcttacgc tttcaggtca gaagggttct atctctgttg gccagaatgt cccttttatt 5100
actggtcgtg tgactggtga atctgccaat gtaaataatc catttcagac gattgagcgt 5160
caaaatgtag gtatttccat gagcgttttt cctgttgcaa tggctggcgg taatattgtt 5220
ctggatatta ccagcaaggc cgatagtttg agttcttcta ctcaggcaag tgatgttatt 5280
actaatcaaa gaagtattgc tacaacggtt aatttgcgtg atggacagac tcttttactc 5340
ggtggcctca ctgattataa aaacacttct caggattctg gcgtaccgtt cctgtctaaa 5400
atccctttaa tcggcctcct gtttagctcc cgctctgatt ctaacgagga aagcacgtta 5460
tacgtgctcg tcaaagcaac catagtacgc gccctgtagc ggcgcattaa gcgcggcggg 5520
tgtggtggtt acgcgcagcg tgaccgctac acttgccagc gccctagcgc ccgctccttt 5580
cgctttcttc ccttcctttc tcgccacgtt cgccggcttt ccccgtcaag ctctaaatcg 5640
ggggctccct ttagggttcc gatttagtgc tttacggcac ctcgacccca aaaaacttga 5700
tttgggtgat ggttcacgta gtgggccatc gccctgatag acggtttttc gccctttgac 5760
gttggagtcc acgttcttta atagtggact cttgttccaa actggaacaa cactcaaccc 5820
tatctcgggc tattcttttg atttataagg gattttgccg atttcggaac caccatcaaa 5880
caggattttc gcctgctggg gcaaaccagc gtggaccgct tgctgcaact ctctcagggc 5940
caggcggtga agggcaatca gctgttgccc gtctcactgg tgaaaagaaa aaccaccctg 6000
gcgcccaata cgcaaaccgc ctctccccgc gcgttggccg attcattaat gcagctggca 6060
cgacaggttt cccgactgga aagcgggcag tgagcgcaac gcaattaatg tgagttagct 6120

cactcattag gcaccccagg ctttacactt tatgcttccg gctcgtatgt tgtgtggaat 6180

tgtgagcgga taacaatttc acacaggaaa cagctatgac catgattacg aattcgagct 6240

cggtacccgg ggatccttat acgggtacta gccatgcgta tacggtcgct agcggacttg 6300

cctcgctatc aaaggtctag agtcgacctg caggcatgca agcttggcac tggccgtcgt 6360

tttacaacgt cgtgactggg aaaaccctgg cgttacccaa cttaatcgcc ttgcagcaca 6420

tccccctttc gccagctggc gtaatagcga agaggcccgc accgatcgcc cttcccaaca 6480

gttgcgcagc ctgaatggcg aatggcgctt tgcctggttt ccggcaccag aagcggtgcc 6540

ggaaagctgg ctggagtgcg atcttcctga ggccgatact gtcgtcgtcc cctcaaactg 6600

gcagatgcac ggttacgatg cgcccatcta caccaacgtg acctatccca ttacggtcaa 6660

tccgccgttt gttcccacgg agaatccgac gggttgttac tcgctcacat ttaatgttga 6720

tgaaagctgg ctacaggaag gccagacgcg aattattttt gatggcgttc ctattggtta 6780

aaaaatgagc tgatttaaca aaaatttaat gcgaatttta acaaaatatt aacgtttaca 6840

atttaaatat ttgcttatac aatcttcctg tttttggggc ttttctgatt atcaaccggg 6900

gtacatatga ttgacatgct agttttacga ttaccgttca tcgattctct tgtttgctcc 6960

agactctcag gcaatgacct gatagccttt gtagatctct caaaaatagc taccctctcc 7020

ggcattaatt tatcagctag aacggttgaa tatcatattg atggtgattt gactgtctcc 7080

ggcctttctc acccttttga atctttacct acacattact caggcattgc atttaaaata 7140

tatgagggtt ctaaaaattt ttatccttgc gttgaaataa aggcttctcc cgcaaaagta 7200

ttacagggtc ataatgtttt tggtacaacc gatttagctt tatgctctga ggctttattg 7260

cttaattttg ctaattcttt gccttgcctg tatgatttat tggatgtt 7308

<210> 1259
<211> 21
<212> DNA
<213> Artificial Sequence

<220>
<223> DNA Farbstoff Adapter Rot, 3′ Modifikation: Atto 647N

<400> 1259
gtgatgtagg tggtagagga a 21

<210> 1260
<211> 21
<212> DNA
<213> Artificial Sequence

<220>
<223> DNA Farbstoff Adapter Gruen, 3′ Modifikation: Atto 565

```
<400> 1260
tatgagaagt taggaatgtt a                                              21


<210> 1261
<211> 21
<212> DNA
<213> Artificial Sequence


<220>
<223> DNA Farbstoff Adapter Blau, 3' Modifikation: Atto 488

<400> 1261
cgagtttagg agagatggta a                                              21


<210> 1262
<211> 21
<212> DNA
<213> Artificial Sequence


<220>
<223> DNA Traeger adapter bzw. Zwischen-Traegeradapter, 5'
      Modifikation: biotin

<400> 1262
gaatcggtca cagtacaacc g                                              21


<210> 1263
<211> 68
<212> DNA
<213> Artificial Sequence


<220>
<223> DNA T1 Ziel Adapter 3D

<400> 1263
taattttaaa agtctcgtat gtcagtatta acaccaccag cagccttgag acgatgttga     60

ccttaacc                                                              68


<210> 1264
<211> 68
<212> DNA
<213> Artificial Sequence


<220>
<223> DNA T2 Ziel Adapter 3D

<400> 1264
taattttaaa agtctcgtat gtcagtatta acaccaccag cagccttcat gtcaggagat     60

tttcagcc                                                              68


<210> 1265
<211> 68
```

<212> DNA
<213> Artificial Sequence

<220>
<223> DNA T3 Ziel Adapter 3D

<400> 1265
taattttaaa agtctcgtat gtcagtatta acaccaccag cagcctttac cctatctgag 60

tgagtagc 68

<210> 1266
<211> 61
<212> DNA
<213> Artificial Sequence

<220>
<223> DNA T1 Ziel Adapter 2D

<400> 1266
ttgtgtcgtg acgagaaaca ccaaatttca actttaattt gagacgatgt tgaccttaac 60

c 61

<210> 1267
<211> 61
<212> DNA
<213> Artificial Sequence

<220>
<223> DNA T2 Ziel Adapter 2D

<400> 1267
ttgtgtcgtg acgagaaaca ccaaatttca actttaattt catgtcagga gattttcagc 60

c 61

<210> 1268
<211> 61
<212> DNA
<213> Artificial Sequence

<220>
<223> DNA T3 Ziel Adapter 2D

<400> 1268
ttgtgtcgtg acgagaaaca ccaaatttca actttaattt taccctatct gagtgagtag 60

c 61

<210> 1269
<211> 87
<212> DNA
<213> Artificial Sequence

<220>
<223> DNA Zielstruktur

<400> 1269
gctactcact cagatagggt atggctgaaa atctcctgac atgtggttaa ggtcaacatc 60

gtctctcggt tgtactgtga ccgattc 87

<210> 1270
<211> 72
<212> DNA
<213> Artificial Sequence

<220>
<223> DNA T4_gapdh Ziel Adapter 3D

<400> 1270
ttgtgtcgtg acgagaaaca ccaaatttca actttttttt ccattgatga caagcttccc 60

gttctcagcc tt 72

<210> 1271
<211> 73
<212> DNA
<213> Artificial Sequence

<220>
<223> DNA T4_tfrc Ziel Adapter 3D

<400> 1271
ttgtgtcgtg acgagaaaca ccaaatttca actttttcga gttttgagcg ctgtctttga 60

cctgaatctt aac 73

<210> 1272
<211> 70
<212> DNA
<213> Artificial Sequence

<220>
<223> DNA T4_actb Ziel Adapter 3D

<400> 1272
ttgtgtcgtg acgagaaaca ccaaatttca actttttaat gatcttgatc ttcattgtgc 60

tgggtgccag 70

<210> 1273
<211> 57
<212> DNA
<213> Artificial Sequence

<220>
<223> DNA Traeger adapter gapdh

<400> 1273
gccatgggtg gaatcatatt ggaacatgta aaccttcggt tgtactgtga ccgattc 57

<210> 1274
<211> 54
<212> DNA
<213> Artificial Sequence

<220>
<223> DNA Traeger adapter tfrc

<400> 1274
cacgccagac tttgctgagt ttaaattcac gttcggttgt actgtgaccg attc 54

<210> 1275
<211> 58
<212> DNA
<213> Artificial Sequence

<220>
<223> DNA Traeger adapter actb

<400> 1275
atgatggagt tgaaggtagt ttcgtggatg ccacattcgg ttgtactgtg accgattc 58

<210> 1276
<211> 76
<212> DNA
<213> Artificial Sequence

<220>
<223> DNA T5_gapdh Ziel Adapter 3D

<400> 1276
ttgtgtcgtg acgagaaaca ccaaatttca acttttctgg gtggcagtga tggcatggac 60

tgtggtcatg agtcct 76

<210> 1277
<211> 74
<212> DNA
<213> Artificial Sequence

<220>
<223> DNA T5_tfrc Ziel Adapter 3D

<400> 1277
ttgtgtcgtg acgagaaaca ccaaatttca actttttacc catcttttaa gaccatatct 60

gagaacatct gggc 74

<210> 1278
<211> 70

```
<212> DNA
<213> Artificial Sequence

<220>
<223> DNA T5_actb Ziel Adapter 3D

<400> 1278
ttgtgtcgtg acgagaaaca ccaaatttca actttttcag tgtacaggta agccctggct      60

gcctccaccc                                                               70
```